(19) Europäisches Patentamt
European Patent Office
Office européen des brevets

(11) **EP 4 659 592 A1**

(12) **EUROPEAN PATENT APPLICATION**
published in accordance with Art. 153(4) EPC

(43) Date of publication:
**10.12.2025 Bulletin 2025/50**

(21) Application number: **24750370.9**

(22) Date of filing: **31.01.2024**

(51) International Patent Classification (IPC):
***A23L 27/10*** *(2016.01)* ***A23L 23/10*** *(2016.01)*
***A23L 27/00*** *(2016.01)* ***A23L 27/18*** *(2016.01)*
***C07K 5/06*** *(2006.01)*

(52) Cooperative Patent Classification (CPC):
**A23L 23/10; A23L 27/00; A23L 27/10; A23L 27/18; C07K 5/06**

(86) International application number:
**PCT/JP2024/003190**

(87) International publication number:
**WO 2024/162421 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**31.01.2023 JP 2023013575**
**31.01.2023 JP 2023013576**
**31.01.2023 JP 2023013623**
**28.04.2023 JP 2023074823**

(71) Applicants:
- **House Foods Corporation**
**Higashi-Osaka-shi, Osaka 577-8520 (JP)**
- **House Foods Group Inc.**
**Higashiosaka-shi, Osaka 577-8520 (JP)**

(72) Inventors:
- **KOBAYASHI, Runo**
**Higashiosaka-shi, Osaka 577-8520 (JP)**
- **SAKANE, Kumi**
**Higashiosaka-shi, Osaka 577-8520 (JP)**
- **MIYAZAKI, Kinuko**
**Higashiosaka-shi, Osaka 577-8520 (JP)**
- **SHIRAMIZU, Takashi**
**Higashiosaka-shi, Osaka 577-8520 (JP)**
- **ISHIKAWA, Chikako**
**Higashiosaka-shi, Osaka 577-8520 (JP)**
- **IKEDA, Masaki**
**Higashiosaka-shi, Osaka 577-8520 (JP)**
- **MATSUBARA, Keiko**
**Higashiosaka-shi, Osaka 577-8520 (JP)**

(74) Representative: **Global IP Europe Patentanwaltskanzlei**
**Pfarrstraße 14**
**80538 München (DE)**

# (54) FLAVOR ENHANCING COMPOSITION, MANUFACTURING METHOD THEREFOR, AND METHOD FOR ENHANCING FLAVOR OF FOOD PRODUCT

(57) The present invention provides a flavor enhancing composition that can enhance a flavor of a food product when blended with the aforesaid food product, a method for producing the same, and a method for enhancing a flavor of a food product. A first embodiment of the present invention relates to a flavor enhancing composition containing one or more heat-treated spices selected from cumin, paprika, asafoetida and mustard seed. A second embodiment of the present invention relates to a method for producing the aforesaid flavor enhancing composition, comprising heating, under a specified condition, the one or more spices selected from cumin, paprika, asafoetida and mustard seed. A third embodiment of the present invention relates to a method for enhancing a flavor of a food product, comprising blending the aforesaid flavor enhancing composition with the food product.

EP 4 659 592 A1

1 CYCLIC(Phe-Leu/Ile)

| | Ave. | SD |
|---|---|---|
| Comp. Ex. 201 | 0.383 | 0.045 |
| Comp. Ex. 202 | 0.424 | 0.008 |
| Ex. 201 | 0.538 | 0.013 |
| Ex. 202 | 0.528 | 0.062 |
| Ex. 203 | 0.575 | 0.002 |
| Ex. 204 | 0.588 | 0.056 |
| Ex. 205 | 0.651 | 0.014 |
| Ex. 206 | 0.675 | 0.006 |
| Ex. 207 | 0.653 | 0.027 |
| Ex. 208 | 0.712 | 0.065 |
| Ex. 209 | 0.685 | 0.057 |
| Ex. 210 | 0.753 | 0.019 |
| Ex. 211 | 0.638 | 0.021 |
| Ex. 212 | 0.612 | 0.011 |
| Ex. 213 | 0.688 | 0.006 |
| Ex. 214 | 0.544 | 0.023 |
| Ex. 215 | 0.501 | 0.013 |
| Ex. 216 | 0.531 | 0.001 |

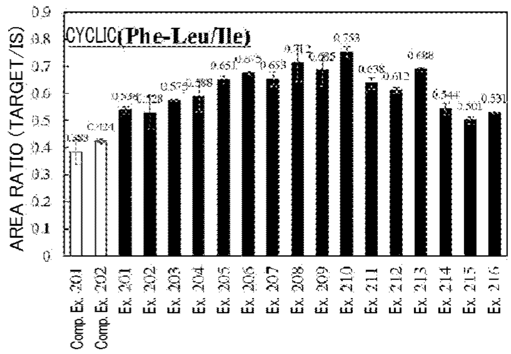


2 CYCLIC(Pro-Asn)

| | Ave. | SD |
|---|---|---|
| Comp. Ex. 201 | 0.014 | 0.008 |
| Comp. Ex. 202 | 0.010 | 0.003 |
| Ex. 201 | 0.041 | 0.012 |
| Ex. 202 | 0.051 | 0.012 |
| Ex. 203 | 0.041 | 0.003 |
| Ex. 204 | 0.101 | 0.011 |
| Ex. 205 | 0.052 | 0.002 |
| Ex. 206 | 0.065 | 0.008 |
| Ex. 207 | 0.057 | 0.010 |
| Ex. 208 | 0.089 | 0.017 |
| Ex. 209 | 0.058 | 0.012 |
| Ex. 210 | 0.099 | 0.006 |
| Ex. 211 | 0.030 | 0.001 |
| Ex. 212 | 0.040 | 0.001 |
| Ex. 213 | 0.026 | 0.000 |
| Ex. 214 | 0.062 | 0.001 |
| Ex. 215 | 0.064 | 0.000 |
| Ex. 216 | 0.062 | 0.001 |

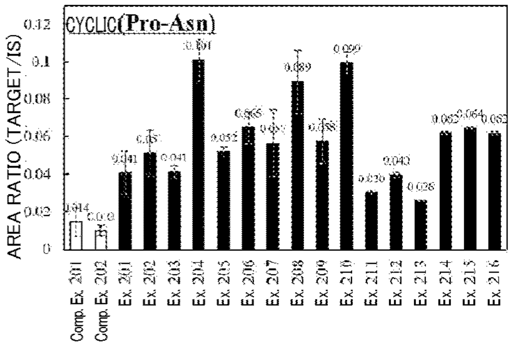


3 CYCLIC(Leu/Ile-Leu/Ile)

| | Ave. | SD |
|---|---|---|
| Comp. Ex. 201 | 0.273 | 0.025 |
| Comp. Ex. 202 | 0.296 | 0.003 |
| Ex. 201 | 0.401 | 0.012 |
| Ex. 202 | 0.400 | 0.042 |
| Ex. 203 | 0.445 | 0.022 |
| Ex. 204 | 0.449 | 0.027 |
| Ex. 205 | 0.484 | 0.048 |
| Ex. 206 | 0.512 | 0.002 |
| Ex. 207 | 0.512 | 0.010 |
| Ex. 208 | 0.547 | 0.036 |
| Ex. 209 | 0.507 | 0.034 |
| Ex. 210 | 0.566 | 0.037 |
| Ex. 211 | 0.584 | 0.008 |
| Ex. 212 | 0.438 | 0.013 |
| Ex. 213 | 0.599 | 0.014 |
| Ex. 214 | 0.394 | 0.014 |
| Ex. 215 | 0.326 | 0.020 |
| Ex. 216 | 0.386 | 0.002 |

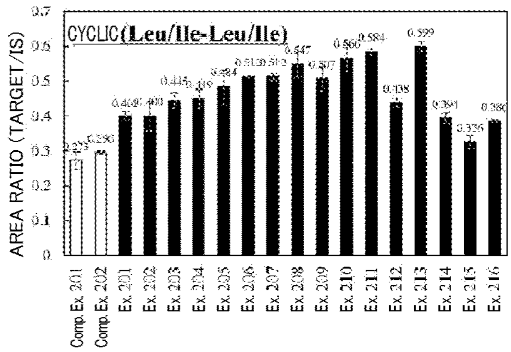


4 CYCLIC(Thr-Leu/Ile)

| | Ave. | SD |
|---|---|---|
| Comp. Ex. 201 | 0.012 | 0.000 |
| Comp. Ex. 202 | 0.012 | 0.000 |
| Ex. 201 | 0.027 | 0.001 |
| Ex. 202 | 0.023 | 0.000 |
| Ex. 203 | 0.025 | 0.002 |
| Ex. 204 | 0.019 | 0.001 |
| Ex. 205 | 0.021 | 0.001 |
| Ex. 206 | 0.025 | 0.001 |
| Ex. 207 | 0.029 | 0.003 |
| Ex. 208 | 0.029 | 0.001 |
| Ex. 209 | 0.019 | 0.002 |
| Ex. 210 | 0.022 | 0.000 |
| Ex. 211 | 0.030 | 0.001 |
| Ex. 212 | 0.041 | 0.001 |
| Ex. 213 | 0.039 | 0.000 |
| Ex. 214 | 0.047 | 0.002 |
| Ex. 215 | 0.027 | 0.002 |
| Ex. 216 | 0.018 | 0.001 |

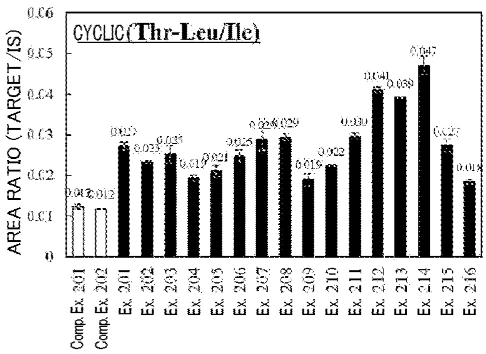


FIG. 1

## Description

### Technical Field

One or more aspects of the present invention relate to a flavor-enhancing composition, a method for producing the same, and a method for enhancing a flavor of a food product.

One or more other aspects of the present invention relate to heat-treated mustard seed, a food product composition including the same, and a method for producing the same.

### Background Art

Sodium chloride imparts a preferable taste to food products and is used as a source of chlorine and sodium, which are elements essential for sustaining life. On the other hand, sodium chloride intake is desired to be controlled because excessive sodium chloride intake is known to be a cause of many diseases such as hypertension.

Patent Literature 1 discloses a saltiness-enhancing spice mixture that contains paprika, yuzu rind and/or chenpi, ginger, and allspice in a specified ratio, and may further contain at least one spice selected from red chili pepper, cumin, coriander, and celery seed. The Patent Literature 1 describes that the aforesaid saltiness-enhancing spice mixture when including a smoked spice, can further enhance the saltiness-enhancing effect. Smoked paprika is described as a specific example of the smoked spice. According to the Patent Literature 1, the aforesaid saltiness-enhancing spice mixture, when added together with sodium chloride to a food product, exhibits the saltiness-enhancing effect on it.

Patent Literature 2 discloses a saltiness-enhancer containing a mixture of pepper, ginger, clove and cinnamon, and a saltiness enhancer containing a mixture of pepper, ginger, clove, cinnamon and capsicum.

Patent Literature 3 discloses a method for producing a caramelized-flavor-enhanced spice comprising a step of heating at least one spice selected from the group consisting of coriander, cumin, chenpi, anise, celery, turmeric, fenugreek, garlic, chili pepper, paprika, fennel, black pepper, ginger and asafoetida under a gauge pressure of 0.05 MPa or higher and a condition whereby a heating value is 15 to 170, and a method for producing an almond-flavor-enhanced spice comprising a step of heating at least one spice selected from the group consisting of turmeric, chili, fenugreek, cumin, coriander, chenpi, garlic, paprika, fennel, anise, celery, black pepper, ginger, fenugreek leaves and bay leaves under a gauge pressure of 0.05 MPa or lower and a condition whereby the heating value is 50 to 180. The Patent Literature 3 also discloses that a seared-flavor-enhanced spice can be obtained by heating coriander under a condition whereby the heating value is 800 or more.

Mustard seed is a spice having a distinctive flavor, including pungency, and is used as an ingredient in mustard and dressings. Mustard seed roasted in oil is commonly used as a starter spice in Indian cuisine.

Patent Literature 4 describes a seasoning composition including seeds and/or nuts as well as curry leaf and/or the extract thereof. The Patent Literature 4 describes that the aforesaid seasoning composition may further include roasted mustard seed in order to enhance the roasted flavor of the aforesaid seeds and/or nuts. However, the Patent Literature 4 does not describe any condition whereby the mustard seed is roasted.

### Citation List

#### Patent Literature

Patent Literature 1: JP 2018-102142
Patent Literature 2: JP 2012-239398
Patent Literature 3: JP 2020-103257
Patent Literature 4: JP 2020-202765

### Summary of Invention

#### Technical Problem

One or more aspects of the present invention provide a flavor-enhancing composition capable of enhancing the flavor of a food product by blending the composition with the aforesaid food product, and a method for producing the same. One or more aspects of the present invention also provide a method for enhancing a flavor of a food product.

One or more other aspects of the present invention provide mustard seed having a further enhanced flavor such as sweetness and roasted flavor, a food product composition including the mustard seed, and a method for producing mustard seed having further enhanced flavors such as sweetness and roasted flavor.

Solution to Problem

The present inventors have found the following means as a flavor-enhancing composition capable of enhancing a flavor of a food product, a method for producing the flavor-enhancing composition and a method for enhancing the flavor of the food product.

The present inventors have also found the following means as mustard seed having enhanced a flavor such as sweetness and roasted flavor, a food product composition including the mustard seed, a method for producing mustard seed having an enhanced flavor such as sweetness and roasted flavor, a flavor-enhancing composition and a method for enhancing a flavor of a food product.

[1] A flavor-enhancing composition containing one or more selected from the group consisting of heat-treated cumin, heat-treated paprika, heat-treated asafoetida and heat-treated mustard seed.
In the flavor-enhancing composition, said flavor can be one or more selected from saltiness and richness.
[2] The flavor-enhancing composition according to [1], containing said heat-treated cumin.
[3] The flavor-enhancing composition according to [2], wherein said heat-treated cumin is obtained by heating cumin without the addition of oil or fat under a condition whereby a heating value is 15 or more.
[4] The flavor-enhancing composition according to [3], wherein said heating value is 60 or more and 500 or less.
[5] The flavor-enhancing composition according to [2], wherein said heat-treated cumin is obtained by heating cumin together with oil or fat under a condition whereby the heating value is 1 or more.
[6] The flavor-enhancing composition according to [5], wherein said heating value is 20 or more and 4000 or less.
[7] The flavor-enhancing composition according to any one of [2] to [6], wherein said heat-treated cumin contains one or more selected the group consisting of cyclic (Phe-Tyr), cyclic (Phe-Ala), cyclic (Tyr-Ser), cyclic (Leu/Ile-Ser), cyclic (Ala-Tyr), cyclic (Gly-Tyr), cyclic (Ala-Pro), cyclic (Gly-Val), cyclic (hyPro-Pro) and cyclic (Glu-Glu).
[8] The flavor-enhancing composition according to any one of [2] to [7], wherein when said heat-treated cumin (representing a mass converted to that of the cumin excluding oil or fat when said heat-treated cumin is cumin heat-treated together with the oil or fat), with the addition of 47 μg/g of L-lysine-$^{13}C_6$ monohydrochloride, is analyzed by liquid chromatography-mass spectrometry (LC-MS/MS) according to the following method, said heat-treated cumin satisfies one or more of the following:

(1) a peak area of 0.024 or more derived from cyclic (Phe-Phe),
(2) a peak area of 0.040 or more derived from cyclic (Phe-Leu/Ile),
(3) a peak area of 0.015 or more derived from cyclic (Leu/Ile-Val),
(4) a peak area of 0.020 or more derived from cyclic (Phe-Pro),
(5) a peak area of 0.0020 or more derived from cyclic (Phe-Tyr),
(6) a peak area of 0.040 or more derived from cyclic (Pro-Leu/Ile),
(7) a peak area of 0.004 or more derived from cyclic (Val-Val),
(8) a peak area of 0.002 or more derived from cyclic (Phe-Ala),
(9) a peak area of 0.002 or more derived from cyclic (Tyr-Ser),
(10) a peak area of 0.013 or more derived from cyclic (Pro-Val),
(11) a peak area of 0.009 or more derived from cyclic (Pro-Tyr),
(12) a peak area of 0.050 or more derived from cyclic (Pro-Pro),
(13) a peak area of 0.010 or more derived from cyclic (Leu/Ile-Ser),
(14) a peak area of 0.10 or more derived from cyclic (Arg-Leu/Ile),
(15) a peak area of 0.007 or more derived from cyclic (Ala-Tyr),
(16) a peak area of 0.002 or more derived from cyclic (Gly-Tyr),
(17) a peak area of 0.020 or more derived from cyclic (Ala-Pro),
(18) a peak area of 0.001 or more derived from cyclic (Gly-Val),
(19) a peak area of 0.010 or more derived from cyclic (hyPro-Pro),
(20) a peak area of 0.040 or more derived from cyclic (Thr-Pro),
(21) a peak area of 0.060 or more derived from cyclic (Val-Arg),
(22) a peak area of 0.040 or more derived from cyclic (Pro-Asp),
(23) a peak area of 0.035 or more derived from cyclic (Arg-Pro),
(24) a peak area of 0.050 or more derived from cyclic (Glu-Tyr),
(25) a peak area of 0.020 or more derived from cyclic (Pro-His),
(26) a peak area of 0.080 or more derived from cyclic (Glu-Asp),
(27) a peak area of 0.50 or more derived from cyclic (Gly-Arg),

(28) a peak area of 0.005 or more derived from cyclic (Gly-His),
(29) a peak area of 0.070 or more derived from cyclic (Leu/Ile-Leu/Ile),
(30) a peak area of 0.010 or more derived from cyclic (Glu-Glu),
(31) a peak area of 0.010 or more derived from cyclic (Leu/Ile-Asp),
(32) a peak area of 0.012 or more derived from cyclic (Phe-Asp),
(33) a peak area of 0.30 or more derived from sulfurol(4-methyl-5-thiazoleethanol) and
(34) a peak area of 0.025 or more derived from sulfurol acetate,

as a ratio to a peak area derived from L-lysine-$^{13}C_6$ monohydrochloride in a chromatogram obtained.

(Measurement method of LC-MS/MS)

A 10-mL test tube containing 200 mg of said heat-treated cumin (representing a mass converted to that of the cumin excluding oil or fat when said heat-treated cumin is cumin heat-treated together with the oil or fat) and 5 mL of water is kept heated in a thermostatic water bath at 80 °C for 30 minutes, to prepare a water extract. To said water extract in said test tube are added 5 mL of acetonitrile and L-lysine-$^{13}C_6$ monohydrochloride in an amount of 47 μg/g with respect to said heat-treated cumin (representing a mass converted to that of the cumin excluding oil or fat when said heat-treated cumin is cumin heat-treated together with the oil or fat), followed by stirring, and then a solid portion is removed and a liquid portion is collected, to prepare a specimen sample. Said specimen sample is analyzed by LC-MS/MS to obtain a chromatogram. In this context, said heat-treated cumin used as the analysis sample is preferably a pulverized product.

[9] The flavor-enhancing composition according to any one of [2] to [8], wherein when said heat-treated cumin (representing a mass converted to that of the cumin excluding oil or fat when said heat-treated cumin is cumin heat-treated together with the oil or fat), with the addition of 99 μg/g of ribitol, is analyzed by liquid chromatography-mass spectrometry (LC-MS/MS) according to the method described below, said heat-treated cumin satisfies one or more of the following:

(35) a peak area of 0.180 or more derived from cis-aconitic acid,
(36) a peak area of 0.250 or more derived from *trans-aconitic* acid,
(37) a peak area of 0.550 or more derived from tartaric acid,
(38) a peak area of 22.0 or more derived from malic acid and
(39) a peak area of 35.0 or more derived from citric acid,
as a ratio to a peak area derived from ribitol in a chromatogram obtained.

(Measurement method of LC-MS/MS)

A 10-mL test tube containing 200 mg of said heat-treated cumin (representing a mass converted to that of the cumin excluding oil or fat when said heat-treated cumin is cumin heat-treated together with the oil or fat) and 5 mL of water is kept heated in a thermostatic water bath at 80 °C for 30 minutes, to prepare a water extract. To said water extract in said test tube are added 5 mL of acetonitrile and ribitol in an amount of 99 μg/g with respect to said heat-treated cumin (representing a mass converted to that of the cumin excluding oil or fat when said heat-treated cumin is cumin heat-treated together with the oil or fat), followed by stirring, and then a solid portion is removed and a liquid portion is collected, to prepare a specimen sample. Said specimen sample is analyzed by LC-MS/MS to obtain a chromatogram. In this context, said heat-treated cumin used as the analysis sample is preferably a pulverized product.

[10] The flavor-enhancing composition according to any one of [2] to [9],wherein when said heat-treated cumin (representing a mass converted to that of the cumin excluding oil or fat when said heat-treated cumin is cumin heat-treated together with the oil or fat), with the addition of 625 μg/g of 4-methylthiazole, is analyzed by gas chromatography-mass spectrometry (CG-MS) according to the method described below, said heat-treated cumin satisfies one or more of the following:

(40) a peak area of 0.05 or more derived from carveol,
(41) a peak area of 0.18 or more derived from nerolidol and
(42) a peak area of 100 or more derived from α-terpinene-7-al,
as a ratio to a peak area derived from 4-methylthiazole in a chromatogram obtained.

(Measurement method of GC-MS)

Fifteen milligrams of said heat-treated cumin (representing a mass converted to that of the cumin excluding oil or fat when said heat-treated cumin is cumin heat-treated together with the oil or fat), 4-methylthiazole in an amount of 625 μg/g with respect to said heat-treated cumin (representing a mass converted to that of cumin excluding oil or fat when said heat-treated cumin is cumin heat-treated together with the oil or fat), 5 mL of acetone and 5 mL of methanol contained in a 10-mL test tube are stirred, and then a solid portion is removed and a liquid portion is collected, to which is added 1 mL of acetone per 0.1 mL of said liquid portion, to prepare a GC-MS specimen sample. Said GC-MS specimen sample is analyzed by GC-MS, to obtain a chromatogram. In this context, said heat-treated cumin used as the analysis sample is preferably a pulverized product.

[11] The flavor-enhancing composition according to any one of [1] to [10], containing said heat-treated paprika.

[12] The flavor-enhancing composition according to [11], wherein said heat-treated paprika is obtained by heating paprika without the addition of oil or fat under a condition whereby the heating value is 50 or more.

[13] The flavor-enhancing composition according to [12], wherein said heating value is 200 or more and 600 or less.

[14] The flavor-enhancing composition according to [11], wherein said heat-treated paprika is obtained by heating paprika together with oil or fat under a condition whereby the heating value is 0.3 or more.

[15] The flavor-enhancing composition according to [14], wherein said heating value is 2.5 or more and 450 or less.

[16] The flavor-enhancing composition according to any one of [11] to [15], wherein said heat-treated paprika contains one or more selected from the group consisting of cyclic (Gly-His), cyclic (Gly-Ser), cyclic (hyPro-Pro), cyclic (Gly-Val), cyclic (Tyr-Asp) and cyclic (Ala-Asp).

[17] The flavor-enhancing composition according to any one of [11] to [16],wherein when said heat-treated paprika (representing a mass converted to that of the paprika excluding oil or fat when said heat-treated paprika is paprika heat-treated together with the oil or fat), with the addition of 47 μg/g of L-lysine-$^{13}C_6$ monohydrochloride, is analyzed by liquid chromatography-mass spectrometry (LC-MS/MS) according to the method described below, said heat-treated paprika satisfies one or more of the following:

(1) a peak area of 0.45 or more derived from cyclic (Phe-Leu/Ile),
(2) a peak area of 0.020 or more derived from cyclic (Pro-Asn),
(3) a peak area of 0.30 or more derived from cyclic (Leu/Ile-Leu/Ile),
(4) a peak area of 0.014 or more derived from cyclic (Thr-Leu/Ile),
(5) a peak area of 0.025 or more derived from cyclic (Phe-Ser),
(6) a peak area of 0.0020 or more derived from cyclic (Gly-His),
(7) a peak area of 0.0010 or more derived from cyclic (Gly-Ser),
(8) a peak area of 0.080 or more derived from cyclic (Gly-Arg),
(9) a peak area of 0.0140 or more derived from cyclic (Ala-His),
(10) a peak area of 0.040 or more derived from cyclic (Glu-His),
(11) a peak area of 0.21 or more derived from cyclic (Glu-Arg),
(12) a peak area of 0.24 or more derived from cyclic (Ala-Arg),
(13) a peak area of 0.020 or more derived from cyclic (Glu-Glu),
(14) a peak area of 0.39 or more derived from cyclic (Glu-Gly),
(15) a peak area of 0.19 or more derived from cyclic (Glu-Asp),
(16) a peak area of 0.12 or more derived from cyclic (Pro-His),
(17) a peak area of 0.40 or more derived from cyclic (Glu-Tyr),
(18) a peak area of 0.100 or more derived from cyclic (Leu/Ile-Asp),
(19) a peak area of 0.10 or more derived from cyclic (Pro-Asp),
(20) a peak area of 0.15 or more derived from cyclic (Val-Arg),
(21) a peak area of 0.40 or more derived from cyclic (Thr-Pro),
(22) a peak area of 0.020 or more derived from cyclic (hyPro-Pro),
(23) a peak area of 0.010 or more derived from cyclic (Gly-Val),
(24) a peak area of 0.10 or more derived from cyclic (Ala-Pro),
(25) a peak area of 0.025 or more derived from cyclic (Ala-Tyr),
(26) a peak area of 0.60 or more derived from cyclic (Arg-Leu/Ile),
(27) a peak area of 0.050 or more derived from cyclic (Leu/Ile-Ser),
(28) a peak area of 0.040 or more derived from cyclic (Ala-Val),
(29) a peak area of 0.30 or more derived from cyclic (Pro-Pro),
(30) a peak area of 0.025 or more derived from cyclic (Gly-Leu/Ile),
(31) a peak area of 0.060 or more derived from cyclic (Gly-Phe),
(32) a peak area of 0.060 or more derived from cyclic (Pro-Tyr),
(33) a peak area of 0.040 or more derived from cyclic (Phe-Asp),
(34) a peak area of 0.070 or more derived from cyclic (Pro-Val),
(35) a peak area of 0.080 or more derived from cyclic (Ala-Leu/Ile),

(36) a peak area of 0.020 or more derived from cyclic (Val-Tyr),
(37) a peak area of 0.20 or more derived from cyclic (Tyr-Ser),
(38) a peak area of 0.025 or more derived from cyclic (Phe-Ala),
(39) a peak area of 0.050 or more derived from cyclic (Val-Val),
(40) a peak area of 0.45 or more derived from cyclic (Pro-Leu/Ile),
(41) a peak area of 0.160 or more derived from cyclic (Phe-Tyr),
(42) a peak area of 0.020 or more derived from cyclic (Phe-Thr),
(43) a peak area of 0.45 or more derived from cyclic (Phe-Pro),
(44) a peak area of 0.09 or more derived from cyclic (Leu/Ile-Val),
(45) a peak area of 0.010 or more derived from cyclic (Val-Phe),
(46) a peak area of 0.020 or more derived from cyclic (Tyr-Asp),
(47) a peak area of 0.005 or more derived from cyclic (Ala-Asp),
(48) a peak area of 0.070 or more derived from cyclic (Val-Ser),
(49) a peak area of 0.090 or more derived from sulfurol(4-methyl-5-thiazoleethanol) and
(50) a peak area of 0.011 or more derived from sulfurol acetate,

as a ratio to a peak area derived from L-lysine-$^{13}C_6$ monohydrochloride in a chromatogram obtained.

(Measurement method of LC-MS/MS)

A 10-mL test tube containing 200 mg of said heat-treated paprika (representing a mass converted to that of the paprika excluding oil or fat when said heat-treated paprika is paprika heat-treated together with the oil or fat) and 5 mL of water is kept heated in a thermostatic water bath at 80 °C for 30 minutes, to prepare a water extract. To said water extract in said test tube are added 5 mL of acetonitrile and L-lysine-$^{13}C_6$ monohydrochloride in an amount of 47 μg/g with respect to said heat-treated paprika (representing a mass converted to that of the paprika excluding oil or fat when said heat-treated paprika is paprika heat-treated together with the oil or fat), followed by stirring, and then a solid portion is removed and a liquid portion is collected, to prepare a specimen sample. Said specimen sample is analyzed by LC-MS/MS to obtain a chromatogram. In this context, said heat-treated paprika used as the analysis sample is preferably a pulverized product.

[18] The flavor-enhancing composition according to any one of [11] to [17], wherein when said heat-treated paprika (representing a mass converted to that of paprika excluding the oil or fat when said heat-treated paprika is paprika heat-treated together with the oil or fat), with the addition of 99 μg/g of ribitol, is analyzed by liquid chromatography-mass spectrometry (LC-MS/MS) according to the following method, said heat-treated paprika satisfies one or more of the following:

(51) a peak area of 0.60 or more derived from tartaric acid and
(52) a peak area of 0.35 or more derived from *trans-aconitic* acid,
as a ratio to a peak area derived from ribitol in a chromatogram obtained.

(Measurement method of LC-MS/MS)

A 10-mL test tube containing 200 mg of said heat-treated paprika (representing a mass converted to that of the paprika excluding oil or fat when said heat-treated paprika is paprika heat-treated together with the oil or fat) and 5 mL of water is kept heated in a thermostatic water bath at 80 °C for 30 minutes, to prepare a water extract. To said water extract in said test tube are added 5 mL of acetonitrile and ribitol in an amount of 99 μg/g with respect to the heat-treated paprika (representing a mass converted to that of the paprika excluding oil or fat when said heat-treated paprika is paprika heat-treated together with the oil or fat), followed by stirring, and then a solid portion is removed and a liquid portion is collected, to prepare a specimen sample. Said specimen sample is analyzed by LC-MS/MS to obtain a chromatogram. In this context. said heat-treated paprika used as the analysis sample is preferably a pulverized product.

[19] The flavor-enhancing composition according to any one of [1] to [18], containing said heat-treated asafoetida.

[20] The flavor-enhancing composition according to [19], wherein said heat-treated asafoetida is obtained by heating asafoetida without the addition of oil or fat under a condition whereby the heating value is 15 or more.

[21] The flavor-enhancing composition according to [19], wherein said heat-treated asafoetida is obtained by heating asafoetida together with oil or fat under a condition whereby the heating value is 5 or more.

[22] The flavor-enhancing composition according to any one of [19] to [21], wherein said heat-treated asafoetida contains one or more selected from the group consisting of cyclic (Val-Ser), cyclic (Pro-Pro) and cyclic (Pro-Glu).

[23] The flavor-enhancing composition according to any one of [19] to [22], wherein when said heat-treated asafoetida, with the addition of 47 μg/g of L-lysine-$^{13}C_6$ monohydrochloride, is analyzed by liquid chromatography-mass spectrometry (LC-MS/MS) according to the following method, said heat-treated asafoetida satisfies one or more of the following:

(1) a peak area of 0.028 or more derived from cyclic (Gly-Thr),
(2) a peak area of 0.003 or more derived from cyclic (Gly-His),
(3) a peak area of 0.022 or more derived from cyclic (Ala-Asp),
(4) a peak area of 0.040 or more derived from cyclic (Val-Ser),
(5) a peak area of 0.020 or more derived from cyclic (Gly-Arg),
(6) a peak area of 0.005 or more derived from cyclic (Ala-His),
(7) a peak area of 0.008 or more derived from cyclic (Glu-Asp),
(8) a peak area of 0.050 or more derived from cyclic (Phe-Phe),
(9) a peak area of 0.025 or more derived from cyclic (Leu/Ile-Leu/Ile),
(10) a peak area of 0.025 or more derived from cyclic (Ala-Arg),
(11) a peak area of 0.020 or more derived from cyclic (Leu/Ile-Val),
(12) a peak area of 0.015 or more derived from cyclic (Phe-Pro),
(13) a peak area of 0.040 or more derived from cyclic (Phe-Leu/Ile),
(14) a peak area of 0.010 or more derived from cyclic (Pro-Pro),
(15) a peak area of 0.050 or more derived from cyclic (Glu-Phe),
(16) a peak area of 0.045 or more derived from cyclic (Arg-Leu/Ile),
(17) a peak area of 0.080 or more derived from cyclic (Pro-Leu/Ile),
(18) a peak area of 0.005 or more derived from cyclic (Pro-Glu),
(19) a peak area of 0.015 or more derived from cyclic (Glu-Leu/Ile),
(20) a peak area of 0.028 or more derived from cyclic (Ala-Pro),
(21) a peak area of 0.020 or more derived from cyclic (Pro-Val),
(22) a peak area of 0.040 or more derived from cyclic (Pro-His) and
(23) a peak area of 0.25 or more derived from sulfurol(4-methyl-5-thiazoleethanol),

as a ratio to a peak area derived from L-lysine-$^{13}C_6$ monohydrochloride in a chromatogram obtained.

(Measurement method of LC-MS/MS)

A 10-mL test tube containing 200 mg of said heat-treated asafoetida (mass converted under the assumption that the asafoetida resin content is 12% by mass) and 5 mL of water is kept heated in a thermostatic water bath at 80 °C for 30 minutes, to prepare a water extract. To said water extract in said test tube are added 5 mL of acetonitrile and L-lysine-$^{13}C_6$ monohydrochloride in an amount of 47 μg/g with respect to said heat-treated asafoetida (mass converted under the assumption that the asafoetida resin content is 12% by mass), followed by stirring, and then a solid portion is removed and a liquid portion is collected. After contacting said liquid portion with a solid-phase extraction carrier that carries an octadecyl group, an eluate by acetonitrile is collected, to prepare a specimen sample from said eluate. Said specimen sample is analyzed by LC-MS/MS to obtain a chromatogram. In this context, said heat-treated asafoetida used as the analysis sample is preferably a pulverized product.

[24] The flavor-enhancing composition according to any one of [19] to [23], wherein when said heat-treated asafoetida, with the addition of 99 μg/g of ribitol, is analyzed by liquid chromatography-mass spectrometry (LC-MS/MS) according to the method described below, said heat-treated asafoetida satisfies one or more of the following:

(24) a peak area of 0.12 or more derived from tartaric acid,
(25) a peak area of 1.10 or more derived from malic acid and
(26) a peak area of 1.00 or more derived from citric acid,
as a ratio to a peak area derived from ribitol in a chromatogram obtained.

(Measurement method of LC-MS/MS)

A 10-mL test tube containing 200 mg of said heat-treated asafoetida (mass converted under the assumption that the asafoetida resin content is 12% by mass) and 5 mL of water is kept heated in a thermostatic water bath at 80 °C for 30 minutes, to prepare a water extract. To said water extract in said test tube are added 5 mL of acetonitrile and ribitol in an amount of 99 μg/g with respect to said heat-treated asafoetida (mass converted under the assumption that the asafoetida resin content is 12% by mass), followed by stirring, and then a solid portion is removed and a liquid portion is collected. After contacting said liquid portion with a solid-phase extraction carrier that carries an octadecyl group, an eluate by acetonitrile is collected, to prepare a specimen sample from said eluate. Said specimen sample is analyzed by LC-MS/MS to obtain a chromatogram. In this context, said heat-treated asafoetida used as the analysis sample is preferably a pulverized product.

[25] The flavor-enhancing composition according to any one of [1] to [24], containing said heat-treated mustard seed.

[26] The flavor-enhancing composition according to [25], wherein when said heat-treated mustard seed (representing a mass converted to that of the mustard seed excluding oil or fat when said heat-treated mustard seed is mustard seed heat-treated together with the oil or fat), with the addition of 47 μg/g of L-lysine-$^{13}C_6$ monohydrochloride, is analyzed by liquid chromatography-mass spectrometry (LC-MS/MS) according to the method described below, said heat-treated mustard seed satisfies one or more of the following:

(1) a peak area of 0.20 or more derived from cyclic (Glu-His),
(2) a peak area of 0.29 or more derived from cyclic (Gly-Arg),
(3) a peak area of 0.15 or more derived from cyclic (Glu-Gly),
(4) a peak area of 0.12 or more derived from cyclic (Ala-His),
(5) a peak area of 0.20 or more derived from cyclic (Ala-Arg),
(6) a peak area of 0.13 or more derived from cyclic (Glu-Asp),
(7) a peak area of 0.035 or more derived from cyclic (Leu/Ile-Asp),
(8) a peak area of 0.50 or more derived from cyclic (Thr-Pro),
(9) a peak area of 0.09 or more derived from cyclic (Pro-His),
(10) a peak area of 0.50 or more derived from cyclic (Pro-Pro),
(11) a peak area of 0.070 or more derived from cyclic (Glu-Tyr),
(12) a peak area of 0.015 or more derived from cyclic (Arg-Leu/Ile),
(13) a peak area of 0.70 or more derived from cyclic (Ala-Pro),
(14) a peak area of 0.65 or more derived from cyclic (Pro-Val),
(15) a peak area of 0.060 or more derived from cyclic (Phe-Ala),
(16) a peak area of 0.070 or more derived from cyclic (Phe-Pro),
(17) a peak area of 0.18 or more derived from cyclic (Phe-Tyr),
(18) a peak area of 0.02 or more derived from cyclic (Tyr-His),
(19) a peak area of 0.05 or more derived from cyclic (Leu/Ile-His),
(20) a peak area of 0.32 or more derived from cyclic (Pro-Met) and
(21) a peak area of 0.10 or more derived from cyclic (Val-Glu),

as a ratio to a peak area derived from L-lysine-$^{13}C_6$ monohydrochloride in a chromatogram obtained.

(Measurement method of LC-MS/MS)

A 10-mL test tube containing 200 mg of said heat-treated mustard seed (representing a mass converted to that of the mustard seed excluding oil or fat when said heat-treated mustard seed is mustard seed heat-treated together with the oil or fat) and 5 mL of water is kept heated in a thermostatic water bath at 80 °C for 30 minutes, to prepare a water extract. To said water extract in said test tube are added 5 mL of acetonitrile and L-lysine-$^{13}C_6$ monohydrochloride in an amount of 47 μg/g with respect to said heat-treated mustard seed (representing a mass converted to that of the mustard seed excluding oil or fat when said heat-treated mustard seed is mustard seed heat-treated together with the oil or fat), followed by stirring, and then a solid portion is removed and a liquid portion is collected, to prepare an LC-MS/MS specimen sample. Said LC-MS/MS specimen sample is analyzed by LC-MS/MS to obtain a chromatogram. In this context, said heat-treated mustard seed used as the analysis sample is preferably a pulverized product.

[27] The flavor-enhancing composition according to [25] or [26], wherein when said heat-treated mustard seed (representing a mass converted to that of the mustard seed excluding oil or fat when said heat-treated mustard seed is mustard seed heat-treated together with the oil or fat), with the addition of 625 μg/g of 4-methylthiazole, is analyzed by gas chromatography-mass spectrometry (GC-MS) according to the following method, said heat-treated mustard seed satisfies one or more of the following:

(22) a peak area of 0.006 or more derived from allylmethyl disulfide,
(23) a peak area of 0.60 or more derived from 2,5-dimethylpyrazine,
(24) a peak area of 0.15 or more derived from 2-ethyl-6-methylpyrazine,
(25) a peak area of 0.15 or more derived from 2,3,5-trimethylpyrazine,
(26) a peak area of 0.01 or more derived from 2,6-diethylpyrazine,
(27) a peak area of 0.20 or more derived from 2,5-diethylpyrazine,
(28) a peak area of 0.03 or more derived from 2,3-diethylpyrazine,

(29) a peak area of 0.025 or more derived from allylmethyl trisulfide,
(30) a peak area of 0.70 or more derived from 3-methyl-1,2,4-trithiane,
(31) a peak area of 0.10 or more derived from 2-dodecenal and
(32) a peak area of 0.50 or more derived from sulfurol,
as a ratio to a peak area derived from 4-methylthiazole in a chromatogram obtained.

(Measurement method of GC-MS)

Fifteen milligrams of said heat-treated mustard seed (representing a mass converted to that of the mustard seed excluding oil or fat when said heat-treated mustard seed is mustard seed heat-treated together with the oil or fat), 4-methylthiazole in an amount of 625 μg/g with respect to said heat-treated mustard seed (representing a mass converted to that of the mustard seed excluding oil or fat when said heat-treated mustard seed is mustard seed heat-treated together with the oil or fat), 5 mL of acetone and 5 mL of methanol contained in a 10-mL test tube are stirred, and then a solid portion is removed and a liquid portion is collected, to which is added 1 mL of acetone per 0.1 mL of said liquid portion, to prepare a GC-MS specimen sample. Said GC-MS specimen sample is analyzed by GC-MS, to obtain a chromatogram. In this context. said heat-treated mustard seed used as the analysis sample is preferably a pulverized product.

[28] A method for producing the flavor-enhancing composition according to any one of [2] to [10], comprising: heating cumin without the addition of oil or fat under a condition whereby the heating value is 15 or more to obtain said heat-treated cumin.

[29] The method according to [28], wherein said heating value is 60 or more and 500 or less.

[30] A method for producing the flavor-enhancing composition according to any one of [2] to [10], comprising: heating cumin together with oil or fat under a condition whereby the heating value is 1 or more to obtain said heat-treated cumin.

[31] The method according to [30], wherein said heating value is 20 or more and 4000 or less.

[32] A method for producing the flavor-enhancing composition according to any one of [11] to [18], comprising: heating paprika without the addition of oil or fat under a condition whereby the heating value is 50 or more to obtain said heat-treated paprika.

[33] The method according to [32], wherein said heating value is 200 or more and 600 or less.

[34] A method for producing the flavor-enhancing composition according to any one of [11] to [18], comprising: heating paprika together with oil or fat under a condition whereby the heating value is 0.3 or more to obtain said heat-treated paprika.

[35] The method according to [34], wherein said heating value is 2.5 or more and 450 or less.

[36] A method for producing the flavor-enhancing composition according to any one of [19] to [24], comprising: heating asafoetida without the addition of oil or fat under a condition whereby the heating value is 15 or more to obtain said heat-treated asafoetida.

[37] A method for producing the flavor-enhancing composition according to any one of [19] to [24], comprising: heating asafoetida together with the addition of oil or fat under a condition whereby the heating value is 5 or more to obtain said heat-treated asafoetida.

[38] A method for producing the flavor-enhancing composition according to any one of [25] to [27], comprising: heating mustard seed in an open system under a condition whereby the heating value is 200 or more to obtain said heat-treated mustard seed.

[39] A method for enhancing a flavor of a food product, comprising blending the flavor-enhancing composition according to any one of [1] to [27]. In this method, said flavor can be one or more flavors selected from saltiness and richness.

[40] Use of one or more spices selected from the group consisting of heat-treated cumin, heat-treated paprika, heat-treated asafoetida and heat-treated mustard seed for enhancing a flavor of a food product. In the use, said flavor can be one or more flavors selected from, for example, saltiness and richness.

[41] The use according to [40], wherein said spice includes said heat-treated cumin,
which is the heat-treated cumin as defined in any of [2] to [10].

[42] The use according to [40] or [41], wherein said spice includes said heat-treated paprika,
which is the heat-treated paprika as defined in any of [11] to [18].

[43] The use according to [40] to [42], wherein said spice includes said heat-treated asafoetida,
which is the heat-treated asafoetida as defined in any of [19] to [24].

[44] The use according to any of [40] to [43], wherein said spice includes said heat-treated mustard seed,
which is the heat-treated mustard seed as defined in any of [25] to [27].

[45] A method for enhancing a flavor of a food product, comprising blending one or more spices selected from the group consisting of heat-treated cumin, heat-treated paprika, heat-treated asafoetida and heat-treated mustard seed. In this method, said flavor can be one or more flavors selected from, for example, saltiness and richness.

[46] The method according to [45], wherein said spice includes said heat-treated cumin, which is the heat-treated cumin as defined in any of [2] to [10].

[47] The method according to [45] or [46], wherein said spice includes said heat-treated paprika, which is the heat-treated paprika as defined in any of [11] to [18].

[48] The method according to any of [45] to [47], wherein said spice includes said heat-treated asafoetida, which is the heat-treated asafoetida as defined in any of [19] to [24].

[49] The method according to any of [45] to [48], wherein said spice includes said heat-treated mustard seed, which is said heat-treated mustard seed as defined in any of [25] to [27].

[50] One or more spices selected from the group consisting of heat-treated cumin, heat-treated paprika, heat-treated asafoetida and heat-treated mustard seed for use in enhancing a flavor of a food product. In this spice, said flavor can be one or more flavors selected from, for example, saltiness and richness.

[51] The spice according to [50], wherein said spice includes said heat-treated cumin, which is the heat-treated cumin as defined in any of [2] to [10].

[52] The spice according to [50] or [51], wherein said spice includes said heat-treated paprika, which is the heat-treated paprika as defined in any of [11] to [18].

[53] The spice according to any of [50] to [52], wherein said spice includes said heat-treated asafoetida, and which is the heat-treated asafoetida as defined in any of [19] to [24].

[54] The spice according to any of [50] to [53], wherein said spice includes said heat-treated mustard seed, which is the heat-treated mustard seed as defined in any of [25] to [27].

[55] Use of one or more spices selected from the group consisting of heat-treated cumin, heat-treated paprika, heat-treated asafoetida and heat-treated mustard seed in producing an additive for use in enhancing a flavor of a food product. In the use, said flavor can be one or more flavors selected from, for example, saltiness and richness.

[56] The use according to [55], wherein said spice includes said heat-treated cumin, which is the heat-treated cumin as defined in any of [2] to [10].

[57] The use according [55] or [56], wherein said spice includes said heat-treated paprika, which is the heat-treated paprika as defined in any of [11] to [18].

[58] The use according to any of [55] to [57], wherein said spice includes said heat-treated asafoetida, which is the heat-treated asafoetida as defined in any of [19] to [24].

[59] The use according to any of [55] to [58], wherein said spice includes said heat-treated mustard seed, which is the heat-treated mustard seed as defined in any of [25] to [27].

[60] The flavor-enhancing composition according to any one of [1] to [27], the use according to any one of [40] to [44], the method according to any of [45] to [49], the spice according to any one of [50] to [54], or the use according to any one of [55] to [59], wherein said one or more selected from the group consisting of the heat-treated cumin, heat-treated paprika, heat-treated asafoetida and heat-treated mustard seed are not combined with seeds or nuts, or said flavor-enhancing composition is not used for enhancing the flavor of seeds or nuts.

[61] The method according to [39], the use according to any one of [40] to [44], the method according to any one of [45] to [49], the spice according to any one of [50] to [54] or the use according to any one of [55] to [59], wherein said food product does not include seeds or nuts.

[62] A heat-treated mustard seed, wherein when said heat-treated mustard seed (representing a mass converted to that of the mustard seed excluding oil or fat when said heat-treated mustard is mustard seed heat-treated together with the oil or fat), with the addition of 47 μg/g of L-lysine-$^{13}C_6$ monohydrochloride, is analyzed by liquid chromatography-mass spectrometry (LC-MS/MS) according to the method described below, said heat-treated mustard seed satisfies one or more of the following:

(1) a peak area of 0.20 or more derived from cyclic (Glu-His),
(2) a peak area of 0.29 or more derived from cyclic (Gly-Arg),
(3) a peak area of 0.15 or more derived from cyclic (Glu-Gly),
(4) a peak area of 0.12 or more derived from cyclic (Ala-His),
(5) a peak area of 0.20 or more derived from cyclic (Ala-Arg),
(6) a peak area of 0.13 or more derived from cyclic (Glu-Asp),
(7) a peak area of 0.035 or more derived from cyclic (Leu/Ile-Asp),
(8) a peak area of 0.50 or more derived from cyclic (Thr-Pro),
(9) a peak area of 0.09 or more derived from cyclic (Pro-His),
(10) a peak area of 0.50 or more derived from cyclic (Pro-Pro),
(11) a peak area of 0.070 or more derived from cyclic (Glu-Tyr),
(12) a peak area of 0.015 or more derived from cyclic (Arg-Leu/Ile),
(13) a peak area of 0.70 or more derived from cyclic (Ala-Pro),
(14) a peak area of 0.65 or more derived from cyclic (Pro-Val),

(15) a peak area of 0.060 or more derived from cyclic (Phe-Ala),
(16) a peak area of 0.070 or more derived from cyclic (Phe-Pro),
(17) a peak area of 0.18 or more derived from cyclic (Phe-Tyr),
(18) a peak area of 0.02 or more derived from cyclic (Tyr-His),
(19) a peak area of 0.05 or more derived from cyclic (Leu/Ile-His),
(20) a peak area of 0.32 or more derived from cyclic (Pro-Met) and
(21) a peak area of 0.10 or more derived from cyclic (Val-Glu),

as a ratio to a peak area derived from L-lysine-$^{13}C_6$ monohydrochloride in a chromatogram obtained.

(Measurement method of LC-MS/MS)

A 10-mL test tube containing 200 mg of said heat-treated mustard seed (representing a mass converted to that of the mustard seed excluding oil or fat when said heat-treated mustard seed is mustard seed heat-treated together with the oil or fat) and 5 mL of water is kept heated in a thermostatic water bath at 80 °C for 30 minutes, to prepare a water extract. To said water extract in said test tube are added 5 mL of acetonitrile and L-lysine-$^{13}C_6$ monohydrochloride in an amount of 47 μg/g with respect to said heat-treated mustard seed (representing a mass converted to that of the mustard seed excluding oil or fat when said heat-treated mustard seed is mustard seed heat-treated together with the oil or fat), followed by stirring, and then a solid portion is removed and a liquid portion is collected, to prepare an LC-MS/MS specimen sample. Said LC-MS/MS specimen sample is analyzed by LC-MS/MS to obtain a chromatogram. In this context, said heat-treated mustard seed used as the analysis sample is preferably a pulverized product.
[63] The heat-treated mustard seed according to [62], wherein said heat-treated mustard seed (representing a mass converted to that of the mustard seed excluding oil or fat when said heat-treated mustard seed is mustard seed heat-treated together with the oil or fat), with the addition of 625 μg/g of 4-methylthiazole, is analyzed by gas chromatography-mass spectrometry (GC-MS) according to the method described below, said heat-treated mustard seed satisfies one or more of the following:

(22) a peak area of 0.006 or more derived from allylmethyl disulfide,
(23) a peak area of 0.60 or more derived from 2,5-dimethylpyrazine,
(24) a peak area of 0.15 or more derived from 2-ethyl-6-methylpyrazine,
(25) a peak area of 0.15 or more derived from 2,3,5-trimethylpyrazine,
(26) a peak area of 0.01 or more derived from 2,6-diethylpyrazine,
(27) a peak area of 0.20 or more derived from 2,5-diethylpyrazine,
(28) a peak area of 0.03 or more derived from 2,3-diethylpyrazine,
(29) a peak area of 0.025 or more derived from allylmethyl trisulfide,
(30) a peak area of 0.70 or more derived from 3-methyl-1,2,4-trithiane,
(31) a peak area of 0.10 or more derived from 2-dodecenal and
(32) a peak area of 0.50 or more derived from sulfurol,
as a ratio to a peak area derived from 4-methylthiazole in a chromatogram obtained.

(Measurement method of GC-MS)

Fifteen milligrams of said heat-treated mustard seed (representing a mass converted to that of the mustard seed excluding oil or fat when said heat-treated mustard seed is mustard seed heat-treated together with the oil or fat), 4-methylthiazole in an amount of 625 μg/g with respect to said heat-treated mustard seed (representing a mass converted to that of the mustard seed excluding oil or fat when said heat-treated mustard seed is mustard seed heat-treated together with the oil or fat), 5 mL of acetone and 5 mL of methanol contained in a 10-mL test tube are stirred, and then a solid portion is removed and a liquid portion is collected, to which is added 1 mL of acetone per 0.1 mL of said liquid portion, to prepare a GC-MS specimen sample. Said GC-MS specimen sample is analyzed by GC-MS, to obtain a chromatogram. In this context, said heat-treated mustard seed used as the analysis sample is preferably a pulverized product.
[64] A food product composition containing the mustard seed according to [62] or [63].
[65] A method for producing a heat-treated mustard seed, comprising
heat-treating the mustard seed in an open system under a condition whereby a heating value is 200 or more.
[66] The method according to [65], wherein said heat-treated mustard seed to be produced is the mustard seed according to [62] or [63].
This specification encompasses the content of the disclosure of Japanese Patent Application No. 2023-013575, JP 2023-013576, JP 2023-013623 and JP 2023-074823, on which the priority of the present application is based. All publications, patents and patent applications cited herein are to be incorporated herein by reference in their entirety.

Advantageous Effect of Invention

The flavor-enhancing composition according to one or more embodiments of the present invention can enhance a flavor of a food product when blended with the aforesaid food product.

According to the method for producing a flavor-enhancing composition according to one or more embodiments of the present invention, the aforesaid flavor-enhancing composition can be produced.

According to the method for enhancing a flavor of a food product according to one or more embodiments of the present invention, the flavor of a food product can be enhanced by blending the aforesaid flavor-enhancing composition with the aforesaid food product.

The heat-treated mustard seed according to one or more embodiments of the present invention exhibits increased sweetness and roasted flavor, and can enhance the flavor of a food product when blended with the food product.

The method for producing the heat-treated mustard seed according to one or more embodiments of the present invention can produce mustard seed having the desirable property as described above.

Brief Description of Drawings

Figure 1 shows the ratio of the peak area of cyclic (Phe-Leu/Ile) (1), cyclic (Pro-Asn) (2), cyclic (Leu/Ile-Leu/Ile) (3) and cyclic (Thr-Leu/Ile) (4), to the peak area of an internal standard (47 μg/g of L-lysine-$^{13}C_6$ monohydrochloride) in an extracted ion chromatogram obtained by LC-MS/MS, in unheated paprika (Comparative Examples 201, 202) and in paprika heat-treated under a specified condition (Examples 201 to 216).

Figure 2 shows the ratio of the peak area of cyclic (Phe-Ser) (5), cyclic (Gly-His) (6), cyclic (Gly-Ser) (7) and cyclic (Gly-Arg) (8), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 3 shows the ratio of the peak area of cyclic (Ala-His) (9), cyclic (Glu-His) (10), cyclic (Glu-Arg) (11) and cyclic (Ala-Arg) (12), to the peak area of the internal standard (47 μg/g of L-lysine-$^{13}C_6$ monohydrochloride) in an extracted ion chromatogram obtained by LC-MS/MS, in unheated paprika (Comparative Examples 201, 202) and in paprika heat-treated under a specified condition (Examples 201 to 216).

Figure 4 shows the ratio of the peak area of cyclic (Glu-Glu) (13), cyclic (Glu-Gly) (14), cyclic (Glu-Asp) (15) and cyclic (Pro-His) (16), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 5 shows the ratio of the peak area of cyclic (Glu-Tyr) (17), cyclic (Leu/Ile-Asp) (18), cyclic (Pro-Asp) (19) and cyclic (Val-Arg) (20), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 6 shows the ratio of the peak area of cyclic (Thr-Pro) (21), cyclic (hyPro-Pro) (22), cyclic (Gly-Val) (23) and cyclic (Ala-Pro) (24), to the peak area of the internal standard (47 μg/g L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 7 shows the ratio of the peak area of cyclic (Ala-Tyr) (25), cyclic (Arg-Leu/Ile) (26), cyclic (Leu/Ile-Ser) (27) and cyclic (Ala-Val) (28), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 8 shows the ratio of the peak area of cyclic (Pro-Pro) (29), cyclic (Gly-Leu/Ile) (30), cyclic (Gly-Phe) (31) and cyclic (Pro-Tyr) (32), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 9 shows the ratio of the peak area of cyclic (Phe-Asp) (33), cyclic (Pro-Val) (34), cyclic (Ala-Leu/Ile) (35) and cyclic (Val-Tyr) (36), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 10 shows the ratio of the peak area of cyclic (Tyr-Ser) (37), cyclic (Phe-Ala) (38), cyclic (Val-Val) (39) and cyclic (Pro-Leu/Ile) (40), to the peak area of the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 11 shows the ratio of the peak area of cyclic (Phe-Tyr) (41), cyclic (Phe-Thr) (42), cyclic (Phe-Pro) (43) and cyclic (Leu/Ile-Val) (44), to that for the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 12 shows the ratio of the peak area of cyclic (Val-Phe) (45), cyclic (Tyr-Asp) (46), cyclic (Ala-Asp) (47) and cyclic (Val-Ser) (48), to that for the internal standard (47 μg/g of L-lysine-$^{13}C_6$ monohydrochloride) in an extracted ion chromatogram obtained by LC-MS/MS, in unheated paprika (Comparative Examples 201 and 202) and in paprika heat-treated under a specified condition (Examples 201 to 216).

Figure 13 shows the ratio of the peak area of sulfurol and sulfurol acetate, to the peak area of the internal standard (47 μg/g of L-lysine-$^{13}C_6$ monohydrochloride) in an extracted ion chromatogram obtained by LC-MS/MS, and the ratio of the peak area of tartaric acid and *trans*-aconitic acid, to that for the internal standard (99 μg/g ribitol) in an extracted ion chromatogram obtained by LC-MS/MS, in unheated paprika (Comparative Examples 201 and 202) and in paprika heat-treated under a specified condition (Examples 201 to 216).

Figure 14 shows the ratio of the peak area of cyclic (Phe-Phe) (1), cyclic (Phe-Leu/Ile) (2) and cyclic (Leu/Ile-Val) (3), to the peak area of the internal standard (47 μg/g of L-lysine-$^{13}C_6$ monohydrochloride) in an extracted ion chromatogram obtained by LC-MS/MS, in an unheated cumin (Comparative Example 301) and in cumin heat-treated under a specified condition (Examples 301 to 319).

Figure 15 shows the ratio of the peak area of cyclic (Phe-Pro) (4), cyclic (Phe-Tyr) (5) and cyclic (Pro-Leu/Ile) (6), to the peak area of the aforesaid internal standard in an extracted ion chromatogram obtained by LC-MS/MS, in an unheated cumin (Comparative Example 301) and in cumin heat-treated under a specified condition (Examples 301 to 319).

Figure 16 shows the ratio of the peak area of cyclic (Val-Val) (7), cyclic (Phe-Ala) (8) and cyclic (Tyr-Ser) (9), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 17 shows the ratio of the peak area of cyclic (Pro-Val) (10), cyclic (Pro-Tyr) (11) and cyclic (Pro-Pro) (12), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 18 shows the ratio of the peak area of cyclic (Leu/Ile-Ser) (13), cyclic (Arg-Leu/Ile) (14) and cyclic (Ala-Tyr) (15), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 19 shows the ratio of the peak area of cyclic (Gly-Tyr) (16), cyclic (Ala-Pro) (17) and cyclic (Gly-Val) (18), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 20 shows the ratio of the peak area of cyclic (hyPro-Pro) (19), cyclic (Thr-Pro) (20) and cyclic (Val-Arg) (21), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 21 shows the ratio of the peak area of cyclic (Pro-Asp) (22), cyclic (Arg-Pro) (23) and cyclic (Glu-Tyr) (24), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 22 shows the ratio of the peak area of cyclic (Pro-His) (25), cyclic (Glu-Asp) (26) and cyclic (Gly-Arg) (27), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 23 shows the ratio of the peak area of cyclic (Gly-His) (28), cyclic (Leu/Ile-Leu/Ile) (29) and cyclic (Glu-Glu) (30), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 24 shows the ratio of the peak area of cyclic (Leu/Ile-Asp) (31) and cyclic (Phe-Asp) (32), to the peak area of the aforesaid internal standard in an extracted ion chromatogram obtained by LC-MS/MS, in unheated cumin (Comparative Example 301) and in cumin heat-treated under a specified condition (Examples 301 to 319).

Figure 25 shows the ratio of the peak area of sulfurol (top) and sulfurol acetate (middle), to the peak area of the internal standard (47 μg/g of L-lysine-$^{13}C_6$ monohydrochloride) in an extracted ion chromatogram obtained by LC-MS/MS, and the ratio of the peak area of cis-aconitic acid (bottom), to the peak area of the internal standard (99 μg/g of ribitol) in an extracted ion chromatogram obtained by LC-MS/MS, in unheated cumin (Comparative Example 301) and in cumin heat-treated under a specified condition (Examples 301 to 319).

Figure 26 shows the ratio of the peak area of *trans-aconitic* acid (top), tartaric acid (middle) and malic acid (bottom), to the peak area of the internal standard (99 μg/g of ribitol) in an extracted ion chromatogram obtained by LC-MS/MS, in unheated cumin (Comparative Example 301) and in cumin heat-treated under a specified condition (Examples 301 to 319).

Figure 27 shows the ratio of the peak area of citric acid to the peak area of the internal standard (99 μg/g of ribitol) in an extracted ion chromatogram obtained by LC-MS/MS, in unheated cumin (Comparative Example 301) and in cumin heat-treated under a specified condition (Examples 301 to 319).

Figure 28 shows the ratio of the peak area of carveol (top), nerolidol (middle) and α-terpinene-7-al (bottom), to the peak area of the internal standard (625 μg/g of 4-methylthiazole) in an extracted ion chromatogram obtained by GC-MS, in unheated cumin (Comparative Example 301) and in cumin heat-treated under a specified condition (Examples 301 to 319).

Figure 29 shows the ratio of the peak area of cyclic (Gly-Thr) (1), cyclic (Gly-His) (2), cyclic (Ala-Asp) (3) and cyclic (Val-Ser) (4), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 30 shows the ratio of the peak area of cyclic (Gly-Arg) (5), cyclic (Ala-His) (6), cyclic (Glu-Asp) (7) and cyclic (Phe-Phe) (8), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 31 shows the ratio of the peak area of cyclic (Leu/Ile-Leu/Ile) (9), cyclic (Ala-Arg) (10) and cyclic (Leu/Ile-Val) (11) and cyclic (Phe-Pro) (12), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 32 shows the ratio of the peak area of cyclic (Phe-Leu/Ile) (13), cyclic (Pro-Pro) (14), cyclic (Glu-Phe) (15) and cyclic (Arg-Leu/Ile) (16), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 33 shows the ratio of the peak area of cyclic (Pro-Leu/Ile) (17), cyclic (Pro-Glu) (18), cyclic (Glu-Leu/Ile) (19) and cyclic (Ala-Pro) (20), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 34 shows the ratio of the peak area of cyclic (Pro-Val) (21) and cyclic (Pro-His) (22), to the peak area of the internal standard (47 μg/g of L-lysine-$^{13}C_6$ monohydrochloride) in an extracted ion chromatogram obtained by LC-MS/MS, in unheated asafoetida (Comparative Example 401) and in asafoetida heat-treated under a specified condition (Examples 401 to 404).

Figure 35 shows the ratio of the peak area of sulfurol to the peak area of the internal standard (47 μg/g of L-lysine-$^{13}C_6$ monohydrochloride) in an extracted ion chromatogram obtained by LC-MS/MS, and the ratio of the peak area of tartaric acid, citric acid and malic acid, to the peak area of the internal standard (99 μg/g of ribitol) in an extracted ion chromatogram obtained by LC-MS/MS, in unheated asafoetida (Comparative Example 401) and in asafoetida heat-treated under a specified condition (Examples 401 to 404).

Figure 36 shows the ratio of the peak area of specified cyclic dipeptides, to the peak area of the internal standard (47 μg/g of L-lysine-$^{13}C_6$ monohydrochloride) in an extracted ion chromatogram obtained by LC-MS/MS, in the mustard seed of Examples and Comparative Examples.

Figure 37 shows the ratio of the peak area of specified cyclic dipeptides, to the peak area of the internal standard (47 μg/g of L-lysine-$^{13}C_6$ monohydrochloride) in an extracted ion chromatogram obtained by LC-MS/MS, in the mustard seed of Examples and Comparative Examples.

Figure 38 shows the ratio of the peak area of specified cyclic dipeptides, to the peak area of the internal standard (47 μg/g of L-lysine-$^{13}C_6$ monohydrochloride) in an extracted ion chromatogram obtained by LC-MS/MS, in the mustard seed of Examples and Comparative Examples.

Figure 39 shows the ratio of the peak area of specified cyclic dipeptides, to the internal standard (47 μg/g of L-lysine-$^{13}C_6$ monohydrochloride) in an extracted ion chromatogram obtained by LC-MS/MS, in the mustard seed of Examples and Comparative Examples.

Figure 40 shows the ratio of the peak area of specified cyclic dipeptides, to the peak area of the internal standard (47 μg/g of L-lysine-$^{13}C_6$ monohydrochloride) in an extracted ion chromatogram obtained by LC-MS/MS, in the mustard seed of Examples and Comparative Examples.

Figure 41 shows the ratio of the peak area of specified cyclic dipeptides, to the peak area of the internal standard (47 μg/g of L-lysine-$^{13}C_6$ monohydrochloride) in an extracted ion chromatogram obtained by LC-MS/MS, in the mustard seed of Examples and Comparative Examples.

Figure 42 shows the ratio of the peak area of specified constituents to the peak area of the internal standard (625 μg/g of 4-methylthiazole) in an extracted ion chromatogram obtained by GC-MS, in the mustard seed of Examples and Comparative Examples.

Figure 43 shows the ratio of the peak area of specified constituents to the internal standard (625 μg/g of 4-methylthiazole) in an extracted ion chromatogram obtained by GC-MS, in the mustard seed of Examples and Comparative Examples.

Figure 44 shows the ratio of the peak area of specified constituents to the internal standard (625 μg/g of 4-methylthiazole) in an extracted ion chromatogram obtained by GC-MS, in the mustard seed of Examples and Comparative Examples.

## Description of Embodiments

This specification discloses one or more spices selected from the group consisting of heat-treated cumin, heat-treated paprika, heat-treated asafoetida and heat-treated mustard seed, applications of the aforesaid spices and a method for producing the aforesaid spices.

In this specification, one or more aspects of the present invention in which the aforesaid spice includes heat-treated paprika will be explained as a first aspect of the present invention.

In this specification, one or more aspects of the present invention in which the aforesaid spice includes heat-treated cumin will be explained as a second aspect of the present invention.

In this specification, one or more aspects of the present invention in which the aforesaid spice includes heat-treated asafoetida will be explained as a third aspect of the present invention.

In this specification, one or more aspects of the present invention in which the aforesaid spice includes heat-treated mustard seed will be explained as a fourth aspect of the present invention.

### <Flavor-enhancement>

In this specification, "flavor" refers to a flavor possessed by food products, and for example, it can be one or more selected from saltiness, sweetness, sourness, bitterness, umami, richness, sweet umami, spiciness, astringency, flavor spreading and roasted flavor. In addition, the "enhancement" of flavor refers to enhancing a flavor of a food product perceived when the food product is consumed and, for example, it refers to enhancing a weakened flavor of a food product including the flavor constituent in a reduced amount from usual (e.g., reduced-sodium food product) perceived when the food product is consumed.

In this specification, the flavor perceived when a food product is consumed can be divided into three stages: "top" perceived first, "middle" perceived second and "last" perceived last. The enhancement of flavor in this specification refers to enhancing at least one of these flavors.

Saltiness among flavors is a flavor perceived when sodium chloride is consumed. Saltiness encompasses the flavor of sodium chloride itself and a flavor perceived when sodium chloride is combined with a foodstuff other than table salt. For example, the top flavor of food products including sodium chloride is exemplified by "sharp saltiness (shiokado)", which is a sharp taste of table salt, the middle flavor is exemplified by "full-bodied taste" and "savoriness", and the last flavor is exemplified by "metal-like complexity" and "aftertaste." Saltiness may also include a taste owing to "boosting of flavor", in which the flavor of foodstuffs other than sodium chloride is enhanced by table salt. The enhancement of saltiness herein refers to enhancing at least one of these types of saltiness.

Richness among flavors can be expressed as "thickness" or "full-body" of taste perceived when a food product is consumed. Therefore, "richness-enhancement" can also be expressed as "thickness-impartation" or "full-body impartation".

In this specification, the enhancement of flavor is preferably the enhancement of a flavor derived from one or more flavor constituents selected from table salt, sucrose, citric acid, tartaric acid, naringin, glutamic acid or its salts, aspartic acid or its salts, succinic acid or its salts, inosinic acid or its salts, guanylic acid or its salts, glycine or its salts, alanine or its salts, chili pepper, black pepper, animal- or plant-derived extracts and seasonings. Salts in the aforesaid one or more of flavor constituents are exemplified by respective sodium salts. The aforesaid animal- or plant-derived extracts are exemplified by one or more extracts selected from beef extract, chicken extract, pork extract, seafood extract, garlic extract and onion extract. The aforesaid seasonings are exemplified by one or more selected from tomato paste, banana paste, apple paste, honey, soy sauce, miso, ketchup, Worcestershire sauce, mayonnaise, cheese, noodle soup base, nonfat soybean, nonfat milk powder, yeast extract, protein hydrolysis products and curry powder. The enhancement of flavor herein refers more preferably to the enhancement of a flavor derived from the aforesaid one or more flavor constituents in food products including the aforesaid one or more flavor constituents described-above, especially in food products including the aforesaid one or more flavor constituents described-above in a smaller amount than usual.

### <1. First Aspect of Present Invention>

This chapter explains a preferred embodiment of the first aspect of the present invention.

<Flavor-enhancing Composition>

A first embodiment of the first aspect of the present invention relates to a flavor-enhancing composition containing heat-treated paprika.

The flavor-enhancing composition according to this embodiment can enhance a flavor of a food product by blending the composition with the aforesaid food product. For example, a food product which includes the one or more flavor constituents as described above in a reduced amount from usual (e.g., a reduced-sodium food product including a reduced amount of sodium chloride from usual) and the flavor-enhancing composition according to this embodiment blended therewith can have, in comparison with the food product without the flavor-enhancing composition, a flavor close to that of a food product which includes the one or more flavor constituents in their usual amount, and more preferably, can have a flavor comparable to that of the food product including the one or more of flavor constituents in their usual amount. The flavor-enhancing composition according to this embodiment is more preferably a saltiness-enhancing composition.

In this specification, "paprika" refers to a spice (paprika powder) made by drying and powdering the rind and pulp of the fruit of paprika (*Capsicum annuum var. grossum*), a perennial plant of the nightshade family. Hereinafter, "paprika" refers to paprika powder that can be used as a spice, unless otherwise clearly stated.

The heat-treatment for producing heat-treated paprika is preferably a heat-treatment that comprises heating paprika without the addition of oil or fat (hereinafter it may be referred to as "first heat-treatment") or a heat-treatment that comprises heating paprika together with oil or fat (hereinafter it may be referred to as "second heat-treatment").

A preferred embodiment of the aforesaid first heat-treatment will be explained below.

The aforesaid first heat-treatment can comprise heating paprika only or a mixture of paprika and water. Heat-treated paprika from the mixture of paprika and water is preferred because of its particularly strong flavor-enhancing effect. When the mixture of paprika and water is heat-treated, the mixing ratio of paprika and water is not particularly limited, but preferably the aforesaid mixing ratio is set so that the water activity of the paprika after heating is within the range described below.

For the aforesaid first heat-treatment, its temperature and time period are preferably set so that a heating value may be preferably 15 or more, more preferably 50 or more, even more preferably 130 or more, even more preferably 190 or more, most preferably 200 or more, and furthermore, the heating value may be preferably 15 or more and 1000 or less, more preferably 50 or more and 600 or less, even more preferably 130 or more and 600 or less, even more preferably 190 or more and 600 or less, even more preferably 200 or more and 600 or less, even more preferably 200 or more and 270 or less. Paprika heat-treated without the addition of oil or fat under a condition whereby the heating value is within the aforesaid range is preferred because of its particularly strong flavor-enhancing effect. The heating condition whereby the heating value is within the aforesaid range can preferably comprise heating under the temperature and time period condition described below.

The heating value is given by the integral of the value expressed by the formula (hereinafter "CV value"),

$$\text{(Formula): CV value} = 10^{[(\text{product temperature - reference temperature}) / \text{Z value}]},$$

with respect to the heating time (min).

In this specification, "reference temperature" is 110 °C and "Z value" is 30 °C.

The aforesaid first heat-treatment to obtain the aforesaid heat-treated paprika can be carried out at a temperature of, for example, 110 °C or higher, preferably 120 °C or higher, more preferably 125 °C or higher, especially preferably 128 °C or higher, and for example, 110 °C or higher and 180 °C or lower, preferably 120 °C or higher and 160 °C or lower, more preferably 125 °C or higher and 150 °C or lower, especially preferably 128 °C or higher and 150 °C or lower. The aforesaid first heat-treatment can be carried out so that the heating time at a temperature within the aforesaid temperature range may be, for example, 20 minutes or longer, preferably 30 minutes or longer, more preferably 45 minutes or longer, especially preferably 55 minutes or longer, and for example 20 minutes or longer and 120 minutes or shorter, preferably 30 minutes or longer and 90 minutes or shorter, more preferably 45 minutes or longer and 75 minutes or shorter, most preferably 55 minutes or longer and 75 minutes or shorter. The heat-treated paprika obtained by the first heat-treatment preferably at a temperature of 120 °C or higher, more preferably 125 °C or higher, for 45 minutes or longer and 120 minutes or shorter, is preferred because of its particularly strong flavor-enhancing effect.

The aforesaid first heat-treatment can be carried out under a pressure condition in which gauge pressure is preferably 0.05 MPa or higher, more preferably 0.2 MPa or higher. The upper limit of the pressure during heating is not particularly limited, but a gauge pressure of 0.6 MPa or lower is preferred from the viewpoint of equipment.

Various types of means such as pressure-tight heating, superheated steam heating, oven heating, etc. can be used for the aforesaid first heat-treatment, but pressure-tight heating is preferably used. Heating equipment used for pressure-tight heating can be exemplified by pressure-tight kettles and retort sterilizers. When heating other than pressure-tight heating is utilized, ovens, flat-bottom roasting machines, vertical heating mixers, microwave heating equipment, superheated steam eddy current mixers, superheated steam sterilizers, etc. can be used appropriately.

Next, a preferred embodiment of the aforesaid second heat-treatment will be explained.

In the aforesaid second heat-treatment, paprika is heated together with oil or fat. The heat-treated paprika obtained by the aforesaid second heat-treatment is preferred because of its particularly strong flavor-enhancing effect. The amount of oil or fat used in the aforesaid second heat-treatment is not particularly limited, but for example, 10 parts by mass or more and 500 parts by mass or less, preferably 50 parts by mass or more and 200 parts by mass or less, of oil or fat can be used with respect to 100 parts by mass of paprika. The aforesaid oil or fat is not limited so long as it is edible oil or fat derived from plants, animals, etc. acceptable as food products. The oil or fat may be an oil or fat that has a melting point adjusted by a technique such as esterification or hydrogenation for fatty acids.

For the aforesaid second heat-treatment, its temperature and time period are preferably set so that the heating value may be preferably 0.3 or more, more preferably 2.5 or more, even more preferably 30 or more. The heating value in the aforesaid second heat-treatment is exemplified by, for example, 0.3 or more and 6,000 or less, preferably 2.5 or more and 450 or less, more preferably 30 or more and 110 or less. Paprika that has been subjected to the aforesaid second heat-treatment under these conditions is preferred because of its particularly strong flavor-enhancing effect. The heating condition whereby the heating value is within the aforesaid range can preferably comprise heating under the temperature and time period condition described below. The definition of the heating value is as described above with regard to the aforesaid first heat-treatment.

In the aforesaid second heat-treatment, the mixture of paprika and oil or fat can be heated at a temperature of, for example, 90 °C or higher, preferably 100 °C or higher, more preferably 110 °C or higher, more preferably 120 °C or higher, especially preferably 125 °C or higher, and for example 90 °C or higher and 190 °C or lower, preferably 100 °C or higher and 190 °C or lower, more preferably 110 °C or higher and 190 °C or lower, more preferably 120 °C or higher and 180 °C or lower, especially preferably 125 °C or higher and 180 °C or lower. The aforesaid second heat-treatment can be carried out so that the heating time at a temperature within the aforesaid temperature range may be, for example, 1 minute or longer, preferably 2 minutes or longer, preferably 3 minutes or longer, and for example, 1 minute or longer and 10 minutes or shorter, preferably 2 minutes or longer and 7 minutes or shorter, more preferably 3 minutes or longer and 7 minutes or shorter. Heat-treated paprika obtained by the aforesaid second heat-treatment under these conditions is preferred because of its particularly strong flavor-enhancing effect.

The aforesaid second heat-treatment can be carried out in an either open or closed system.

The aforesaid second heat-treatment can be carried out by heating with superheated steam or by heating with an oven. Heating equipment used for the aforesaid second heat-treatment can be exemplified by ovens, flat-bottom roasting machines, vertical heating mixers, microwave heating equipment, etc.

Next, a more preferred embodiment of the aforesaid heat-treated paprika will be explained.

The aforesaid heat-treated paprika may be further subjected to a maturing treatment after the heat-treatment. The aforesaid maturing treatment is a storage treatment at a temperature higher than room temperature under an air atmosphere, and can be a storage treatment, for example, in an airtight container filled with air, for example, at 30 °C to 50 °C, preferably 35 °C to 45 °C, for example, for 5 days to 15 days, preferably 7 days to 10 days.

The aforesaid heat-treated paprika preferably has a water activity measured at 25 °C of 0.85 or less, more preferably 0.81 or less, especially preferably 0.80 or less, and the lower limit is not particularly limited, but is, for example, 0.50 or more. The aforesaid heat-treated paprika can be prepared by heat-treating paprika under a condition whereby the water activity is within this range after heating.

The aforesaid heat-treated paprika, in a more preferred embodiment, contains an increased amount of one or more selected from cyclic (Phe-Leu/Ile), cyclic (Pro-Asn), cyclic (Leu/Ile-Leu/Ile), cyclic (Thr-Leu/Ile), cyclic (Phe-Ser), cyclic (Gly-His), cyclic (Gly-Ser), cyclic (Gly-Arg), cyclic (Ala-His), cyclic (Glu-His), cyclic (Glu-Arg), cyclic (Ala-Arg), cyclic (Glu-Glu), cyclic (Glu Glu-Gly), cyclic (Glu-Asp), cyclic (Pro-His), cyclic (Glu-Tyr), cyclic (Leu/Ile-Asp), cyclic (Pro-Asp), cyclic (Val-Arg), cyclic (Thr-Pro), cyclic (hyPro-Pro), cyclic (Gly-Val), cyclic (Ala-Pro), cyclic (Ala-Tyr), cyclic (Arg-Leu/Ile), cyclic (Leu/Ile-Ser), cyclic (Ala-Val), cyclic (Pro-Pro), cyclic (Gly-Leu/Ile), cyclic (Gly-Phe), cyclic (Pro-Tyr), cyclic (Phe-Asp), cyclic (Pro-Val), cyclic (Ala-Leu/Ile), cyclic (Val-Tyr), cyclic (Tyr-Ser), cyclic (Phe-Ala), cyclic (Val-Val), cyclic (Pro-Leu/Ile), cyclic (Phe-Tyr), cyclic (Phe-Thr), cyclic (Phe-Pro), cyclic (Leu/Ile-Val), cyclic (Val-Phe), cyclic (Tyr-Asp), cyclic (Ala-Asp), cyclic (Val-Ser), sulfurol(4-methyl-5-thiazoleethanol), sulfurol acetate, tartaric acid and *trans*-aconitic acid in comparison with the paprika before the heat-treatment. The present inventors have found that the amount of the aforesaid compounds included in the aforesaid heat-treated paprika correlates to the strength of the flavor-enhancing effect.

That is, in a more preferred embodiment, when the aforesaid heat-treated paprika, with the addition of 47 μg/g of L-lysine-$^{13}C_6$ monohydrochloride, is analyzed by liquid chromatography-mass spectrometry (LC-MS/MS) according to the method described below, the aforesaid heat-treated paprika satisfies one or more, more preferably ten or more, even more preferably twenty or more, especially preferably thirty or more, especially preferably forty or more, and most preferably all, of the following:

(1) a peak area of 0.45 or more, preferably 0.50 or more, and more preferably 0.45 or more and 5.50 or less, and even more preferably 0.50 or more and 5.50 or less derived from cyclic (Phe-Leu/Ile),
(2) a peak area of 0.020 or more, preferably 0.030 or more, and more preferably 0.020 or more and 0.35 or less, even more preferably 0.030 or more and 0.35 or less derived from cyclic (Pro-Asn),
(3) a peak area of 0.30 or more, preferably 0.35 or more, and more preferably 0.30 or more and 4.0 or less, even more preferably 0.35 or more and 4.0 or less derived from cyclic (Leu/Ile-Leu/Ile),
(4) a peak area of 0.014 or more, and more preferably 0.014 or more and 0.14 or less derived from cyclic (Thr-Leu/Ile),
(5) a peak area of 0.025 or more, preferably 0.030 or more, and more preferably 0.025 or more and 0.35 or less, even more preferably 0.030 or more and 0.35 or less derived from cyclic (Phe-Ser),
(6) a peak area of 0.0020 or more, and more preferably 0.0020 or more and 0.050 or less derived from cyclic (Gly-His),
(7) a peak area of 0.0010 or more, preferably 0.0030 or more, and more preferably 0.0010 or more and 0.100 or less, even more preferably 0.0030 or more and 0.050 or less derived from cyclic (Gly-Ser),
(8) a peak area of 0.080 or more, and more preferably 0.080 or more and 1.20 or less derived from cyclic (Gly-Arg),
(9) a peak area of 0.0140 or more, and more preferably 0.0140 or more and 0.20 or less derived from cyclic (Ala-His),
(10) a peak area of 0.040 or more, and more preferably 0.040 or more and 0.50 or less derived from cyclic (Glu-His),
(11) a peak area of 0.21 or more, and more preferably 0.21 or more and 2.5 or less derived from cyclic (Glu-Arg),
(12) a peak area of 0.24 or more, preferably 0.40 or more, and more preferably 0.24 or more and 6.0 or less derived from cyclic (Ala-Arg),
(13) a peak area of 0.020 or more, and more preferably 0.020 or more and 0.25 or less derived from cyclic (Glu-Glu),
(14) a peak area of 0.39 or more, preferably 0.40 or more, and more preferably 0.39 or more and 4.5 or less, even more preferably 0.40 or more and 4.5 or less derived from cyclic (Glu-Gly),
(15) a peak area of 0.19 or more, preferably 0.40 or more, and more preferably 0.19 or more and 6.0 or less, even more preferably 0.40 or more and 6.0 or less derived from cyclic (Glu-Asp),
(16) a peak area of 0.12 or more, preferably 0.15 or more, and more preferably 0.12 or more and 1.9 or less, even more preferably 0.15 or more and 1.9 or less derived from cyclic (Pro-His),
(17) a peak area of 0.40 or more, preferably 0.60 or more, and more preferably 0.40 or more and 9.0 or less, even more preferably 0.60 or more and 9.0 or less derived from cyclic (Glu-Tyr),
(18) a peak area of 0.100 or more, and more preferably 0.100 or more and 1.50 or less, even more preferably 0.100 or more and 0.30 or less derived from cyclic (Leu/Ile-Asp),
(19) a peak area of 0.10 or more, preferably 0.12 or more, and more preferably 0.10 or more and 1.7 or less, even more preferably 0.12 or more and 1.7 or less derived from cyclic (Pro-Asp),
(20) a peak area of 0.15 or more, preferably 0.25 or more, and more preferably 0.15 or more and 6.0 or less, even more preferably 0.25 or more and 6.0 or less derived from cyclic (Val-Arg),
(21) a peak area of 0.40 or more, and more preferably 0.40 or more and 6.0 or less derived from cyclic (Thr-Pro),
(22) a peak area of 0.020 or more, and more preferably 0.020 or more and 0.50 or less, more preferably 0.020 or more and 0.30 or less derived from cyclic (HyPro-Pro),
(23) a peak area of 0.010 or more, preferably 0.020 or more, and more preferably 0.010 or more and 0.40 or less, even more preferably 0.020 or more and 0.40 or less derived from cyclic (Gly-Val),
(24) a peak area of 0.10 or more, preferably 0.15 or more, and more preferably 0.10 or more and 2.0 or less, even more preferably 0.15 or more and 2.0 or less derived from cyclic (Ala-Pro),
(25) a peak area of 0.025 or more, preferably 0.030 or more, and more preferably 0.025 or more and 0.40 or less, even more preferably 0.030 or more and 0.40 or less derived from cyclic (Ala-Tyr),
(26) a peak area of 0.60 or more, and more preferably 0.60 or more and 8.0 or less derived from cyclic (Arg-Leu/Ile),
(27) a peak area of 0.050 or more, preferably 0.070 or more, and more preferably 0.050 or more and 1.20 or less, even more preferably 0.070 or more and 1.20 or less derived from cyclic (Leu/Ile-Ser),
(28) a peak area of 0.040 or more, preferably 0.050 or more, and more preferably 0.040 or more and 0.60 or less, even more preferably 0.050 or more and 0.60 or less derived from cyclic (Ala-Val),
(29) a peak area of 0.30 or more, preferably 0.40 or more, and more preferably 0.30 or more and 5.0 or less, even more preferably 0.40 or more and 5.0 or less derived from cyclic (Pro-Pro),
(30) a peak area of 0.025 or more, preferably 0.035 or more, and more preferably 0.025 or more and 0.50 or less, even more preferably 0.035 or more and 0.50 or less derived from cyclic (Gly-Leu/Ile),
(31) a peak area of 0.060 or more, and more preferably 0.060 or more and 0.80 or less derived from cyclic (Gly-Phe),
(32) a peak area of 0.060 or more, preferably 0.080 or more, and more preferably 0.060 or more and 1.0 or less, even more preferably 0.080 or more and 1.0 or less derived from cyclic (Pro-Tyr),
(33) a peak area of 0.040 or more, preferably 0.050 or more, and more preferably 0.040 or more and 1.0 or less, even more preferably 0.050 or more and 0.30 or less derived from cyclic (Phe-Asp),
(34) a peak area of 0.070 or more, and more preferably 0.070 or more and 0.80 or less derived from cyclic (Pro-Val),
(35) a peak area of 0.080 or more, preferably 0.10 or more, and more preferably 0.080 or more and 1.20 or less, even more preferably 0.10 or more and 1.20 or less derived from cyclic (Ala-Leu/Ile),

(36) a peak area of 0.020 or more, preferably 0.040 or more, and more preferably 0.020 or more and 0.60 or less, even more preferably 0.040 or more and 0.60 or less derived from cyclic (Val-Tyr),
(37) a peak area of 0.20 or more, preferably 0.30 or more, and more preferably 0.20 or more and 4.0 or less, even more preferably 0.30 or more and 4.0 or less derived from cyclic (Tyr-Ser),
(38) a peak area of 0.025 or more, preferably 0.030 or more, and more preferably 0.025 or more and 0.40 or less, even more preferably 0.030 or more and 0.40 or less derived from cyclic (Phe-Ala),
(39) a peak area of 0.50 or more, preferably 0.080 or more, and more preferably 0.50 or more and 0.90 or less, even more preferably 0.080 or more and 0.90 or less derived from cyclic (Val-Val),
(40) a peak area of 0.45 or more, preferably 0.50 or more, and more preferably 0.45 or more and 6.0 or less, even more preferably 0.50 or more and 6.0 or less derived from cyclic (Pro-Leu/Ile),
(41) a peak area of 0.160 or more, and more preferably 0.160 or more and 5.0 or less, even more preferably 0.160 or more and 1.70 or less derived from cyclic (Phe-Tyr),
(42) a peak area of 0.020 or more, and more preferably 0.020 or more and 0.60 or less derived from cyclic (Phe-Thr),
(43) a peak area of 0.45 or more, preferably 0.50 or more, and more preferably 0.45 or more and 5.5 or less, even more preferably 0.50 or more and 5.5 or less derived from cyclic (Phe-Pro),
(44) a peak area of 0.09 or more, preferably 0.10 or more, and more preferably 0.09 or more and 1.5 or less, even more preferably 0.10 or more and 1.5 or less derived from cyclic (Leu/Ile-Val),
(45) a peak area of 0.010 or more, preferably 0.0130 or more, and more preferably 0.010 or more and 0.20 or less, even more preferably 0.0130 or more and 0.20 or less derived from cyclic (Val-Phe),
(46) a peak area of 0.020 or more, and more preferably 0.020 or more and 0.50 or less derived from cyclic (Tyr-Asp),
(47) a peak area of 0.005 or more, and more preferably 0.005 or more and 0.310 or less derived from cyclic (Ala-Asp),
(48) a peak area of 0.070 or more, preferably 0.080 or more, and more preferably 0.070 or more and 2.0 or less, even more preferably 0.080 or more and 2.0 or less derived from cyclic (Val-Ser),
(49) a peak area of 0.090 or more, and more preferably 0.090 or more and 1.10 or less derived from sulfurol(4-methyl-5-thiazoleethanol) and
(50) a peak area of 0.011 or more, preferably 0.020 or more, and more preferably 0.011 or more and 0.25 or less, even more preferably 0.020 or more and 0.25 or less derived from sulfurol acetate,

as a ratio to a peak area derived from L-lysine-$^{13}C_6$ monohydrochloride in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated paprika (representing a mass converted to that of the paprika excluding oil or fat when the aforesaid heat-treated paprika is paprika heat-treated together with the oil or fat) and 5 mL of water is kept heated in a thermostatic water bath at 80 °C for 30 minutes, to prepare a water extract. To the aforesaid water extract in the aforesaid test tube are added 5 mL of acetonitrile and L-lysine-$^{13}C_6$ monohydrochloride in an amount of 47 μg/g with respect to the aforesaid heat-treated paprika (representing a mass converted to that of the paprika excluding oil or fat when the aforesaid heat-treated paprika is paprika heat-treated together with the oil or fat), followed by stirring, and then a solid portion is removed and a liquid portion is collected, to prepare a specimen sample. The aforesaid specimen sample is analyzed by LC-MS/MS, to obtain a chromatogram. In this context, the aforesaid heat-treated paprika used as the analysis sample is preferably a pulverized product.

In respective disclosures herein with regard to the first, second, third and fourth aspects of the present invention, cyclic (Phe-Phe), etc. represent a cyclic dipeptide consisting of two amino acids. The expression "Leu/Ile" represents one or both of leucine (Leu) and isoleucine (Ile), and for example, "peak area derived from cyclic (Phe-Leu/Ile)" in aforesaid (1) refers to the total of the peak area derived from cyclic (Phe-Leu) and that from cyclic (Phe-Ile). In respective disclosures herein with regard to the first, second, third and fourth aspects of the present invention, "hyPro" refers to γ-hydroxy-proline. In respective disclosures herein with regard to the first, second, third and fourth aspects of the present invention, any amino acid constituting the cyclic dipeptide may be an L-enantiomer, a D-enantiomer, or a mixture of L- and D-enantiomers.

The peak area in an extracted ion chromatogram for respective product ions with the m/z value described in Examples can be used as the peak area derived from L-lysine-$^{13}C_6$ monohydrochloride and the peak area of the respective compounds in aforesaid (1) to (50).

In a particularly preferred embodiment of the aforesaid heat-treated paprika obtained by the aforesaid first heat-treatment (heat-treatment carried out without the addition of oil or fat), the heat-treated paprika satisfies one or more, more preferably ten or more, even more preferably twenty or more, especially preferably thirty or more, especially preferably forty or more, and most preferably all, of the following:

(1') a peak area of 0.55 or more, and more preferably 0.55 or more and 5.50 or less derived from cyclic (Phe-Leu/Ile),
(2') a peak area of 0.035 or more, and more preferably 0.035 or more and 0.35 or less derived from cyclic (Pro-Asn),
(3') a peak area of 0.40 or more, and more preferably 0.40 or more and 4.0 or less derived from cyclic (Leu/Ile-Leu/Ile),

(4') a peak area of 0.014 or more, and more preferably 0.014 or more and 0.14 or less derived from cyclic (Thr-Leu/Ile),
(5') a peak area of 0.035 or more, and more preferably 0.035 or more and 0.35 or less derived from cyclic (Phe-Ser),
(6') a peak area of 0.0050 or more, and more preferably 0.0050 or more and 0.050 or less derived from cyclic (Gly-His),
(7') a peak area of 0.0050 or more, and more preferably 0.0050 or more and 0.100 or less derived from cyclic (Gly-Ser),
(8') a peak area of 0.120 or more, and preferably 0.120 or more and 1.20 or less derived from cyclic (Gly-Arg),
(9') a peak area of 0.020 or more, and more preferably 0.020 or more and 0.20 or less derived from cyclic (Ala-His),
(10') a peak area of 0.050 or more, and more preferably 0.050 or more and 0.50 or less derived from cyclic (Glu-His),
(11') a peak area of 0.25 or more, and more preferably 0.25 or more and 2.5 or less derived from cyclic (Glu-Arg),
(12') a peak area of 0.60 or more, and more preferably 0.60 or more and 6.0 or less derived from cyclic (Ala-Arg),
(13') a peak area of 0.025 or more, and more preferably 0.025 or more and 0.25 or less derived from cyclic (Glu-Glu),
(14') a peak area of 0.45 or more, and more preferably 0.45 or more and 4.5 or less derived from cyclic (Glu-Gly),
(15') a peak area of 0.60 or more, and more preferably 0.60 or more and 6.0 or less derived from cyclic (Glu-Asp),
(16') a peak area of 0.19 or more, and more preferably 0.19 or more and 1.9 or less derived from cyclic (Pro-His),
(17') a peak area of 0.90 or more, and more preferably 0.90 or more and 9.0 or less derived from cyclic (Glu-Tyr),
(18') a peak area of 0.150 or more, and more preferably 0.150 or more and 1.50 or less derived from cyclic (Leu/Ile-Asp),
(19') a peak area of 0.17 or more, and more preferably 0.17 or more and 1.7 or less derived from cyclic (Pro-Asp),
(20') a peak area of 0.30 or more, preferably 0.60 or more, and more preferably 0.30 or more and 6.0 or less, even more preferably 0.60 or more and 6.0 or less derived from cyclic (Val-Arg),
(21') a peak area of 0.50 or more, more preferably 0.60 or more, and more preferably 0.50 or more and 6.0 or less, even more preferably 0.60 or more and 6.0 or less derived from cyclic (Thr-Pro),
(22') a peak area of 0.030 or more, and more preferably 0.030 or more and 0.30 or less derived from cyclic (hyPro-Pro),
(23') a peak area of 0.040 or more, and more preferably 0.040 or more and 0.40 or less derived from cyclic (Gly-Val),
(24') a peak area of 0.20 or more, and more preferably 0.20 or more and 2.0 or less derived from cyclic (Ala-Pro),
(25') a peak area of 0.040 or more, and more preferably 0.040 or more and 0.40 or less derived from cyclic (Ala-Tyr),
(26') a peak area of 0.80 or more, and more preferably 0.80 or more and 8.0 or less derived from cyclic (Arg-Leu/Ile),
(27') a peak area of 0.120 or more, and more preferably 0.120 or more and 1.20 or less derived from cyclic (Leu/Ile-Ser),
(28') a peak area of 0.060 or more, and more preferably 0.060 or more and 0.60 or less derived from cyclic (Ala-Val),
(29') a peak area of 0.50 or more, and more preferably 0.50 or more and 5.0 or less derived from cyclic (Pro-Pro),
(30') a peak area of 0.050 or more, and more preferably 0.050 or more and 0.50 or less derived from cyclic (Gly-Leu/Ile),
(31') a peak area of 0.080 or more, and more preferably 0.080 or more and 0.80 or less derived from cyclic (Gly-Phe),
(32') a peak area of 0.10 or more, and more preferably 0.10 or more and 1.0 or less derived from cyclic (Pro-Tyr),
(33') a peak area of 0.100 or more, and more preferably 0.100 or more and 1.0 or less derived from cyclic (Phe-Asp),
(34') a peak area of 0.080 or more, and more preferably 0.080 or more and 0.80 or less derived from cyclic (Pro-Val),
(35') a peak area of 0.120 or more, and more preferably 0.120 or more and 1.20 or less derived from cyclic (Ala-Leu/Ile),
(36') a peak area of 0.060 or more, and more preferably 0.060 or more and 0.60 or less derived from cyclic (Val-Tyr),
(37') a peak area of 0.40 or more, and more preferably 0.40 or more and 4.0 or less derived from cyclic (Tyr-Ser),
(38') a peak area of 0.040 or more, and more preferably 0.040 or more and 0.40 or less derived from cyclic (Phe-Ala),
(39') a peak area of 0.090 or more, and more preferably 0.090 or more and 0.90 or less derived from cyclic (Val-Val),
(40') a peak area of 0.60 or more, and more preferably 0.60 or more and 6.0 or less derived from cyclic (Pro-Leu/Ile),
(41') a peak area of 0.170 or more, and more preferably 0.170 or more and 1.70 or less derived from cyclic (Phe-Tyr),
(42') a peak area of 0.060 or more, and more preferably 0.060 or more and 0.60 or less derived from cyclic (Phe-Thr),
(43') a peak area of 0.55 or more, and more preferably 0.55 or more and 5.5 or less derived from cyclic (Phe-Pro),
(44') a peak area of 0.15 or more, and more preferably 0.15 or more and 1.5 or less derived from cyclic (Leu/Ile-Val),
(45') a peak area of 0.020 or more, and more preferably 0.020 or more and 0.20 or less derived from cyclic (Val-Phe),
(46') a peak area of 0.050 or more, and more preferably 0.050 or more and 0.50 or less derived from cyclic (Tyr-Asp),
(47') a peak area of 0.014 or more, and more preferably 0.014 or more and 0.310 or less derived from cyclic (Ala-Asp),
(48') a peak area of 0.100 or more, preferably 0.160 or more, and more preferably 0.100 or more and 2.0 or less, even more preferably 0.160 or more and 2.0 or less derived from cyclic (Val-Ser),
(49') a peak area of 0.110 or more, and more preferably 0.110 or more and 1.10 or less derived from sulfurol(4-methyl-5-thiazoleethanol) and
(50') a peak area of 0.025 or more, and more preferably 0.025 or more and 0.25 or less derived from sulfurol acetate,
as a ratio to a peak area derived from L-lysine-$^{13}C_6$ monohydrochloride in the chromatogram described above.

In a particularly preferred embodiment of the aforesaid heat-treated paprika obtained by the aforesaid second heat-treatment (heat-treatment carried out with the addition of oil or fat), the heat-treated paprika satisfies one or more, more preferably ten or more, even more preferably twenty or more, especially preferably thirty or more, especially preferably forty or more, and most preferably all, of the following:

(1") a peak area of 0.45 or more, and more preferably 0.45 or more and 5.50 or less derived from cyclic (Phe-Leu/Ile),
(2") a peak area of 0.035 or more, and more preferably 0.035 or more and 0.35 or less derived from cyclic (Pro-Asn),
(3") a peak area of 0.30 or more, and more preferably 0.30 or more and 4.0 or less derived from cyclic (Leu/Ile-Leu/Ile),
(4") a peak area of 0.014 or more, and more preferably 0.014 or more and 0.14 or less derived from cyclic (Thr-Leu/Ile),
(5") a peak area of 0.025 or more, and more preferably 0.025 or more and 0.35 or less derived from cyclic (Phe-Ser),
(6") a peak area of 0.0050, and more preferably 0.0050 or more and 0.050 or less derived from cyclic (Gly-His),
(7") a peak area of 0.0030 or more, and more preferably 0.0030 or more and 0.100 or less derived from cyclic (Gly-Ser),
(8") a peak area of 0.100 or more, and more preferably 0.100 or more and 1.20 or less derived from cyclic (Gly-Arg),
(9") a peak area of 0.015 or more, and more preferably 0.015 or more and 0.20 or less derived from cyclic (Ala-His),
(10") a peak area of 0.040 or more, and more preferably 0.040 or more and 0.50 or less derived from cyclic (Glu-His),
(11") a peak area of 0.25 or more, and more preferably 0.25 or more and 2.5 or less derived from cyclic (Glu-Arg),
(12") a peak area of 0.30 or more, and more preferably 0.30 or more and 6.0 or less derived from cyclic (Ala-Arg),
(13") a peak area of 0.020 or more, and more preferably 0.020 or more and 0.25 or less derived from cyclic (Glu-Glu),
(14") a peak area of 0.45 or more, and more preferably 0.45 or more and 4.5 or less derived from cyclic (Glu-Gly),
(15") a peak area of 0.30 or more, and more preferably 0.30 or more and 6.0 or less derived from cyclic (Glu-Asp),
(16") a peak area of 0.13 or more, and more preferably 0.13 or more and 1.9 or less derived from cyclic (Pro-His),
(17") a peak area of 0.70 or more, and more preferably 0.70 or more and 9.0 or less derived from cyclic (Glu-Tyr),
(18") a peak area of 0.120 or more, and more preferably 0.120 or more and 1.50 or less derived from cyclic (Leu/Ile-Asp),
(19") a peak area of 0.12 or more, and more preferably 0.12 or more and 1.7 or less derived from cyclic (Pro-Asp),
(20") a peak area of 0.15 or more, and more preferably 0.15 or more and 6.0 or less derived from cyclic (Val-Arg),
(21") a peak area of 0.60 or more, and more preferably 0.60 or more and 6.0 or less derived from cyclic (Thr-Pro),
(22") a peak area of 0.050 or more, and more preferably 0.050 or more and 0.50 or less derived from cyclic (hyPro-Pro),
(23") a peak area of 0.020 or more, and more preferably 0.020 or more and 0.40 or less derived from cyclic (Gly-Val),
(24") a peak area of 0.20 or more, and more preferably 0.20 or more and 2.0 or less derived from cyclic (Ala-Pro),
(25") a peak area of 0.030 or more, and more preferably 0.030 or more and 0.40 or less derived from cyclic (Ala-Tyr),
(26") a peak area of 0.70 or more, and more preferably 0.70 or more and 8.0 or less derived from cyclic (Arg-Leu/Ile),
(27") a peak area of 0.050 or more, and more preferably 0.050 or more and 1.20 or less derived from cyclic (Leu/Ile-Ser),
(28") a peak area of 0.050 or more, and more preferably 0.050 or more and 0.60 or less derived from cyclic (Ala-Val),
(29") a peak area of 0.40 or more, and more preferably 0.40 or more and 5.0 or less derived from cyclic (Pro-Pro),
(30") a peak area of 0.050 or more, and more preferably 0.050 or more and 0.50 or less derived from cyclic (Gly-Leu/Ile),
(31") a peak area of 0.070 or more, and more preferably 0.070 or more and 0.80 or less derived from cyclic (Gly-Phe),
(32") a peak area of 0.10 or more, and more preferably 0.10 or more and 1.0 or less derived from cyclic (Pro-Tyr),
(33") a peak area of 0.100 or more, and more preferably 0.100 or more and 1.0 or less derived from cyclic (Phe-Asp),
(34") a peak area of 0.080 or more, and more preferably 0.080 or more and 0.80 or less derived from cyclic (Pro-Val),
(35") a peak area of 0.100 or more, and more preferably 0.100 or more and 1.20 or less derived from cyclic (Ala-Leu/Ile),
(36") a peak area of 0.040 or more, and more preferably 0.040 or more and 0.60 or less derived from cyclic (Val-Tyr),
(37") a peak area of 0.30 or more, and more preferably 0.30 or more and 4.0 or less derived from cyclic (Tyr-Ser),
(38") a peak area of 0.025 or more, and more preferably 0.025 or more and 0.40 or less derived from cyclic (Phe-Ala),
(39") a peak area of 0.080 or more, and more preferably 0.080 or more and 0.90 or less derived from cyclic (Val-Val),
(40") a peak area of 0.80 or more, and more preferably 0.80 or more and 6.0 or less derived from cyclic (Pro-Leu/Ile),
(41") a peak area of 0.160 or more, and more preferably 0.160 or more and 1.70 or less derived from cyclic (Phe-Tyr),
(42") a peak area of 0.10 or more, and more preferably 0.10 or more and 0.60 or less derived from cyclic (Phe-Thr),
(43") a peak area of 0.80 or more, and more preferably 0.80 or more and 5.5 or less derived from cyclic (Phe-Pro),
(44") a peak area of 0.15 or more, and more preferably 0.15 or more and 1.5 or less derived from cyclic (Leu/Ile-Val),
(45") a peak area of 0.010 or more, and more preferably 0.010 or more and 0.20 or less derived from cyclic (Val-Phe),
(46") a peak area of 0.060 or more, and more preferably 0.060 or more and 0.50 or less derived from cyclic (Tyr-Asp),
(47") a peak area of 0.015 or more, and more preferably 0.015 or more and 0.310 or less derived from cyclic (Ala-Asp),
(48") a peak area of 0.070 or more, and more preferably 0.070 or more and 2.0 or less derived from cyclic (Val-Ser),

(49") a peak area of 0.150 or more, and more preferably 0.150 or more and 1.10 or less derived from sulfurol(4-methyl-5-thiazoleethanol) and

(50") a peak area of 0.025 or more, and more preferably 0.025 or more and 0.25 or less derived from sulfurol acetate, as a ratio to a peak area derived from L-lysine-$^{13}C_6$ monohydrochloride in the chromatogram described above.

In addition, the aforesaid heat-treated paprika, in a more preferred embodiment, is characterized in that it contains one or more selected from the group consisting of cyclic (Gly-His), cyclic (Gly-Ser), cyclic (hyPro-Pro), cyclic (Gly-Val), cyclic (Tyr-Asp) and cyclic (Ala-Asp), and more preferably all members of the aforesaid group. These cyclic dipeptides are characteristic constituents of heat-treated paprika having the flavor-enhancing effect, which are not detected in unheated paprika.

In a more preferred embodiment, when the aforesaid heat-treated paprika, with the addition of 99 μg/g of ribitol, is analyzed by liquid chromatography-mass spectrometry (LC-MS/MS) according to the method described below, the aforesaid heat-treated paprika satisfies one or more, more preferably both, of the following:

(51) a peak area of 0.60 or more, and preferably 0.60 or more and 10.0 or less, even more preferably 0.60 or more and 7.0 or less derived from tartaric acid and

(52) a peak area of 0.35 or more, and more preferably 0.35 or more and 10.0 or less, even more preferably 0.35 or more and 4.0 or less derived from *trans-aconitic* acid,

as a ratio to a peak area derived from ribitol in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated paprika (representing a mass converted to that of the paprika excluding oil and fat when the aforesaid heat-treated paprika is paprika heat-treated together with the oil and fat) and 5 mL of water is kept heated in a thermostatic water bath at 80 °C for 30 minutes, to prepare a water extract. To the aforesaid water extract in the aforesaid test tube are added 5 mL of acetonitrile and ribitol in an amount of 99 μg/g with respect to the aforesaid heat-treated paprika (representing a mass converted to that of the paprika excluding oil or fat when the aforesaid heat-treated paprika is paprika heat-treated together with the oil or fat), followed by stirring, and then a solid portion is removed and a liquid portion is collected, to prepare a specimen sample. The aforesaid specimen sample is analyzed by LC-MS/MS to obtain a chromatogram. In this context, the aforesaid heat-treated paprika used as the analysis sample is preferably a pulverized product.

The peak area in an extracted ion chromatogram for respective product ions with an m/z value described in Examples can be used as the peak area derived from ribitol, the peak area derived from tartaric acid and the peak area derived from *trans*-aconitic acid.

In a particularly preferred embodiment of the aforesaid heat-treated paprika obtained by the aforesaid first heat-treatment (heat-treatment carried out without the addition of oil or fat), the heat-treated paprika satisfies one or more, more preferably both, of the following:

(51') a peak area of 0.70 or more, and more preferably 0.70 or more and 7.0 or less derived from tartaric acid and

(52') a peak area of 0.40 or more, and more preferably 0.40 or more and 4.0 or less derived from *trans-aconitic* acid,

as a ratio to a peak area derived from ribitol in the chromatogram described above.

In a particularly preferred embodiment of the aforesaid heat-treated paprika obtained by the aforesaid second heat-treatment (heat-treatment carried out with the addition of oil or fat), the heat-treated paprika satisfies one or more, more preferably both, of the following:

(51') a peak area of 1.50 or more, and more preferably 1.50 or more and 10.0 or less derived from tartaric acid and

(52') a peak area of 1.00 or more, and more preferably 1.00 or more and 10.0 or less derived from *trans*-aconitic acid,

as a ratio to a peak area derived from ribitol in the chromatogram described above.

The heat-treated paprika in the flavor-enhancing composition according to this embodiment is preferably in the form of powder, and the particle size is not particularly limited, but can be, for example, 1000 μm or less, preferably 500 μm or less. The particle size can be determined by the opening of a standard sieve as specified in JIS. The heat-treated paprika in the flavor-enhancing composition according to this embodiment may also be provided in the form of a mixture of the heat-treated paprika and oil or fat. The aforesaid mixture may be solid at room temperature or liquid at room temperature, depending on the melting point of the aforesaid oil or fat.

The flavor-enhancing composition according to this embodiment may consist only of the heat-treated paprika, or may include the heat-treated paprika and an additional material. The additional material may be exemplified by one or more materials having the flavor-enhancing effect and one or more materials acceptable as food products. The flavor-enhancing composition according to this embodiment can contain the heat-treated paprika at a percentage of preferably 30% by mass or more and 100% by mass or less, more preferably 50% by mass or more and 100% by mass or less, even more preferably 80% by mass or more 100% by mass or less, and most preferably 95% by mass or more and 100% by mass or less. The flavor-enhancing composition according to this embodiment can be in the form of powder, granule, paste, liquid, etc., and can include one or more materials acceptable as food products, such as excipients, carriers, etc., as necessary, in order to achieve a desired form of the composition.

<Method for Producing Flavor-enhancing Composition>

A first example of the second embodiment of the first aspect of the present invention relates to

a method for producing the flavor-enhancing composition according to the first embodiment of the first aspect of the present invention, comprising
heating paprika without the addition of oil or fat under a condition whereby the heating value is 50 or more to obtain the aforesaid heat-treated paprika.

According to the aforesaid first example of this embodiment, the flavor-enhancing composition according to the first embodiment of the present invention can be produced.
In the aforesaid first example of this embodiment, the step of heating paprika without the addition of oil or fat under a condition whereby the heating value is 50 or more to obtain the aforesaid heat-treated paprika is preferably provided with the feature described with regard to the aforesaid first heat-treatment for obtaining the heat-treated paprika of the flavor-enhancing composition according to the first embodiment of the first aspect of the present invention.
A second example of the second embodiment of the first aspect of the present invention relates to

a method for producing the flavor-enhancing composition according to the first embodiment of the first aspect of the present invention, comprising
heating paprika together with oil or fat under a condition whereby the heating value is 0.3 or more to obtain the aforesaid heat-treated paprika.

According to the aforesaid second example of this embodiment, the flavor-enhancing composition according to the first embodiment of the first aspect of the present invention can be produced.
In the aforesaid second example of this embodiment, the step of heating paprika together with oil or fat under a condition whereby the heating value is 0.3 or more to obtain the aforesaid heat-treated paprika is preferably provided with the feature described with regard to the aforesaid second heat-treatment for obtaining the heat-treated paprika of the flavor-enhancing composition according to the first embodiment of the first aspect of the present invention.
In this embodiment encompassing the aforesaid first and second examples (hereinafter "this embodiment"), the aforesaid heat-treated paprika may be further matured or pulverized. The maturing step preferably has the feature described with regard to the maturing step that can be performed to obtain the aforesaid heat-treated paprika of the flavor-enhancing composition according to the first embodiment of the first aspect of the present invention.
The method for producing the flavor-enhancing composition according to this embodiment can further comprise pulverizing the aforesaid heat-treated paprika. A preferred range of particle size of the aforesaid heat-treated paprika after pulverizing is as described with regard to the aforesaid heat-treated paprika of the flavor-enhancing composition according to the first embodiment of the first aspect of the present invention.
The method for producing the flavor-enhancing composition according to this embodiment may use the aforesaid heat-treated paprika as is as the aforesaid flavor-enhancing composition, or may further comprise preparing the aforesaid flavor-enhancing composition by combining the aforesaid heat-treated paprika with an additional material. Preferred examples of the aforesaid additional material are as described with regard to the flavor-enhancing composition according to the first embodiment of the first aspect of the present invention.
The method for producing the flavor-enhancing composition according to this embodiment may comprise processing the obtained flavor-enhancing composition into the form of powder, granule, paste, liquid, etc.

<Method for Enhancing Flavor>

A third embodiment of the first aspect of the present invention relates to
a method for enhancing a flavor of a food product, comprising blending the flavor-enhancing composition according to the first embodiment of the first aspect of the present invention.
As the method according to this embodiment can enhance the flavor of food products, it can be suitably used to enhance the flavor of a food product including one or more of such flavor constituents as described above in a reduced amount from usual (e.g., a reduced-sodium food product in which the sodium chloride amount is reduced from usual).

The blending amount of the flavor-enhancing composition according to the first embodiment of the first aspect of the present invention is not particularly limited and can be adjusted appropriately, according to the form of the food product. For example, for the purpose of enhancing saltiness, the flavor-enhancing composition according to the first embodiment of the first aspect of the present invention can be blended so that the amount of the heat-treated paprika may be, for example, 0.5 g or more, preferably 1 g or more, preferably 2 g or more, more preferably 4 g or more, and for example 0.5 g or more and 50 g or less, preferably 1 g or more and 20 g or less, more preferably 2 g or more and 10 g or less, especially preferably 4 g or more and 10 g or less per 100 g of the sodium chloride equivalent of the food product.

The type of the aforesaid food product is not limited, but the examples are curry sauces, stew sauces, meat products, prepared foods and confectionery. The aforesaid food product may be a food product including such one or more flavor constituents as described above in a reduced amount from usual. The aforesaid food product may be a food product containing such one or more flavor constituents as described above, e.g., table salt.

<One or More Further Embodiments>

One or more further embodiments of the first aspect of the present invention relates to

use of heat-treated paprika, for enhancing the flavor of a food product,
a method for enhancing the flavor of a food product, comprising blending the heat-treated paprika with the food product,
heat-treated paprika for use in enhancing a flavor of a food product, or
use of the heat-treated paprika in producing an additive for use in enhancing a flavor of a food product. In this embodiment, the aforesaid heat-treated paprika is preferably provided with the feature described with regard to the heat-treated paprika included in the flavor-enhancing composition according to the first embodiment of the first aspect of the invention. In this embodiment, the aforesaid heat-treated paprika can preferably be produced by the method for producing the heat-treated paprika described in the method for producing a flavor-enhancing composition according to the second embodiment of the first aspect of the present invention. In this embodiment, the aforesaid food product is preferably provided with the feature described with regard to the method of the third embodiment of the first aspect of the present invention.

<2. Second Aspect of Present Invention>

In this section, a preferred embodiment of the second aspect of the present invention will be described.

<Flavor-enhancing Composition>

A first embodiment of the second aspect of the present invention relates to
a flavor-enhancing composition containing heat-treated cumin.

The flavor-enhancing composition according to this embodiment can enhance a flavor of a food product by blending the composition with the aforesaid food product. For example, a food product which includes the one or more flavor constituents as described above in a reduced amount from usual (e.g., a reduced-sodium food product including a reduced amount of sodium chloride from usual) and the flavor-enhancing composition according to this embodiment blended therewith can have, in comparison with the food product without the flavor-enhancing composition, a flavor close to that of a food product which includes the one or more flavor constituents in their usual amount, and more preferably, can have a flavor comparable to that of a food product including the one or more of flavor constituents in their usual amount. The flavor-enhancing composition according to this embodiment is more preferably a saltiness-enhancing composition.

The heat-treated cumin is the heat-treated seed of cumin (*Cuminum cyminum*), an annual herb of the family *Seriaceae*.

The cumin seed to be subjected to heat-treatment may be whole seed or a pulverized product obtained by pulverizing whole seed.

The heat-treatment of producing the aforesaid heat-treated cumin is preferably a heat-treatment that comprises heating cumin without the addition of oil or fat (hereinafter it may be referred to as "first heat-treatment"), or a heat-treatment that comprises heating cumin together with oil or fat (hereinafter it may be referred to as "second heat-treatment").

A preferred embodiment of the aforesaid first heat-treatment will be explained below.

In the aforesaid first heat-treatment, cumin only or a mixture of cumin and water, preferably a mixture of cumin and water, can be subjected to the heat-treatment. The cumin from the heat-treated mixture of cumin and water is preferred because of its strong flavor-enhancing effect. When the mixture of cumin and water is subjected to the heat-treatment, the mixing ratio of cumin and water is not particularly limited, but preferably the aforesaid mixing ratio is set so that the water activity after heating may be within the range described below. In this context, cumin to be subjected to the aforesaid first heat-treatment is preferably in the form of whole seed. Cumin obtained by the heat-treatment from whole seed only or a mixture of whole seed and water has a particularly strong flavor-enhancing effect.

For the aforesaid first heat-treatment, its temperature and time period are preferably set so that the heating value may be preferably 15 or more, more preferably 60 or more, even more preferably 100 or more, even more preferably 190 or more, most preferably 200 or more, and furthermore, the heating value may be preferably 15 or more and 1000 or less, more preferably 60 or more and 500 or less, even more preferably 100 or more and 410 or less, even more preferably 190 or more and 410 or less, most preferably 200 or more and 410 or less. Another preferred aspect of the aforesaid heating value is, for example, 15 or more and 170 or less, preferably 60 or more and 170 or less, more preferably 100 or more and 170 or less. Cumin heat-treated without the addition of oil or fat under a condition whereby the heating value is within the aforesaid range is preferred because of its particularly strong flavor-enhancing effect. The heating condition whereby the heating value is within the aforesaid range can preferably comprise heating at the temperature for the time described below.

In this context, the definition of the heating value and the CV value is as described with regard to the first embodiment of the first aspect of the present invention.

The aforesaid first heat-treatment for obtaining the aforesaid heat-treated cumin can be carried out at, for example, 110 °C or higher, preferably 120 °C or higher, more preferably 125 °C or higher, especially preferably 128 °C or higher, and for example, 110 °C or higher and 180 °C or lower, preferably 120 °C or higher and 160 °C or lower, more preferably 125 °C or higher and 150 °C or lower, most preferably 128 °C or higher and 150 °C or lower. The aforesaid first heat-treatment can be carried out so that the heating time at a temperature within the aforesaid temperature range may be, for example, 15 minutes or longer, preferably 20 minutes or longer, more preferably 30 minutes or longer, and for example, 15 minutes or longer and 120 minutes or shorter, preferably 20 minutes or longer and 90 minutes or shorter, more preferably 30 minutes or longer and 60 minutes or shorter. Cumin heat-treated at a temperature of 125 °C or higher is preferred because of its particularly strong flavor-enhancing effect.

The aforesaid first heat-treatment can be carried out under a pressure condition in which gauge pressure is preferably 0.05 MPa or higher, more preferably 0.2 MPa or higher. The upper limit of the pressure during heating is not particularly limited, but a gauge pressure of 0.6 MPa or lower is preferred from the viewpoint of equipment.

Various types of means such as pressure-tight heating, superheated steam heating, oven heating, etc. can be used for the aforesaid first heat-treatment, but pressure-tight heating is preferably used. Heating equipment used for pressure-tight heating can be exemplified by pressure-tight kettles and retort sterilizers. When heating other than pressure-tight heating is utilized, ovens, flat-bottom roasting machines, vertical heating mixers, microwave heating equipment, superheated steam eddy current mixers, superheated steam sterilizers, etc. can be used appropriately.

Next, a preferred embodiment of the aforesaid second heat-treatment will be explained.

In the aforesaid second heat-treatment, cumin is heated together with oil or fat. The heat-treated cumin obtained by the aforesaid second heat-treatment is preferred because of its particularly strong flavor-enhancing effect. The amount of oil or fat used in the aforesaid second heat-treatment is not particularly limited, but for example, 10 parts by mass or more and 500 parts by mass or less, preferably 50 parts by mass or more and 200 parts by mass or less, of oil or fat can be used with respect to 100 parts by mass of cumin. The aforesaid oil or fat is not limited so long as it is edible oil or fat derived from plants, animals etc. acceptable as food products. The oil or fat may be an oil or fat that has a melting point adjusted by a technique such as esterification or hydrogenation for fatty acids. In this context, the cumin used in the aforesaid second heat-treatment may be in the form of whole seed or a pulverized product of cumin seed, but the pulverized product of cumin seed is preferred. The pulverized product of cumin seed heat-treated together with oil or fat has a particularly strong flavor-enhancing effect.

For the aforesaid second heat-treatment, its temperature and time period are preferably set so that the heating value may be preferably 1 or more, more preferably 20 or more, more preferably 100 or more, even more preferably 200 or more. The heating value in the aforesaid second heat-treatment can be, for example, 1 or more and 6000 or less, preferably 20 or more and 4000 or less, more preferably 100 or more and 1000 or less, especially preferably 200 or more and 600 or less. Cumin subjected to the aforesaid second heat-treatment under these conditions is preferred because of its particularly strong flavor-enhancing effect. The heating condition whereby the heating value is within the aforesaid range can preferably comprise heating under the temperature and time period condition described below. The definition of the heating value is as described above with regard to the aforesaid first heat-treatment.

In the aforesaid second heat-treatment, the mixture of cumin and oil or fat can be heated at a temperature of, for example, 90 °C or higher, preferably 100 °C or higher, more preferably 120 °C or higher, more preferably 130 °C or higher, more preferably 140 °C or higher, more preferably 150 °C or higher, and for example, 90 °C or higher and 230 °C or lower, preferably 100 °C or higher and 220 °C or lower, more preferably 120 °C or higher and 220 °C or lower, more preferably 130 °C or higher and 210 °C or lower, more preferably 150 °C or higher and 210 °C or lower, especially preferably 160 °C or higher and 210 °C or lower. The aforesaid second heat-treatment can be carried out so that the heating time at a temperature within the aforesaid temperature range may be, for example, 1 minute or longer, preferably 2 minutes or longer, preferably 3 minutes or longer, and for example, 1 minute or longer and 10 minutes or shorter, preferably 2 minutes or longer and 7 minutes or shorter, more preferably 3 minutes or longer and 7 minutes or shorter. The heat-treated cumin obtained by the aforesaid second heat-treatment under these conditions is preferred because of its particularly high flavor-enhancing effect.

The aforesaid second heat-treatment can be carried out in an either open or closed system.

The aforesaid second heat-treatment can be carried out by heating with superheated steam or by heating with an oven. Heating equipment used for the aforesaid second heat-treatment can be exemplified by ovens, flat-bottom roasting machines, vertical heating mixers, microwave heating equipment, etc.

Next, a more preferred embodiment of the aforesaid heat-treated cumin will be described.

The aforesaid heat-treated cumin preferably has a water activity measured at 25 °C of 0.85 or less, more preferably 0.82 or less, and the lower limit is not particularly limited, but it is, for example, 0.50 or more. The aforesaid heat-treated cumin can be prepared by heat-treating cumin under a condition whereby the water activity after heating is within this range.

The aforesaid heat-treated cumin may be further subjected to a maturing treatment after the heat-treatment. The aforesaid maturing treatment is a storage treatment at a temperature higher than room temperature under an air atmosphere, and can be a storage treatment, for example, in an airtight container filled with air, for example, at 30 °C to 50 °C, preferably 35 °C to 45 °C, for example, for 5 days to 15 days, preferably 7 days to 10 days.

The aforesaid heat-treated cumin, in a more preferred embodiment, contains an increased amount of one or more selected from cyclic (Phe-Phe), cyclic (Phe-Leu/Ile), cyclic (Leu/Ile-Val), cyclic (Phe-Pro), cyclic (Phe-Tyr), cyclic (Pro-Leu/Ile), cyclic (Val-Val), cyclic (Phe-Ala), cyclic (Tyr-Ser), cyclic (Pro-Val), cyclic (Pro-Tyr), cyclic (Pro-Pro), cyclic (Leu/Ile-Ser), cyclic (Arg-Leu/Ile), cyclic (Ala-Tyr), cyclic (Gly-Tyr), cyclic (Ala-Pro), cyclic (Gly-Val), cyclic (hyPro-Pro), cyclic (Thr-Pro), cyclic (Val-Arg), cyclic (Pro-Asp), cyclic (Arg-Pro), cyclic (Glu-Tyr), cyclic (Pro-His), cyclic (Glu-Asp), cyclic (Gly-Arg), cyclic (Gly-His), cyclic (Leu/Ile-Leu/Ile), cyclic (Glu-Glu), cyclic (Leu/Ile-Asp), cyclic (Phe-Asp), sulfurol(4-methyl-5-thiazoleethanol), sulfurol acetate, carveol, nerolidol, $\alpha$-terpinene-7-al, tartaric acid, malic acid, citric acid, cis-aconitic acid and *trans*-aconitic acid in comparison with the cumin before the heat-treatment. The present inventors have found that the amount of the aforesaid compounds included in the aforesaid heat-treated cumin correlates with the strength of the flavor-enhancing effect.

That is, in a more preferred embodiment, when the aforesaid heat-treated cumin, with the addition of 47 $\mu$g/g of L-lysine-$^{13}C_6$ monohydrochloride, is analyzed by liquid chromatography-mass spectrometry (LC-MS/MS) according to the method described below, the aforesaid heat-treated cumin satisfies one or more, more preferably ten or more, especially preferably twenty or more, especially preferably thirty or more, and most preferably all, of the following:

(1) a peak area of 0.024 or more, preferably 0.029 or more, and more preferably 0.024 or more and 0.30 or less, even more preferably 0.029 or more and 0.30 or less derived from cyclic (Phe-Phe),
(2) a peak area of 0.040 or more, preferably 0.050 or more, and more preferably 0.040 or more and 1.10 or less, even more preferably 0.050 or more and 1.10 or less derived from cyclic (Phe-Leu/Ile),
(3) a peak area of 0.015 or more, preferably 0.020 or more, and more preferably 0.015 or more and 0.60 or less, more preferably 0.020 or more and 0.60 or less derived from cyclic (Leu/Ile-Val),
(4) a peak area of 0.020 or more, preferably 0.030 or more, and more preferably 0.020 or more and 1.00 or less, even more preferably 0.030 or more and 1.00 or less derived from cyclic (Phe-Pro),
(5) a peak area of 0.0020 or more, and more preferably 0.0020 or more and 0.05 or less derived from cyclic (Phe-Tyr),
(6) a peak area of 0.040 or more, and more preferably 0.040 or more and 1.50 or less derived from cyclic (Pro-Leu/Ile),
(7) a peak area of 0.004 or more, and more preferably 0.004 or more and 0.10 or less derived from cyclic (Val-Val),
(8) a peak area of 0.002 or more, preferably 0.003 or more, and more preferably 0.002 or more and 0.10 or less, even more preferably 0.003 or more and 0.10 or less derived from cyclic (Phe-Ala),
(9) a peak area of 0.002 or more, and more preferably 0.002 or more and 0.15 or less derived from cyclic (Tyr-Ser),
(10) a peak area of 0.013 or more, preferably 0.015 or more, and more preferably 0.013 or more and 0.25 or less, even more preferably 0.015 or more and 0.25 or less derived from cyclic (Pro-Val),
(11) a peak area of 0.009 or more, and more preferably 0.009 or more and 0.25 or less derived from cyclic (Pro-Tyr),
(12) a peak area of 0.050 or more, and more preferably 0.050 or more and 2.0 or less derived from cyclic (Pro-Pro),
(13) a peak area of 0.010 or more, and more preferably 0.010 or more and 0.20 or less derived from cyclic (Leu/Ile-Ser),
(14) a peak area of 0.10 or more, and more preferably 0.10 or more and 2.20 or less derived from cyclic (Arg-Leu/Ile),
(15) a peak area of 0.007 or more, and more preferably 0.007 or more and 0.20 or less derived from cyclic (Ala-Tyr),
(16) a peak area of 0.002 or more, and more preferably 0.002 or more and 0.20 or less, even more preferably 0.002 or more and 0.09 or less derived from cyclic (Gly-Tyr),

(17) a peak area of 0.020 or more, and more preferably 0.020 or more and 0.60 or less derived from cyclic (Ala-Pro),
(18) a peak area of 0.001 or more, and more preferably 0.001 or more and 0.09 or less derived from cyclic (Gly-Val),
(19) a peak area of 0.010 or more, preferably 0.020 or more, and more preferably 0.010 or more and 0.50 or less, more preferably 0.020 or more and 0.50 or less derived from cyclic (HyPro-Pro),
(20) a peak area of 0.040 or more, and more preferably 0.040 or more and 0.90 or less derived from cyclic (Thr-Pro),
(21) a peak area of 0.060 or more, and more preferably 0.060 or more and 1.00 or less derived from cyclic (Val-Arg),
(22) a peak area of 0.040 or more, and more preferably 0.040 or more and 0.90 or less derived from cyclic (Pro-Asp),
(23) a peak area of 0.035 or more, and more preferably 0.035 or more and 1.00 or less derived from cyclic (Arg-Pro),
(24) a peak area of 0.050 or more, and more preferably 0.050 or more and 1.50 or less derived from cyclic (Glu-Tyr),
(25) a peak area of 0.020 or more, and more preferably 0.020 or more and 0.90 or less derived from cyclic (Pro-His),
(26) a peak area of 0.080 or more, and more preferably 0.080 or more and 1.10 or less derived from cyclic (Glu-Asp),
(27) a peak area of 0.50 or more, preferably 0.90 or more, and more preferably 0.50 or more and 20.0 or less, even more preferably 0.90 or more and 20.0 or less derived from cyclic (Gly-Arg),
(28) a peak area of 0.005 or more, preferably 0.007 or more, and more preferably 0.005 or more and 0.18 or less, even more preferably 0.007 or more and 0.18 or less derived from cyclic (Gly-His),
(29) a peak area of 0.070 or more, and more preferably 0.070 or more and 1.30 or less derived from cyclic (Leu/Ile-Leu/Ile),
(30) a peak area of 0.010 or more, preferably 0.015 or more, and more preferably 0.010 or more and 0.30 or less, even more preferably 0.015 or more and 0.30 or less derived from cyclic (Glu-Glu),
(31) a peak area of 0.010 or more, and more preferably 0.010 or more and 0.30 or less derived from cyclic (Leu/Ile-Asp),
(32) a peak area of 0.012 or more, and more preferably 0.012 or more and 0.25 or less derived from cyclic (Phe-Asp),
(33) a peak area of 0.30 or more, and more preferably 0.30 or more and 4.00 or less derived from sulfurol(4-methyl-5-thiazoleethanol) and
(34) a peak area of 0.025 or more, and more preferably 0.025 or more and 0.80 or less derived from sulfurol acetate,

as a ratio to a peak area derived from L-lysine-$^{13}C_6$ monohydrochloride in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated cumin (representing a mass converted to that of the cumin excluding oil or fat when the aforesaid heat-treated cumin is cumin heat-treated together with the oil or fat) and 5 mL of water is kept heated in a thermostatic water bath at 80 °C for 30 minutes, to prepare a water extract. To the aforesaid water extract in the aforesaid test tube are added 5 mL of acetonitrile and L-lysine-$^{13}C_6$ monohydrochloride in an amount of 47 μg/g with respect to the heat-treated cumin (representing a mass converted to that of the cumin excluding oil or fat when the aforesaid heat-treated cumin is cumin heat-treated together with the oil or fat), followed by stirring, and then a solid portion is removed and a liquid portion is collected, to prepare a specimen sample. The aforesaid specimen sample is analyzed by LC-MS/MS to obtain a chromatogram. In this context, the aforesaid heat-treated cumin used as the analysis sample is preferably a pulverized product.

The peak area in an extracted ion chromatogram for respective product ions with an m/z value described in Examples can be used as the peak area derived from L-lysine-$^{13}C_6$ monohydrochloride and the peak area of the respective compounds in aforesaid (1) to (34).

In a particularly preferred embodiment of the aforesaid heat-treated cumin obtained by the aforesaid first heat-treatment (heat-treatment without the addition of oil or fat), the heat-treated cumin satisfies one or more, more preferably ten or more, especially preferably twenty or more, especially preferably thirty or more, and most preferably all, of the following:

(1') a peak area of 0.030 or more, and more preferably 0.030 or more and 0.30 or less derived from cyclic (Phe-Phe),
(2') a peak area of 0.110 or more, and more preferably 0.110 or more and 1.10 or less derived from cyclic (Phe-Leu/Ile),
(3') a peak area of 0.060 or more, and more preferably 0.060 or more and 0.60 or less derived from cyclic (Leu/Ile-Val),
(4') a peak area of 0.100 or more, and more preferably 0.100 or more and 1.00 or less derived from cyclic (Phe-Pro),
(5') a peak area of 0.005 or more, and more preferably 0.005 or more and 0.05 or less derived from cyclic (Phe-Tyr),
(6') a peak area of 0.150 or more, and more preferably 0.150 or more and 1.50 or less derived from cyclic (Pro-Leu/Ile),
(7') a peak area of 0.010 or more, and more preferably 0.010 or more and 0.10 or less derived from cyclic (Val-Val),
(8') a peak area of 0.010 or more, and more preferably 0.010 or more and 0.10 or less derived from cyclic (Phe-Ala),
(9') a peak area of 0.015 or more, and more preferably 0.015 or more and 0.15 or less derived from cyclic (Tyr-Ser),
(10') a peak area of 0.025 or more, and more preferably 0.025 or more and 0.25 or less derived from cyclic (Pro-Val),
(11') a peak area of 0.025 or more, and more preferably 0.025 or more and 0.25 or less derived from cyclic (Pro-Tyr),
(12') a peak area of 0.20 or more, and more preferably 0.20 or more and 2.0 or less derived from cyclic (Pro-Pro),

(13') a peak area of 0.020 or more, and more preferably 0.020 or more and 0.20 or less derived from cyclic (Leu/Ile-Ser),
(14') a peak area of 0.220 or more, and more preferably 0.220 or more and 2.20 or less derived from cyclic (Arg-Leu/Ile),
(15') a peak area of 0.020 or more, and more preferably 0.020 or more and 0.20 or less derived from cyclic (Ala-Tyr),
(16') a peak area of 0.009 or more, and more preferably 0.009 or more and 0.09 or less derived from cyclic (Gly-Tyr),
(17') a peak area of 0.060 or more, and more preferably 0.060 or more and 0.60 or less derived from cyclic (Ala-Pro),
(18') a peak area of 0.009 or more, and more preferably 0.009 or more and 0.09 or less derived from cyclic (Gly-Val),
(19') a peak area of 0.050 or more, and more preferably 0.050 or more and 0.50 or less derived from cyclic (hyPro-Pro),
(20') a peak area of 0.090 or more, and more preferably 0.090 or more and 0.90 or less derived from cyclic (Thr-Pro),
(21') a peak area of 0.100 or more, and more preferably 0.100 or more and 1.00 or less derived from cyclic (Val-Arg),
(22') a peak area of 0.090 or more, and more preferably 0.090 or more and 0.90 or less derived from cyclic (Pro-Asp),
(23') a peak area of 0.100 or more, and more preferably 0.100 or more and 1.00 or less derived from cyclic (Arg-Pro),
(24') a peak area of 0.150 or more, and more preferably 0.150 or more and 1.50 or less derived from cyclic (Glu-Tyr),
(25') a peak area of 0.090 or more, and more preferably 0.090 or more and 0.90 or less derived from cyclic (Pro-His),
(26') a peak area of 0.110 or more, and more preferably 0.110 or more and 1.10 or less derived from cyclic (Glu-Asp),
(27') a peak area of 2.00 or more, and more preferably 2.00 or more and 20.0 or less derived from cyclic (Gly-Arg),
(28') a peak area of 0.018 or more, and more preferably 0.018 or more and 0.18 or less derived from cyclic (Gly-His),
(29') a peak area of 0.130 or more, and more preferably 0.130 or more and 1.30 or less derived from cyclic (Leu/Ile-Leu/Ile),
(30') a peak area of 0.030 or more, and more preferably 0.030 or more and 0.30 or less derived from cyclic (Glu-Glu),
(31') a peak area of 0.030 or more, and more preferably 0.030 or more and 0.30 or less derived from cyclic (Leu/Ile-Asp),
(32') a peak area of 0.025 or more, and more preferably 0.025 or more and 0.25 or less derived from cyclic (Phe-Asp),
(33') a peak area of more than 0.400, and more preferably 0.400 or more and 4.00 or less derived from sulfurol(4-methyl-5-thiazoleethanol) and
(34') a peak area of 0.080 or more, and more preferably 0.080 or more and 0.80 or less derived from sulfurol acetate,
as a ratio to a peak area derived from L-lysine-$^{13}C_6$ monohydrochloride in the chromatogram described above.

In a particularly preferred embodiment of the aforesaid heat-treated paprika obtained by the aforesaid second heat-treatment (heat-treatment carried out with the addition of oil or fat), the heat-treated paprika satisfies one or more, more preferably ten or more, especially preferably twenty or more, especially preferably thirty or more, and most preferably all, of the following:

(1") a peak area of 0.025 or more, and more preferably 0.025 or more and 0.30 or less derived from cyclic (Phe-Phe),
(2") a peak area of 0.100 or more, and more preferably 0.100 or more and 1.10 or less derived from cyclic (Phe-Leu/Ile),
(3") a peak area of 0.080 or more, and more preferably 0.080 or more and 0.60 or less derived from cyclic (Leu/Ile-Val),
(4") a peak area of 0.090 or more, and more preferably 0.090 or more and 1.00 or less derived from cyclic (Phe-Pro),
(5") a peak area of 0.008 or more, and more preferably 0.008 or more and 0.05 or less derived from cyclic (Phe-Tyr),
(6") a peak area of 0.200 or more, and more preferably 0.200 or more and 1.50 or less derived from cyclic (Pro-Leu/Ile),
(7") a peak area of 0.007 or more, and more preferably 0.007 or more and 0.10 or less derived from cyclic (Val-Val),
(8") a peak area of 0.010 or more, and more preferably 0.010 or more and 0.10 or less derived from cyclic (Phe-Ala),
(9") a peak area of 0.015 or more, and more preferably 0.015 or more and 0.15 or less derived from cyclic (Tyr-Ser),
(10") a peak area of 0.030 or more, and more preferably 0.025 or more and 0.25 or less derived from cyclic (Pro-Val),
(11") a peak area of 0.040 or more, and more preferably 0.040 or more and 0.25 or less derived from cyclic (Pro-Tyr),
(12") a peak area of 0.10 or more, and more preferably 0.10 or more and 2.0 or less derived from cyclic (Pro-Pro),
(13") a peak area of 0.020 or more, and more preferably 0.020 or more and 0.20 or less derived from cyclic (Leu/Ile-Ser),
(14") a peak area of 0.150 or more, and more preferably 0.150 or more and 2.20 or less derived from cyclic (Arg-Leu/Ile),
(15") a peak area of 0.020 or more, and more preferably 0.020 or more and 0.20 or less derived from cyclic (Ala-Tyr),
(16") a peak area of 0.020 or more, and more preferably 0.020 or more and 0.20 or less derived from cyclic (Gly-Tyr),
(17") a peak area of 0.060 or more, and more preferably 0.060 or more and 0.60 or less derived from cyclic (Ala-Pro),
(18") a peak area of 0.005 or more, and more preferably 0.005 or more and 0.09 or less derived from cyclic (Gly-Val),
(19") a peak area of 0.060 or more, and more preferably 0.060 or more and 0.50 or less derived from cyclic (hyPro-Pro),
(20") a peak area of 0.060 or more, and more preferably 0.060 or more and 0.90 or less derived from cyclic (Thr-Pro),

(21") a peak area of 0.060 or more, and more preferably 0.060 or more and 1.00 or less derived from cyclic (Val-Arg),
(22") a peak area of 0.110 or more, and more preferably 0.110 or more and 0.90 or less derived from cyclic (Pro-Asp),
(23") a peak area of 0.100 or more, and more preferably 0.100 or more and 1.00 or less derived from cyclic (Arg-Pro),
(24") a peak area of 0.200 or more, and more preferably 0.200 or more and 1.50 or less derived from cyclic (Glu-Tyr),
(25") a peak area of 0.060 or more, and more preferably 0.060 or more and 0.90 or less derived from cyclic (Pro-His),
(26") a peak area of 0.100 or more, and more preferably 0.100 or more and 1.10 or less derived from cyclic (Glu-Asp),
(27") a peak area of 1.00 or more, and more preferably 1.00 or more and 20.0 or less derived from cyclic (Gly-Arg),
(28") a peak area of 0.020 or more, and more preferably 0.020 or more and 0.18 or less derived from cyclic (Gly-His),
(29") a peak area of 0.130 or more, and more preferably 0.130 or more and 1.30 or less derived from cyclic (Leu/Ile-Leu/Ile),
(30") a peak area of 0.012 or more, and more preferably 0.012 or more and 0.30 or less derived from cyclic (Glu-Glu),
(31") a peak area of 0.020 or more, and more preferably 0.020 or more and 0.30 or less derived from cyclic (Leu/Ile-Asp),
(32") a peak area of 0.018 or more, and more preferably 0.018 or more and 0.25 or less derived from cyclic (Phe-Asp),
(33") a peak area of 0.400 or more, and more preferably 0.400 or more and 4.00 or less derived from sulfurol(4-methyl-5-thiazoleethanol) and
(34") a peak area of 0.100 or more, and more preferably 0.100 or more and 0.80 or less derived from sulfurol acetate,
as a ratio to a peak area derived from L-lysine-$^{13}C_6$ monohydrochloride in the chromatogram described above.

In addition, the aforesaid heat-treated cumin, in a more preferred embodiment, is characterized in that it contains one or more selected from the group consisting of cyclic (Phe-Tyr), cyclic (Phe-Ala), cyclic (Tyr-Ser), cyclic (Leu/Ile-Ser), cyclic (Ala-Tyr), cyclic (Gly-Tyr), cyclic (Ala-Pro), cyclic (Gly-Val), cyclic (hyPro-Pro) and cyclic (Glu-Glu), and more preferably all members of the aforesaid group. These cyclic dipeptides are characteristic constituents of heat-treated cumin having the flavor-enhancing effect, which are not detected in unheated cumin.

In a more preferred embodiment, when the aforesaid heat-treated cumin, with the addition of 99 μg/g of ribitol, is analyzed by liquid chromatography-mass spectrometry (LC-MS/MS) according to the method described below, the aforesaid heat-treated cumin satisfies one or more, more preferably two or more, more preferably three or more, even more preferably four or more, and most preferably all, of the following:

(35) a peak area of 0.180 or more, and more preferably 0.180 or more and 2.50 or less derived from *cis*-aconitic acid,
(36) a peak area of 0.250 or more, and more preferably 0.250 or more and 5.00 or less derived from *trans*-aconitic acid,
(37) a peak area of 0.550 or more, preferably 0.660 or more, and more preferably 0.550 or more and 7.0 or less, more preferably 0.660 or more and 7.0 or less derived from tartaric acid,
(38) a peak area of 22.0 or more, and more preferably 22.0 or more and 230.0 or less derived from malic acid and
(39) a peak area of 35.0 or more, and more preferably 35.0 or more and 400.0 or less derived from citric acid,
as a ratio to a peak area derived from ribitol in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated cumin (representing a mass converted to that of the cumin excluding oil or fat when the aforesaid heat-treated cumin is cumin heat-treated together with the oil or fat) and 5 mL of water is kept heated in a thermostatic water bath at 80 °C for 30 minutes, to prepare a water extract. To the aforesaid water extract in the aforesaid test tube are added 5 mL of acetonitrile and ribitol in an amount of 99 μg/g with respect to the heat-treated cumin (representing a mass converted to that of the cumin excluding oil or fat when the aforesaid heat-treated cumin is cumin heat-treated together with the oil or fat), followed by stirring, and then a solid portion is removed and a liquid portion is collected, to prepare a specimen sample. The aforesaid specimen sample is analyzed by LC-MS/MS, to obtain a chromatogram. In this context, the aforesaid heat-treated cumin used as the analysis sample is preferably a pulverized product.

The peak area in an extracted ion chromatogram for respective product ions with an m/z value described in Examples can be used as the peak area derived from ribitol and of the peak area of the respective compounds in aforesaid (35) to (39).

In a particularly preferred embodiment of the aforesaid heat-treated cumin obtained by the aforesaid first heat-treatment (heat-treatment without the addition of oil or fat), the heat-treated cumin satisfies one or more, more preferably two or more, more preferably three or more, even more preferably four or more, and most preferably all, of the following:

(35') a peak area of 0.250 or more, and more preferably 0.250 or more and 2.50 or less derived from *cis*-aconitic acid,
(36') a peak area of 0.500 or more, and more preferably 0.500 or more and 5.00 or less derived from *trans*-aconitic acid,

(37') a peak area of 0.70 or more, and more preferably 0.70 or more and 7.0 or less derived from tartaric acid,
(38') a peak area of 23.00 or more, and more preferably 23.00 or more and 230.0 or less derived from malic acid and
(39') a peak area of 40.00 or more, and more preferably 40.00 or more and 400.0 or less derived from citric acid,
as a ratio to a peak area derived from ribitol in the chromatogram described above.

In a particularly preferred embodiment of the aforesaid heat-treated cumin obtained by the aforesaid second heat-treatment (heat-treatment with the addition of oil or fat), the heat-treated cumin satisfies one or more, more preferably two or more, more preferably three or more, even more preferably four or more, and most preferably all, of the following:

(35") a peak area of 0.200 or more, and more preferably 0.200 or more and 2.50 or less derived from *cis*-aconitic acid,
(36") a peak area of 0.700 or more, and more preferably 0.500 or more and 5.00 or less derived from *trans*-aconitic acid,
(37") a peak area of 0.55 or more, and more preferably 0.55 or more and 7.0 or less derived from tartaric acid,
(38") a peak area of 25.00 or more, and more preferably 25.00 or more and 230.0 or less derived from malic acid and
(39") a peak area of 37.00 or more, and more preferably 37.00 or more and 400.0 or less derived from citric acid,
as a ratio to a peak area derived from ribitol in the aforesaid chromatogram.

In a more preferred embodiment, when the aforesaid heat-treated cumin according to the this embodiment (representing a mass converted to that of the cumin excluding oil or fat when the aforesaid heat-treated cumin is cumin heat-treated together with the oil or fat), with the addition of 625 μg/g of 4-methylthiazole, is analyzed by gas chromatography-mass spectrometry (GC-MS) according to the method described below, the heat-treated cumin satisfies one or more, more preferably two or more, and more preferably all, of the following:

(40) a peak area of 0.05 or more, and more preferably 0.05 or more and 0.80 or less derived from carveol,
(41) a peak area of 0.18 or more, and more preferably 0.18 or more and 2.0 or less derived from nerolidol and
(42) a peak area of 100 or more, and more preferably 100 or more and 1500 or less derived from α-terpinene-7-al,
as a ratio to a peak area derived from 4-methylthiazole in a chromatogram obtained.

In this context, the measurement method of GC-MS is as follows, and more preferably, it is the measurement method of GC-MS described in the Examples. In this specification, operations where temperature is not specified can be carried out at room temperature, specifically at 23 °C.

(Measurement method of GC-MS)

Fifteen milligrams of the aforesaid heat-treated cumin (mass converted to that of the cumin excluding oil or fat when the aforesaid heat-treated cumin is cumin heat-treated together with the oil or fat), 4-methylthiazole in an amount of 625 μg/g with respect to the aforesaid heat-treated cumin (representing a mass converted to that of cumin excluding oil or fat when the aforesaid heat-treated cumin is cumin heat-treated together with the oil or fat), 5 mL of acetone and 5 mL of methanol contained in a 10-mL test tube are stirred, and then a solid portion is removed and a liquid portion is collected, to which is added 1 mL of acetone per 0.1 mL of the liquid portion, to prepare a GC-MS specimen sample. The aforesaid GC-MS specimen sample is analyzed by GC-MS, to obtain a chromatogram. In this context, the aforesaid heat-treated cumin used as the analysis sample is preferably a pulverized product.

The peak area in an extracted ion chromatogram for respective ions corresponding to the precise mass of constituents described in Examples can be used as the peak area derived from 4-methylthiazole and the peak area derived from the constituents specified in aforesaid (40) to (42).

In a particularly preferred embodiment of the aforesaid heat-treated cumin obtained by the aforesaid first heat-treatment (heat-treatment carried out without the addition of oil or fat), the heat-treated cumin satisfies one or more, more preferably two or more, more preferably all, of the following:

(40') a peak area of 0.08 or more, and more preferably 0.08 or more and 0.80 or less derived from carveol,
(41') a peak area of 0.20 or more, and more preferably 0.20 or more and 2.0 or less derived from nerolidol and
(42') a peak area of 150 or more, and more preferably 150 or more and 1500 or more derived from α-terpinene-7-al,
as a ratio to a peak area derived from 625 μg/g of 4-methylthiazole in the chromatogram described above.

In a particularly preferred embodiment of the aforesaid heat-treated cumin obtained by the aforesaid second heat-treatment (heat-treatment with the addition of oil or fat), the heat-treated cumin satisfies one or more, more preferably two or more, more preferably all, of the following:

(40") a peak area of 0.06 or more, and more preferably 0.06 or more and 0.80 or less derived from carveol,
(41") a peak area of 0.18 or more, and more preferably 0.18 or more and 2.0 or less derived from nerolidol and
(42") a peak area of 120 or more, and more preferably 120 or more and 1500 or less derived from α-terpinene-7-al,
as a ratio to a peak area derived from 625 μg/g of 4-methylthiazole in the chromatogram described above.

The flavor-enhancing composition according to this embodiment may include the heat-treated cumin in the form of whole seed or in the form of a pulverized product made by pulverizing whole seed. The flavor-enhancing compositions according to this embodiment including the heat-treated cumin in the form of a pulverized product is preferred because of its particularly strong flavor-enhancing effect. The heat-treated cumin in the form of a pulverized product is preferably in the form of powder, and the particle size is not particularly limited, but can be, for example, 1000 μm or less, preferably 500 μm or less, more preferably 300 μm or less. The particle size can be determined by the opening of a standard sieve as specified in JIS. The heat-treated cumin in the flavor-enhancing composition according to this embodiment may also be provided in the form of a mixture of the heat-treated cumin and oil or fat. The aforesaid mixture may be solid at room temperature or liquid at room temperature, depending on the melting point of the aforesaid oil or fat.
The flavor-enhancing composition according to this embodiment may consist only of the heat-treated cumin, or may include the heat-treated cumin and an additional material. The additional material may be exemplified by one or more materials having the flavor-enhancing effect and one or more materials acceptable as food products. The flavor-enhancing composition according to this embodiment can contain the heat-treated cumin at a percentage of preferably 30% by mass or more and 100% by mass or less, more preferably 50% by mass or more and 100% by mass or less, even more preferably 80% by mass or more 100% by mass or less, and most preferably 95% by mass or more and 100% by mass or less. The flavor-enhancing composition according to this embodiment can be in the form of powder, granule, paste, liquid, etc., and can include one or more materials acceptable as food products, such as excipients, carriers, etc., as necessary, in order to achieve a desired form of the composition.

<Method for Producing Flavor-enhancing Composition>

A first example of the second embodiment of the second aspect of the present invention relates to

a method for producing the flavor-enhancing composition according to the first embodiment of the second aspect of the present invention, comprising
heating cumin without the addition of oil or fat under a condition whereby the heating value is 15 or more to obtain the aforesaid heat-treated cumin.

According to the aforesaid first example of this embodiment, the flavor-enhancing composition according to the first embodiment of the second aspect of the present invention can be produced.
In the aforesaid first example of this embodiment, the step of heating cumin without the addition of oil or fat under a condition whereby the heating value is 15 or more to obtain the heat-treated cumin is preferably provided with the feature described with regard to the aforesaid first heat-treatment for obtaining the heat-treated cumin of the flavor-enhancing composition according to the first embodiment of the second aspect of the present invention.
A second example of the second embodiment of the second aspect of the present invention relates to

a method for producing the flavor-enhancing composition according to the first embodiment of the second aspect of the present invention, comprising
heating cumin together with oil or fat under a condition whereby the heating value is 1 or more to obtain the aforesaid heat-treated cumin.

According to the aforesaid second example of this embodiment, the flavor-enhancing composition according to the first embodiment of the second aspect of the present invention can be produced.
In the aforesaid second example of this embodiment, the step of heating cumin together with oil or fat under a condition whereby the heating value is 1 or more to obtain the heat-treated cumin is preferably provided with the feature described with regard to the second heat-treatment for obtaining the heat-treated cumin of the flavor-enhancing composition according to the first embodiment of the second aspect of the present invention.
In this embodiment encompassing the aforesaid first and second examples above (hereinafter "this embodiment"), the aforesaid heat-treated cumin may be further matured or pulverized. The maturing step is preferably provided with the feature described with regard to the maturing step that can be carried out to obtain the aforesaid heat-treated cumin of the flavor-enhancing composition of the first embodiment of the second aspect of the present invention.
The method for producing a flavor-enhancing composition according to this embodiment can further include pulverizing the aforesaid heat-treated cumin. A preferred range of particle size of the aforesaid heat-treated cumin after pulverizing is as described with regard to the aforesaid heat-treated cumin of the flavor-enhancing composition according to the first embodiment of the second aspect of the present invention.

The method for producing a flavor-enhancing composition according to this embodiment may use the aforesaid heat-treated cumin as is as the aforesaid flavor-enhancing composition, or may further comprise preparing the aforesaid flavor-enhancing composition by combining the aforesaid heat-treated cumin with an additional material. Preferred examples of the aforesaid above-described additional material are as described with regard to the flavor-enhancing composition according to the first embodiment of the second aspect of the present invention.

The method for producing a flavor-enhancing composition according to this embodiment may comprise processing the obtained flavor-enhancing composition into the form of powder, granule, paste, liquid, etc.

<Method for Enhancing Flavor>

A third embodiment of the second aspect of the present invention relates to a method for enhancing the flavor of a food product, comprising

blending the flavor-enhancing composition according to the first embodiment of the second aspect of the present invention with the food product.

As the method according to this embodiment can enhance the flavor of food products, it can be suitably used to enhance the flavor of a food product including one or more of such flavor constituents as described above in a reduced amount from usual (e.g., a reduced-sodium food product in which the sodium chloride amount is reduced from usual).

The blending amount of the flavor-enhancing composition according to the first embodiment of the second aspect of the present invention is not particularly limited and can be adjusted appropriately, according to the form of the food product. For example, for the purpose of enhancing saltiness, the flavor-enhancing composition according to the first embodiment of the second aspect of the present invention can be blended so that the amount of the heat-treated cumin may be, for example, 0.5 g or more, preferably 1 g or more, preferably 3 g or more, and for example 0.5 g or more and 50 g or less, preferably 1 g or more and 20 g or less, more preferably 3 g or more and 10 g or less per 100 g of the sodium chloride equivalent of the food product.

The type of the aforesaid food product is not limited, but the examples are curry sauces, stew sauces, meat products, prepared foods and confectionery. The aforesaid food product may be a food product including one or more of such flavor constituents as described above in a reduced amount from usual. The aforesaid food product may be a food product containing one or more of such flavor constituents as described above, e.g., table salt.

<One or More Further Embodiments>

One or more further embodiments of the second aspect of the present invention relates to

use of heat-treated cumin for enhancing the flavor of a food product,
a method for enhancing the flavor of a food product, comprising blending the heat-treated cumin with the food product,
heat-treated cumin for use in enhancing the flavor of a food product; or
use of the heat-treated cumin in producing an additive for use in enhancing the flavor of a food product.

In this embodiment, the aforesaid heat-treated cumin is preferably provided with the feature described with regard to the heat-treated cumin included in the flavor-enhancing composition according to the first embodiment of the second aspect of the present invention. In this embodiment, the aforesaid heat-treated cumin can preferably be produced by the method for producing the heat-treated cumin described in the method for producing a flavor-enhancing composition according to the second embodiment of the second aspect of the present invention. In this embodiment, the aforesaid food product is preferably provided with the feature described with regard to the method of the third embodiment of the second aspect of the present invention.

<3. Third Aspect of Present Invention>

In this section, a preferred embodiment of the third aspect of the present invention is described.

<Flavor-enhancing Composition>

A first embodiment of the third aspect of the present invention relates to
a flavor-enhancing composition containing heat-treated asafoetida.

The flavor-enhancing composition according to this embodiment can enhance a flavor of a food product by blending the composition with the aforesaid food product. For example, a food product which includes the one or more flavor constituents as described above in a reduced amount from usual (e.g., a reduced-sodium food product including a reduced amount of sodium chloride from usual) and the flavor-enhancing composition according to this embodiment blended therewith can have, in comparison with the food product without the flavor-enhancing composition, a flavor close to that of a food product which includes the one or more flavor constituents in their usual amount, and more preferably, can have a flavor comparable to that of a food product including the one or more of flavor constituents in their usual amount. The flavor-enhancing composition according to this embodiment is more preferably a saltiness-enhancing composition.

In this specification, asafoetida is a dried resin used as a spice, collected from asafoetida (*Ferula assa-foetida*), a member of the family *Seriaceae.* Asafoetida used as a spice may include, in addition to the asafoetida resin, an additional additive such as gum arabic and rice flour. For example, powdered asafoetida including asafoetida resin and the additive is commercially available as a spice. In this specification, asafoetida refers to asafoetida that is used as a spice and may include the aforesaid additive, unless otherwise stated. The mass of asafoetida used in the respective embodiments described below is expressed in a mass converted under the assumption that the asafoetida resin content is 12% by mass. For example, "200 mg of heat-treated asafoetida" is, when the asafoetida resin content in used asafoetida is 6% by mass, equivalent to 400 mg of the mass as a mass converted under the assumption that the asafoetida resin content is 12% by mass. The heat-treated asafoetida is asafoetida that has been heated.

The heat-treatment for producing the aforesaid heat-treated asafoetida is preferably a heat-treatment that comprises heating asafoetida without the addition of oil or fat (hereinafter it may be referred to as "first heat-treatment"), or a heat-treatment that comprises heating asafoetida together with oil or fat (hereinafter it may be referred to as "second heat-treatment").

A preferred aspect of the aforesaid first heat-treatment will be described below.

Asafoetida only or a mixture of asafoetida and water may be subjected to the aforesaid first heat-treatment.

For the aforesaid first heat-treatment, its temperature and time period are preferably set so that the heating value may be preferably 15 or more, more preferably 50 or more, even more preferably 100 or more, and furthermore so that the heating value may be preferably 15 or more and 1000 or less, more preferably 50 or more and 500 or less, most preferably 100 or more and 300 or less. The preferred range of the heating value is exemplified by 50 or more and 300 or less. Asafoetida subjected to the aforesaid first heat-treatment under these conditions is preferred because of its particularly strong flavor-enhancing effect. The heating condition whereby the heating value is within the aforesaid range can preferably comprise heating under the temperature and time period condition described below.

In this context, the definition of the heating value and the CV value is as described with regard to the first embodiment of the first aspect of the present invention.

In the aforesaid first heat-treatment, heating can be carried out at a temperature of, for example, 110 °C or higher, preferably 120 °C or higher, more preferably 125 °C or higher, especially preferably 128 °C or higher, and for example, 110 °C or higher and 180 °C or lower, preferably 120 °C or higher and 160 °C or lower, more preferably 125 °C or higher and 150 °C or lower, especially preferably 128 °C or higher and 150 °C or lower. The aforesaid first heat-treatment can be carried out so that the heating time at a temperature within the aforesaid temperature range may be, for example, 20 minutes or longer, preferably 30 minutes or longer, and for example, 20 minutes or longer and 120 minutes or shorter, preferably 30 minutes or longer and 90 minutes or shorter, more preferably 30 minutes or longer and 60 minutes or shorter. Heat-treated asafoetida obtained by the aforesaid first heat-treatment under these conditions is preferred because of its particularly strong flavor-enhancing effect.

The aforesaid first heat-treatment can be carried out under a pressure condition in which gauge pressure is preferably 0.05 MPa or higher, more preferably 0.2 MPa or higher. The upper limit of the pressure during heating is not particularly limited, but a gauge pressure of 0.6 MPa or lower is preferred from the viewpoint of equipment.

Various types of means such as pressure-tight heating, superheated steam heating, oven heating, etc. can be used for the aforesaid first heat-treatment, but pressure-tight heating is preferably used. Heating equipment used for pressure-tight heating can be exemplified by pressure-tight kettles and retort sterilizers. When heating other than pressure-tight heating is utilized, ovens, flat-bottom roasting machines, vertical heating mixers, microwave heating equipment, superheated steam eddy current mixers, superheated steam sterilizers, etc. can be used appropriately.

Next, a preferred aspect of the aforesaid second heat-treatment will be described below.

In the aforesaid second heat-treatment, asafoetida is heated together with oil or fat. The heat-treated asafoetida obtained by the aforesaid second heat-treatment is preferred because of its particularly strong flavor-enhancing effect. The amount of oil or fat used in the aforesaid second heat-treatment is not particularly limited, but for example, 10 parts by mass or more and 500 parts by mass or less, preferably 50 parts by mass or more and 200 parts by mass or less, of oil or fat can be used with respect to 100 parts by mass of asafoetida. The aforesaid oil or fat is not limited so long as it is edible oil or fat derived from plants, animals etc. acceptable as food products. The oil or fat may be an oil or fat that has a melting point adjusted by a technique such as esterification or hydrogenation for fatty acids.

For the aforesaid second heat-treatment, its temperature and time period are preferably set so that the heating value may be preferably 5 or more, more preferably 10 or more, even more preferably 20 or more. The upper limit of the heating value in the aforesaid second heat-treatment is exemplified by 100 or less, preferably 50 or less, more preferably 40 or less. That is, the aforesaid heating value can preferably be 5 or more and 100 or less, more preferably 10 or more and 50 or less, even more preferably 20 or more and 40 or less. Asafoetida subjected to the aforesaid second heat-treatment under these conditions is preferred because of its particularly strong flavor-enhancing effect. The heating condition whereby the heating value is within the aforesaid range can preferably comprise heating under the temperature and time period condition described below. The definition of the heating value is as described above with regard to the aforesaid first heat-treatment.

In the aforesaid second heat-treatment, the mixture of asafoetida and oil or fat can be heated at a temperature of, for example, 90 °C or higher, preferably 100 °C or higher, more preferably 110 °C or higher, more preferably 120 °C or higher, especially preferably 125 °C or higher, and for example, 90 °C or higher and 180 °C or lower, preferably 100 °C or higher and 160 °C or lower, more preferably 110 °C or higher and 150 °C or lower, more preferably 120 °C or higher and 150 °C or lower, especially preferably 125 °C or higher and 150 °C or lower. The aforesaid second heat-treatment can be carried out so that the heating time at a temperature within the above-described temperature range may be, for example, 2 minutes or longer, preferably 3 minutes or longer, and for example, 3 minutes or longer and 10 minutes or shorter, preferably 3 minutes or longer and 7 minutes or shorter. Heat-treated asafoetida obtained by the aforesaid second heat-treatment under these conditions is preferred because of its particularly strong flavor-enhancing effect.

The aforesaid second heat-treatment can be carried out in an either open or closed system.

The aforesaid second heat-treatment can be carried out by heating with superheated steam or by heating with an oven. Heating equipment used for the aforesaid second heat-treatment can be exemplified by ovens, flat-bottom roasting machines, vertical heating mixers, microwave heating equipment, etc.

Next, a more preferred embodiment of the aforesaid heat-treated asafoetida will be described.

The aforesaid heat-treated asafoetida preferably has a water activity measured at 25 °C of 0.70 or less, more preferably 0.60 or less, especially preferably 0.50 or less.

The aforesaid heat-treated asafoetida, in a more preferred embodiment, contains an increased amount of one or more selected from cyclic (Gly-Thr), cyclic (Gly-His), cyclic (Ala-Asp), cyclic (Val-Ser), cyclic (Gly-Arg), cyclic (Ala-His), cyclic (Glu-Asp), cyclic (Phe-Phe), cyclic (Leu/Ile-Leu/Ile), cyclic (Ala-Arg), cyclic (Leu/Ile-Val), cyclic (Phe-Pro), cyclic (Phe-Leu/Ile), cyclic (Pro-Pro), cyclic (Glu-Phe), cyclic (Arg-Leu/Ile), cyclic (Pro-Leu/Ile), cyclic (Pro-Glu), cyclic (Glu-Leu/Ile), cyclic (Ala-Pro), cyclic (Pro-Val), cyclic (Pro-His), tartaric acid, malic acid, citric acid, and sulfurol(4-methyl-5-thiazoleethanol) in comparison with the asafoetida before the heat-treatment. The present inventors have found that the amount of the aforesaid compounds included in the aforesaid heat-treated asafoetida correlates with the strength of the flavor-enhancing effect.

That is, in a more preferred embodiment, when the aforesaid heat-treated asafoetida, with the addition of 47 μg/g of L-lysine-$^{13}C_6$ monohydrochloride, is analyzed by liquid chromatography-mass spectrometry (LC-MS/MS) according to the method described below, the aforesaid heat-treated asafoetida satisfies one or more, more preferably ten or more, even more preferably twenty or more, especially preferably thirty or more, especially preferably forty or more, and most preferably all, of the following:

(1) a peak area of 0.028 or more, and more preferably 0.028 or more and 0.40 or less derived from cyclic (Gly-Thr),
(2) a peak area of 0.003 or more, and more preferably 0.003 or more and 0.40 or less derived from cyclic (Gly-His),
(3) a peak area of 0.022 or more, and more preferably 0.022 or more and 0.30 or less derived from cyclic (Ala-Asp),
(4) a peak area of 0.040 or more, and more preferably 0.040 or more and 1.0 or less derived from cyclic (Val-Ser),
(5) a peak area of 0.020 or more, and more preferably 0.60 or less derived from cyclic (Gly-Arg),
(6) a peak area of 0.005 or more, and more preferably 0.020 or more and 0.15 or less derived from cyclic (Ala-His),
(7) a peak area of 0.008 or more, and more preferably 0.008 or more and 0.15 or less derived from cyclic (Glu-Asp),
(8) a peak area of 0.050 or more, and more preferably 0.050 or more and 1.0 or less derived from cyclic (Phe-Phe),
(9) a peak area of 0.025 or more, and more preferably 0.025 or more and 0.40 or less derived from cyclic (Leu/Ile-Leu/Ile),
(10) a peak area of 0.025 or more, and more preferably 0.025 or more and 0.50 or less derived from cyclic (Ala-Arg),
(11) a peak area of 0.020 or more, and more preferably 0.020 or more and 0.25 or less derived from cyclic (Leu/Ile-Val),
(12) a peak area of 0.015 or more, and more preferably 0.015 or more and 0.30 or less derived from cyclic (Phe-Pro),
(13) a peak area of 0.040 or more, and more preferably 0.040 or more and 0.55 or less derived from cyclic (Phe-Leu/Ile),
(14) a peak area of 0.010 or more, and more preferably 0.010 or more and 0.30 or less derived from cyclic (Pro-Pro),
(15) a peak area of 0.050 or more, and more preferably 0.050 or more and 2.0 or less derived from cyclic (Glu-Phe),
(16) a peak area of 0.045 or more, and more preferably 0.045 or more and 0.55 or less of derived from cyclic (Arg-Leu/Ile),
(17) a peak area of 0.080 or more, and more preferably 0.080 or more and 1.10 or less derived from cyclic (Pro-Leu/Ile),
(18) a peak area of 0.005 or more, and more preferably 0.005 or more and 0.20 or less of derived from cyclic (Pro-Glu),

(19) a peak area of 0.015 or more, and more preferably 0.015 or more and 0.20 or less derived from cyclic (Glu-Leu/Ile),

(20) a peak area of 0.028 or more, and more preferably 0.028 or more and 0.35 or less derived from cyclic (Ala-Pro),

(21) a peak area of 0.020 or more, and more preferably 0.020 or more and 0.40 or less of derived from cyclic (Pro-Val),

(22) a peak area of 0.040 or more, and more preferably 0.040 or more and 0.80 or less derived from cyclic (Pro-His) and

(23) a peak area of 0.25 or more, and more preferably 0.25 or more and 3.5 or less derived from sulfurol(4-methyl-5-thiazoleethanol),

as a ratio to a peak area derived from L-lysine-$^{13}C_6$ monohydrochloride in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated asafoetida (mass converted under the assumption that the asafoetida resin content is 12% by mass) and 5 mL of water is kept heated in a thermostatic water bath at 80 °C for 30 minutes, to prepare a water extract. To the aforesaid water extract in the aforesaid test tube are added 5 mL of acetonitrile and L-lysine-$^{13}C_6$ monohydrochloride in an amount of 47 $\mu$g/g with respect to the aforesaid heat-treated asafoetida (mass converted under the assumption that the asafoetida resin content is 12% by mass), followed by stirring, and then a solid portion is removed and a liquid portion is collected. After contacting the aforesaid liquid portion with a solid-phase extraction carrier that carries an octadecyl group, an eluate by acetonitrile is collected, to prepare a specimen sample from the aforesaid eluate. The aforesaid specimen sample is analyzed by LC-MS/MS, to obtain the chromatogram. In this context, the aforesaid heat-treated asafoetida used as the analysis sample is preferably a pulverized product.

The peak area in an extracted ion chromatogram for respective product ions with an m/z value described in Examples can be used as the peak area derived from L-lysine-$^{13}C_6$ monohydrochloride and the peak area of respective compounds in aforesaid (1) to (23).

In a particularly preferred embodiment of the aforesaid heat-treated asafoetida, the heat-treated asafoetida satisfies one or more, more preferably ten or more, even more preferably twenty or more, and most preferably all, of the following:

(1') a peak area of 0.040 or more, and more preferably 0.040 or more and 0.40 or less of derived from cyclic (Gly-Thr),

(2') a peak area of 0.004 or more, and more preferably 0.004 or more and 0.40 or less derived from cyclic (Gly-His),

(3') a peak area of 0.030 or more, and more preferably 0.030 or more and 0.30 or less derived from cyclic (Ala-Asp),

(4') a peak area of 0.10 or more, and more preferably 0.10 or more and 1.0 or less derived from cyclic (Val-Ser),

(5') a peak area of 0.060 or more, and more preferably 0.060 or more and 0.60 or less derived from cyclic (Gly-Arg),

(6') a peak area of 0.015 or more, and more preferably 0.015 or more and 0.15 or less derived from cyclic (Ala-His),

(7') a peak area of 0.012 or more, and more preferably 0.012 or more and 0.15 or less derived from cyclic (Glu-Asp),

(8') a peak area of 0.10 or more, and more preferably 0.10 or more and 1.0 or less derived from cyclic (Phe-Phe),

(9') a peak area of 0.040 or more, and more preferably 0.040 or more and 0.40 or less derived from cyclic (Leu/Ile-Leu/Ile),

(10') a peak area of 0.050 or more, and more preferably 0.050 or more and 0.50 or less derived from cyclic (Ala-Arg),

(11') a peak area of 0.025 or more, and more preferably 0.025 or more and 0.25 or less derived from cyclic (Leu/Ile-Val),

(12') a peak area of 0.030 or more, and more preferably 0.030 or more and 0.30 or less derived from cyclic (Phe-Pro),

(13') a peak area of 0.055 or more, and more preferably 0.055 or more and 0.55 or less derived from cyclic (Phe-Leu/Ile),

(14') a peak area of 0.030 or more, and more preferably 0.030 or more and 0.30 or less derived from cyclic (Pro-Pro),

(15') a peak area of 0.20 or more, and more preferably 0.20 or more and 2.0 or less derived from cyclic (Glu-Phe),

(16') a peak area of 0.055 or more, and more preferably 0.055 or more and 0.55 or less derived from cyclic (Arg-Leu/Ile),

(17') a peak area of 0.100 or more, and more preferably 0.100 or more and 1.10 or less derived from cyclic (Pro-Leu/Ile),

(18') a peak area of 0.020 or more, and more preferably 0.020 or more and 0.20 or less derived from cyclic (Pro-Glu),

(19') a peak area of 0.020 or more, and more preferably 0.020 or more and 0.20 or less derived from cyclic (Glu-Leu/Ile),

(20') a peak area of 0.035 or more, and more preferably 0.035 or more and 0.35 or less derived from cyclic (Ala-Pro),

(21') a peak area of 0.040 or more, and more preferably 0.040 or more and 0.40 or less derived from cyclic (Pro-Val),

(22') a peak area of 0.080 or more, and more preferably 0.080 or more and 0.80 or less derived from cyclic (Pro-His) and

(23') a peak area of 0.35 or more, and more preferably 0.35 or more and 3.5 or less derived from sulfurol(4-methyl-5-thiazoleethanol),
as a ratio to a peak area derived from L-lysine-$^{13}C_6$ monohydrochloride in the chromatogram described above.

In addition, the aforesaid heat-treated asafoetida, in a more preferred embodiment, is characterized in that it contains one or more selected from the group consisting of cyclic (Val-Ser), cyclic (Pro-Pro) and cyclic (Pro-Glu), and more preferably all members of the aforesaid group. These cyclic dipeptides are characteristic constituents of the heat-treated asafoetida having the flavor-enhancing effect, which are not detected in unheated asafoetida.

In a more preferred embodiment, when the aforesaid heat-treated asafoetida, with the addition of 99 μg/g of ribitol, is analyzed by liquid chromatography-mass spectrometry (LC-MS/MS) according to the method described below, the aforesaid asafoetida satisfies one or more, more preferably two or more, and most preferably all, of the following:

(24) a peak area of 0.12 or more, and more preferably 0.12 or more and 1.5 or less derived from tartaric acid,
(25) a peak area of 1.10 or more, and more preferably 1.10 or more and 13.0 or less derived from malic acid and
(26) a peak area of 1.00 or more, and more preferably 1.00 or more and 20.0 or less derived from citric acid,
as a ratio to a peak area derived from ribitol in a chromatogram obtained.

In this context, the measurement method of LC-MS/MS is as follows, and more preferably, it is the measurement method of LC-MS/MS described in Examples.

A 10-mL test tube containing 200 mg of the aforesaid heat-treated asafoetida (mass converted under the assumption that the asafoetida resin content is 12% by mass) and 5 mL of water is kept heated in a thermostatic water bath at 80 °C for 30 minutes, to prepare a water extract. To the aforesaid water extract in the aforesaid test tube are added 5 mL of acetonitrile and ribitol in an amount of 99 μg/g with respect to the aforesaid heat-treated asafoetida (mass converted under the assumption that the asafoetida resin content is 12% by mass), followed by stirring, and then a solid portion is removed and a liquid portion is collected. After contacting the aforesaid liquid portion with a solid-phase extraction carrier that carries an octadecyl group, an eluate by acetonitrile is collected, to prepare a specimen sample from the aforesaid eluate. The aforesaid specimen sample is analyzed by LC-MS/MS, to obtain the chromatogram. In this context, the aforesaid heat-treated asafoetida used as the analysis sample is preferably a pulverized product.

The peak area in an extracted ion chromatogram for respective product ions with an m/z value described in Examples can be used as the peak area derived from ribitol, the peak area derived from tartaric acid, the peak area derived from malic acid and the peak area derived from citric acid.

In a particularly preferred embodiment of the aforesaid heat-treated asafoetida, the heat-treated asafoetida satisfies one or more, more preferably two or more, and most preferably all, of the following:

(24') a peak area of 0.15 or more, and more preferably 0.15 or more and 1.5 or less derived from tartaric acid,
(25') a peak area of 1.30 or more, and more preferably 1.30 or more and 13.0 or less derived from malic acid and
(26') a peak area of 2.00 or more, and more preferably 2.00 or more and 20.0 or less derived from citric acid,
as a ratio to a peak area derived from ribitol in the chromatogram described above.

The heat-treated asafoetida in the flavor-enhancing composition according to this embodiment is preferably in the form of powder including the aforesaid additive, and the particle size is preferably 1000 μm or less. The particle size can be determined by the opening of a standard sieve as specified in JIS. The heat-treated asafoetida in the flavor-enhancing composition according to this embodiment may also be provided in the form of a mixture of the heat-treated asafoetida and oil or fat. The aforesaid mixture may be solid at room temperature or liquid at room temperature, depending on the melting point of the aforesaid oil or fat.

The flavor-enhancing composition according to this embodiment may consist only of the heat-treated asafoetida or may include the heat-treated asafoetida and an additional material. The additional material may be exemplified by one or more materials having the flavor-enhancing effect and one or more materials acceptable as food products. The flavor-enhancing composition according to this embodiment can contain the heat-treated asafoetida at a percentage of preferably 30% by mass or more and 100% by mass or less, more preferably 50% by mass or more and 100% by mass or less, even more preferably 80% by mass or more 100% by mass or less, most preferably 95% by mass or more and 100% by mass or less. The flavor-enhancing composition according to this embodiment can be in the form of powder, granule, paste, liquid, etc., and can include one or more materials acceptable as food products, such as excipients, carriers, etc., as necessary, in order to achieve a desired form of the composition.

<Method for Producing Flavor-enhancing Composition>

A second embodiment of the third aspect of the present invention relates to

a method for producing the flavor-enhancing composition according to the first embodiment of the third aspect of the present invention, comprising
heating asafoetida without the addition of oil or fat under a condition whereby the heating value of is 15 or more to obtain the aforesaid heat-treated asafoetida.

A third embodiment of the third aspect of the present invention relates to

a method for producing the flavor-enhancing composition according to the first embodiment of the third aspect of the present invention, comprising
heating asafoetida together with oil or fat under a condition whereby the heating value is 5 or more to obtain the aforesaid heat-treated asafoetida.

According to the second or third embodiment of the third aspect of the present invention, the flavor-enhancing composition according to the first embodiment of the third aspect of the present invention can be produced.
In the second embodiment of the third aspect of the present invention, the step of heating asafoetida without the addition of oil or fat under a condition whereby the heating value is 15 or more to obtain the aforesaid heat-treated asafoetida is preferably provided with the feature described with regard to the aforesaid first heat-treatment for obtaining the heat-treated asafoetida of the flavor-enhancing composition according to the first embodiment of the third aspect of the present invention.
In the third embodiment of the third aspect of the present invention, the step of heating the asafoetida together with oil or fat under a condition whereby the heating value is 5 or more to obtain the aforesaid heat-treated asafoetida is preferably provided with the feature described with regard to the aforesaid second heat-treatment for obtaining the heat-treated asafoetida of the flavor-enhancing composition according to the first embodiment of the third aspect of the present invention.
The method for producing a flavor-enhancing composition according to the second or third embodiment of the third aspect of the present invention can further comprise pulverizing the aforesaid heat-treated asafoetida. A preferred range of the particle size of the aforesaid heat-treated asafoetida after pulverizing is as described with regard to the aforesaid heat-treated asafoetida of the flavor-enhancing composition according to the first embodiment of the third aspect of the present invention.
The method for producing a flavor-enhancing composition according to the second or third embodiment of the third aspect of the present invention may use the aforesaid heat-treated asafoetida as is, or may further comprise preparing the aforesaid flavor-enhancing composition by combining the aforesaid heat-treated asafoetida with an additional material. Preferred examples of the aforesaid additional material are as described with regard to the flavor-enhancing composition according to the first embodiment of the third aspect of the present invention.
The method for producing a flavor-enhancing composition according to this embodiment may comprise processing the obtained flavor-enhancing composition into the form of powder, granule, paste, liquid, etc.

<Method for Enhancing Flavor>

A fourth embodiment of the third aspect of the present invention relates to
a method for enhancing the flavor of a food product, comprising blending the flavor-enhancing composition according to the first embodiment of the third aspect of the present invention.
As the method according to this embodiment can enhance the flavor of food products, it can be suitably used to enhance the flavor of a food product including one or more of the above-described flavor constituents in a reduced amount from usual (e.g., a reduced-sodium food product in which the food product is reduced from usual).
The blending amount of the flavor-enhancing composition according to the first embodiment of the third aspect of the present invention is not particularly limited and can be adjusted appropriately, according to the form of the food product. For example, for the purpose of enhancing saltiness, the flavor-enhancing composition according to the first embodiment of the third aspect of the present invention can be blended so that the amount of the heat-treated asafoetida may be, for example, 0.5 g or more, preferably 1 g or more, more preferably 3 g or more, and for example 0.5 g or more and 50 g or less, preferably 1 g or more and 20 g or less, more preferably 3 g or more and 10 g or less per 10,000 g of the sodium chloride equivalent of the food product.
The type of the food product is not limited, but the examples are curry sauces, stew sauces, meat products, prepared foods and confectionery. The aforesaid food product may be a food product including one or more of such flavor constituents as described above in a reduced amount from usual. The aforesaid food product may be a food product containing one or more flavor constituents as described above, e.g., table salt.

<One or More Further Embodiments>

One or more further embodiments of the third aspect of the present invention relates to

use of heat-treated asafoetida, for enhancing the flavor of a food product,
a method for enhancing the flavor of a food product, comprising blending the heat-treated asafoetida with the food product,
heat-treated asafoetida for use in enhancing the flavor of a food product; or
use of heat-treated asafoetida in producing an additive for use in enhancing the flavor of a food product.

In this embodiment, the aforesaid heat-treated asafoetida is preferably provided with the feature described for the heat-treated asafoetida included in the flavor-enhancing composition according to the first embodiment of the third aspect of the present invention. In this embodiment, the aforesaid heat-treated asafoetida can preferably be produced by the method for producing the heat-treated asafoetida described in the method for producing a flavor-enhancing composition according to the second embodiment of the third aspect of the present invention. In this embodiment, the aforesaid food product is preferably provided with the feature described with regard to the method of the third embodiment of the third aspect of the present invention.

<4. Fourth Aspect of Present Invention>

In this section, a preferred embodiment of the fourth aspect of the present invention will be described.

<Heat-treated Mustard Seed>

A first embodiment of the fourth aspect of the present invention relates to

heat-treated mustard seed, wherein
when the aforesaid mustard seed (representing a mass converted to that of the mustard seed excluding oil or fat when the aforesaid heat-treated mustard seed is mustard seed heat-treated together with the oil or fat), with the addition of 47 μg/g of L-lysine-$^{13}C_6$ monohydrochloride, is analyzed by liquid chromatography-mass spectrometry (LC-MS/MS) according to the method described below, the heat-treated mustard seed satisfies one or more, more preferably five or more, more preferably ten or more, more preferably fifteen or more, more preferably twenty or more, and most preferably all, of the following:

(1) a peak area of 0.20 or more, and more preferably 0.20 or more and 10.0 or less, even more preferably 0.20 or more and 3.0 or less derived from cyclic (Glu-His),
(2) a peak area of 0.29 or more, and more preferably 0.29 or more and 6.0 or less, even more preferably 0.29 or more and 2.0 or less derived from cyclic (Gly-Arg),
(3) a peak area of 0.15 or more, and more preferably 0.15 or more and 6.0 or less, even more preferably 0.15 or more and 2.0 or less derived from cyclic (Glu-Gly),
(4) a peak area of 0.12 or more, and more preferably 0.12 or more and 3.0 or less, even more preferably 0.12 or more and 1.0 or less derived from cyclic (Ala-His),
(5) a peak area of 0.20 or more, and more preferably 0.20 or more and 5.0 or less, even more preferably 0.20 or more and 1.5 or less derived from cyclic (Ala-Arg),
(6) a peak area of 0.13 or more, and more preferably 0.13 or more and 5.0 or less, even more preferably 0.13 or more and 1.5 or less derived from cyclic (Glu-Asp),
(7) a peak area of 0.035 or more, and more preferably 0.035 or more and 1.0 or less, even more preferably 0.035 or more and 0.30 or less derived from cyclic (Leu/Ile-Asp),
(8) a peak area of 0.50 or more, and more preferably 0.50 or more and 12.0 or less, even more preferably 0.50 or more and 4.0 or less derived from cyclic (Thr-Pro),
(9) a peak area of 0.09 or more, and more preferably 0.09 or more and 3.0 or less, even more preferably 0.09 or more and 1.0 or less derived from cyclic (Pro-His),
(10) a peak area of 0.50 or more, and more preferably 0.50 or more and 13.0 or less, even more preferably 0.50 or more and 4.0 or less derived from cyclic (Pro-Pro),
(11) a peak area of 0.070 or more, and more preferably 0.070 or more and 4.5 or less, even more preferably 0.070 or more and 1.5 or less derived from cyclic (Glu-Tyr),
(12) a peak area of 0.020 or more, and more preferably 0.020 or more and 3.6 or less, even more preferably 0.020 or more and 1.2 or less derived from cyclic (Arg-Leu/Ile),

(13) a peak area of 0.70 or more, and more preferably 0.70 or more and 15.0 or less, even more preferably 0.70 or more and 4.0 or less derived from cyclic (Ala-Pro),
(14) a peak area of 0.65 or more, and more preferably 0.65 or more and 25.0 or less, even more preferably 0.65 or more and 8.0 or less derived from cyclic (Pro-Val),
(15) a peak area of 0.015 or more, preferably 0.060 or more, and more preferably 0.015 or more and 1.7 or less, even more preferably 0.060 or more and 0.5 or less derived from cyclic (Phe-Ala),
(16) a peak area of 0.070 or more, and more preferably 0.070 or more and 2.4 or less, even more preferably 0.070 or more and 0.8 or less derived from cyclic (Phe-Pro),
(17) a peak area of 0.18 or more, and more preferably 0.18 or more and 3.0 or less, even more preferably 0.18 or more and 0.9 or less derived from cyclic (Phe-Tyr),
(18) a peak area of 0.02 or more, and more preferably 0.02 or more and 0.60 or less, even more preferably 0.02 or more and 0.15 or less derived from cyclic (Tyr-His),
(19) a peak area of 0.05 or more, and more preferably 0.05 or more and 0.6 or less, even more preferably 0.05 or more and 0.2 or less derived from cyclic (Leu/Ile-His),
(20) a peak area of 0.32 or more, and more preferably 0.32 or more and 7.5 or less, even more preferably 0.32 or more and 2.5 or less derived from cyclic (Pro-Met) and
(21) a peak area of 0.10 or more, and more preferably 0.10 or more and 9.0 or less, even more preferably 0.10 or more and 3.0 or less derived from cyclic (Val-Glu),

as a ratio to a peak area derived from L-lysine-$^{13}C_6$ monohydrochloride in a chromatogram obtained.

The present inventors have found that the amount of the aforesaid cyclic dipeptides in the aforesaid heat-treated mustard seed relate to sweetness and roasted flavor, as well as the strength of the flavor-enhancing effect. The heat-treated mustard seed according to this embodiment exhibits increased sweetness and roasted flavor, and can enhance their flavor of food products when blended therewith. The aforesaid flavor is, for example, richness.

In this specification, mustard seed is the seed of brown mustard (*Brassica juncea*), black mustard (*Brassica nigra*) or white mustard (*Sinapis alba*), and is preferably the seed of brown mustard. The heat-treated mustard seed according to this embodiment can be produced by heat-treating the mustard seed in the form of whole seed, and may be pulverized accordingly after the heat-treatment.

In this embodiment, the measurement method of LC-MS/MS is as follows, and more preferably, it is the measurement method of LC-MS/MS described in Examples.

In this specification, operations where temperature is not specified can be carried out at room temperature, specifically at 23 °C.

(Measurement method of LC-MS/MS)

A 10-mL test tube containing 200 mg of the heat-treated mustard seed (representing a mass converted to that of the mustard seed excluding oil or fat when the aforesaid heat-treated mustard seed is mustard seed heat-treated together with the oil or fat) and 5 mL of water is kept heated in a thermostatic water bath at 80 °C for 30 minutes, to prepare a water extract. To the aforesaid water extract in the aforesaid test tube are added 5 mL of acetonitrile and L-lysine-$^{13}C_6$ monohydrochloride in an amount of 47 μg/g with respect to the aforesaid heat-treated mustard seed (representing a mass converted to that of the mustard seed excluding oil or fat when the aforesaid heat-treated mustard seed is mustard seed heat-treated together with the oil or fat), followed by stirring, and then a solid portion is removed and a liquid portion is collected, to prepare a LC-MS/MS specimen sample. The aforesaid LC-MS/MS specimen sample is analyzed by LC-MS/MS, to obtain a chromatogram. In this context, the aforesaid heat-treated mustard seed used as the analysis sample is preferably a pulverized product.

The peak area in an extracted ion chromatogram for respective product ions with an m/z value described in Examples can be used as the peak area derived from L-lysine-$^{13}C_6$ monohydrochloride and the peak area of respective cyclic dipeptides described in aforesaid (1) to (21).

In a particularly preferred embodiment of the aforesaid heat-treated mustard seed, the heat-treated mustard seed satisfies one or more, more preferably five or more, more preferably ten or more, more preferably fifteen or more, more preferably twenty or more, most preferably all, of the following:

(1') a peak area of 0.30 or more, and more preferably 0.30 or more and 10.0 or less, even more preferably 0.30 or more and 3.0 or less derived from cyclic (Glu-His),
(2') a peak area of 0.30 or more, and more preferably 0.30 or more and 6.0 or less, even more preferably 0.30 or more and 2.0 or less derived from cyclic (Gly-Arg),
(3') a peak area of 0.30 or more, and more preferably 0.15 or more and 6.0 or less, even more preferably 0.15 or more and 2.0 or less derived from cyclic (Glu-Gly),
(4') a peak area of 0.20 or more, and more preferably 0.20 or more and 3.0 or less, even more preferably 0.20 or more and 1.0 or less derived from cyclic (Ala-His),
(5') a peak area of 0.30 or more, and more preferably 0.30 or more and 5.0 or less, even more preferably 0.30 or more and 1.5 or less derived from cyclic (Ala-Arg),
(6') a peak area of 0.20 or more, and more preferably 0.20 or more and 5.0 or less, even more preferably 0.20 or more and 1.5 or less derived from cyclic (Glu-Asp),
(7') a peak area of 0.060 or more, and more preferably 0.060 or more and 1.0 or less, even more preferably 0.060 or more and 0.30 or less derived from cyclic (Leu/Ile-Asp),
(8') a peak area of 0.60 or more, and more preferably 0.60 or more and 12.0 or less, even more preferably 0.60 or more and 4.0 or less derived from cyclic (Thr-Pro),
(9') a peak area of 0.60 or more, and more preferably 0.60 or more and 3.0 or less, even more preferably 0.60 or more and 1.0 or less derived from cyclic (Pro-His),
(10') a peak area of 0.80 or more, and more preferably 0.80 or more and 13.0 or less, even more preferably 0.80 or more and 4.0 or less derived from cyclic (Pro-Pro),
(11') a peak area of 0.090 or more, and more preferably 0.090 or more and 4.5 or less, even more preferably 0.090 or more and 1.5 or less derived from cyclic (Glu-Tyr),
(12') a peak area of 0.020 or more, and more preferably 0.020 or more and 3.6 or less, even more preferably 0.020 or more and 1.2 or less derived from cyclic (Arg-Leu/Ile),
(13') a peak area of 0.80 or more, and more preferably 0.80 or more and 15.0 or less, even more preferably 0.80 or more and 4.0 or less derived from cyclic (Ala-Pro),
(14') a peak area of 1.50 or more, and more preferably 1.50 or more and 25.0 or less, even more preferably 1.50 or more and 8.0 or less derived from cyclic (Pro-Val),
(15') a peak area of 0.10 or more, and more preferably 0.10 or more and 1.7 or less, even more preferably 0.10 or more and 0.5 or less derived from cyclic (Phe-Ala),
(16') a peak area of 0.10 or more, and more preferably 0.10 or more and 2.4 or less, even more preferably 0.10 or more and 0.8 or less derived from cyclic (Phe-Pro),
(17') a peak area of 0.19 or more, and more preferably 0.19 or more and 3.0 or less, even more preferably 0.19 or more and 0.9 or less derived from cyclic (Phe-Tyr),
(18') a peak area of 0.04 or more, and more preferably 0.04 or more and 0.60 or less, even more preferably 0.04 or more and 0.15 or less derived from cyclic (Tyr-His),
(19') a peak area of 0.15 or more, and more preferably 0.15 or more and 0.6 or less, even more preferably 0.15 or more and 0.2 or less derived from cyclic (Leu/Ile-His),
(20') a peak area of 0.40 or more, and more preferably 0.40 or more and 7.5 or less, even more preferably 0.40 or more and 2.5 or less derived from cyclic (Pro-Met) and
(21') a peak area of 0.30 or more, and more preferably 0.30 or more and 9.0 or less, even more preferably 0.30 or more and 3.0 or less derived from cyclic (Val-Glu),
as a ratio to a peak area derived from L-lysine-$^{13}C_6$ monohydrochloride in the chromatogram described above.

In a more preferred embodiment, when the aforesaid heat-treated mustard seed according to this embodiment (representing a mass converted to that of the mustard seed excluding oil or fat when the aforesaid heat-treated mustard seed is mustard seed heat-treated together with the oil or fat), with the addition of 625 μg/g of 4-methylthiazole, is analyzed by gas chromatography-mass spectrometry (GC-MS) according to the method described below, the heat-treated mustard seed satisfies one or more, more preferably five or more, more preferably ten or more, most preferably all, of the following:

(22) a peak area of 0.006 or more, and more preferably 0.006 or more and 0.20 or less, even more preferably 0.006 or more and 0.60 or less derived from allylmethyl disulfide,
(23) a peak area of 0.60 or more, and more preferably 0.60 or more and 10.0 or less, even more preferably 0.60 or more and 3.0 or less derived from 2,5-dimethylpyrazine,
(24) a peak area of 0.15 or more, and more preferably 0.15 or more and 3.0 or less, even more preferably 0.15 or more and 1.0 or less derived from 2-ethyl-6-methylpyrazine,
(25) a peak area of 0.15 or more, and more preferably 0.15 or more and 3.0 or less, even more preferably 0.15 or more and 1.0 or less derived from 2,3,5-trimethylpyrazine,
(26) a peak area of 0.01 or more, and more preferably 0.01 or more and 0.30 or less, even more preferably 0.01 or more and 0.10 or less derived from 2,6-diethylpyrazine,
(27) a peak area of 0.20 or more, and more preferably 0.20 or more and 10.0 or less, even more preferably 0.20 or more and 3.0 or less derived from 2,5-diethylpyrazine,
(28) a peak area of 0.03 or more, and more preferably 0.03 or more and 0.70 or less, even more preferably 0.03 or more and 0.25 or less derived from 2,3-diethylpyrazine,

(29) a peak area of 0.025 or more, and more preferably 0.025 or more and 1.0 or less, even more preferably 0.025 or more and 0.30 or less derived from allylmethyl trisulfide,

(30) a peak area of 0.70 or more, and more preferably 0.70 or more and 15.0 or less, even more preferably 0.70 or more and 4.5 or less derived from 3-methyl-1,2,4-trithiane,

(31) a peak area of 0.10 or more, and more preferably 0.10 or more and 2.0 or less, even more preferably 0.10 or more and 0.6 or less of derived from 2-dodecenal and

(32) a peak area of 0.50 or more, and more preferably 0.50 or more and 10.0 or less, even more preferably 0.50 or more and 3.0 or less derived from sulfurol,

as a ratio to a peak area derived from 4-methylthiazole in a chromatogram obtained.

In this context, the measurement method of GC-MS is as follows, and more preferably, it is the measurement method of GC-MS described in the Examples. In this specification, operations where temperature is not specified can be carried out at room temperature, specifically at 23 °C.

(Measurement method of GC-MS)

Fifteen milligrams of the aforesaid heat-treated mustard seed (representing a mass converted to that of the mustard seed excluding oil or fat when the aforesaid heat-treated mustard seed is mustard seed heat-treated together with the oil or fat), 4-methylthiazole in an amount of 625 μg/g with respect to the aforesaid heat-treated mustard seed (representing a mass converted to that of the mustard seed excluding oil or fat when the aforesaid heat-treated mustard seed is mustard seed heat-treated together with the oil or fat), 5 mL of acetone and 5 mL of methanol contained in a 10-mL test tube are stirred, and then a solid portion is removed and a liquid portion is collected, to which is added 1 mL of acetone per 0.1 mL of the liquid portion, to prepare a GC-MS specimen sample. The aforesaid GC-MS specimen sample is analyzed by GC-MS, to obtain a chromatogram. In this context, the aforesaid heat-treated mustard seed used as the analysis sample is preferably a pulverized product.

The peak area in an extracted ion chromatogram for ions corresponding to the precise mass of respective constituents described in the Examples may be used as the peak area derived from 4-methylthiazole and the peak area derived from the respective constituents specified in aforesaid (22) to (32).

The present inventors have found that the amount of respective constituents specified in aforesaid (22) to (32) included in the aforesaid heat-treated mustard seed relates to the strength of the enhancing effect of flavors, including sweetness, roasted flavor and richness. The heat-treated mustard seed satisfying one or more of aforesaid (22) to (32) exhibits particularly increased sweetness and roasted flavor, and has a particularly strong effect of enhancing a flavor such as richness, when blended with a food product.

In a particularly preferred embodiment of the aforesaid heat-treated mustard seed, the heat-treated mustard seed satisfies one or more, more preferably five or more, more preferably ten or more, and most preferably all, of the following:

(22') a peak area of 0.010 or more, and preferably 0.010 or more and 0.20 or less, more preferably 0.010 or more and 0.60 or less derived from allylmethyl disulfide,

(23') a peak area of 0.70 or more, and more preferably 0.70 or more and 10.0 or less, even more preferably 0.70 or more and 3.0 or less derived from 2,5-dimethylpyrazine,

(24') a peak area of 0.19 or more, and more preferably 0.19 or more and 3.0 or less, more preferably 0.19 or more and 1.0 or less derived from 2-ethyl-6-methylpyrazine,

(25') a peak area of 0.20 or more, and more preferably 0.20 or more and 3.0 or less, even more preferably 0.20 or more and 1.0 or less derived from 2,3,5-trimethylpyrazine,

(26') a peak area of 0.015 or more, and more preferably 0.015 or more and 0.30 or less, even more preferably 0.015 or more and 0.10 or less derived from 2,6-diethylpyrazine,

(27') a peak area of 0.30 or more, and more preferably 0.30 or more and 10.0 or less, even more preferably 0.30 or more and 3.0 or less derived from 2,5-diethylpyrazine,

(28') a peak area of 0.04 or more, and more preferably 0.04 or more and 0.70 or less, even more preferably 0.04 or more and 0.25 or less derived from 2,3-diethylpyrazine,

(29') a peak area of 0.030 or more, and more preferably 0.030 or more and 1.0 or less, even more preferably 0.030 or more and 0.30 or less derived from allylmethyl trisulfide,

(30') a peak area of 0.90 or more, and more preferably 0.90 or more and 15.0 or less, even more preferably 0.90 or more and 4.5 or less derived from 3-methyl-1,2,4-trithiane,

(31') a peak area of 0.12 or more, and more preferably 0.12 or more and 2.0 or less, even more preferably 0.12 or more and 0.6 or less derived from 2-dodecenal and

(32') a peak area of 0.60 or more, and more preferably 0.60 or more and 10.0 or less, even more preferably 0.60 or more and 3.0 or less derived from sulfurol,
as a ratio to a peak area derived from 625 μg/g of 4-methylthiazole in the chromatogram described above.

The heat-treated mustard seed according to this embodiment preferably has a water activity measured at 25 °C of 0.50 or less, more preferably 0.20 or less, and the lower limit is not particularly limited, but is for example 0.02 or more.

The heat-treated mustard seed according to this embodiment may be provided in the form of whole seed or in the form of powder. When the heat-treated mustard seed is in the form of powder, the particle size is not particularly limited, but can be, for example, 1000 μm or less, preferably 500 μm or less. The particle size can be determined by the opening of a standard sieve as specified in JIS.

The heat-treated mustard seed according to this embodiment can be blended with a food product together with an additional material to form a food product composition. The additional material only has to be one or more materials acceptable as food products. The aforesaid food product composition can contain the heat-treated mustard seed according to this embodiment in a percentage of preferably 30% by mass or more and 100% by mass or less, more preferably 50% by mass or more and 100% by mass or less, more preferably 80% by mass or more and 100% by mass or less, most preferably 95% by mass or more and 100% by mass or less. The aforesaid food product composition can be in the form of powder, granule, paste, liquid, etc., and can include one or more materials acceptable as food products, such as excipients, carriers, etc., as necessary, to achieve a desired form of the composition.

<Method for Producing Heat-treated Mustard Seed>

A second embodiment of the fourth aspect of the present invention relates to

a method for producing heat-treated mustard seed, comprising
heat-treating the mustard seed in an open system under a condition whereby the heating value is 200 or more.

According to this embodiment, mustard seed having enhanced sweetness and roasted flavor, which can enhance a flavor such as richness when blended in a food product, can be produced. The heat-treated mustard seed produced according to this embodiment is preferably the heat-treated mustard seed according to the first embodiment of the fourth aspect of the present invention.

For the heat-treatment of the mustard seed in this embodiment, its heating temperature and time period are set so that the heating value may be 200 or more, more preferably 600 or more, even more preferably 800 or more, most preferably 950 or more. Furthermore, the temperature and time period are preferably set so that the heating value may be preferably 200 or more and 10000 or less, more preferably 600 or more and 6000 or less, even more preferably 800 or more and 6000 or less, most preferably 950 or more and 6000 or less. Mustard seed heat-treated in an open system under a condition whereby the heating value is within the aforesaid range exhibits significantly increased sweetness and roasted flavor in comparison with mustard seed heat-treated under a condition whereby the heating value of 200 or less, and can enhance flavory such as richness when blended with a food product. The heating condition whereby the heating value is within the aforesaid range can preferably comprise heating under the temperature and time period condition described below.

In this context, the definition of the heating value and the CV value is as described with regard to the first embodiment of the first aspect of the present invention.

In the heat-treatment in this embodiment, the maximum reached temperature of the temperature of the mustard seed (product temperature) can be, for example, 130 °C or higher, preferably 140 °C or higher, more preferably 160 °C or higher, and for example, 130 °C or higher and 200 °C or lower, preferably 140 °C or higher and 200 °C or lower, more preferably 160 °C or higher and 200 °C or lower. In this embodiment, the heating time at a temperature within the temperature range described above can be adjusted accordingly so that the heating value may be within a specified range, and it can be, for example, within the range of time from 3 minutes or longer and 30 minutes or shorter.

The mustard seed to be subjected to the heat-treatment in this embodiment is preferably mustard seed in the form of whole seed.

The heat-treatment of mustard seed in this embodiment is carried out in an open system. The heat-treatment in the open system refers to a heating treatment in an environment that is not sealed and in which moisture and volatile constituents including aromatic constituents, can volatilize into surrounding atmosphere during the heat-treatment. The process of heating mustard seed in oil or fat (roasting in oil) does not fall under the category of the heat-treatment in an open system because the oil prevents the volatilization of moisture and volatile constituents from the mustard seed. The present inventors found that mustard seed heat-treated in an open system at a heating value of 200 or more is superior in sweetness and roasted flavor in comparison with mustard seed heat-treated in a closed container or mustard seed roasted in oil, and has a particularly strong effect of enhancing a flavor such as richness when blended with a food product. Heat-treatment equipment that can be used for the heat-treatment in an open system in this embodiment is exemplified by roasting machines equipped with an open container such as a flat cauldron, a rotary cylindrical cauldron, a pot, etc., ovens with its interior sealed, hot air roasters and the like. Heat-treatment in such an open system can be referred to as "roasting." The heating process in the open system can be carried out under a non-pressurized condition.

The step of heat-treating mustard seed in this embodiment in an open system is preferably a dry heat-treatment step in which the mustard seed is heat-treated without mixing the mustard seed with a liquid portion such as oil or fat, water, etc., and is even more preferably a step in which only mustard seed is heat-treated.

The method for producing heat-treated mustard seed according to this embodiment may further include pulverizing the aforesaid heat-treated mustard seed. A preferred range of the particle size of the aforesaid heat-treated mustard seed after pulverizing is as described with regard to the heat-treated mustard seed according to the first embodiment of the fourth aspect of the present invention.

<Flavor-enhancing Composition>

A third embodiment of the fourth aspect of the present invention relates to
a flavor-enhancing composition containing heat-treated mustard seed.

The present inventors have found that heat-treated mustard seed exhibits increased sweetness and roasted flavor in comparison with unheated mustard seed, and has a stronger effect of enhancing a flavor such as richness of the food products. The flavor-enhancing composition according to this embodiment is more preferably a richness-enhancing composition.

As described above, "richness" in this specification can be expressed as "thickness" or "full-bodied taste" perceived when food products are eaten.

The heat-treated mustard seed included in the flavor-enhancing composition according to this embodiment is preferably mustard seed heat-treated so that the heating value may be 100 or more, more preferably 150 or more, especially preferably 200 or more, and it is more preferably heat-treated mustard seed produced by the method according to the second embodiment of the fourth aspect of the present invention. The definition of the heating value and the more preferred method for heat-treatment is as described with regard to the second embodiment of the fourth aspect of the present invention.

The heat-treated mustard seed included in the flavor-enhancing composition according to this embodiment is especially preferably the heat-treated mustard seed according to the first embodiment of the fourth aspect of the present invention.

<Method for Enhancing Flavor>

A fourth embodiment of the fourth aspect of the present invention relates to
a method for enhancing the flavor of a food product, comprising blending the flavor-enhancing composition according to the third embodiment of the fourth aspect of the present invention with a food product.

As the method according to this embodiment can enhance a flavor such as richness of a food product, it can be suitably used for enhancing a flavor such as richness of a lightly flavored food product having one or more above-described flavor constituents in a reduced used amount from usual.

In the method according to this embodiment, the blending amount of the flavor-enhancing composition according to the third embodiment is not particularly limited and can be adjusted appropriately, according to the form of the food product. For example, the flavor-enhancing composition according to the third embodiment can be blended so that the heat-treated mustard seed may be, for example, 0.1 g or more, preferably 1 g or more, more preferably 2 g or more, and for example, 0.1 g or more and 50 g or less, preferably 1 g or more and 20 g or less, more preferably 2 g or more and 10 g or less per 100 g of the food product.

The type of a food product to be flavor-enhanced is not limited, but it can be exemplified by curry sauces, stew sauces, meat products, prepared foods and confectionery.

<One or More Further Embodiments>

One or more further embodiments of the fourth aspect of the present invention relate to

use of heat-treated mustard seed, for enhancing the flavor of a food product,
a method for enhancing the flavor of a food product, comprising blending the heat-treated mustard seed with the food product,
heat-treated mustard seed for use in enhancing the flavor of a food product, or
use of the heat-treated mustard seed in producing an additive for use in enhancing the flavor of a food product.

In this embodiment, the aforesaid heat-treated mustard seed is preferably provided with the feature described with regard to the heat-treated mustard seed according to the first embodiment of the fourth aspect of the present invention, or the heat-treated mustard seed included in the flavor-enhancing composition according to the third embodiment of the fourth aspect of the invention. In this embodiment, the aforesaid heat-treated mustard seed can preferably be produced by the method for producing heat-treated mustard seed according to the second embodiment of the fourth aspect of the present invention. In this embodiment, the aforesaid food product is preferably provided with the feature described with regard to the method of the fourth embodiment of the fourth aspect of the present invention.

Examples

Experiment 1 described below relates to the first aspect of the present invention.
Experiment 2 described below relates to the second aspect of the present invention.
Experiment 3 described below relates to the third aspect of the present invention.
Experiment 4 described below relates to the fourth aspect of the present invention.

1. Experiment 1: Flavor-enhancing Effect of Heat-treated Paprika

1. 1. Preparation of Heat-treated Paprika

Heat-treated paprika was prepared by heat-treating paprika powder under the conditions shown in the table described below. The heating values are values given by the integral of the CV value according to the aforesaid formula ("reference temperature" is 110 °C and "Z value" is 30 (°C)) with respect to the heating time (min), as previously described. The temperatures and time periods described in the heating condition column are maximum reached temperatures and their holding times, and the heating values were calculated on the basis of temperature-change over time, including the values of the temperature and time during temperature rise and fall.

[Table 1]

| | Heating condition | Heating value | Maturing condition | Aw | Temp. (° C) |
|---|---|---|---|---|---|
| Comp. Ex. 201 | unheated | 0 | | 0.5177 | 25.02 |
| Comp. Ex. 202 | unheated, matured | 0 | kept heated in air at 40° C for 8 days | 0.5177 | 25.13 |
| Ex. 201 | 130° C for 30 min, w/o water | 168 | | 0.6452 | 24.9 |
| Ex. 202 | 130° C for 30 min, w/ water | 168 | | 0.7871 | 24.99 |
| Ex. 203 | 130° C for 60 min, w/o water | 264 | | 0.6695 | 24.84 |
| Ex. 204 | 130° C for 60 min, w/ water | 264 | | 0.8001 | 24.92 |
| Ex. 205 | 130° C for 90 min, w/o water | 402 | | 0.6751 | 24.93 |
| Ex. 206 | 130° C for 90 min, w/ water | 402 | | 0.8042 | 24.94 |
| Ex. 207 | 130° C for 60 min, w/o water, matured | 264 | kept heated in air at 40° C for 8 days | 0.6576 | 24.98 |
| Ex. 208 | 130° C for 60 min, w/ water, matured | 264 | kept heated in air at 40° C for 8 days | 0.8009 | 25.02 |
| Ex. 209 | 140° C for 60 min, w/o water, matured | 586 | kept heated in air at 40° C for 8 days | 0.6882 | 25.26 |
| Ex. 210 | 140° C for 60 min, w/ water, matured | 586 | kept heated in air at 40° C for 8 days | 0.7997 | 25.08 |
| Ex. 211 | heated in oil at reached temp. of 90° C | 0.3 | | | |
| Ex. 212 | heated in oil at reached | 0.5 | | | |

(continued)

| | Heating condition | Heating value | Maturing condition | Aw | Temp. (° C) |
|---|---|---|---|---|---|
| | temp. of 100° C | | | | |
| Ex. 213 | heated in oil at reached temp. of 110° C | 2.5 | | | |
| Ex. 214 | heated in oil at 130° C for 5 min | 32 | | | |
| Ex. 215 | heated in oil at 150° C for 5 min | 101.6 | | | |
| Ex. 216 | heated in oil at 170° C for 5 min | 446.7 | | | |

Comparative Example 201 is paprika powder without heat-treatment including heat sterilization.

Comparative Example 202 is paprika powder that was matured by keeping the paprika powder of Comparative Example 201 at 40 °C for 8 days by the same method as in Examples 207 to 210 described below.

Heating paprika powder for "w/o water" under a specified temperature and time period condition was carried out according to the following procedure. An aluminum foil pouch was filled with 100 g of paprika powder and sealed. The sealed pouch was heat-treated in a retort sterilizer under the specified temperature and time period condition, and then cooled in water. The heat-treatment in the retort sterilizer was carried out under a gauge pressure of 0.2 MPa.

Heating paprika powder for "w/ water" under a specified temperature and time period condition was carried out according to the following procedure. An aluminum foil pouch was filled with 100 g of paprika powder and 10 g of water, and sealed. The sealed pouch was heat-treated in a retort sterilizer under the specified temperature and time period condition, and then cooled in water. The heat-treatment in the retort sterilizer was carried out under a gauge pressure of 0.2 MPa.

For the paprika powder of Examples 207 to 210, the aluminum foil pouch was filled with the paprika powder heated under a specified condition, and sealed such that air was included therein. The sealed pouch was stored and matured at 40 °C for 8 days.

The paprika powder of Examples 211 to 216 was heated in oil according to the following procedure. One hundred grams of palm oil (melting point of 45 °C) was heated, and upon 80 °C was reached, 100 g of paprika powder was mixed therewith, and the resulting mixture was heated with stirring to the specified temperature shown in the table described above, held at the temperature for a specified time period, and then the mixture was cooled. Cooling was carried out down to about 60 °C with stirring to the extent that the paprika powder did not separate from the mixture, and then the mixture was kept cooled in a refrigerator until it solidified. Note that "reached temperature" in Examples 211 to 213 means that upon the mixture reached the specified temperature, heating was immediately stopped and cooling was started.

The water activity (Aw) of the paprika powder of Comparative Example 201 and Examples 201 to 210 was measured under the respective temperature conditions described in the table described above.

1.2. Flavor-enhancing Effect

(1) Preparation of Common Curry Roux

Flour roux was prepared by putting 20 g of flour and 30 g of beef tallow in a pan, then heating with stirring at 120 °C. To this flour roux were added 10 g of table salt, 10 g of sugar, 10 g of cornstarch, 5 g of curry powder and 15 g of other seasoning ingredients (vegetable and fruit extracts, yeast extract and seafood extract), and after heat-treatment to 105 °C, the mixture was cooled and solidified, to form a block of common curry roux.

The sodium chloride equivalent per 100 g of this curry roux was 10.6 g.

(2) Preparation of Reduced-sodium Curry Roux

Reduced-sodium curry roux was prepared according to the same procedure, except that the amount of sodium chloride was reduced to 7 g from the amount in the common curry roux in (1).

The sodium chloride equivalent per 100 g of this reduced-sodium curry roux was 7.7 g.

(3) Setting Amount of Spice to Be Added for Sensory Evaluation

The amount of spice to be added was set so that the flavor-enhancing effect could be felt, within a range such that the characteristic aroma of the spice did not affect the flavor of the curry roux.

The reduced-sodium curry roux in (2) was dispersed in hot water and boiled with stirring, to make base sauce.

The heat-treated paprika powder of Example 203 was added to the sauce so as to be in a final concentration of 0.062% by mass, and a sensory evaluation was done. The amount of added paprika was reduced from the aforesaid concentration to an amount such that the characteristic aroma of the spice did not affect the flavor of the roux, and a final concentration of 0.0464% by mass was set as the amount of paprika to be added.

(4) Sensory Evaluation

Two dispersions of 44 g of the reduced-sodium curry roux in (2) dispersed in 300 g of hot water were prepared.

To one of them was added the paprika powder of any of Comparative Examples or any of Examples so as to be in the concentration set in (3).

Forty-four grams of the common curry roux in (1) was dispersed in 300 g of hot water.

The dispersion of reduced-sodium curry roux dispersed in hot water and the dispersion of common curry roux dispersed in hot water were used as comparative controls, and the strength of the flavor was evaluated by three evaluators (evaluators 1, 2 and 3) on the basis of the following evaluation criteria from the viewpoint of "middle fullness." The "flavor (middle fullness)" in this experiment is a middle flavor concerted by the combination of sodium chloride and other foodstuffs in curry roux, and is an aspect of "saltiness." Thus, the "flavor (middle fullness)" in this experiment can also be expressed as "saltiness (middle fullness)."

The scores of 1, 2, 3, 4 and 5 points for the flavor (middle fullness) were determined as follows, and the three evaluators evaluated the flavor of respective samples in 0.1-point increments, to give an average score.

1 point: Comparable flavor to that of reduced-sodium curry roux
2 points: Slightly stronger flavor than reduced-sodium curry roux
3 points: Stronger flavor than reduced-sodium curry roux
4 points: Much stronger flavor than reduced-sodium curry roux
5 points: Comparable strength of flavor to that of common curry roux

The average of the evaluated scores was defined as "C" when it was higher than those of Comparative Examples 201 and 202 and was 2.0 or lower, "B" when it was 2.1 or higher and 2.5 or lower, "A" when it was 2.6 or higher and 3.0 or lower, and "AA" when it was 3.1 or higher and 5.0 or lower.

The evaluation results are shown in the table described below. The heat-treated paprika of Examples 201 to 216 was confirmed to have a stronger effect of enhancing the "middle fullness" of the flavor in comparison with the paprika of Comparative Examples 201, 202.

[Table 2]

| Evaluation of "middle fullness" | | | | | |
|---|---|---|---|---|---|
| | Average | Evaluator 1 | Evaluator 2 | Evaluator 3 | Evaluation |
| Comp. Ex. 201 | 1.5 | 1.2 | 1.8 | 1.5 | - |
| Comp. Ex. 202 | 1.6 | 1.5 | 1.5 | 1.7 | - |
| Ex. 201 | 1.9 | 2.0 | 1.8 | 2.0 | C |
| Ex. 202 | 2.1 | 2.0 | 2.0 | 2.3 | B |
| Ex. 203 | 3.0 | 3.5 | 2.0 | 3.5 | A |
| Ex. 204 | 3.3 | 3.0 | 3.0 | 4.0 | AA |
| Ex. 205 | 2.7 | 3.0 | 3.0 | 2.0 | A |
| Ex. 206 | 2.5 | 2.5 | 3.0 | 2.0 | B |
| Ex. 207 | 2.8 | 3.0 | 2.5 | 3.0 | A |
| Ex. 208 | 3.1 | 3.0 | 3.5 | 2.7 | AA |
| Ex. 209 | 2.3 | 2.5 | 2.0 | 2.5 | B |
| Ex. 210 | 2.4 | 2.3 | 2.7 | 2.2 | B |
| Ex. 211 | 3.0 | 2.5 | 3.0 | 3.5 | A |

(continued)

| Evaluation of "middle fullness" | | | | | |
|---|---|---|---|---|---|
| | Average | Evaluator 1 | Evaluator 2 | Evaluator 3 | Evaluation |
| Ex. 212 | 3.0 | 3.0 | 2.5 | 3.5 | A |
| Ex. 213 | 3.0 | 3.0 | 2.5 | 3.5 | A |
| Ex. 214 | 3.2 | 3.2 | 2.5 | 3.8 | AA |
| Ex. 215 | 3.2 | 3.5 | 2.5 | 3.5 | AA |
| Ex. 216 | 2.7 | 3.2 | 2.0 | 3.0 | A |

1.3. Constituent Analysis

Constituents included in the paprika sample of Comparative Examples 201 and 202, and of Examples 201 to 216 were analyzed according to the following procedure.

(1) LC-MS/MS Test Solution Preparation

Into a 10-mL test tube was taken 200 mg of a paprika sample (representing a mass converted to that of the paprika sample excluding oil or fat when the aforesaid paprika sample was a paprika sample heat-treated together with the oil or fat), and 5 mL of ultrapure water was added. The test tube was kept heated for 30 minutes in a thermostatic water bath set to 80 °C and then left to stand down to room temperature. To the test tube was added 5 mL of acetonitrile (FUJIFILM Wako Pure Chemicals), and furthermore, L-lysine-$^{13}C_6$ monohydrochloride (FUJIFILM Wako Pure Chemicals) and ribitol (Fujifilm Wako Pure Chemicals) were added as an internal standard in Positive mode and an internal standard in Negative mode, respectively. L-lysine-$^{13}C_6$ monohydrochloride and ribitol were added so as to be 47 μg/g and 99 μg/g with respect to the paprika sample (mass converted to that of paprika), respectively. The test tube was shaken with a high-speed shaker (CM-1000, TOKYO RIKAKIKAI CO., LTD.) at room temperature and 1,800 rpm for 30 minutes, and after centrifugation, 0.5 mL of the solution in the test tube was transferred to an ultrafiltration filter (Nanosep Centrifugal Filtration Device 3K, Nippon Paul). After centrifuging the ultrafiltration filter at room temperature and 15,000 rpm x 20 minutes, 0.5 mL of ultrapure water was added to a filtrate under the filter, which was then vortexed for 10 seconds. The solution after passing through a 0.2 μm filter was used as an LC-MS/MS specimen (n=2). Two hundred milligrams of the paprika sample described above means a mass converted to that of the paprika sample excluding oil or fat, means 200 mg as the mass of the heat-treated paprika sample of Examples 201 to 210, in which paprika only or a mixture of paprika and water was heat-treated, and refers to 400 mg as the mass of the heat-treated paprika sample of Examples 211 to 216, in which a mixture of paprika and oil or fat was heat-treated.

(2) LC-MS/MS Analysis Condition

The analysis condition for LC-orbitrap-MS is shown below.
Analytical Apparatus:

LC: Vanquish Flex (Thermo Fisher Scientific)
MS: ID-X (Thermo Fisher Scientific)

Analysis column: Unison UK-C18, 3 μm [particle size], 250 mm [length] x 4.6 mm [inside diameter] (Imtakt)
LC Condition:
Column temperature: 40 °C
Injection volume: 5 μL
Mode: ESI positive, ESI negative
Flow rate: 0.3 mL/min
Mobile phase:

Liquid A 0.1% of formic acid solution (formic acid: LCMS grade, FUJIFILM Wako Pure Chemicals)
Liquid B acetonitrile (LCMS grade, FUJIFILM Wako Pure Chemicals)
Mobile phase composition - analysis time 70 min.

[Table 3]

| LC-MS/MS mobile phase composition | | | | | | | | | |
|---|---|---|---|---|---|---|---|---|---|
| | 0 min. | 15 min. | 35 min. | 40 min. | 53 min. | 54 min. | 59 min. | 60 min. | 70 min. |
| Liquid A (%) | 100 | 100 | 70 | 50 | 50 | 0 | 0 | 100 | 100 |
| Liquid B (%) | 0 | 0 | 30 | 50 | 50 | 100 | 100 | 0 | 0 |

MS Condition:

Ion source temperature: 230 °C, collision energy: 30 eV

Monitoring ions: shown in the table described below.

(3) Data Analysis

From LC-MS/MS chromatogram, the precise mass of the precursor ion > the product ion (table described below) of respective constituents was extracted and the peak area was obtained. Constituents in respective specimens were compared by calculating the ratio of the peak area of product ions (= the peak area of the product ion of the respective constituents / the peak area of the product ion of the internal standard). In the table described below, A1 and A2 indicate two amino acids constituting respective cyclic dipeptides.

[Table 4]

| Constituents detected in LC-MS/MS analysis (other than cyclic dipeptides) and analysis condition (Positive mode) | | | |
|---|---|---|---|
| Constituent name | Retention time (Min) | [M+H] | |
| | | Precursor Ion | Product Ion |
| internal standard: L-lysine-$^{13}C_6$ | 8.0 | 153.1327 | 89.0974 |
| 4-methyl-5-thiazoleethanol(sulfurol) | 35.5 | 144.0477 | 113.0293 |
| sulfurol acetate | 49.8 | 186.0583 | 126.0372 |

[Table 5]

| Constituents detected in LC-MS/MS analysis (cyclic dipeptides) and analysis condition (Positive mode) | | | | |
|---|---|---|---|---|
| Constituent name : cyclic (A1-A2) | | Retention time (min) | [M+H] | |
| A1 | A2 | | Precursor Ion | Product Ion |
| Phe | Leu/Ile | 50.7 | 261.1598 | 120.0806 |
| Pro | Asn | 33.8 | 212.1030 | 212.1028 |
| Leu/Ile | Leu/Ile | 50.5 | 227.1755 | 86.0962 |
| Thr | Leu/Ile | 38.9 | 215.1391 | 169.1337 |
| Phe | Ser | 40.7 | 235.1078 | 120.0806 |
| Gly | His | 9.5 | 195.0877 | 122.0712 |
| Gly | Ser | 11.0 | 145.0608 | 85.0395 |
| Gly | Arg | 12.8 | 214.1299 | 155.0815 |
| Ala | His | 11.3 | 209.1034 | 110.0713 |
| Glu | His | 14.1 | 267.1088 | 110.0712 |
| Glu | Arg | 16.0 | 286.1510 | 227.1022 |
| Ala | Arg | 15.9 | 228.1456 | 169.0970 |

(continued)

| Constituents detected in LC-MS/MS analysis (cyclic dipeptides) and analysis condition (Positive mode) | | | | |
|---|---|---|---|---|
| Constituent name : cyclic (A1-A2) | | Retention time (min) | [M+H] | |
| A1 | A2 | | Precursor Ion | Product Ion |
| Glu | Glu | 13.0 | 259.0925 | 195.0761 |
| Glu | Gly | 18.2 | 187.0714 | 84.0441 |
| Glu | Asp | 22.1 | 245.0769 | 84.0441 |
| Pro | His | 29.5 | 235.1190 | 110.0712 |
| Glu | Tyr | 34.5 | 293.1133 | 247.1078 |
| Leu/Ile | Asp | 41.0 | 229.1183 | 141.1022 |
| Pro | Asp | 34.7 | 213.0870 | 125.0706 |
| Val | Arg | 34.4 | 256.1769 | 197.1284 |
| Thr | Pro | 35.7 | 199.1078 | 125.0707 |
| hyPro | Pro | 35.5 | 211.1079 | 70.0650 |
| Gly | Val | 35.8 | 157.0972 | 72.0805 |
| Ala | Pro | 36.6 | 169.0968 | 70.0649 |
| Ala | Tyr | 37.6 | 235.1072 | 136.0756 |
| Arg | Leu/Ile | 37.0 | 270.1925 | 211.1438 |
| Leu/Ile | Ser | 37.9 | 201.1234 | 86.0962 |
| Ala | Val | 38.9 | 171.1125 | 72.0806 |
| Pro | Pro | 39.2 | 195.1128 | 70.0649 |
| Gly | Leu/Ile | 40.0 | 171.1128 | 86.0962 |
| Gly | Phe | 42.1 | 205.0972 | 120.0806 |
| Pro | Tyr | 41.9 | 261.1234 | 136.0756 |
| Phe | Asp | 42.6 | 263.1027 | 175.0863 |
| Pro | Val | 42.9 | 197.1285 | 70.0650 |
| Ala | Leu/Ile | 43.5 | 185.1285 | 86.0963 |
| Val | Tyr | 43.9 | 263.1391 | 136.0756 |
| Tyr | Ser | 35.6 | 251.1027 | 107.0487 |
| Phe | Ala | 44.9 | 219.1128 | 120.0806 |
| Val | Val | 45.9 | 199.1442 | 72.0806 |
| Pro | Leu/Ile | 46.9 | 211.1442 | 70.0650 |
| Phe | Tyr | 47.6 | 311.1391 | 136.0754 |
| Phe | Thr | 42.8 | 249.1234 | 91.0542 |
| Phe | Pro | 48.3 | 245.1285 | 120.0806 |
| Leu/Ile | Val | 48.5 | 213.1599 | 72.0806 |
| Val | Phe | 49.5 | 247.1442 | 120.0806 |
| Tyr | Asp | 48.7 | 279.0976 | 136.0756 |
| Ala | Asp | 17.3 | 187.0714 | 99.0550 |
| Val | Ser | 33.9 | 187.1078 | 72.0807 |

[Table 6]

| Constituents detected in LC-MS/MS analysis and analysis condition (Negative mode) | | | |
|---|---|---|---|
| Constituent name | Retention time (Min) | [M-H] | |
| | | Precursor Ion | Product Ion |
| internal standard: ribitol | 9.7 | 151.0607 | 71.0138 |
| tartaric acid | 10.6 | 149.0086 | 72.9931 |
| *trans*-aconitic acid | 33.6 | 173.0091 | 111.0087 |

The ratios of the peak area of cyclic peptides, organic acids (tartaric acid and trans-aconitic acid), sulfurol and sulfurol acetate detected in LC-MS/MS specimens prepared from the respective samples, to the peak area of the internal standard are shown in Figures 1 to 13. Bars in the figures represent standard deviations.

The results shown in Figures 1 to 13 indicate that the heat-treated paprika of Examples 201 to 216 included a larger amount of cyclic (Phe-Leu/Ile), cyclic (Pro-Asn), cyclic (Leu/Ile-Leu/Ile), cyclic (Thr-Leu/Ile), cyclic (Phe-Ser), cyclic (Gly-His), cyclic (Gly-Ser), cyclic (Gly-Arg), cyclic (Ala-His), cyclic (Glu-His), cyclic (Glu-Arg), cyclic (Ala-Arg), cyclic (Glu-Glu), cyclic (Glu-Gly), cyclic (Glu-Asp), cyclic (Pro-His), cyclic (Glu-Tyr), cyclic (Leu/Ile-Asp), cyclic (Pro-Asp), cyclic (Val-Arg), cyclic (Thr-Pro), cyclic (hyPro-Pro), cyclic (Gly-Val), cyclic (Ala-Pro), cyclic (Ala-Tyr), cyclic (Arg-Leu/Ile), cyclic (Leu/Ile-Ser), cyclic (Ala-Val), cyclic (Pro-Pro), cyclic (Gly-Leu/Ile), cyclic (Gly-Phe), cyclic (Pro-Tyr), cyclic (Phe-Asp), cyclic (Pro-Val), cyclic (Ala-Leu/Ile), cyclic (Val-Tyr), cyclic (Tyr-Ser), cyclic (Phe-Ala), cyclic (Val-Val), cyclic (Pro-Leu/Ile), cyclic (Phe-Tyr), cyclic (Phe-Thr), cyclic (Phe-Pro), cyclic (Leu/Ile-Val), cyclic (Val-Phe), cyclic (Tyr-Asp), cyclic (Ala-Asp), cyclic (Val-Ser), sulfurol, sulfurol acetate, tartaric acid and trans-aconitic acid in comparison with the unheated paprika of Comparative Examples 201 and 202. The amount of these constituents showed a tendency to correlate with the strength of the flavor-enhancing effect. In particular, cyclic (Gly-His), cyclic (Gly-Ser), cyclic (hyPro-Pro), cyclic (Gly-Val), cyclic (Tyr-Asp) and cyclic (Ala-Asp) are constituents that are not included in the unheated paprika (below detection limit) and are specific to the heat-treated paprika.

1.4. Flavor-enhancing Effect on Various Foodstuffs

(1) Sample Preparation

Foodstuffs shown in the "basic taste", "dashi ingredient", "seasoning" and "ingredient" columns of the table described below were each added to water and diluted so as to be in the concentrations (% (w/w)) described in the table described below, to prepare foodstuff dilutions. The heat-treated paprika powder of Example 203 was mixed with each of the aforesaid foodstuff dilutions so that the heat-treated paprika powder was in the final concentration of 0.05% (w/w), to prepare a sample. The aforesaid foodstuff dilutions without the addition of paprika powder were used as negative control samples. For the respective foodstuffs, a foodstuff dilution in approximately 1.2 times the concentration shown in the table described below was also prepared as a positive control sample.

Food products, such as curry, shown in the "food product" column of the table described below were prepared using commercially available instant food kits. The respective food products were prepared with approximately 1.2 times the amount of water specified in the aforesaid kits in order to facilitate evaluation of flavor-enhancement. The heated paprika powder of Example 203 was mixed with the respective prepared food products so that the heated paprika powder was in a final concentration within the range of 0.03 to 0.07% (w/w), to prepare samples. The aforesaid respective food products without the addition of paprika powder were used as negative control samples. The respective food products were also prepared as positive control samples by using the amount of water specified in the aforesaid kits.

(2) Sensory Evaluation

Three evaluators (evaluators 1, 2, and 3) tasted the sample prepared from the respective foodstuffs and evaluated the strength of the flavor of the sample.

The scores of 1, 2, 3, 4 and 5 points were determined as follows. The strength of the flavor of the respective samples was evaluated in 0.5-point increments by the three evaluators, to give an average score.

1 point: Comparable flavor to that of negative control sample
2 points: Slightly stronger flavor than negative control sample
3 points: Stronger flavor than negative control sample
4 points: Much stronger flavor than negative control sample

5 points: Comparable strength of flavor to that of positive control sample

The average of the evaluated scores was defined as "C" when it was higher than 1.0 point (negative control sample) and 2.0 or lower, "B" when it was 2.1 or higher and 2.5 or lower, "A" when it was 2.6 or higher and 3.0 or lower, and "AA" when it was 3.1 or higher and 5.0 or lower.

The evaluation results are shown in the table described below. The heat-treated paprika powder of Example 203 was confirmed to have a strong effect of enhancing the flavor of various foodstuffs.

[Table 7]

| | | | Foodstuff concentration | Example 203 (paprika 130° C for 60 min. w/o water) | | | | |
|---|---|---|---|---|---|---|---|---|
| | | | | Average | Evaluator 1 | Evaluator 2 | Evaluator 3 | Evaluation |
| Basic taste | saltiness | sodium chloride | 0.5% | 4.0 | 4. 0 | 4.0 | 4.0 | AA |
| | sweetness | granulated sugar | 1.0% | 4.0 | 4.0 | 4.0 | 4.0 | AA |
| | sourness | tartaric acid | 0. 05% | 2.0 | 1.0 | 4.0 | 1.0 | C |
| | *umami* | mono sodium glutamate (MSG) | 0.25% | 2.0 | 2.5 | 1.0 | 2.5 | C |
| | | disodium succinate | 0.1% | 2.5 | 1.0 | 4.0 | 2.5 | B |
| | | sodium inosinate + sodium guanylate | each 0.05% | 2.0 | 1.0 | 2.5 | 2.5 | C |
| | sweet *umami* | glycine + alanine | each 0.125% | 1.5 | 1.0 | 2.5 | 1.0 | C |
| | pungency | chili pepper | 0.01% | 2.0 | 1.0 | 4.0 | 1.0 | C |
| | | black pepper | 0.05% | 2.0 | 1.0 | 1.0 | 4.0 | C |
| | | | | | | | | |
| *Dashi* ingredient | beef extract | | 0.5% | 1.5 | 1.0 | 1.0 | 2.5 | C |
| | chicken extract | | 0.5% | 2.5 | 1.0 | 4.0 | 2.5 | B |
| | pork extract | | 0.5% | 1.5 | 1.0 | 1.0 | 2.5 | C |
| | bonito extract | | 0.5% | 1.5 | 1.0 | 1.0 | 2.5 | C |
| | small dried sardine extract | | 0.5% | 2.5 | 1.0 | 2. 5 | 4.0 | B |
| | tomato paste | | 1.5% | 2.5 | 1.0 | 2.5 | 4.0 | B |
| | garlic extract | | 0.5% | 2.0 | 2.5 | 1.0 | 2.5 | C |
| | onion extract | | 0.5% | 3.0 | 4.0 | 2.5 | 2.5 | A |
| | | | | | | | | |

(continued)

| | | Foodstuff concentration | Example 203 (paprika 130° C for 60 min. w/o water) | | | | |
|---|---|---|---|---|---|---|---|
| | | | Average | Evaluator 1 | Evaluator 2 | Evaluator 3 | Evaluation |
| Seasoning | soy sauce | 1.2% | 2.5 | 1.0 | 4.0 | 2 5 | B |
| | *miso* paste | 3.3% | 2.5 | 1.0 | 4. 0 | 2.5 | B |
| | ketchup | 6.0% | 3.0 | 2.5 | 4.0 | 2.5 | A |
| | worcestershire sauce | 2.2% | 1.5 | 1.0 | 2. 5 | 1.0 | C |
| | mayonnaise | 10.0% | 1.5 | 1.0 | 1.0 | 2.5 | C |
| | noodle soup base | 1.65% | 2.5 | 1.0 | 4.0 | 2.5 | B |
| | cheese | 1.65% | 2.5 | 4.0 | 1.0 | 2.5 | B |
| Ingredient | caramel | 0.07% | 1.5 | 1.0 | 2. 5 | 1.0 | C |
| | banana paste | 2.5% | 1.5 | 1.0 | 2.5 | 1.0 | C |
| | apple paste | 2.5% | 2.0 | 1.0 | 2. 5 | 2.5 | C |
| | honey | 1.25% | 2.0 | 1.0 | 2. 5 | 2.5 | C |
| | soybean skimmed | 1.5% | 1.5 | 2.5 | 1.0 | 1.0 | C |
| | full-fat powdered milk | 1.5% | 1.5 | 2.5 | 1.0 | 1.0 | C |
| | roasted skimmed milk powder | 1.5% | 3.0 | 2.5 | 4.0 | 2.5 | A |
| | yeast extract (main ingredient: baker's yeast) | 0.1% | 2.0 | 1.0 | 4.0 | 1.0 | C |
| | protein hydrolysate | 0.1% | 2.0 | 2.5 | 1.0 | 2.5 | C |
| | curry powder A | 0.5% | 1.5 | 2.5 | 1.0 | 1.0 | C |
| | curry powder B | 0.5% | 1.5 | 1.0 | 2.5 | 1.0 | C |
| | | | | | | | |
| Food product | curry (medium-hot) | | 3.5 | 4 | 4 | 2.5 | AA |
| | cream stew | | 3 | 4 | 4 | 1 | A |
| | spaghetti with meat sauce | | 3.5 | 4 | 4 | 2.5 | AA |
| | consomme soup with onion extract | | 3 | 2.5 | 4 | 2.5 | A |
| | *miso* soup (ingredients: *tofu* and *wakame* | | 2 | 1 | 2.5 | 2.5 | C |
| | *Sanuki* udon noodle | | 2.5 | 1 | 4 | 2.5 | B |
| | Ramen noodle with soy sauce | | 3 | 4 | 2.5 | 2.5 | A |

2. Experiment 2: Flavor-enhancing Effect of Heat-treated Cumin

2.1. Preparation of Heat-treated Cumin

Whole seed of cumin (cumin seed) was heat-treated under the conditions shown in the table described below. The heating values are values given by the integral of the CV value according to the aforesaid formula ("reference temperature" is 110 °C and "Z value" is 30 (°C)) with respect to the heating time (min), as previously described. The temperatures and time periods described in the heating condition column are maximum reached temperatures and their holding times, and the heating values were calculated on the basis of temperature-change over time, including the values of the temperature and time during temperature rise and fall.

[Table 8]

| | Heating condition | Heating value | Maturing condition | Aw | Temp. (° C) |
|---|---|---|---|---|---|
| Comp. Ex. 301 | unheated | 0 | | 0.5439 | 25.08 |
| Ex. 301 | 120° C for 20 min, w/o water | 53 | | 0.6016 | 25. 11 |
| Ex. 302 | 120° C for 20 min, w/ water | 53 | | 0. 8063 | 25 |
| Ex. 303 | 120° C for 30 min, w/o water | 74 | | 0.6021 | 25. 14 |
| Ex. 304 | 120° C for 30 min, w/ water | 74 | | 0.7991 | 24. 98 |
| Ex. 305 | 130° C for 30 min, w/o water | 168 | | 0.6019 | 25.01 |
| Ex. 306 | 130° C for 30 min, w/ water | 168 | | 0. 8069 | 24. 95 |
| Ex. 307 | 130° C for 30 min, w/ water, matured, (pulverized → matured) | 168 | kept heated in air at 40° C for 8 days | 0. 8187 | 24. 9 |
| Ex. 308 | 130° C for 30 min, w/o water, matured (pulverized → matured) | 168 | kept heated in air at 40° C for 8 days | 0.6253 | 24. 91 |
| Ex. 309 | 130° C for 60 min, w/o water | 264 | | 0.6548 | 25. 04 |
| Ex. 310 | 130° C for 60 min, w/ water | 264 | | 0. 7837 | 25.02 |
| Ex. 311 | 140° C for 30 min, w/o water | 407 | | | |
| Ex. 312 | 140° C for 30 min, w/ water | 407 | | | |
| Ex. 313 | 140° C for 30 min, w/ water, matured, (pulverized → matured) | 407 | kept heated in air at 40° C for 8 days | 0. 7995 | 24. 97 |
| Ex. 314 | 140° C for 30 min, w/o water, matured, (pulverized → matured) | 407 | kept heated in air at 40° C for 8 days | 0.6234 | 25.082 |
| Ex. 315 | heated in oil at reached temp. of 100° C | 1 | | | |
| Ex. 316 | heated in oil at 130° C for 5 min | 34 | | | |
| Ex. 317 | heated in oil at 150° C for 5 min | 183 | | | |
| Ex. 318 | heated in oil at 170° C for 5 min | 577 | | | |
| Ex. 319 | heated in oil at 200° C for 5 min | 4340 | | | |

Comparative Example 301 is cumin seed without heat-treatment including heat sterilization.

Heating cumin seed for "w/o water" under a specified temperature and time period condition was carried out according to the following procedure. An aluminum foil pouch was filled with 100 g of cumin seed and sealed. The sealed pouch was heat-treated in a retort sterilizer under the specified temperature and time period condition, and then cooled in water. The heat-treatment in the retort sterilizer was carried out under a gauge pressure of 0.2 MPa.

Heating cumin seed for "w/ water" under a specified temperature and time period condition was carried out according to the following procedure. An aluminum foil pouch was filled with 100 g of cumin seed and 10 g of water, and sealed. The sealed pouch was heat-treated in a retort sterilizer under the specified temperature and time period condition, and then cooled in water. The heat-treatment in the retort sterilizer was carried out under a gauge pressure of 0.2 MPa.

The cumin seed of Examples 307, 308, 313 and 314 was, after heating, pulverized and matured according to the following procedure. The cumin seed after heating was pulverized into powder using a stamp mill for 3 minutes per 20 g. An aluminum foil pouch was filled with the product, and sealed such that air was included therein. The sealed pouch was stored and matured at 40 °C for 8 days.

The cumin of Comparative Example and Examples other than Examples 307, 308, 313 and 314 was also pulverized into powder under the same condition and used as a sample in Water Activity Measurement, Confirmation of Flavor-enhancing Effect and Constituent Analysis below. The cumin of Examples 307, 308, 313 and 314 was used as a sample as is after maturing.

Examples 315 to 319 are pulverized products of cumin seed heated in oil according to the following procedure.

Unheated cumin seed was pulverized into powder using a stamp mill for 5 minutes per 75 g.

One hundred grams of palm oil (melting point of 45 °C) was heated, and upon 80 °C was reached, 100 g of the pulverized cumin seed product was mixed therewith, and the resulting mixture was heated with stirring to the specified temperature indicated in the table described above, kept at the temperature for a specified time period, and then the mixture was cooled. Cooling was carried out down to about 60 °C with stirring to the extent that the pulverized cumin seed product did not separate from the mixture, and then the mixture was kept cooled in a refrigerator until it solidified. Note that "reached temperature" in Example 315 means that upon the mixture reached the specified temperature, heating was immediately stopped and cooling was started.

The water activity (Aw) of the cumin of Comparative Example 301 and Examples 301 to 314 was measured under the respective temperature conditions shown in the table described above.

2.2. Flavor-enhancing Effect

Common and reduced-sodium curry rouxes were prepared according to the procedures described in (1) and (2) of 1.2. described above.

The aforesaid reduced-sodium curry roux was dispersed in hot water and boiled with stirring, to make base sauce.

The heat-treated cumin of Example 305 was added to the sauce so as to be in a final concentration of 0.062% by mass, and a sensory evaluation was done. The amount of added cumin was reduced from the aforesaid concentration to an amount such that the characteristic aroma of spice did not affect the flavor of the roux, and a final concentration of 0.0619% by mass was set as the amount of cumin to be added.

Two dispersions of 44 g of the aforesaid reduced-sodium curry roux dispersed in 300 g of hot water were prepared.

To one of them was added cumin of Comparative Example or any of Examples so as to be in the concentration set above.

Forty-four grams of the aforesaid common curry roux was dispersed in 300 g of hot water.

The dispersion of reduced-sodium curry roux dispersed in hot water and the dispersion of common curry roux dispersed in hot water were used as comparative controls, and the strength of the flavor was evaluated by three evaluators (evaluators 1, 2 and 3) on the basis of the following evaluation criteria from the viewpoint of "top sharp saltiness" and "middle fullness." The "flavor (top sharp saltiness)" and "flavor (middle fullness)" in this experiment are top and middle flavors each concerted by the combination of sodium chloride and other foodstuffs in curry roux, and are aspects of "saltiness." Thus, the "flavor (top sharp saltiness)" and the "flavor (middle fullness)" in this experiment can also be expressed as "saltiness (top sharp saltiness)" and "saltiness (middle fullness)", respectively.

The scores of 1, 2, 3, 4 and 5 points for the flavors ("top sharp saltiness" and "middle fullness") were determined as follows, and the three evaluators evaluated the flavor of respective samples in 0.1-point increments, to give an average score.

1 point: Comparable flavor to that of reduced-sodium curry roux
2 points: Slightly stronger flavor than reduced-sodium curry roux
3 points: Stronger flavor than reduced-sodium curry roux
4 points: Much stronger flavor than reduced-sodium curry roux
5 points: Comparable strength of flavor to that of common curry roux

For both evaluation items, "top sharp saltiness" and "middle fullness", the average of the evaluated scores was defined as "C" when it was higher than that of Comparative Example 301 and 2.0 or lower, "B" when it was 2.1 or higher and 2.5 or lower, "A" when it was 2.6 or higher and 3.0 or lower, and "AA" when it was 3.1 or higher and 5.0 or lower.

The evaluation results are shown in the table described below. The heat-treated cumin of Examples 301 to 319 was confirmed to have a stronger effect of enhancing flavor in comparison with the cumin of Comparative Example 301, from the viewpoint of both "top sharp saltiness" and "middle fullness."

[Table 9]

| Evaluation of "top sharp saltiness (*shiokado*)" | | | | | |
|---|---|---|---|---|---|
| | Average | Evaluator 1 | Evaluator 2 | Evaluator 3 | Evaluation |
| Comp. Ex. 301 | 1.5 | 1.5 | 2.0 | 1.0 | - |
| Ex. 301 | 2.0 | 2.0 | 2.0 | 2.0 | C |
| Ex. 302 | 1. 7 | 1.5 | 2.0 | 1.5 | C |

(continued)

| Evaluation of "top sharp saltiness (*shiokado*)" | | | | | |
|---|---|---|---|---|---|
| | Average | Evaluator 1 | Evaluator 2 | Evaluator 3 | Evaluation |
| Ex. 303 | 2. 2 | 2. 5 | 2. 5 | 1.5 | B |
| Ex. 304 | 2. 2 | 2. 5 | 2. 5 | 1.5 | B |
| Ex. 305 | 2. 8 | 3.0 | 2. 5 | 3.0 | A |
| Ex. 306 | 2. 7 | 2. 5 | 2.0 | 3.5 | A |
| Ex. 307 | 2. 5 | 2.8 | 2. 5 | 2.2 | B |
| Ex. 308 | 2. 5 | 3.0 | 2.0 | 2.5 | B |
| Ex. 309 | 2.8 | 2. 5 | 3.0 | 2.8 | A |
| Ex. 310 | 2.0 | 2.0 | 2.0 | 2.0 | C |
| Ex. 311 | 2. 2 | 2.2 | 2.0 | 2.5 | B |
| Ex. 312 | 1.9 | 2.0 | 1.8 | 2.0 | C |
| Ex. 313 | 2. 5 | 2. 5 | 3.0 | 2.0 | B |
| Ex. 314 | 2. 5 | 2. 7 | 2.8 | 2.0 | B |
| Ex. 315 | 2. 2 | 2.0 | 2.0 | 2.5 | B |
| Ex. 316 | 2. 2 | 2.0 | 2. 5 | 2.0 | B |
| Ex. 317 | 3.2 | 3.5 | 3.0 | 3.2 | AA |
| Ex. 318 | 2. 8 | 3.0 | 2. 5 | 2.8 | A |
| Ex. 319 | 2. 3 | 2. 5 | 2.0 | 2.5 | B |

[Table 10]

| Evaluation of "middle fullness" | | | | | |
|---|---|---|---|---|---|
| | Average | Evaluator 1 | Evaluator 2 | Evaluator 3 | Evaluation |
| Comp. Ex. 301 | 1.1 | 1.0 | 1.2 | 1.1 | - |
| Ex. 301 | 1.2 | 1.0 | 1.5 | 1.1 | C |
| Ex. 302 | 1.3 | 1. 1 | 1.5 | 1.3 | C |
| Ex. 303 | 1.3 | 1. 1 | 1.5 | 1.3 | C |
| Ex. 304 | 1.5 | 1.2 | 1.8 | 1.4 | C |
| Ex. 305 | 1.6 | 1.5 | 1.8 | 1.5 | C |
| Ex. 306 | 1.8 | 2.0 | 1.8 | 1.5 | C |
| Ex. 307 | 2. 2 | 2.0 | 2.0 | 2.5 | B |
| Ex. 308 | 1.8 | 2.0 | 1.5 | 2.0 | C |
| Ex. 309 | 2. 1 | 2. 5 | 1.8 | 2.0 | B |
| Ex. 310 | 2. 3 | 2. 5 | 1.5 | 3.0 | B |
| Ex. 311 | 1.9 | 2.0 | 2.3 | 1.5 | C |
| Ex. 312 | 2.4 | 2. 5 | 2.3 | 2.5 | B |
| Ex. 313 | 2.8 | 3.0 | 2.0 | 3.3 | A |
| Ex. 314 | 2. 5 | 2. 5 | 2.0 | 3.0 | A |
| Ex. 315 | 2.0 | 2.0 | 2.0 | 2.0 | C |
| Ex. 316 | 2. 3 | 3.0 | 2. 5 | 1.5 | B |

(continued)

| Evaluation of "middle fullness" | | | | | |
|---|---|---|---|---|---|
| | Average | Evaluator 1 | Evaluator 2 | Evaluator 3 | Evaluation |
| Ex. 317 | 2. 8 | 3.0 | 2.8 | 2.5 | A |
| Ex. 318 | 2. 6 | 3.0 | 2.5 | 2.3 | A |
| Ex. 319 | 2. 2 | 2. 5 | 2.0 | 2.0 | B |

2.3. Constituent Analysis

Constituents included in the cumin (cumin sample) of Comparative Example 301 and of Examples 301 to 319 were analyzed according to the following procedure.

2.3.1. Constituent Analysis by LC-MS/MS

(1) LC-MS/MS Test Solution Preparation

Into a 10-mL test tube was taken 200 mg of a cumin sample (representing a mass converted to that of the cumin sample excluding oil or fat when the aforesaid cumin sample was a cumin sample heat-treated together with the oil or fat), and 5 mL of ultrapure water was added. The test tube was kept heated for 30 minutes in a thermostatic water bath set to 80 °C and then left to stand down to room temperature. To the test tube was added 5 mL of acetonitrile (FUJIFILM Wako Pure Chemicals), and furthermore, L-lysine-$^{13}C_6$ monohydrochloride (FUJIFILM Wako Pure Chemicals) and ribitol (Fujifilm Wako Pure Chemicals) were added as an internal standard in Positive mode and an internal standard in Negative mode, respectively. L-lysine-$^{13}C_6$ monohydrochloride and ribitol were added so as to be 47 μg/g and 99 μg/g with respect to the cumin sample (representing a mass converted to that of the cumin sample excluding oil or fat when the aforesaid cumin sample was a cumin sample heat-treated together with the oil or fat), respectively. The test tube was shaken with a high-speed shaker (CM-1000, TOKYO RIKAKIKAI CO., LTD.) at room temperature and 1,800 rpm for 30 minutes, and after centrifugation, 0.5 mL of the solution in the test tube was transferred to an ultrafiltration filter (Nanosep Centrifugal Filtration Device 3K, Nippon Paul). After centrifuging the ultrafiltration filter at room temperature and 15,000 rpm x 20 minutes, 0.5 mL of ultrapure water was added to a filtrate under the filter, which was then vortexed for 10 seconds. The solution after passing through a 0.2 μm filter was used as an LC-MS/MS specimen (n=2). Two hundred milligrams of the cumin sample described above means a mass converted to that of the cumin sample excluding oil or fat, means 200 mg as the mass of the heat-treated cumin sample of Examples 301 to 314, in which cumin only or a mixture of cumin and water was heat-treated, and refers to 400 mg as the mass of the heat-treated cumin sample of Examples 315 to 319, in which a mixture of cumin and oil or fat was heat-treated.

(2) LC-MS/MS Analysis Condition

LC-MS/MS analysis was carried out under the condition described in (2) of 1.3. described above.

(3) Data Analysis

From LC-MS/MS ion chromatogram, the precise mass of the precursor ion > the product ion (table described below) of respective constituents was extracted and the peak area was obtained. Constituents in respective specimens were compared by calculating the ratio of the peak area of product ions (= the peak area of the product ion of respective constituents / the peak area of the product ion of the internal standard). In the table described below, A1 and A2 indicate two amino acids constituting respective cyclic dipeptides.

[Table 11]

| Constituents detected in LC-MS/MS analysis (other than cyclic dipeptides) and analysis condition (Positive mode) | | | |
|---|---|---|---|
| Constituent name | Retention time (min) | [M+H] | |
| | | Precursor Ion | Product Ion |
| internal standard: L-lysine-$^{13}C_6$ | 8.0 | 153.1327 | 89. 0974 |
| 4-methyl-5-thiazole ethanol (sulfurol) | 35. 5 | 144.0477 | 113.0293 |

(continued)

| Constituents detected in LC-MS/MS analysis (other than cyclic dipeptides) and analysis condition (Positive mode) | | | |
|---|---|---|---|
| Constituent name | Retention time (min) | [M+H] | |
| | | Precursor Ion | Product Ion |
| sulfurol acetate | 49. 8 | 186.0583 | 126.0372 |

[Table 12]

| Constituents detected in LC-MS/MS analysis (cyclic dipeptides) and analysis condition (Positive mode) | | | | |
|---|---|---|---|---|
| Constituent name : cyclic (A1-A2) | | Retention time (min) | [M+H] | |
| A1 | A2 | | Precursor Ion | Product Ion |
| Phe | Phe | 51. 6 | 295. 1442 | 120.0806 |
| Phe | Leu/Ile | 50. 7 | 261.1598 | 120.0806 |
| Leu/Ile | Val | 48. 5 | 213.1599 | 72. 0806 |
| Phe | Pro | 48. 3 | 245.1285 | 120.0806 |
| Phe | Tyr | 47. 6 | 311.1391 | 136. 0754 |
| Pro | Leu/Ile | 46. 9 | 211.1442 | 70. 0650 |
| Val | Val | 45. 9 | 199.1442 | 72. 0806 |
| Phe | Ala | 44. 9 | 219.1128 | 120.0806 |
| Tyr | Ser | 35. 6 | 251.1027 | 107. 0487 |
| Pro | Val | 42. 9 | 197.1285 | 70.0650 |
| Pro | Tyr | 41.9 | 261.1234 | 136.0756 |
| Pro | Pro | 39.2 | 195.1128 | 70.0649 |
| Leu/Ile | Ser | 37.9 | 201.1234 | 86.0962 |
| Arg | Leu/Ile | 37.0 | 270.1925 | 211.1438 |
| Ala | Tyr | 37.6 | 235.1072 | 136.0756 |
| Gly | Tyr | 36.4 | 221.0921 | 107.0487 |
| Ala | Pro | 36.6 | 169.0968 | 70.0649 |
| Gly | Val | 35.8 | 157.0972 | 72.0805 |
| hyPro | Pro | 35.5 | 211.1079 | 70.0650 |
| Thr | Pro | 35.7 | 199.1078 | 125.0707 |
| Val | Arg | 34.4 | 256.1769 | 197.1284 |
| Pro | Asp | 34.7 | 213.0870 | 125.0706 |
| Arg | Pro | 34.2 | 254.1607 | 195.1128 |
| Glu | Tyr | 34.5 | 293.1133 | 247.1078 |
| Pro | His | 29.5 | 235.1190 | 110.0712 |
| Glu | Asp | 22.1 | 245.0769 | 84.0441 |
| Gly | Arg | 12.8 | 214.1299 | 155.0815 |
| Gly | His | 9.5 | 195.0877 | 122.0712 |
| Leu/Ile | Leu/Ile | 50.5 | 227.1755 | 86.0962 |
| Glu | Glu | 13.0 | 259.0925 | 195.0761 |
| Leu/Ile | Asp | 41.0 | 229.1183 | 141.1022 |

(continued)

| Constituents detected in LC-MS/MS analysis (cyclic dipeptides) and analysis condition (Positive mode) | | | | |
|---|---|---|---|---|
| Constituent name : cyclic (A1-A2) | | Retention time (min) | [M+H] | |
| A1 | A2 | | Precursor Ion | Product Ion |
| Phe | Asp | 42.6 | 263.1027 | 175.0863 |

[Table 13]

| Constituents detected in LC-MS/MS analysis and analysis condition (Negative mode) | | | |
|---|---|---|---|
| Constituent name | Retention time (min) | [M-H] | |
| | | Precursor Ion | Product Ion |
| internal standard: ribitol | 9. 7 | 151. 0607 | 71.0138 |
| tartaric acid | 10.6 | 149.0086 | 72.9931 |
| malic acid | 13. 1 | 133.0142 | 71.0138 |
| citric acid | 19.0 | 191.0197 | 111.0087 |
| *cis*-aconitic acid | 26. 2 | 173.0091 | 111.0087 |
| *trans*-aconitic acid | 33. 6 | 173.0091 | 111. 0087 |

2.3.2. Constituent Analysis by GC-MS

(1) GC-MS Test Solution Preparation

Into a 10-mL test tube was taken 15 mg of a cumin sample (representing a mass converted to that of the cumin sample excluding oil or fat when the aforesaid cumin sample was a heat-treated cumin sample heat-treated together with the oil or fat), and 5 mL of acetone (FUJIFILM Wako Pure Chemicals) and 5 mL of methanol (FUJIFILM Wako Pure Chemicals) were added. As an internal standard, 4-methylthiazole (TOKYO CHEMICAL INDUSTRY CO., LTD) was added so as to be 625 μg/g with respect to the cumin sample (mass converted as cumin). The test tube was shaken with a high-speed shaker (CM-1000, TOKYO RIKAKIKAI CO., LTD.) at room temperature and 1,800 rpm for 30 minutes. After stirring, centrifugation was carried out, and after centrifugation 0.1 mL of the solution in the test tube was taken into a GC-MS vial, and 1 mL of acetone was further added, which was used as a GC-MS specimen (n=2). Fifteen milligrams of the cumin sample described above means a mass converted to that of the cumin sample excluding oil or fat, means 15 mg as the mass of the heat-treated cumin sample of Examples 301 to 314, in which cumin only or a mixture of cumin and water was heat-treated, and refers to 30 mg as the mass of the heat-treated cumin sample of Examples 315 to 319, in which a mixture of cumin and oil or fat was heat-treated.

(2) GC-MS Analysis Condition

The analysis condition for GC-orbitrap-MS is shown below.

Analytical Apparatus:

GC: TRACE1310 (Thermo Fisher Scientific)
MS: QExactiveGC (Thermo Fisher Scientific)

Analysis column: TG-WAXMS [length] 60 m [inner diameter] 0.25 mm [film thickness] 0.25 μm (Thermo Fisher Scientific)

Autosampler: TRIPLUS RSH (Thermo Fisher Scientific)
GC condition:

Injection method: Liquid injection
Injection volume: 1 μL

Gas: helium, 130 kPa (pressure)
Inlet temperature: 240 °C
Oven temperature: 40 °C (1 minute hold) - 10 °C/min - 110 °C - 2 °C/min - 180 °C - 3 °C/min - 240 °C - 10 °C/min-250 °C (6 min hold), 70 min in total

MS Condition:

Transfer temperature: 240 °C
Ion source temperature: 230 °C
Ionization method: EI positive
MS scan: m/z 30-450
Monitoring ions: shown in the table described below.

(3) Data Analysis

From GC-MS chromatogram, the precise mass of respective constituents (table described below) was extracted and the peak area was obtained. Constituents in respective specimen were compared by calculating the ratio of the peak area (= the peak area of the respective constituents / the peak area of the internal standard).

[Table 14]

| Constituents detected in GC-MS analysis and analysis condition | | |
|---|---|---|
| Constituent name | Retention time (min) | Precise mass |
| internal standard: 4-methylthiazole | 13.2 | 99.0137 |
| $\alpha$-terpinene-7-al | 32. 8 | 79. 0543 |
| carveol | 34. 2 | 109.0648 |
| nerol idol | 43. 6 | 67. 0543 |

2.3.3. Results of Constituent Analysis

The ratios of the peak area of cyclic peptides, organic acids (tartaric acid, malic acid, citric acid, *cis*-aconitic acid and *trans*-aconitic acid), sulfurol, sulfurol acetate, carveol, nerolidol and $\alpha$-terpinene-7-acetate detected in the specimen prepared from respective samples, to the peak area of the internal standard are shown in Figures 14 to 28. Bars in the figures represent standard deviations.

The results shown in Figures 14 to 28 indicate that the heat-treated cumin of Examples 301 to 319 included a larger amount of cyclic (Phe-Phe), cyclic (Phe-Leu/Ile), cyclic (Leu/Ile-Val), cyclic (Phe-Pro), cyclic (Phe-Tyr), cyclic (Pro-Leu/Ile), cyclic (Val-Val), cyclic (Phe-Ala), cyclic (Tyr-Ser), cyclic (Pro-Val), cyclic (Pro-Tyr), cyclic (Pro-Pro), cyclic (Leu/Ile-Ser), cyclic (Arg-Leu/Ile), cyclic (Ala-Tyr), cyclic (Gly-Tyr), cyclic (Ala-Pro), cyclic (Gly-Val), cyclic (hyPro-Pro), cyclic (Thr-Pro), cyclic (Val-Arg), cyclic (Pro-Asp), cyclic (Arg-Pro), cyclic (Glu-Tyr), cyclic (Pro-His), cyclic (Glu-Asp), cyclic (Gly-Arg), cyclic (Gly-His), cyclic (Leu/Ile-Leu/Ile), cyclic (Glu-Glu), cyclic (Leu/Ile-Asp), cyclic (Phe-Asp), tartaric acid, malic acid, citric acid, cis-aconitic acid, trans-aconitic acid, sulfurol, sulfurol acetate, carveol, nerolidol and $\alpha$-terpinene-7-al in comparison with the unheated cumin of Comparative Example 301. The heat-treated cumin of Examples 305 to 314 among others, which was heat-treated at 130 °C or 140 °C, exhibited especially significant increase in the aforesaid constituents. In particular, cyclic (Phe-Tyr), cyclic (Phe-Ala), cyclic (Tyr-Ser), cyclic (Leu/Ile-Ser), cyclic (Ala-Tyr), cyclic (Gly-Tyr), cyclic (Ala-Pro), cyclic (Gly-Val), cyclic (hyPro-Pro) and cyclic (Glu-Glu) are constituents specific to the heat-treated cumin, which are not present in the unheated cumin (below detection limit).

2.4. Flavor-enhancing Effect on Various Foodstuffs

(1) Sample Preparation

A sample of each of the foodstuff dilutions or of the food products shown in the table described below to which the heat-treated cumin of Example 305 was added, and its negative and positive control samples, were prepared in such a way as to add the heat-treated cumin of Example 305, instead of the heat-treated paprika powder of Example 203, in the method in (1) of 1.4. described above, so that the final concentration of the heat-treated cumin was 0.05% (w/w) in the foodstuff dilution sample or 0.03 to 0.07% (w/w) in the food product sample.

(2) Sensory Evaluation

Three evaluators tasted the samples prepared from the respective foodstuffs and evaluated the strength of the flavor of the samples. The criteria and method for the evaluation are as described in (2) of 1.4. described above.

The evaluation results are shown in the table described below. The heat-treated cumin of Example 305 was confirmed to have a strong effect of enhancing the flavor of various foodstuffs.

[Table 15]

| | | | Foodstuff concentration | Example 305 (cumin 130° C for 30 min, w/o water) | | | | |
|---|---|---|---|---|---|---|---|---|
| | | | | Average | Evaluator 1 | Evaluator 2 | Evaluator 3 | Evaluation |
| Basic taste | saltiness | sodium chloride | 0.5% | 4.0 | 4.0 | 4.0 | 4.0 | AA |
| | sweetness | granulated sugar | 1.0% | 1.5 | 1.0 | 2. 5 | 1.0 | C |
| | sourness | citric acid | 0.05% | 3.5 | 4.0 | 4.0 | 2.5 | AA |
| | | tartaric acid | 0.05% | 3.0 | 4.0 | 4.0 | 1.0 | A |
| | *umami* | mono sodium glutamate (MSG) | 0.25% | 2.0 | 1.0 | 4.0 | 1.0 | C |
| | | disodium succinate | 0.1% | 2. 5 | 1.0 | 4.0 | 2.5 | B |
| | | sodium inosinate | 0.1% | 2.0 | 1.0 | 1.0 | 4.0 | C |
| | | sodium inosinate + sodium guanylate | each 0.05% | 3.0 | 1.0 | 4.0 | 4.0 | A |
| | sweet *umami* | glycine + alanine | each 0.125% | 1.5 | 1.0 | 2. 5 | 1.0 | C |
| | pungency | chili pepper | 0.01% | 2.0 | 1.0 | 4.0 | 1.0 | C |
| | | black pepper | 0.05% | 2.0 | 1.0 | 1.0 | 4.0 | C |
| | | | | | | | | |
| *Dashi* ingredient | beef extract | | 0.5% | 2. 2 | 1.0 | 4. 5 | 1.0 | B |
| | chicken extract | | 0.5% | 2. 5 | 1.0 | 4.0 | 2.5 | B |
| | pork extract | | 0.5% | 1.5 | 1.0 | 1.0 | 2.5 | C |
| | bonito extract | | 0.5% | 1.5 | 1.0 | 1.0 | 2.5 | C |
| | small dried sardine extract | | 0.5% | 2.5 | 1.0 | 2. 5 | 4.0 | B |
| | tomato paste | | 1.5% | 2.5 | 1.0 | 2.5 | 4.0 | B |
| | garlic extract | | 0.5% | 2.0 | 2. 5 | 1.0 | 2.5 | C |
| | onion extract | | 0.5% | 2.5 | 1.0 | 4.0 | 2.5 | B |
| | | | | | | | | |

(continued)

| | | Foodstuff concentration | Example 305 (cumin 130° C for 30 min, w/o water) | | | | |
|---|---|---|---|---|---|---|---|
| | | | Average | Evaluator 1 | Evaluator 2 | Evaluator 3 | Evaluation |
| Seasoning | soy sauce | 1.2% | 3.0 | 1.0 | 4.0 | 4.0 | A |
| | *miso* paste | 3.3% | 2.5 | 1.0 | 4.0 | 2.5 | B |
| | ketchup | 6.0% | 2.5 | 1.0 | 4.0 | 2.5 | B |
| | worcestershire sauce | 2.2% | 2.5 | 1.0 | 2. 5 | 4.0 | B |
| | mayonnaise | 10.0% | 2.5 | 2. 5 | 1.0 | 4.0 | B |
| | noodle soup base | 1.65% | 2.5 | 1.0 | 4.0 | 2.5 | B |
| | cheese | 1.65% | 2.5 | 4.0 | 1.0 | 2.5 | B |
| Ingredient | caramel | 0.07% | 1.5 | 1.0 | 2. 5 | 1.0 | C |
| | banana paste | 2.5% | 2.0 | 1.0 | 2. 5 | 2.5 | C |
| | apple paste | 2.5% | 1.5 | 1.0 | 1.0 | 2.5 | C |
| | honey | 1.25% | 2.0 | 1.0 | 2. 5 | 2.5 | C |
| | roasted skimmed milk powder | 1.5% | 2.0 | 2. 5 | 1.0 | 2.5 | C |
| | yeast extract (main ingredient: baker's | 0.1% | 2. 5 | 1.0 | 4.0 | 2.5 | B |
| | protein hydrolysate | 0.1% | 1.5 | 1.0 | 1.0 | 2.5 | C |
| | curry powder A | 0.5% | 1.5 | 1.0 | 1.0 | 2.5 | C |
| | | | | | | | |
| Food product | curry (medium-hot) | | 3.0 | 4.0 | 2.5 | 2.5 | A |
| | cream stew | | 2.5 | 4.0 | 2. 5 | 1.0 | B |
| | spaghetti with meat sauce | | 3.0 | 2. 5 | 4.0 | 2.5 | A |
| | consomme soup with onion extract | | 2.5 | 2. 5 | 2.5 | 2.5 | B |
| | *miso* soup (ingredients: *tofu* and *wakame* | | 2.5 | 1.0 | 2. 5 | 4.0 | B |
| | *Sanuki* udon noodle | | 2.0 | 1.0 | 2. 5 | 2.5 | C |
| | Ramen noodle with soy sauce | | 3.5 | 4.0 | 2. 5 | 4.0 | AA |

3. Experiment 3: Flavor-enhancing Effect of Heat-treated Asafoetida

3.1. Preparation of Heat-treated Asafoetida

The asafoetida powder used in the following experiment includes 12% by mass of asafoetida (resin), 60% by mass of gum arabic and 28% by mass of rice flour. The asafoetida powder is a product made by dissolving asafoetida, mixing it with gum arabic and rice flour, drying the mixture, and then powdering the mixture. The aforesaid asafoetida powder was heat-treated under the conditions shown in the table described below. The heating values are values given by the integral of the CV value according to the aforesaid formula ("reference temperature" is 110 °C and "Z value" is 30 (°C)) with respect to the heating time (min), as previously described. The temperatures and time periods described in the heating condition column are maximum reached temperatures and their holding times, and the heating values were calculated on the basis of temperature-change over time, including the values of the temperature and time during temperature rise and fall.

[Table 16]

| | Heating condition | Heatin g value | Aw | Temp. (° C) |
|---|---|---|---|---|
| Comp. Ex. 401 | unheated | 0 | 0.4684 | 25.12 |
| Ex. 401 | 130° C for 30 min, w/o water | 168 | - | - |
| Ex. 402 | heated in oil at 110° C for 5 min | 8 | - | - |
| Ex. 403 | heated in oil at 130° C for 5 min | 27 | - | - |
| Ex. 404 | heated in oil at reached temp. of 140° C | 12 | - | - |

Comparative Example 401 is asafoetida powder without heat-treatment including heat sterilization.

Heating the asafoetida powder of Example 401 (130 °C for 30 min w/o water) was carried out according to the following procedure. An aluminum foil pouch was filled with 100 g of asafoetida powder and sealed. The sealed pouch was heat-treated in a retort sterilizer under the specified temperature and time period condition, and then cooled in water. The heat-treatment in the retort sterilizer was carried out under a gauge pressure of 0.2 MPa.

Heating asafoetida powder in oil in Examples 402, 403, or 404 was carried out according to the following procedure. Asafoetida powder (12% by mass of asafoetida) and palm oil (melting point of 45 °C) were mixed at a mass ratio of 50:50, heated with stirring under the specified temperature and time period condition, and then cooled, to obtain heated-in-oil asafoetida that was a curry-roux-like solid at room temperature.

The water activity (Aw) of the asafoetida powder of Comparative Example 401 was measured under the temperature condition shown in the table described above.

3.2. Flavor-enhancing Effect

Common and reduced-sodium curry rouxes were prepared according to the procedures described in (1) and (2) of 1.2. described above.

The aforesaid reduced-sodium curry roux was dispersed in hot water and boiled with stirring, to make base sauce.

The heat-in-oil asafoetida of Example 403 was added to the sauce so as to be in a final concentration of 0.0031% by mass, and a sensory evaluation was done. The amount of added asafoetida was reduced from the aforesaid concentration to an amount such that the characteristic aroma of spice did not affect the flavor of the roux, and a final concentration of 0.00077% by mass was set as the amount of asafoetida powder to be added of Comparative Example 401, asafoetida powder of Example 401, or heated-in-oil asafoetida (including oil or fat) of Examples 402, 403 or 404.

Two dispersions of 44 g of the aforesaid reduced-sodium curry roux dispersed in 300 g of hot water were prepared.

To one of them was added asafoetida of Comparative Example or any of Examples so as to be in the concentration set above.

Forty-four grams of the aforesaid common curry roux was dispersed in 300 g of hot water.

The dispersion of reduced-sodium curry roux dispersed in hot water and the dispersion of common curry roux dispersed in hot water were used as comparative controls, and the strength of the flavor was evaluated by three evaluators (evaluators 1, 2 and 3) on the basis of the following evaluation criteria from the viewpoint of "boosting of middle and last flavors." The "boosting of middle and last flavors" in this experiment are middle and last flavors concerted by the combination of sodium chloride and other foodstuffs in curry roux, and are aspects of "saltiness." Thus, the "flavor (boosting of middle and last flavors)" in this experiment can also be expressed as "saltiness (boosting of middle and last flavors)".

The scores of 1, 2, 3, 4 and 5 points for flavor (boosting of middle and last flavors) were determined as follows, and the three evaluators evaluated the flavor of respective samples in 0.1-point increments, to give an average score.

1 point: Comparable flavor to that of reduced-sodium curry roux
2 points: Slightly stronger flavor than reduced-sodium curry roux
3 points: Stronger flavor than reduced-sodium curry roux
4 points: Much stronger flavor than reduced-sodium curry roux
5 points: Comparable strength of flavor to that of common curry roux

The average of the evaluated scores was defined as "C" when it was higher than that of Comparative Example 401 and 2.0 or lower, "B" when it was 2.1 or higher and 2.5 or lower, "A" when it was 2.6 or higher and 3.0 or lower, and "AA" when it was 3.1 or higher and 5.0 or lower.

The evaluation results are shown in the table described below. The heat-treated asafoetida of Examples 401 to 404 was confirmed to have a stronger effect of enhancing the "boosting of middle and last flavors" in the flavor in comparison with the asafoetida of Comparative Example 401.

[Table 17]

| Evaluation of "boosting of middle- and last-flavors" | | | | | |
|---|---|---|---|---|---|
| | Average | Evaluator 1 | Evaluator 2 | Evaluator 3 | Evaluation |
| Comp. Ex. 401 | 2.0 | 2.0 | 2.0 | 2.0 | - |
| Ex. 401 | 2.7 | 2.5 | 3.0 | 2.5 | A |
| Ex. 402 | 2.9 | 3.3 | 3.0 | 2.5 | A |
| Ex. 403 | 3. 7 | 3.0 | 4.0 | 4.0 | AA |
| Ex. 404 | 2.8 | 2.8 | 2.5 | 3.0 | A |

3.3. Constituent Analysis

Constituents in the asafoetida of Comparative Example 401 and Examples 401 to 404 were analyzed according to the following procedure.

(1) LC-MS/MS Test Solution Preparation

Into a 10-mL test tube was taken 200 mg of the asafoetida of Comparative Example 401, or Example 401, or 400 mg of heated-in-oil asafoetida of Example 402, 403 or 404 (200 mg as a mass converted under the assumption that the asafoetida resin content was 12% by mass), and 5 mL of ultrapure water was added. The test tube was kept heated for 30 minutes in a thermostatic water bath set to 80 °C and then left to stand down to room temperature. To the test tube was added 5 mL of acetonitrile (FUJIFILM Wako Pure Chemicals), and furthermore, L-lysine-$^{13}C_6$ monohydrochloride (FUJIFILM Wako Pure Chemicals) and ribitol (Fujifilm Wako Pure Chemicals) were added as an internal standard in Positive mode and an internal standard in Negative mode, respectively. L-lysine-$^{13}C_6$ monohydrochloride and ribitol were added so as to be 47 μg/g and 99 μg/g with respect to the asafoetida (mass converted under the assumption that the asafoetida resin content is 12% by mass for the heated-in-oil asafoetida of Examples 402, 403 and 404), respectively. The test tube was shaken with a high-speed shaker (CM-1000, TOKYO RIKAKIKAI CO., LTD.) at room temperature and 1,800 rpm for 30 minutes, and after centrifugation, 0.5 mL of the solution in the test tube was transferred to an ultrafiltration filter (Nanosep Centrifugal Filtration Device 3K, Nippon Paul). After centrifuging the ultrafiltration filter at room temperature and 15,000 rpm x 20 minutes, 0.3 mL of the filtrate under the filter was loaded onto a solid phase extraction column InertSep C18 (100 mg/l mL, GL Science) and an eluate was collected. InertSep C18 was conditioned with 1 mL of 50% (w/w) acetonitrile water before loading the filtrate. Then, 0.5 mL of acetonitrile was loaded into InertSep C18 and an eluate was collected. To the eluate was added 1.5 mL of ultrapure water, which was then vortexed for 10 seconds. The solution after passing through a 0.2 μm filter was used as an LC-MS/MS specimen (n=2).

(2) LC-MS/MS Analysis Condition

LC-MS/MS analysis was carried out under the condition described in (2) of 1.3. described above.

(3) Data Analysis

From LC-MS/MS chromatogram, the precise mass of the precursor ion > the product ion (table described below) of respective constituents was extracted and the peak area was obtained. Constituents in respective specimens were compared by calculating the ratio of the peak area of product ions (= the peak area of the product ion of the respective constituents / the peak area of the product ion of the internal standard). In the table described below, A1 and A2 indicate two amino acids constituting respective cyclic dipeptides.

[Table 18]

| Constituents detected in LC-MS/MS analysis (other than cyclic dipeptides) and analysis condition (Positive mode) | | | |
|---|---|---|---|
| Constituent name | Retention time (min) | [M+H] | |
| | | Precursor Ion | Product Ion |
| internal standard: L-lysine-$^{13}C_6$ | 8.0 | 153.1327 | 89.0974 |
| 4-methyl-5- | 35.5 | 144.0477 | 113.0293 |
| thiazoleethanol (sulfurol) | | | |

[Table 19]

| Constituents detected in LC-MS/MS analysis (cyclic dipeptides) and analysis condition (Positive mode) | | | | |
|---|---|---|---|---|
| Const i tuent name : cyclic (A1-A2) | | Retention | [M+H] | |
| A1 | A2 | time (min) | Precursor Ion | Product Ion |
| Gly | Thr | 12.6 | 159.0765 | 113.0710 |
| Gly | His | 9.5 | 195.0877 | 122.0712 |
| Ala | Asp | 17.3 | 187.0714 | 99.0550 |
| Val | Ser | 33. 9 | 187.1078 | 72. 0807 |
| Gly | Arg | 12.8 | 214.1299 | 155.0815 |
| Ala | His | 11.3 | 209.1034 | 110.0713 |
| Glu | Asp | 22. 1 | 245. 0769 | 84.0441 |
| Phe | Phe | 51.6 | 295.1442 | 120.0806 |
| Leu/Ile | Leu/Ile | 50. 5 | 227.1755 | 86. 0962 |
| Ala | Arg | 15. 9 | 228.1456 | 169.0970 |
| Leu/Ile | Val | 48.5 | 213.1599 | 72.0806 |
| Phe | Pro | 48. 3 | 245.1285 | 120.0806 |
| Phe | Leu/Ile | 50. 7 | 261.1598 | 120.0806 |
| Pro | Pro | 39. 2 | 195. 1128 | 70. 0649 |
| Glu | Phe | 46. 1 | 277. 1183 | 120.0806 |
| Arg | Leu/Ile | 37.0 | 270.1925 | 211. 1438 |
| Pro | Leu/Ile | 46. 9 | 211.1442 | 70. 0650 |
| Pro | Glu | 36. 7 | 227.1027 | 84. 0443 |
| Glu | Leu/Ile | 44.5 | 243.1340 | 86. 0962 |
| Ala | Pro | 36. 6 | 169. 0968 | 70. 0649 |
| Pro | Val | 42. 9 | 197.1285 | 70. 0650 |
| Pro | His | 29. 5 | 235. 1190 | 110.0712 |

[Table 20]

| Constituents detected in LC-MS/MS analysis and analysis condition (Negative mode) | | | |
|---|---|---|---|
| Constituent name | Retention time (min) | [M-H] | |
| | | Precursor Ion | Product Ion |
| internal standard: ribitol | 9. 7 | 151. 0607 | 71.0138 |
| tartaric acid | 10.6 | 149.0086 | 72.9931 |

(continued)

| Constituent name | Retention time (min) | [M-H] | |
|---|---|---|---|
| | | Precursor Ion | Product Ion |
| malic acid | 13. 1 | 133.0142 | 71.0138 |
| citric acid | 19.0 | 191.0197 | 111.0087 |

The ratios of the peak area of cyclic peptides, organic acids (tartaric acid, malic acid, citric acid) and sulfurol detected in LC-MS/MS specimens prepared from the respective samples, to the peak area of the internal standard are shown in Figures 29 to 35. Bars in the figures represent standard deviations.

The results shown in Figures 29 to 35 indicate that the heat-treated asafoetida of Examples 401 to 404 included a larger amount of cyclic (Gly-Thr), cyclic (Gly-His), cyclic (Ala -Asp), cyclic (Val-Ser), cyclic (Gly-Arg), cyclic (Ala-His), cyclic (Glu-Asp), cyclic (Phe -Phe), cyclic (Leu/Ile-Leu/Ile), cyclic (Ala-Arg), cyclic (Leu/Ile-Val), cyclic (Phe-Pro), cyclic (Phe-Leu/Ile), cyclic (Pro-Pro), cyclic (Glu-Phe), cyclic (Arg-Leu/Ile), cyclic (Pro-Leu/Ile), cyclic (Pro-Glu), cyclic (Glu-Leu/Ile), cyclic (Ala-Pro), cyclic (Pro-Val), cyclic (Pro-His), tartaric acid, malic acid, citric acid and sulfurol in comparison with the unheated asafoetida of Comparative Example 401. In particular, the aforesaid constituents were significantly increased in the heated-in-oil asafoetida of Examples 402 to 404 that exhibited a strong flavor-enhancing effect. The amount of these constituents showed a tendency to correlate with the strength of the flavor-enhancing effect. In particular, cyclic (Val-Ser), cyclic (Pro-Pro) and cyclic (Pro-Glu) are constituents that are not included in unheated asafoetida (below detection limit) and are specific to heat-treated asafoetida.

3.4. Flavor-enhancing Effect on Various Foodstuffs

(1) Sample Preparation

A sample of each of the foodstuff dilutions or of the food products shown in the table described below to which the heat-treated asafoetida of Example 403 was added, and its negative and positive control samples, were prepared in such a way as to add the heat-treated cumin of Example 403, instead of the heat-treated paprika powder of Example 203, in the method in (1) of 1.4. described above, so that the final concentration of the heat-treated asafoetida was 0.025% (w/w) in the foodstuff dilution sample or 0.015 to 0.035% (w/w) in the food product sample.

(2) Sensory Evaluation

Three evaluators tasted the samples prepared from the respective foodstuffs and evaluated the strength of the flavor of the samples. The criteria and method for the evaluation are described in (2) of 1.4. described above.

The evaluation results are shown in the table described below. The heat-treated asafoetida of Example 403 was confirmed to have a stronger effect of enhancing the flavor of various foodstuffs.

[Table 21]

| | | | Foodstuff concentration | Example 403 (asafoetida heated in oil at 130° C for 5 min) | | | | |
|---|---|---|---|---|---|---|---|---|
| | | | | Average | Evaluator 1 | Evaluator 2 | Evaluator 3 | Evaluation |
| Basic taste | saltiness | sodium chloride | 0.5% | 4.0 | 4.0 | 4.0 | 4.0 | AA |
| | sweetness | granulated sugar | 1.0% | 2.5 | 4.0 | 2.5 | 1.0 | B |
| | sourness | citric acid | 0.05% | 3.0 | 1.0 | 4.0 | 4.0 | A |
| | | tartaric acid | 0.05% | 2. 5 | 4.0 | 2. 5 | 1.0 | B |
| | bitterness | naringin | 0.05% | 3. 2 | 1.0 | 4.0 | 4. 5 | AA |
| | *umami* | mono sodium glutamate (MSG) | 0.25% | 2. 5 | 2. 5 | 4.0 | 1.0 | B |
| | | sodium L-aspartate | 0.25% | 2.0 | 1.0 | 1.0 | 4.0 | C |
| | | disodium succinate | 0.1% | 3. 5 | 2. 5 | 4.0 | 4. 0 | AA |
| | | sodium inosinate | 0.1% | 3. 0 | 1.0 | 4.0 | 4.0 | A |
| | | sodium inosinate + sodium guanylate | each 0.05% | 2.5 | 1.0 | 4.0 | 2. 5 | B |
| | sweet *umami* | glycine + alanine | each 0.125% | 1.5 | 1.0 | 2. 5 | 1.0 | C |
| | pungency | chili pepper | 0.01% | 2.0 | 1.0 | 1.0 | 4. 0 | C |
| | | black pepper | 0.05% | 4.0 | 4.0 | 4.0 | 4.0 | AA |
| | | | | | | | | |
| *Dashi* ingredient | beef extract | | 0.5% | 3. 5 | 2. 5 | 4.0 | 4.0 | AA |
| | chicken extract | | 0.5% | 1.5 | 1.0 | 1.0 | 2. 5 | C |
| | pork extract | | 0.5% | 3.7 | 2. 5 | 4.0 | 4. 5 | AA |
| | bonito extract | | 0.5% | 3. 0 | 1.0 | 4.0 | 4.0 | A |
| | small dried sardine extract | | 0.5% | 3. 5 | 2. 5 | 4.0 | 4.0 | AA |
| | tomato paste | | 1.5% | 3. 5 | 2. 5 | 4.0 | 4.0 | AA |
| | garlic extract | | 0.5% | 3.5 | 4.0 | 2. 5 | 4.0 | AA |
| | onion extract | | 0.5% | 4.0 | 4.0 | 4.0 | 4.0 | AA |
| | | | | | | | | |

(continued)

| | | Foodstuff concentration | Example 403 (asafoetida heated in oil at 130° C for 5 min) | | | | |
|---|---|---|---|---|---|---|---|
| | | | Average | Evaluator 1 | Evaluator 2 | Evaluator 3 | Evaluation |
| Seasoning | soy sauce | 1.2% | 2. 0 | 1.0 | 4.0 | 1.0 | C |
| | *miso* paste | 3.3% | 3.0 | 1.0 | 4.0 | 4.0 | A |
| | ketchup | 6.0% | 2. 7 | 1.0 | 4.5 | 2. 5 | B |
| | worcestershire sauce | 2.2% | 2. 5 | 2. 5 | 1.0 | 4. 0 | B |
| | mayonnaise | 10.0% | 3.2 | 1.0 | 4.5 | 4.0 | AA |
| | noodle soup base | 1.65% | 2. 0 | 1.0 | 4.0 | 1.0 | C |
| | cheese | 1.65% | 4.0 | 4.0 | 4.0 | 4.0 | AA |
| Ingredient | caramel | 0.07% | 2. 5 | 2. 5 | 1.0 | 4.0 | B |
| | banana paste | 2.5% | 1.5 | 1.0 | 1.0 | 2. 5 | C |
| | honey | 1.25% | 2. 0 | 1.0 | 4.0 | 1.0 | C |
| | soybean skimmed | 1.5% | 2. 5 | 1.0 | 4.0 | 2. 5 | B |
| | full-fat powdered milk | 1.5% | 3.5 | 2. 5 | 4.0 | 4.0 | AA |
| | roasted skimmed milk powder | 1.5% | 2.0 | 1.0 | 4.0 | 1.0 | C |
| | yeast extract (main ingredient: | 0.1% | 4.0 | 4.0 | 4.0 | 4.0 | AA |
| | protein hydrolysate | 0.1% | 2. 5 | 2. 5 | 4.0 | 1.0 | B |
| | curry powder A | 0.5% | 2. 5 | 2. 5 | 1.0 | 4.0 | B |
| | curry powder B | 0.5% | 2.0 | 1.0 | 1.0 | 4.0 | C |
| | | | | | | | |
| Food product | curry (medium-hot) | | 4.3 | 4. 5 | 4. 5 | 4.0 | AA |
| | cream stew | | 4.0 | 4.0 | 4.0 | 4.0 | AA |
| | spaghetti with meat sauce | | 3. 5 | 4.0 | 4.0 | 2. 5 | AA |
| | consomme soup with onion extract | | 3. 5 | 4.0 | 4.0 | 2. 5 | AA |
| | *miso* soup (ingredients: *tofu* and | | 3. 5 | 2. 5 | 4.0 | 4. 0 | AA |
| | *Sanuki* udon noodle | | 3. 0 | 1.0 | 4.0 | 4. 0 | A |
| | Ramen noodle with soy sauce | | 3. 5 | 4.0 | 4.0 | 2. 5 | AA |

Experiment 4: Flavor-Enhancing Effect of Heat-treated Mustard Seed

4.1 Preparation of Heat-treated Mustard Seed

Whole mustard seed (seed of brown mustard (*Brassica juncea*)) was heat-treated under the conditions of Examples 501 to 505 and Comparative Examples 501 to 506 shown below. The heating values are values given by the integral of the CV value according to the above-described formula ("reference temperature" is 110 °C and "Z value" is 30 (°C)) with respect to the heating time (min). The temperatures and time periods described in the heating condition column are maximum reached temperatures and their holding times, and the heating values were calculated on the basis of temperature-change over time, including the values of the temperature and time during temperature rise and fall (however, for oven heating in Example 502, the heating value was calculated under the assumption that the product was held at the finished product temperature for 4 minutes). As the experiments described below were carried out in a temperature-controlled room where the temperature was largely controlled within the range of 22 °C to 25 °C by air conditioning at a preset temperature of 23 °C, "room temperature" refers to 22 °C to 25 °C, for example, 23 °C, unless otherwise stated.

Example 501

(1) Into a home-use coffee roaster ("Tiisana-baisen-shokunin" from YAMAYASU INC.) (open system) was put 200 g of whole mustard seed.
(2) The pot was rotated and stirred at 18.5 rpm (maximum rotational speed).
(3) Ignition was turned on and the mustard seed was heated with stirring until the product temperature of the mustard seed reached 190 °C (It took 9 minutes until the temperature was reached).
(4) The product was then heated with stirring at 190 °C for 8 minutes.
At that time, the wall temperature of the roasting machine was 230 °C. The temperature of the mustard seed was measured with a radiation thermometer.
(5) The heat-treated mustard seed was removed to a vat and allowed to cool to room temperature.

Example 502

(1) On a baking tray at room temperature, a cooking paper was laid, on which 50 g of the whole mustard seeds were arranged so as not to overlap each other.
(2) The aforesaid baking tray was placed into the lower section of a preheated 250 °C oven.
(3) The whole mustard seeds were heat-treated in the aforesaid 250 °C oven for 4 minutes.
The product temperature of the mustard seed at the end of the heat-treatment (finished product temperature) was 195 °C. The product temperature was measured with a radiation thermometer.
(4) The heat-treated mustard seed was removed from the oven and allowed to cool to room temperature.

Example 503

(1) Into an electromagnetic heating type flat pot (open system) was loaded 100 kg of whole mustard seed.
(2) The pot was heated for 24 minutes, rotationally stirred at 20 rpm, until the product temperature reached 169 °C.
(3) The heating was stopped and the product was cooled to a product temperature of 90 °C with stirring in the same pot.

Example 504

Example 504 was prepared by heat-treating mustard seed according to the procedure described in Example 501, except that the mustard seed was heated to the product temperature of 160 °C for 6 minutes in (3) and at the product temperature of 160 °C for 13 minutes in (4). The wall temperature of the roasting machine during heating in (3) and (4) was set to be 180 °C.

Example 505

Example 505 was prepared by heat-treating mustard seed according to the procedure described in Example 501, except that the mustard seed was heated to a product temperature of 140 °C for 7 minutes in (3) and at the product temperature of 140 °C for 20 minutes in (4). The wall temperature of the roasting machine during heating in (3) and (4) was set to be 160 °C.

Comparative Example 501

Whole mustard seed used as a raw material in Examples 501 to 505 and Comparative Examples 502 to 506 was used as unheated Comparative Example 501.

Comparative Example 502

Comparative Example 502 was prepared by heat-treating mustard seed according to the procedure described in Example 501, except that the mustard seed was heated to a product temperature of 130 °C for 6 minutes in (3) and at the product temperature of 130 °C for 15 minutes in (4). The wall temperature of the roasting machine during heating in (3) and (4) was set to be 140 °C.

Comparative Example 503

(1) A retort pouch of 1 kg capacity was filled with 100 g of whole mustard seed and sealed.
(2) The pouch was heated in a retort sterilizer to the internal temperature of its chamber of 130 °C for 16 minutes, and further heated for 30 minutes (spray type, pressure 0.2 MPa), with the internal temperature kept at 130 °C. The heating temperature is a value from a temperature sensor inside the chamber.
(3) After heating, the pouch was cooled in water for 10 minutes and then removed from the sterilizer.

Comparative Example 504

Comparative Example 504 was prepared by heat-treating mustard seed according to the procedure described in Comparative Example 503, except that 100 g of whole mustard seed and 10 g of water were put into a retort pouch of 1 kg capacity, mixed uniformly, and sealed in (1).

Comparative Example 505

Comparative example 505 was prepared by heat-treating mustard seed according to the procedure described in Comparative example 503, except that the mustard seed was heated at 130 °C for 60 minutes in (2).

Comparative Example 506

(1) In a household pot, 100 g of solid palm-derived oil that was a solid at room temperature was heated to melt and then allowed to cool to 60 °C.

(2) To the aforesaid molten oil was added 100 g of whole mustard seed and heated over low to medium heat to 180 °C with stirring (it took 8 minutes until reached temperature). The product temperature was measured with a temperature sensor inserted into the molten oil or fat.

(3) After heating, the whole pot was cooled in water and a uniform paste was obtained by stirring.

(4) The aforesaid paste was put into a plastic bag, which was stretched flat, and quenched in a freezer.

The water activity (Aw) of the mustard seed in respective Examples and Comparative Examples was measured under the respective temperature conditions described in the table described below.

[Table 22]

| | Heating condition | Heating value | Aw | Aw mea. temp. (° C) |
|---|---|---|---|---|
| Ex. 501 | product temp. 190° C for 8 min, open system | 5132 | 0. 1780 | 25. 02 |
| Ex. 502 | heated to finished product temp. 195° C for 4min, open system | 2725 | 0.0820 | 25.04 |
| Ex. 503 | heated to product temp. 169° C for 24 min, open system | 1001 | 0.0461 | 25.03 |
| Ex. 504 | product temp. 160° C for 13 min, open system | 701 | 0. 1866 | 25.03 |
| Ex. 505 | product temp. 140° C for 20 min, open system | 220 | 0. 1206 | 25. 10 |

(continued)

| | Heating condition | Heating value | Aw | Aw mea. temp. (° C) |
|---|---|---|---|---|
| Comp. Ex. 501 | unheated | - | 0.4917 | 25.04 |
| Comp. Ex. 502 | product temp. 130° C for 15 min, open system | 91 | 0. 1498 | 25.07 |
| Comp. Ex. 503 | product temp. about 130° C for 30 min, w/o water, pressurized closed system | 167 | 0. 6505 | 25.00 |
| Comp. Ex. 504 | product temp. about 130° C for 30 min, w/ water, pressurized closed system | 167 | 0. 8588 | 24. 98 |
| Comp. Ex. 505 | product temp. about 130° C for 60 min, w/o water, pressurized closed system | 263 | 0.6866 | 25.01 |
| Comp. Ex. 506 | roasted in oil at reached temp. of 180° C (heated for 8 min until reached temp.) | 414 | 0.2128 | 25.04 |

The mustard seed of Examples 501 to 505 and Comparative Examples 501 to 506 described above was pulverized at 30 g/20 seconds using a household mill (Iwatani Corporation, Crack Millser) for Aroma evaluation, Flavor Evaluation and Constituent Analysis below.

4.2. Aroma Evaluation (simple system)

Into a 200-mL beaker was put 1.0 g of the pulverized mustard seed sample of any of Examples and Comparative Examples, and then the beaker was covered with a stainless-steel lid, and left to stand. Two evaluators removed the lid, smelled the aroma, and rated "sweet aroma" and "roasted aroma" according to the following criteria. The lid was removed only when the evaluators smelled the aroma.

Characteristic flavors, i.e., "sweet aroma" and "roasted aroma" were scored according to the following criteria. Scores of down to 3 points were considered to be preferred for roasted mustard seed.

5 points: Strong and characteristic flavor
4 points: Characteristic flavor
3 points: Slightly weak but characteristic flavor
2 points: Weakly characteristic flavor
1 point: Slightly characteristic flavor
0 points: No characteristic flavor

The average scores of "sweet aroma" and "roasted aroma" by the two evaluators according to the criteria described above were calculated. The results and the evaluators' comments are shown in the table described below. The two evaluators also scored balance between "sweet aroma" and "roasted aroma" with the best case scoring 5 points and the worst case scoring 0 points, and the average value of the two is shown in the "overall" column.

[Table 23]

| | Sweet aroma | Roasted aroma | Overall | Comment |
|---|---|---|---|---|
| Ex. 501 | 5 | 5 | 5 | sweet aroma and roasted aroma (almond aroma) |
| Ex. 502 | 4. 5 | 4. 5 | 4.5 | slightly weak |
| Ex. 503 | 5 | 5 | 5 | sweet aroma and roasted aroma (almond aroma) |
| Ex. 504 | 4 | 4 | 4 | slightly weak |
| Ex. 505 | 4 | 2 | 3 | sweet aroma is stronger than roasted aroma |
| Comp. Ex. 501 | 0 | 0 | 0 | almost no aroma |
| Comp. Ex. 502 | 1 | 1 | 1 | slightly roasted aroma, roasted sesame-l ike |
| Comp. Ex. 503 | 2 | 2 | 2 | caramel ized-aroma, exhibiting a mustard-oi l-like sharp aroma |

(continued)

| | Sweet aroma | Roasted aroma | Overall | Comment |
|---|---|---|---|---|
| Comp. Ex. 504 | 3 | 1 | 2 | aroma of Comp. Ex. 503 imparted with matured flavor |
| Comp. Ex. 505 | 1 | 3 | 2 | firm flavor with somewhat more burnt aroma than Comp. Ex. 503 |
| Comp. Ex. 506 | 2 | 2 | 2 | no refreshing aroma |

4.3. Flavor Evaluation (simple system)

Into a 200-mL beaker was put 1.0 g of the pulverized mustard seed sample of any of Examples and Comparative Examples, and 200 mL of hot water was added and stirred. Two evaluators tasted the sample, and evaluated and scored the "sweetness" and "roasted flavor" according to the same criteria as in 4.2. above ("sweetness" and "roasted flavor" were defined as "characteristic flavors" in 4.2. described above).

The average of the evaluated scores of "sweetness" and "roasted flavor" by the two evaluators according to the criteria described above was calculated. The results and the evaluators' comments are shown in the table described below. The two evaluators also scored a balance between "sweetness" and "roasted flavor" with the best case scoring 5 points and the worst case scoring 0 points, and the average value of the two shown in the "overall" column.

[Table 24]

| | Sweetnes s | Roasted flavor | Overall | Comment |
|---|---|---|---|---|
| Ex. 501 | 5 | 5 | 5 | sweet and roasted flavors, with aftertaste |
| Ex. 502 | 5 | 5 | 5 | sweet and roasted flavors, with aftertaste |
| Ex. 503 | 4 | 5 | 4.5 | sweet flavor lost, slightly strong roasted flavor |
| Ex. 504 | 4 | 4 | 4 | both flavors are week |
| Ex. 505 | 4 | 2 | 3 | roasted flavor that is neither strong nor firm, but has soft and sweet aftertaste |
| Comp. Ex. 501 | 0 | 0 | 0 | week and last astringent taste |
| Comp. Ex. 502 | 1 | 1 | 1 | roasted flavor like sweet soybean flour, simply sweet |
| Comp. Ex. 503 | 2 | 0 | 1 | no roasted flavor, a little sweet |
| Comp. Ex. 504 | 2 | 0 | 1 | no roasted flavor, a little sweet |
| Comp. Ex. 505 | 0 | 3 | 2 | only roasted flavor, no sweetness |
| Comp. Ex. 506 | 0 | 3 | 2 | only roasted flavor, no sweetness |

4.4. Effect of Enhancing Richness of Curry

In 300 mL of hot water was dispersed 44.5 g of the commercially available curry roux, which sample was used as a positive control.

In 330 mL of hot water was dispersed 44.5 g of the aforesaid commercially available curry roux (i.e., 90% concentration of the positive control), which sample was used as a negative control.

In 330 mL of hot water (the same amount as that of the negative control) was dispersed 44.5 g of the aforesaid commercially available curry roux, and further 0.11 g of the pulverized product of mustard seed of Example 501, Example 503, Example 505, Comparative Example 501 or Comparative Example 502 was added, which sample was used as a sample of each of the Examples and Comparative Examples.

Three evaluators tasted each of the samples of the positive control, the negative control and respective Examples and Comparative Examples, and scored richness of the sample of respective Example and Comparative Examples according to the following criteria, with the positive control scoring 5 points and the negative control scoring 1 point.

5 points: Comparable to positive control

4 points: Strong
3 points: Slightly strong
2 points: Weak
1 point: Comparable to negative control

The average score of richness evaluated by the three evaluators according to the criteria described above was calculated. The results and the evaluators' comments are shown in the table described below.

[Table 25]

| Sample | Evaluator | Score | Comment | Average score from three evaluators |
|---|---|---|---|---|
| Ex. 501 | 1 | 4.0 | high profile from top, onset of slight roasted flavor | 4.0 |
| | 2 | 4.0 | mild richness and roasted flavor | |
| | 3 | 4.0 | roasted aftertaste | |
| Ex. 503 | 1 | 4.0 | profile close to Ex. 501, suppressed roasted flavor | 3.8 |
| | 2 | 3.5 | middle mild richness, but less lasting probably owing to less roasted flavor than Ex. 501 | |
| | 3 | 4.0 | more expanding and milder than Ex. 501 | |
| Ex. 505 | 1 | 3.5 | slight lower top profile than Ex. 503, but exhibits firm middle-to-last flavor | 3.5 |
| | 2 | 4.0 | mild richness and roasted flavor, top and last saltiness | |
| | 3 | 3.0 | milder | |
| Comp. Ex. 501 | 1 | 2.0 | exhibits no firm taste, watery | 1.8 |
| | 2 | 2.0 | exhibits mustard-like pungency | |
| | 3 | 1.5 | strong top profile owing to pungency | |
| Comp. Ex. 505 | 1 | 2.5 | watery, but stronger flavor than Comp. Ex. 501 | 2.3 |
| | 2 | 2.5 | vague rise of flavor | |
| | 3 | 2.0 | watery, not firm | |

4.5. Analysis of Cyclic Dipeptides

(1) LC-MS/MS Test Solution Preparation

Into a 10-mL test tube was taken 200 mg of the pulverized product of mustard seed of Example 501, 502, 503, 504 or 505, or Comparative Example 501, 502, 503, 504 or 505, or 400 mg of the pulverized heated-in-oil mustard seed of Comparative Example 506 (200 mg as a mass converted to that of mustard seed), and 5 mL of ultrapure water was added. The test tube was kept heated for 30 minutes in a thermostatic water bath set to 80 °C and then left to stand down to room temperature. To the test tube was added 5 mL of acetonitrile (FUJIFILM Wako Pure Chemicals), and L-lysine-$^{13}C_6$ monohydrochloride (FUJIFILM Wako Pure Chemicals) was added as an internal standard so as to be 47 $\mu$g/g with respect to the mustard seed. The test tube was shaken with a high-speed shaker (CM-1000, TOKYO RIKAKIKAI CO., LTD.) at room temperature and 1,800 rpm for 30 minutes. After centrifugation, 0.5 mL of the solution in the test tube was transferred to an ultrafiltration filter (Nanosep Centrifugal Filtration Device 3K, Nippon Paul). After centrifuging the ultrafiltration filter at room temperature and 15,000 rpm x 20 minutes, 0.5 mL of ultrapure water was added to the filtrate under the filter, which was then vortexed for 10 seconds. The solution after passing through a 0.2 $\mu$m filter was used as an LC-MS/MS specimen (n=3).

(2) LC-MS/MS Analysis Condition

LC-MS/MS analysis was carried out under the condition described in (2) of 1.3. described above.

(3) Data Analysis

From LC-MS/MS chromatogram, the precise mass of the precursor ion > the product ion of respective constituents was extracted and the peak area was obtained. Constituents in respective specimens were compared by calculating the ratio of the peak area of product ions (= the peak area of the product ion of the respective constituents / the peak area of the product ion of the internal standard). In the table described below, A1 and A2 indicate two amino acids constituting respective cyclic dipeptides.

[Table 26]

| LC-MS/MS analysis condition for internal standard | | | |
|---|---|---|---|
| Constituent name | Retention time (min) | M+H | |
| | | Precursor Ion | Product Ion |
| internal standard: L-lysine-$^{13}C_6$ | 8.0 | 153. 1327 | 89.0974 |

[Table 27]

| LC-MS/MS analysis condition for cyclic dipeptides | | | | | |
|---|---|---|---|---|---|
| | Cyclic (A1-A2) | | Retention time (min) | M+H | |
| | A1 | A2 | | Precursor Ion | Product Ion |
| 1 | Glu | His | 14.1 | 267. 1088 | 110.0712 |
| 2 | Gly | Arg | 12.8 | 214. 1299 | 155.0815 |
| 3 | Glu | Gly | 18. 2 | 187. 0714 | 84. 0441 |
| 4 | Ala | His | 11.3 | 209. 1034 | 110. 0713 |
| 5 | Ala | Arg | 15. 9 | 228. 1456 | 169. 0970 |
| 6 | Glu | Asp | 22. 1 | 245. 0769 | 84. 0441 |
| 7 | Leu/Ile | Asp | 41. 0 | 229. 1183 | 141. 1022 |
| 8 | Thr | Pro | 35. 7 | 199.1078 | 125. 0707 |
| 9 | Pro | His | 29. 5 | 235. 1190 | 110. 0712 |
| 10 | Pro | Pro | 39. 2 | 195. 1128 | 70. 0649 |
| 11 | Glu | Tyr | 34. 5 | 293. 1133 | 247. 1078 |
| 12 | Arg | Leu/Ile | 37. 0 | 270. 1925 | 211. 1438 |
| 13 | Ala | Pro | 36. 6 | 169. 0968 | 70. 0649 |
| 14 | Pro | Val | 42. 9 | 197. 1285 | 70.0650 |
| 15 | Phe | Ala | 44. 9 | 219. 1128 | 120.0808 |
| 16 | Phe | Pro | 48. 3 | 245. 1285 | 120.0808 |
| 17 | Phe | Tyr | 47. 6 | 311. 1391 | 136. 0754 |
| 18 | Tyr | His | 33. 7 | 301. 1296 | 110. 0713 |
| 19 | Leu/Ile | His | 36. 8 | 251. 1503 | 110. 0713 |
| 20 | Pro | Met | 43. 1 | 229. 1006 | 181. 0972 |
| 21 | Val | Glu | 40. 0 | 229. 1183 | 84. 0441 |

The ratios of the peak area of cyclic peptides detected in LC-MS/MS specimens prepared from the respective samples to the peak area of the internal standard are shown in Figures 36 to 41. Bars in the figures represent standard deviations.

The results shown in Figures 36 to 41 indicate that the mustard seed of Examples 501 to 505 included a larger amount of cyclic (Glu-His), cyclic (Gly-Arg), cyclic (Glu-Gly), cyclic (Ala-His), cyclic (Ala-Arg), cyclic (Glu-Asp), cyclic (Leu/Ile-Asp), cyclic (Thr-Pro), cyclic (Pro-His), cyclic (Pro-Pro), cyclic (Glu-Tyr), cyclic (Arg-Leu/Ile), cyclic (Ala-Pro), cyclic (Pro-Val), cyclic (Phe-Ala), cyclic (Phe-Pro), cyclic (Phe-Tyr), cyclic (Tyr-His), cyclic (Leu/Ile-His), cyclic (Pro-Met) and cyclic (Val-Glu) in comparison with the mustard seed of Comparative Examples 501 and 506. The amount of these cyclic dipeptides is considered to relate to the strength of sweetness and roasted flavor, as well as to the richness-enhancing effect of mustard seed.

4.6. Analysis of Constituents Other than Cyclic Dipeptides

(1) GC-MS Test Solution Preparation

Into a 10-mL test tube was taken 15 mg of the pulverized product of the mustard seed sample of Example 501, 502, 503, 504 or 505, or Comparative Examples 501, 502, 503, 504 or 505, or 30 mg of the pulverized product of the heated-in-oil mustard seed of Comparative Example 506 (15 mg as a mass converted to that of mustard seed), and 5 mL of acetone (FUJIFILM Wako Pure Chemicals) and 5 mL of methanol (FUJIFILM Wako Pure Chemicals) were added. As an internal standard, 4-methylthiazole (TOKYO CHEMICAL INDUSTRY CO., LTD) was added so as to be 625 μg/g with respect to the mustard seed. The test tube was shaken with a high-speed shaker (CM-1000, TOKYO RIKAKIKAI CO., LTD.) at room temperature and 1,800 rpm for 30 minutes. After stirring, centrifugation was carried out, and then 0.1 mL of the solution in the test tube was taken into a GC-MS vial, and 1 mL of acetone was further added, to prepare a GC-MS specimen (n=3).

(2) GC-MS Analysis Condition

The analysis condition for GC-Orbitrap-MS is as described in (2) of 2.3.2 described above. However, the exact mass of the monitoring ions is shown in the table described below.

(3) Data Analysis

From GC-MS chromatogram, the precise mass of respective constituents (table described below) was extracted and the peak area was obtained. Constituents in the respective specimen were compared by calculating the ratio of the peak area (= the peak area of the respective constituents / the peak area of the internal standard).

[Table 28]

| Constituents detected in GC-MS analysis and analysis condition ion | | | |
|---|---|---|---|
| | Constituent name | Retention time (min) | Precise mass |
| | internal standard : 4-methylthiazole | 13.2 | 99.0137 |
| 22 | allyl methyl disulfide | 13.4 | 120. 0063 |
| 23 | 2,5-dimethylpyrazine | 14.3 | 108. 0682 |
| 24 | 2-ethyl-6-methylpyrazine | 16.2 | 121.0761 |
| 25 | 2,3,5-Trimethylpyrazine | 16.6 | 122. 0838 |
| 26 | 2,6-diethylpyrazine | 17. 5 | 135. 0916 |
| 27 | 2,5-diethylpyrazine | 17. 9 | 135. 0916 |
| 28 | 2,3-diethylpyrazine | 18.5 | 135.0916 |
| 29 | allylmethyl trisulfide | 23. 9 | 87. 0264 |
| 30 | 3-methyl-1,2,4-trithiane | 31.0 | 137.9626 |
| 31 | 2-dodecenal | 39. 3 | 67. 0543 |
| 32 | sulfurol | 59. 9 | 112. 0216 |

The ratios of the peak area of respective constituents detected in the GC-MS specimen prepared from respective samples to the peak area of the internal standard are shown in Figures 42 to 44. Bars in the figures represent standard deviations.

The results shown in Figures 42 to 44 indicate that the mustard seed of Examples 501 to 505 included a larger amount of allylmethyl disulfide, 2,5-dimethylpyrazine, 2-ethyl-6-methylpyrazine, 2,3,5-trimethylpyrazine, 2,6-diethylpyrazine, 2,5-diethylpyrazine, 2,3-diethylpyrazine, allylmethyl trisulfide, 3-methyl-1,2,4-trithiane, 2-dodecenal, and sulfurol in comparison with the mustard seed of Comparative Examples 501 to 506. The amount of these compounds is considered to relate to the strength of sweetness and roasted flavor, as well as the richness-enhancing effect of mustard seed.

## Claims

**1.** A flavor-enhancing composition containing one or more selected from the group consisting of heat-treated cumin, heat-treated paprika, heat-treated asafoetida and heat-treated mustard seed.

**2.** The flavor-enhancing composition according to Claim 1, containing said heat-treated cumin.

**3.** The flavor-enhancing composition according to Claim 2, wherein said heat-treated cumin is obtained by heating cumin without the addition of oil or fat under a condition whereby a heating value is 15 or more.

**4.** The flavor-enhancing composition according to Claim 3, wherein said heating value is 60 or more and 500 or less.

**5.** The flavor-enhancing composition according to Claim 2, wherein said heat-treated cumin is obtained by heating cumin together with oil or fat under a condition whereby the heating value is 1 or more.

**6.** The flavor-enhancing composition according to Claim 5, wherein said heating value is 20 or more and 4000 or less.

**7.** The flavor-enhancing composition according to any one of Claims 2 to 6, wherein said heat-treated cumin contains one or more selected the group consisting of cyclic (Phe-Tyr), cyclic (Phe-Ala), cyclic (Tyr-Ser), cyclic (Leu/Ile-Ser), cyclic (Ala-Tyr), cyclic (Gly-Tyr), cyclic (Ala-Pro), cyclic (Gly-Val), cyclic (hyPro-Pro) and cyclic (Glu-Glu).

**8.** The flavor-enhancing composition according to any one of Claims 2 to 7, wherein when said heat-treated cumin, with the addition of 47 $\mu$g/g of L-lysine-$^{13}C_6$ monohydrochloride, is analyzed by liquid chromatography-mass spectrometry (LC-MS/MS), said heat-treated cumin satisfies one or more of the following:

(1) a peak area of 0.024 or more derived from cyclic (Phe-Phe),
(2) a peak area of 0.040 or more derived from cyclic (Phe-Leu/Ile),
(3) a peak area of 0.015 or more derived from cyclic (Leu/Ile-Val),
(4) a peak area of 0.020 or more derived from cyclic (Phe-Pro),
(5) a peak area of 0.0020 or more derived from cyclic (Phe-Tyr),
(6) a peak area of 0.040 or more derived from cyclic (Pro-Leu/Ile),
(7) a peak area of 0.004 or more derived from cyclic (Val-Val),
(8) a peak area of 0.002 or more derived from cyclic (Phe-Ala),
(9) a peak area of 0.002 or more derived from cyclic (Tyr-Ser),
(10) a peak area of 0.013 or more derived from cyclic (Pro-Val),
(11) a peak area of 0.009 or more derived from cyclic (Pro-Tyr),
(12) a peak area of 0.050 or more derived from cyclic (Pro-Pro),
(13) a peak area of 0.010 or more derived from cyclic (Leu/Ile-Ser),
(14) a peak area of 0.10 or more derived from cyclic (Arg-Leu/Ile),
(15) a peak area of 0.007 or more derived from cyclic (Ala-Tyr),
(16) a peak area of 0.002 or more derived from cyclic (Gly-Tyr),
(17) a peak area of 0.020 or more derived from cyclic (Ala-Pro),
(18) a peak area of 0.001 or more derived from cyclic (Gly-Val),
(19) a peak area of 0.010 or more derived from cyclic (hyPro-Pro),
(20) a peak area of 0.040 or more derived from cyclic (Thr-Pro),
(21) a peak area of 0.060 or more derived from cyclic (Val-Arg),
(22) a peak area of 0.040 or more derived from cyclic (Pro-Asp),
(23) a peak area of 0.035 or more derived from cyclic (Arg-Pro),
(24) a peak area of 0.050 or more derived from cyclic (Glu-Tyr),

(25) a peak area of 0.020 or more derived from cyclic (Pro-His),
(26) a peak area of 0.080 or more derived from cyclic (Glu-Asp),
(27) a peak area of 0.50 or more derived from cyclic (Gly-Arg),
(28) a peak area of 0.005 or more derived from cyclic (Gly-His),
(29) a peak area of 0.070 or more derived from cyclic (Leu/Ile-Leu/Ile),
(30) a peak area of 0.010 or more derived from cyclic (Glu-Glu),
(31) a peak area of 0.010 or more derived from cyclic (Leu/Ile-Asp),
(32) a peak area of 0.012 or more derived from cyclic (Phe-Asp),
(33) a peak area of 0.30 or more derived from sulfurol(4-methyl-5-thiazoleethanol) and
(34) a peak area of 0.025 or more derived from sulfurol acetate,
as a ratio to a peak area derived from L-lysine-$^{13}C_6$ monohydrochloride in a chromatogram obtained, wherein the liquid chromatography-mass spectrometry is carried out according to a method
(measurement method of LC-MS/MS), in which:

a 10-mL test tube containing 200 mg of said heat-treated cumin and 5 mL of water is kept heated in a thermostatic water bath at 80 °C for 30 minutes, to prepare a water extract;
to said water extract in said test tube are added 5 mL of acetonitrile and L-lysine-$^{13}C_6$ monohydrochloride in an amount of 47 μg/g with respect to said heat-treated cumin, followed by stirring, and then a solid portion is removed and a liquid portion is collected, to prepare a specimen sample; and
said specimen sample is analyzed by LC-MS/MS, to obtain the chromatogram.

**9.** The flavor-enhancing composition according to any one of Claims 2 to 8, wherein when said heat-treated cumin, with the addition of 99 μg/g of ribitol, is analyzed by liquid chromatography-mass spectrometry (LC-MS/MS), said heat-treated cumin satisfies one or more of the following:

(35) a peak area of 0.180 or more derived from *cis*-aconitic acid,
(36) a peak area of 0.250 or more derived from *trans*-aconitic acid,
(37) a peak area of 0.550 or more derived from tartaric acid,
(38) a peak area of 22.0 or more derived from malic acid and
(39) a peak area of 35.0 or more derived from citric acid,
as a ratio to a peak area derived from ribitol in a chromatogram obtained, wherein the liquid chromatography-mass spectrometry is carried out according to a method
(measurement method of LC-MS/MS), in which:

a 10-mL test tube containing 200 mg of said heat-treated cumin and 5 mL of water is kept heated in a thermostatic water bath at 80 °C for 30 minutes, to prepare a water extract;
to said water extract in said test tube are add 5 mL of acetonitrile and ribitol in an amount of 99 μg/g with respect to said heat-treated cumin, followed by stirring, and then a solid portion is removed and a liquid portion is collected, to prepare a specimen sample; and
said specimen sample is analyzed by LC-MS/MS, to obtain the chromatogram.

**10.** The flavor-enhancing composition according to any one of Claims 2 to 9, wherein when said heat-treated cumin, with the addition of 625 μg/g of 4-methylthiazole, is analyzed by gas chromatography-mass spectrometry (GC-MS), said heat-treated cumin satisfies one or more of the following:

(40) a peak area of 0.05 or more derived from carveol,
(41) a peak area of 0.18 or more derived from nerolidol and
(42) a peak area of 100 or more derived from α-terpinene-7-al,
as a ratio to a peak area derived from 4-methylthiazole in a chromatogram obtained, wherein the gas chromatography-mass spectrometry is carried out according to a method
(measurement method of GC-MS), in which:

fifteen milligrams of said heat-treated cumin, 4-methylthiazole in an amount of 625 μg/g with respect to said heat-treated cumin, 5 mL of acetone and 5 mL of methanol contained in a 10-mL test tube are stirred, and then a solid portion is removed and a liquid portion is collected, to which is added 1 mL of acetone per 0.1 mL of said liquid portion, to prepare a GC-MS specimen sample; and
said GC-MS specimen sample is analyzed by GC-MS, to obtain the chromatogram.

**11.** The flavor-enhancing composition according to any one of Claims 1 to 10, containing said heat-treated paprika.

**12.** The flavor-enhancing composition according to Claim 11, wherein said heat-treated paprika is obtained by heating paprika without the addition of oil or fat under a condition whereby the heating value is 50 or more.

**13.** The flavor-enhancing composition according to Claim 12, wherein said heating value is 200 or more and 600 or less.

**14.** The flavor-enhancing composition according to Claim 11, wherein said heat-treated paprika is obtained by heating paprika together with oil or fat under a condition whereby the heating value is 0.3 or more.

**15.** The flavor-enhancing composition according to Claim 14, wherein said heating value is 2.5 or more and 450 or less.

**16.** The flavor-enhancing composition according to any one of Claims 11 to 15, wherein said heat-treated paprika contains one or more selected from the group consisting of cyclic (Gly-His), cyclic (Gly-Ser), cyclic (hyPro-Pro), cyclic (Gly-Val), cyclic (Tyr-Asp) and cyclic (Ala-Asp).

**17.** The flavor-enhancing composition according to any one of Claims 11 to 16 wherein when said heat-treated paprika, with the addition of 47 μg/g of L-lysine-$^{13}C_6$ monohydrochloride, is analyzed by liquid chromatography-mass spectrometry (LC-MS/MS), said heat-treated paprika satisfies one or more of the following:

(1) a peak area of 0.45 or more derived from cyclic (Phe-Leu/Ile),
(2) a peak area of 0.020 or more derived from cyclic (Pro-Asn),
(3) a peak area of 0.30 or more derived from cyclic (Leu/Ile-Leu/Ile),
(4) a peak area of 0.014 or more derived from cyclic (Thr-Leu/Ile),
(5) a peak area of 0.025 or more derived from cyclic (Phe-Ser),
(6) a peak area of 0.0020 or more derived from cyclic (Gly-His),
(7) a peak area of 0.0010 or more derived from cyclic (Gly-Ser),
(8) a peak area of 0.080 or more derived from cyclic (Gly-Arg),
(9) a peak area of 0.0140 or more derived from cyclic (Ala-His),
(10) a peak area of 0.040 or more derived from cyclic (Glu-His),
(11) a peak area of 0.21 or more derived from cyclic (Glu-Arg),
(12) a peak area of 0.24 or more derived from cyclic (Ala-Arg),
(13) a peak area of 0.020 or more derived from cyclic (Glu-Glu),
(14) a peak area of 0.39 or more derived from cyclic (Glu-Gly),
(15) a peak area of 0.19 or more derived from cyclic (Glu-Asp),
(16) a peak area of 0.12 or more derived from cyclic (Pro-His),
(17) a peak area of 0.40 or more derived from cyclic (Glu-Tyr),
(18) a peak area of 0.100 or more derived from cyclic (Leu/Ile-Asp),
(19) a peak area of 0.10 or more derived from cyclic (Pro-Asp),
(20) a peak area of 0.15 or more derived from cyclic (Val-Arg),
(21) a peak area of 0.40 or more derived from cyclic (Thr-Pro),
(22) a peak area of 0.020 or more derived from cyclic (hyPro-Pro),
(23) a peak area of 0.010 or more derived from cyclic (Gly-Val),
(24) a peak area of 0.10 or more derived from cyclic (Ala-Pro),
(25) a peak area of 0.025 or more derived from cyclic (Ala-Tyr),
(26) a peak area of 0.60 or more derived from cyclic (Arg-Leu/Ile),
(27) a peak area of 0.050 or more derived from cyclic (Leu/Ile-Ser),
(28) a peak area of 0.040 or more derived from cyclic (Ala-Val),
(29) a peak area of 0.30 or more derived from cyclic (Pro-Pro),
(30) a peak area of 0.025 or more derived from cyclic (Gly-Leu/Ile),
(31) a peak area of 0.060 or more derived from cyclic (Gly-Phe),
(32) a peak area of 0.060 or more derived from cyclic (Pro-Tyr),
(33) a peak area of 0.040 or more derived from cyclic (Phe-Asp),
(34) a peak area of 0.070 or more derived from cyclic (Pro-Val),
(35) a peak area of 0.080 or more derived from cyclic (Ala-Leu/Ile),
(36) a peak area of 0.020 or more derived from cyclic (Val-Tyr),
(37) a peak area of 0.20 or more derived from cyclic (Tyr-Ser),
(38) a peak area of 0.025 or more derived from cyclic (Phe-Ala),

(39) a peak area of 0.050 or more derived from cyclic (Val-Val),
(40) a peak area of 0.45 or more derived from cyclic (Pro-Leu/Ile),
(41) a peak area of 0.160 or more derived from cyclic (Phe-Tyr),
(42) a peak area of 0.020 or more derived from cyclic (Phe-Thr),
(43) a peak area of 0.45 or more derived from cyclic (Phe-Pro),
(44) a peak area of 0.09 or more derived from cyclic (Leu/Ile-Val),
(45) a peak area of 0.010 or more derived from cyclic (Val-Phe),
(46) a peak area of 0.020 or more derived from cyclic (Tyr-Asp),
(47) a peak area of 0.005 or more derived from cyclic (Ala-Asp),
(48) a peak area of 0.070 or more derived from cyclic (Val-Ser),
(49) a peak area of 0.090 or more derived from sulfurol(4-methyl-5-thiazoleethanol) and
(50) a peak area of 0.011 or more derived from sulfurol acetate,
as a ratio to a peak area derived from L-lysine-$^{13}C_6$ monohydrochloride in a chromatogram obtained, wherein the liquid chromatography-mass spectrometry is carried out according to a method
(measurement method of LC-MS/MS), in which:

a 10-mL test tube containing 200 mg of said heat-treated paprika and 5 mL of water is kept heated in a thermostatic water bath at 80 °C for 30 minutes, to prepare a water extract;
to said water extract in said test tube are add 5 mL of acetonitrile and L-lysine-$^{13}C_6$ monohydrochloride in an amount of 47 μg/g with respect to the heat-treated paprika, followed by stirring, and then a solid portion is removed and a liquid portion is collected, to prepare a specimen sample; and
said specimen sample is analyzed by LC-MS/MS, to obtain the chromatogram.

**18.** The flavor-enhancing composition according to any one of Claims 11 to 17, wherein when said heat-treated paprika, with the addition of 99 μg/g of ribitol , is analyzed by liquid chromatography-mass spectrometry (LC-MS/MS), said heat-treated paprika satisfies one or more of the following:

(51) a peak area of 0.60 or more derived from tartaric acid and
(52) a peak area of 0.35 or more derived from *trans*-aconitic acid,
as a ratio to a peak area derived from ribitol in a chromatogram obtained, wherein the liquid chromatography-mass spectrometry is carried out according to a method
(measurement method of LC-MS/MS), in which:

a 10-mL test tube containing 200 mg of said heat-treated paprika and 5 mL of water is kept heated in a thermostatic water bath at 80 °C for 30 minutes, to prepare a water extract;
to said water extract in said test tube, are added 5 mL of acetonitrile and ribitol in an amount of 99 μg/g with respect to the heat-treated paprika, followed by stirring, and then a solid portion is removed and a liquid portion is collected, to prepare a specimen sample; and
said specimen sample is analyzed by LC-MS/MS, to obtain the chromatogram.

**19.** The flavor-enhancing composition according to any one of Claims 1 to 18, containing said heat-treated asafoetida.

**20.** The flavor-enhancing composition according to Claim 19, wherein said heat-treated asafoetida is obtained by heating asafoetida without the addition of oil or fat under a condition whereby the heating value is 15 or more.

**21.** The flavor-enhancing composition according to Claim 19, wherein said heat-treated asafoetida is obtained by heating asafoetida together with oil or fat under a condition whereby the heating value is 5 or more.

**22.** The flavor-enhancing composition according to any one of Claims 19 to 21, wherein said heat-treated asafoetida contains one or more selected from the group consisting of cyclic (Val-Ser), cyclic (Pro-Pro) and cyclic (Pro-Glu).

**23.** The flavor-enhancing composition according to any one of Claims 19 to 22, wherein when said heat-treated asafoetida, with the addition of 47 μg/g of L-lysine-$^{13}C_6$ monohydrochloride, is analyzed by liquid chromatography-mass spectrometry (LC-MS/MS), said heat-treated asafoetida satisfies one or more of the following:

(1) a peak area of 0.028 or more derived from cyclic (Gly-Thr),
(2) a peak area of 0.003 or more derived from cyclic (Gly-His),
(3) a peak area of 0.022 or more derived from cyclic (Ala-Asp),

(4) a peak area of 0.040 or more derived from cyclic (Val-Ser),
(5) a peak area of 0.020 or more derived from cyclic (Gly-Arg),
(6) a peak area of 0.005 or more derived from cyclic (Ala-His),
(7) a peak area of 0.008 or more derived from cyclic (Glu-Asp),
(8) a peak area of 0.050 or more derived from cyclic (Phe-Phe),
(9) a peak area of 0.025 or more derived from cyclic (Leu/Ile-Leu/Ile),
(10) a peak area of 0.025 or more derived from cyclic (Ala-Arg),
(11) a peak area of 0.020 or more derived from cyclic (Leu/Ile-Val),
(12) a peak area of 0.015 or more derived from cyclic (Phe-Pro),
(13) a peak area of 0.040 or more derived from cyclic (Phe-Leu/Ile),
(14) a peak area of 0.010 or more derived from cyclic (Pro-Pro),
(15) a peak area of 0.050 or more derived from cyclic (Glu-Phe),
(16) a peak area of 0.045 or more derived from cyclic (Arg-Leu/Ile),
(17) a peak area of 0.080 or more derived from cyclic (Pro-Leu/Ile),
(18) a peak area of 0.005 or more derived from cyclic (Pro-Glu),
(19) a peak area of 0.015 or more derived from cyclic (Glu-Leu/Ile),
(20) a peak area of 0.028 or more derived from cyclic (Ala-Pro),
(21) a peak area of 0.020 or more derived from cyclic (Pro-Val),
(22) a peak area of 0.040 or more derived from cyclic (Pro-His) and
(23) a peak area of 0.25 or more derived from sulfurol(4-methyl-5-thiazoleethanol),
as a ratio to a peak area derived from L-lysine-$^{13}C_6$ monohydrochloride in a chromatogram obtained, wherein the liquid chromatography-mass spectrometry is carried out according to a method
(measurement method of LC-MS/MS), in which:

a 10-mL test tube containing 200 mg of said heat-treated asafoetida (mass converted under the assumption that the asafoetida resin content is 12% by mass) and 5 mL of water is kept heated in a thermostatic water bath at 80 °C for 30 minutes, to prepare a water extract;
to said water extract in said test tube are added 5 mL of acetonitrile and L-lysine-$^{13}C_6$ monohydrochloride in an amount of 47 μg/g with respect to said heat-treated asafoetida (mass converted under the assumption that the asafoetida resin content is 12% by mass), followed by stirring, and then a solid portion is removed and a liquid portion is collected;
after contacting said liquid portion with a solid-phase extraction carrier that carries an octadecyl group, an eluate by acetonitrile is collected, to prepare a specimen sample from said eluate; and
said specimen sample is analyzed by LC-MS/MS, to obtain the chromatogram.

**24.** The flavor-enhancing composition according to any one of Claims 19 to 23, wherein when said heat-treated asafoetida, with the addition of 99 μg/g of ribitol, is analyzed by liquid chromatography-mass spectrometry (LC-MS/MS), said heat-treated asafoetida satisfies one or more of the following:

(24) a peak area of 0.12 or more derived from tartaric acid,
(25) a peak area of 1.10 or more derived from malic acid and
(26) a peak area of 1.00 or more derived from citric acid,
as a ratio to a peak area derived from ribitol in a chromatogram obtained, wherein the liquid chromatography-mass spectrometry is carried out according to a method
(measurement method of LC-MS/MS), in which:

a 10-mL test tube containing 200 mg of said heat-treated asafoetida (mass converted under the assumption that the asafoetida resin content is 12% by mass) and 5 mL of water is kept heated in a thermostatic water bath at 80 °C for 30 minutes, to prepare a water extract;
to said water extract in said test tube are added 5 mL of acetonitrile and ribitol in an amount of 99 μg/g with respect to said heat-treated asafoetida (mass converted under the assumption that the asafoetida resin content is 12% by mass), followed by stirring, and then a solid portion is removed and a liquid portion is collected;
after contacting said liquid portion with a solid-phase extraction carrier that carries an octadecyl group, an eluate by acetonitrile is collected, to prepare a specimen sample from said eluate; and
said specimen sample is analyzed by LC-MS/MS, to obtain the chromatogram.

**25.** The flavor-enhancing composition according to any one of Claims 1 to 24, containing said heat-treated mustard seed.

**26.** The flavor-enhancing composition according to Claim 25, wherein when said mustard seed, with the addition of 47 μg/g of L-lysine-$^{13}C_6$ monohydrochloride, is analyzed by liquid chromatography-mass spectrometry (LC-MS/MS), said heat-treated mustard seed satisfies one or more of the following:

(1) a peak area of 0.20 or more derived from cyclic (Glu-His),
(2) a peak area of 0.29 or more derived from cyclic (Gly-Arg),
(3) a peak area of 0.15 or more derived from cyclic (Glu-Gly),
(4) a peak area of 0.12 or more derived from cyclic (Ala-His),
(5) a peak area of 0.20 or more derived from cyclic (Ala-Arg),
(6) a peak area of 0.13 or more derived from cyclic (Glu-Asp),
(7) a peak area of 0.035 or more derived from cyclic (Leu/Ile-Asp),
(8) a peak area of 0.50 or more derived from cyclic (Thr-Pro),
(9) a peak area of 0.09 or more derived from cyclic (Pro-His),
(10) a peak area of 0.50 or more derived from cyclic (Pro-Pro),
(11) a peak area of 0.070 or more derived from cyclic (Glu-Tyr),
(12) a peak area of 0.015 or more derived from cyclic (Arg-Leu/Ile),
(13) a peak area of 0.70 or more derived from cyclic (Ala-Pro),
(14) a peak area of 0.65 or more derived from cyclic (Pro-Val),
(15) a peak area of 0.060 or more derived from cyclic (Phe-Ala),
(16) a peak area of 0.070 or more derived from cyclic (Phe-Pro),
(17) a peak area of 0.18 or more derived from cyclic (Phe-Tyr),
(18) a peak area of 0.02 or more derived from cyclic (Tyr-His),
(19) a peak area of 0.05 or more derived from cyclic (Leu/Ile-His),
(20) a peak area of 0.32 or more derived from cyclic (Pro-Met) and
(21) a peak area of 0.10 or more derived from cyclic (Val-Glu),
as a ratio to a peak area derived from L-lysine-$^{13}C_6$ monohydrochloride in a chromatogram obtained, wherein the liquid chromatography-mass spectrometry is carried out according to a method
(measurement method of LC-MS/MS), in which:

a 10-mL test tube containing 200 mg of said heat-treated mustard seed and 5 mL of water is kept heated in a thermostatic water bath at 80 °C for 30 minutes, to prepare a water extract;
to said water extract in said test tube are added 5 mL of acetonitrile and L-lysine-$^{13}C_6$ monohydrochloride in an amount of 47 μg/g with respect to said heat-treated mustard seed, followed by stirring, and then a solid portion is removed and a liquid portion is collected, to prepare an LC-MS/MS specimen sample; and
said LC-MS/MS specimen sample is analyzed by LC-MS/MS, to obtain the chromatogram.

**27.** The flavor-enhancing composition according to Claim 25 or 26, wherein when said mustard seed, with the addition of 625 μg/g of 4-methylthiazole, is analyzed by gas chromatography-mass spectrometry (GC-MS), said heat-treated mustard seed satisfies one or more of the following:

(22) a peak area of 0.006 or more derived from allylmethyl disulfide,
(23) a peak area of 0.60 or more derived from 2,5-dimethylpyrazine,
(24) a peak area of 0.15 or more derived from 2-ethyl-6-methylpyrazine,
(25) a peak area of 0.15 or more derived from 2,3,5-trimethylpyrazine,
(26) a peak area of 0.01 or more derived from 2,6-diethylpyrazine,
(27) a peak area of 0.20 or more derived from 2,5-diethylpyrazine,
(28) a peak area of 0.03 or more derived from 2,3-diethylpyrazine,
(29) a peak area of 0.025 or more derived from allylmethyl trisulfide,
(30) a peak area of 0.70 or more derived from 3-methyl-1,2,4-trithiane,
(31) a peak area of 0.10 or more derived from 2-dodecenal and
(32) a peak area of 0.50 or more derived from sulfurol,
as a ratio to a peak area derived from 4-methylthiazole in a chromatogram obtained, wherein the gas chromatography-mass spectrometry is carried out according to a method
(measurement method of GC-MS), in which:

fifteen milligrams of said heat-treated mustard seed, 4-methylthiazole in an amount of 625 μg/g with respect to said heat-treated mustard seed, 5 mL of acetone and 5 mL of methanol contained in a 10-mL test tube are stirred, and then a solid portion is removed and a liquid portion is collected, to which is added 1 mL of acetone per 0.1 mL of said liquid portion, to prepare a GC-MS specimen sample; and
said GC-MS specimen sample is analyzed by GC-MS, to obtain the chromatogram.

**28.** A method for producing the flavor-enhancing composition according to any one of Claims 2 to 10, comprising: heating cumin without the addition of oil or fat under a condition whereby the heating value is 15 or more to obtain said heat-treated cumin.

**29.** The method according to Claim 28, wherein said heating value is 60 or more and 500 or less.

**30.** A method for producing the flavor-enhancing composition according to any one of Claims 2 to 10, comprising: heating cumin together with oil or fat under a condition whereby the heating value is 1 or more to obtain said heat-treated cumin.

**31.** The method according to Claim 30, wherein said heating value is 20 or more and 4000 or less.

**32.** A method for producing the flavor-enhancing composition according to any one of Claims 11 to 18, comprising: heating paprika without the addition of oil or fat under a condition whereby the heating value is 50 or more to obtain said heat-treated paprika.

**33.** The method according to Claim 32, wherein said heating value is 200 or more and 600 or less.

**34.** A method for producing the flavor-enhancing composition according to any one of Claims 11 to 18, comprising: heating paprika together with oil or fat under a condition whereby the heating value is 0.3 or more to obtain said heat-treated paprika.

**35.** The method according to Claim 34, wherein said heating value is 2.5 or more and 450 or less.

**36.** A method for producing the flavor-enhancing composition according to any one of Claims 19 to 24, comprising: heating asafoetida without the addition of oil or fat under a condition whereby the heating value is 15 or more to obtain said heat-treated asafoetida.

**37.** A method for producing the flavor-enhancing composition according to any one of Claims 19 to 24, comprising: heating asafoetida together with oil or fat under a condition whereby the heating value is 5 or more to obtain said heat-treated asafoetida.

**38.** A method for producing the flavor-enhancing composition according to any one of Claims 25 to 27, comprising: heating mustard seed in an open system under a condition whereby the heating value is 200 or more to obtain said heat-treated mustard seed.

**39.** A method for enhancing a flavor of a food product, comprising blending the flavor-enhancing composition according to any one of Claims 1 to 27 with the food product.

1 CYCLIC(Phe-Leu/Ile)

| | Ave. | SD |
|---|---|---|
| Comp. Ex. 201 | 0.383 | 0.045 |
| Comp. Ex. 202 | 0.424 | 0.008 |
| Ex. 201 | 0.538 | 0.013 |
| Ex. 202 | 0.528 | 0.062 |
| Ex. 203 | 0.575 | 0.002 |
| Ex. 204 | 0.588 | 0.056 |
| Ex. 205 | 0.651 | 0.014 |
| Ex. 206 | 0.675 | 0.006 |
| Ex. 207 | 0.653 | 0.027 |
| Ex. 208 | 0.712 | 0.065 |
| Ex. 209 | 0.685 | 0.057 |
| Ex. 210 | 0.753 | 0.019 |
| Ex. 211 | 0.638 | 0.021 |
| Ex. 212 | 0.612 | 0.011 |
| Ex. 213 | 0.688 | 0.006 |
| Ex. 214 | 0.544 | 0.023 |
| Ex. 215 | 0.501 | 0.013 |
| Ex. 216 | 0.531 | 0.001 |

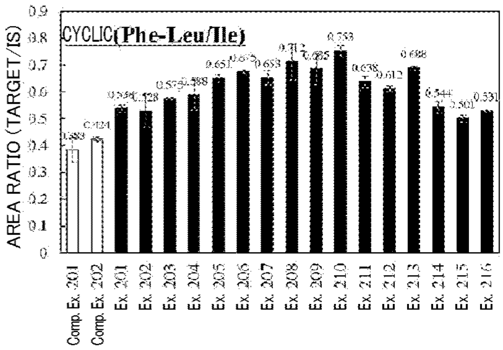


2 CYCLIC(Pro-Asn)

| | Ave. | SD |
|---|---|---|
| Comp. Ex. 201 | 0.014 | 0.008 |
| Comp. Ex. 202 | 0.010 | 0.003 |
| Ex. 201 | 0.041 | 0.012 |
| Ex. 202 | 0.051 | 0.012 |
| Ex. 203 | 0.041 | 0.003 |
| Ex. 204 | 0.101 | 0.011 |
| Ex. 205 | 0.052 | 0.002 |
| Ex. 206 | 0.065 | 0.008 |
| Ex. 207 | 0.057 | 0.010 |
| Ex. 208 | 0.089 | 0.017 |
| Ex. 209 | 0.058 | 0.012 |
| Ex. 210 | 0.099 | 0.006 |
| Ex. 211 | 0.030 | 0.001 |
| Ex. 212 | 0.040 | 0.001 |
| Ex. 213 | 0.026 | 0.000 |
| Ex. 214 | 0.062 | 0.001 |
| Ex. 215 | 0.064 | 0.000 |
| Ex. 216 | 0.062 | 0.001 |

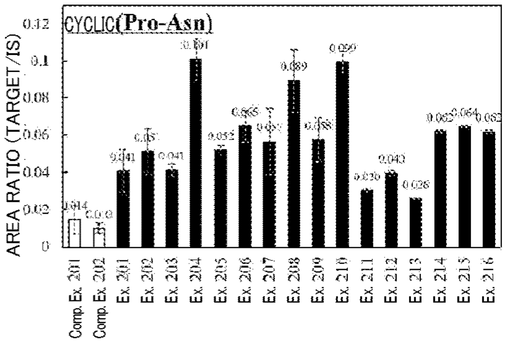


3 CYCLIC(Leu/Ile-Leu/Ile)

| | Ave. | SD |
|---|---|---|
| Comp. Ex. 201 | 0.273 | 0.025 |
| Comp. Ex. 202 | 0.296 | 0.003 |
| Ex. 201 | 0.401 | 0.012 |
| Ex. 202 | 0.400 | 0.042 |
| Ex. 203 | 0.445 | 0.022 |
| Ex. 204 | 0.449 | 0.027 |
| Ex. 205 | 0.484 | 0.048 |
| Ex. 206 | 0.512 | 0.002 |
| Ex. 207 | 0.512 | 0.010 |
| Ex. 208 | 0.547 | 0.036 |
| Ex. 209 | 0.507 | 0.034 |
| Ex. 210 | 0.566 | 0.037 |
| Ex. 211 | 0.584 | 0.008 |
| Ex. 212 | 0.438 | 0.013 |
| Ex. 213 | 0.599 | 0.014 |
| Ex. 214 | 0.394 | 0.014 |
| Ex. 215 | 0.326 | 0.020 |
| Ex. 216 | 0.386 | 0.002 |

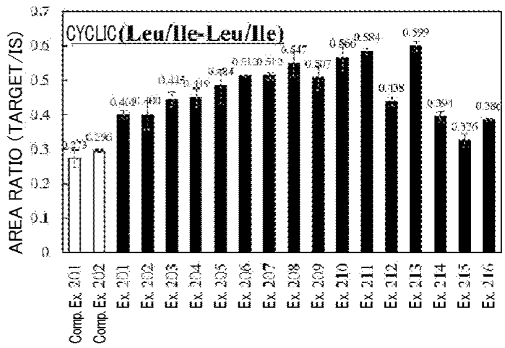


4 CYCLIC(Thr-Leu/Ile)

| | Ave. | SD |
|---|---|---|
| Comp. Ex. 201 | 0.012 | 0.000 |
| Comp. Ex. 202 | 0.012 | 0.000 |
| Ex. 201 | 0.027 | 0.001 |
| Ex. 202 | 0.023 | 0.000 |
| Ex. 203 | 0.025 | 0.002 |
| Ex. 204 | 0.019 | 0.001 |
| Ex. 205 | 0.021 | 0.001 |
| Ex. 206 | 0.025 | 0.001 |
| Ex. 207 | 0.029 | 0.003 |
| Ex. 208 | 0.029 | 0.001 |
| Ex. 209 | 0.019 | 0.002 |
| Ex. 210 | 0.022 | 0.000 |
| Ex. 211 | 0.030 | 0.001 |
| Ex. 212 | 0.041 | 0.001 |
| Ex. 213 | 0.039 | 0.000 |
| Ex. 214 | 0.047 | 0.002 |
| Ex. 215 | 0.027 | 0.002 |
| Ex. 216 | 0.018 | 0.001 |

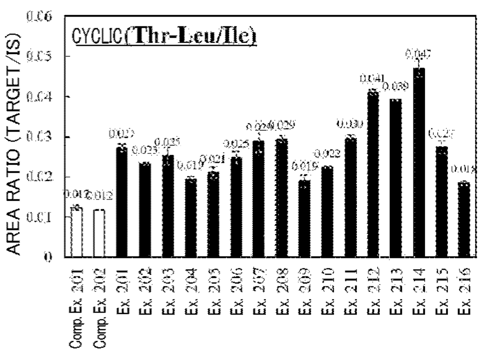


FIG. 1

5 CYCLIC(Phe-Ser)

| | Ave. | SD |
|---|---|---|
| Comp. Ex. 201 | 0.020 | 0.000 |
| Comp. Ex. 202 | 0.022 | 0.001 |
| Ex. 201 | 0.043 | 0.004 |
| Ex. 202 | 0.039 | 0.006 |
| Ex. 203 | 0.042 | 0.004 |
| Ex. 204 | 0.040 | 0.000 |
| Ex. 205 | 0.065 | 0.012 |
| Ex. 206 | 0.057 | 0.004 |
| Ex. 207 | 0.040 | 0.003 |
| Ex. 208 | 0.052 | 0.004 |
| Ex. 209 | 0.064 | 0.007 |
| Ex. 210 | 0.075 | 0.006 |
| Ex. 211 | 0.075 | 0.000 |
| Ex. 212 | 0.101 | 0.001 |
| Ex. 213 | 0.056 | 0.001 |
| Ex. 214 | 0.056 | 0.010 |
| Ex. 215 | 0.042 | 0.003 |
| Ex. 216 | 0.031 | 0.003 |

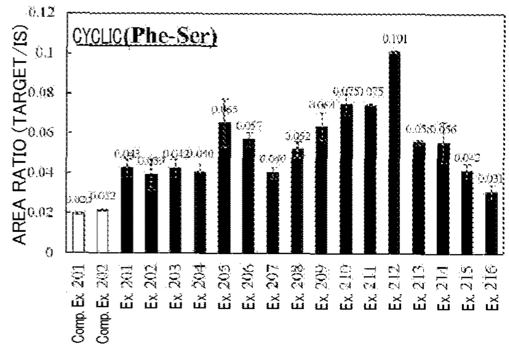


6 CYCLIC(Gly-His)

| | Ave. | SD |
|---|---|---|
| Comp. Ex. 201 | nd | |
| Comp. Ex. 202 | nd | |
| Ex. 201 | 0.004 | 0.002 |
| Ex. 202 | 0.007 | 0.002 |
| Ex. 203 | 0.005 | 0.000 |
| Ex. 204 | 0.008 | 0.004 |
| Ex. 205 | 0.006 | 0.001 |
| Ex. 206 | 0.010 | 0.002 |
| Ex. 207 | 0.004 | 0.001 |
| Ex. 208 | 0.005 | 0.000 |
| Ex. 209 | 0.014 | 0.004 |
| Ex. 210 | 0.016 | 0.003 |
| Ex. 211 | 0.005 | 0.001 |
| Ex. 212 | 0.006 | 0.001 |
| Ex. 213 | 0.007 | 0.000 |
| Ex. 214 | 0.009 | 0.002 |
| Ex. 215 | 0.011 | 0.001 |
| Ex. 216 | 0.012 | 0.000 |

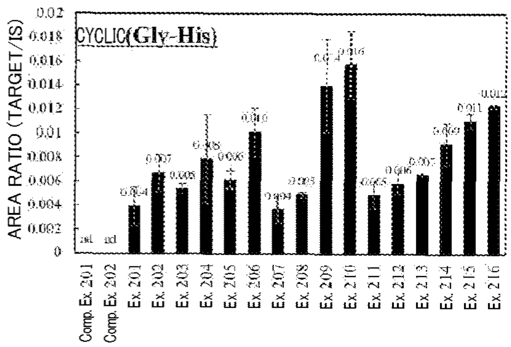


7 CYCLIC(Gly-Ser)

| | Ave. | SD |
|---|---|---|
| Comp. Ex. 201 | nd | |
| Comp. Ex. 202 | nd | |
| Ex. 201 | 0.007 | 0.003 |
| Ex. 202 | 0.006 | 0.003 |
| Ex. 203 | 0.010 | 0.003 |
| Ex. 204 | 0.019 | 0.009 |
| Ex. 205 | 0.014 | 0.001 |
| Ex. 206 | 0.025 | 0.000 |
| Ex. 207 | 0.014 | 0.002 |
| Ex. 208 | 0.024 | 0.003 |
| Ex. 209 | 0.023 | 0.001 |
| Ex. 210 | 0.036 | 0.008 |
| Ex. 211 | 0.004 | 0.001 |
| Ex. 212 | 0.005 | 0.000 |
| Ex. 213 | 0.005 | 0.000 |
| Ex. 214 | 0.009 | 0.001 |
| Ex. 215 | 0.013 | 0.005 |
| Ex. 216 | 0.005 | 0.001 |

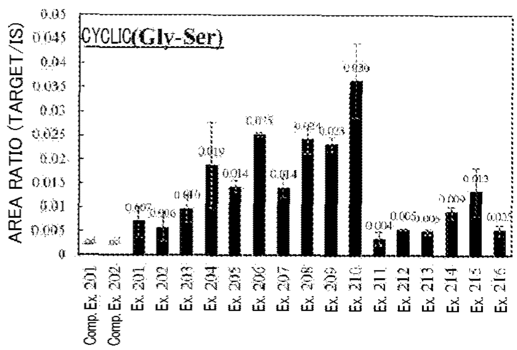


8 CYCLIC(Gly-Arg)

| | Ave. | SD |
|---|---|---|
| Comp. Ex. 201 | 0.055 | 0.012 |
| Comp. Ex. 202 | 0.059 | 0.011 |
| Ex. 201 | 0.133 | 0.002 |
| Ex. 202 | 0.135 | 0.002 |
| Ex. 203 | 0.130 | 0.006 |
| Ex. 204 | 0.138 | 0.009 |
| Ex. 205 | 0.157 | 0.000 |
| Ex. 206 | 0.174 | 0.001 |
| Ex. 207 | 0.110 | 0.011 |
| Ex. 208 | 0.104 | 0.014 |
| Ex. 209 | 0.132 | 0.023 |
| Ex. 210 | 0.158 | 0.005 |
| Ex. 211 | 0.096 | 0.002 |
| Ex. 212 | 0.116 | 0.005 |
| Ex. 213 | 0.093 | 0.009 |
| Ex. 214 | 0.111 | 0.010 |
| Ex. 215 | 0.115 | 0.002 |
| Ex. 216 | 0.136 | 0.004 |

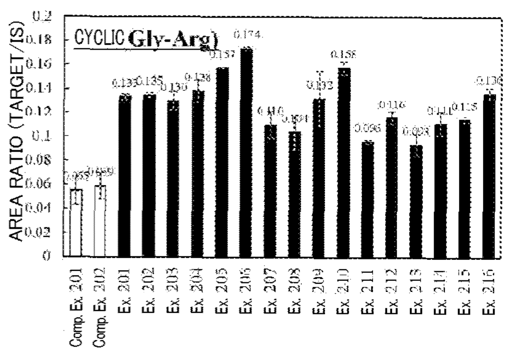


FIG. 2

9 CYCLIC(Ala-His)

| | Ave. | SD |
|---|---|---|
| Comp. Ex. 201 | 0.011 | 0.000 |
| Comp. Ex. 202 | 0.008 | 0.004 |
| Ex. 201 | 0.015 | 0.004 |
| Ex. 202 | 0.019 | 0.007 |
| Ex. 203 | 0.023 | 0.001 |
| Ex. 204 | 0.025 | 0.006 |
| Ex. 205 | 0.022 | 0.007 |
| Ex. 206 | 0.028 | 0.004 |
| Ex. 207 | 0.018 | 0.001 |
| Ex. 208 | 0.025 | 0.003 |
| Ex. 209 | 0.039 | 0.007 |
| Ex. 210 | 0.032 | 0.007 |
| Ex. 211 | 0.017 | 0.000 |
| Ex. 212 | 0.016 | 0.000 |
| Ex. 213 | 0.018 | 0.001 |
| Ex. 214 | 0.021 | 0.003 |
| Ex. 215 | 0.032 | 0.001 |
| Ex. 216 | 0.058 | 0.002 |

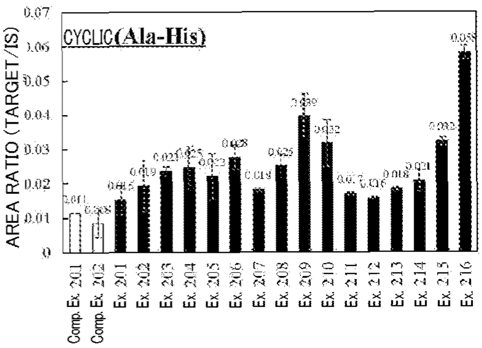


10 CYCLIC(Glu-His)

| | Ave. | SD |
|---|---|---|
| Comp. Ex. 201 | 0.034 | 0.010 |
| Comp. Ex. 202 | 0.026 | 0.005 |
| Ex. 201 | 0.050 | 0.006 |
| Ex. 202 | 0.050 | 0.003 |
| Ex. 203 | 0.065 | 0.017 |
| Ex. 204 | 0.076 | 0.000 |
| Ex. 205 | 0.065 | 0.001 |
| Ex. 206 | 0.067 | 0.005 |
| Ex. 207 | 0.219 | 0.026 |
| Ex. 208 | 0.268 | 0.003 |
| Ex. 209 | 0.094 | 0.005 |
| Ex. 210 | 0.098 | 0.004 |
| Ex. 211 | 0.080 | 0.002 |
| Ex. 212 | 0.085 | 0.002 |
| Ex. 213 | 0.076 | 0.009 |
| Ex. 214 | 0.057 | 0.012 |
| Ex. 215 | 0.050 | 0.007 |
| Ex. 216 | 0.062 | 0.006 |

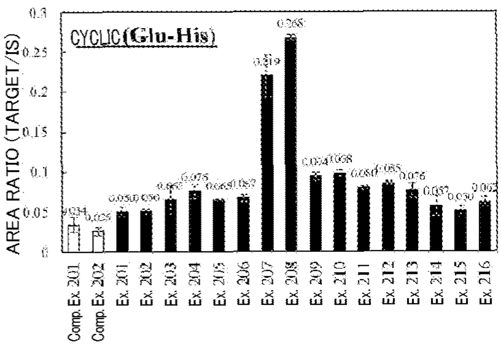


11 CYCLIC(Glu-Arg)

| | Ave. | SD |
|---|---|---|
| Comp. Ex. 201 | 0.215 | 0.017 |
| Comp. Ex. 202 | 0.194 | 0.000 |
| Ex. 201 | 0.250 | 0.007 |
| Ex. 202 | 0.211 | 0.007 |
| Ex. 203 | 0.296 | 0.008 |
| Ex. 204 | 0.246 | 0.007 |
| Ex. 205 | 0.330 | 0.009 |
| Ex. 206 | 0.289 | 0.005 |
| Ex. 207 | 0.341 | 0.000 |
| Ex. 208 | 0.267 | 0.031 |
| Ex. 209 | 0.371 | 0.027 |
| Ex. 210 | 0.310 | 0.008 |
| Ex. 211 | 0.234 | 0.015 |
| Ex. 212 | 0.302 | 0.001 |
| Ex. 213 | 0.298 | 0.005 |
| Ex. 214 | 0.305 | 0.047 |
| Ex. 215 | 0.337 | 0.038 |
| Ex. 216 | 0.358 | 0.077 |

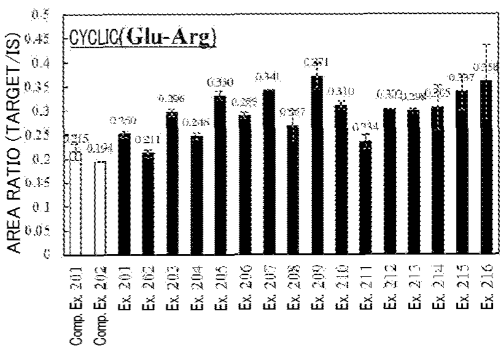


12 CYCLIC(Ala-Arg)

| | Ave. | SD |
|---|---|---|
| Comp. Ex. 201 | 0.203 | 0.001 |
| Comp. Ex. 202 | 0.239 | 0.027 |
| Ex. 201 | 0.721 | 0.004 |
| Ex. 202 | 0.615 | 0.027 |
| Ex. 203 | 0.690 | 0.022 |
| Ex. 204 | 0.587 | 0.007 |
| Ex. 205 | 0.646 | 0.155 |
| Ex. 206 | 0.666 | 0.010 |
| Ex. 207 | 0.650 | 0.032 |
| Ex. 208 | 0.415 | 0.012 |
| Ex. 209 | 0.590 | 0.034 |
| Ex. 210 | 0.513 | 0.091 |
| Ex. 211 | 0.250 | 0.005 |
| Ex. 212 | 0.291 | 0.015 |
| Ex. 213 | 0.258 | 0.018 |
| Ex. 214 | 0.875 | 0.007 |
| Ex. 215 | 0.639 | 0.053 |
| Ex. 216 | 0.364 | 0.053 |

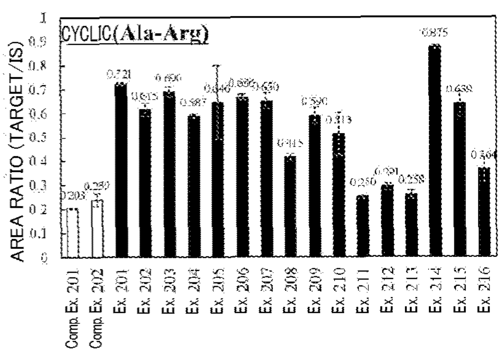


FIG. 3

13 CYCLIC(Glu-Glu)

| | Ave. | SD |
|---|---|---|
| Comp. Ex. 201 | 0.017 | 0.000 |
| Comp. Ex. 202 | 0.015 | 0.006 |
| Ex. 201 | 0.022 | 0.005 |
| Ex. 202 | 0.023 | 0.002 |
| Ex. 203 | 0.026 | 0.004 |
| Ex. 204 | 0.030 | 0.002 |
| Ex. 205 | 0.028 | 0.000 |
| Ex. 206 | 0.025 | 0.011 |
| Ex. 207 | 0.037 | 0.009 |
| Ex. 208 | 0.039 | 0.004 |
| Ex. 209 | 0.026 | 0.004 |
| Ex. 210 | 0.025 | 0.000 |
| Ex. 211 | 0.031 | 0.003 |
| Ex. 212 | 0.026 | 0.003 |
| Ex. 213 | 0.034 | 0.001 |
| Ex. 214 | 0.045 | 0.000 |
| Ex. 215 | 0.034 | 0.003 |
| Ex. 216 | 0.022 | 0.000 |

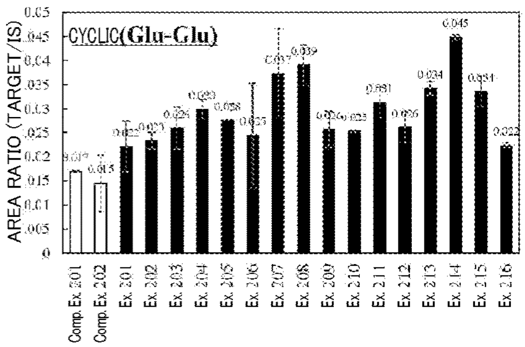


14 CYCLIC(Glu-Gly)

| | Ave. | SD |
|---|---|---|
| Comp. Ex. 201 | 0.361 | 0.008 |
| Comp. Ex. 202 | 0.372 | 0.005 |
| Ex. 201 | 0.440 | 0.035 |
| Ex. 202 | 0.408 | 0.054 |
| Ex. 203 | 0.518 | 0.036 |
| Ex. 204 | 0.463 | 0.015 |
| Ex. 205 | 0.589 | 0.005 |
| Ex. 206 | 0.570 | 0.058 |
| Ex. 207 | 0.453 | 0.015 |
| Ex. 208 | 0.465 | 0.010 |
| Ex. 209 | 0.709 | 0.005 |
| Ex. 210 | 0.729 | 0.005 |
| Ex. 211 | 0.412 | 0.014 |
| Ex. 212 | 0.469 | 0.012 |
| Ex. 213 | 0.415 | 0.050 |
| Ex. 214 | 0.529 | 0.053 |
| Ex. 215 | 0.656 | 0.006 |
| Ex. 216 | 1.178 | 0.056 |

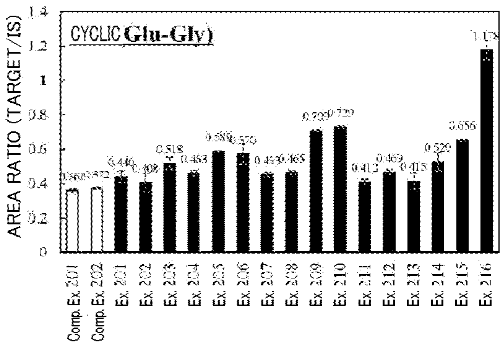


15 CYCLIC(Glu-Asp)

| | Ave. | SD |
|---|---|---|
| Comp. Ex. 201 | 0.178 | 0.004 |
| Comp. Ex. 202 | 0.171 | 0.018 |
| Ex. 201 | 0.488 | 0.030 |
| Ex. 202 | 0.484 | 0.022 |
| Ex. 203 | 0.670 | 0.050 |
| Ex. 204 | 0.718 | 0.009 |
| Ex. 205 | 0.784 | 0.022 |
| Ex. 206 | 0.816 | 0.013 |
| Ex. 207 | 0.886 | 0.031 |
| Ex. 208 | 0.936 | 0.090 |
| Ex. 209 | 0.825 | 0.010 |
| Ex. 210 | 0.938 | 0.014 |
| Ex. 211 | 0.219 | 0.030 |
| Ex. 212 | 0.260 | 0.007 |
| Ex. 213 | 0.268 | 0.050 |
| Ex. 214 | 0.431 | 0.002 |
| Ex. 215 | 0.440 | 0.036 |
| Ex. 216 | 0.407 | 0.006 |

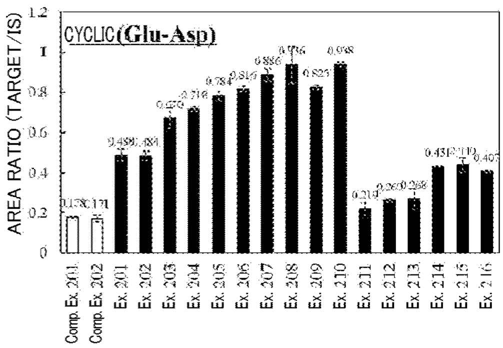


16 CYCLIC(Pro-His)

| | Ave. | SD |
|---|---|---|
| Comp. Ex. 201 | 0.107 | 0.024 |
| Comp. Ex. 202 | 0.109 | 0.030 |
| Ex. 201 | 0.250 | 0.036 |
| Ex. 202 | 0.181 | 0.010 |
| Ex. 203 | 0.232 | 0.020 |
| Ex. 204 | 0.207 | 0.002 |
| Ex. 205 | 0.274 | 0.065 |
| Ex. 206 | 0.291 | 0.018 |
| Ex. 207 | 0.391 | 0.010 |
| Ex. 208 | 0.485 | 0.037 |
| Ex. 209 | 0.375 | 0.010 |
| Ex. 210 | 0.429 | 0.050 |
| Ex. 211 | 0.407 | 0.045 |
| Ex. 212 | 0.328 | 0.004 |
| Ex. 213 | 0.416 | 0.054 |
| Ex. 214 | 0.157 | 0.016 |
| Ex. 215 | 0.220 | 0.009 |
| Ex. 216 | 0.174 | 0.031 |

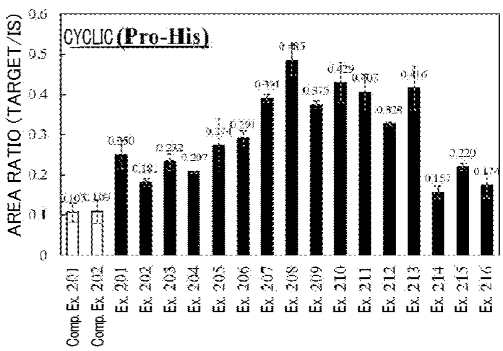


FIG. 4

17 CYCLIC(Glu-Tyr)

| | Ave. | SD |
|---|---|---|
| Comp. Ex. 201 | 0.361 | 0.019 |
| Comp. Ex. 202 | 0.351 | 0.077 |
| Ex. 201 | 1.117 | 0.166 |
| Ex. 202 | 0.733 | 0.002 |
| Ex. 203 | 1.205 | 0.001 |
| Ex. 204 | 0.894 | 0.033 |
| Ex. 205 | 1.178 | 0.009 |
| Ex. 206 | 0.885 | 0.109 |
| Ex. 207 | 1.422 | 0.119 |
| Ex. 208 | 1.094 | 0.060 |
| Ex. 209 | 1.329 | 0.005 |
| Ex. 210 | 1.204 | 0.079 |
| Ex. 211 | 0.437 | 0.003 |
| Ex. 212 | 0.509 | 0.059 |
| Ex. 213 | 0.477 | 0.075 |
| Ex. 214 | 0.980 | 0.026 |
| Ex. 215 | 1.188 | 0.008 |
| Ex. 216 | 1.838 | 0.084 |

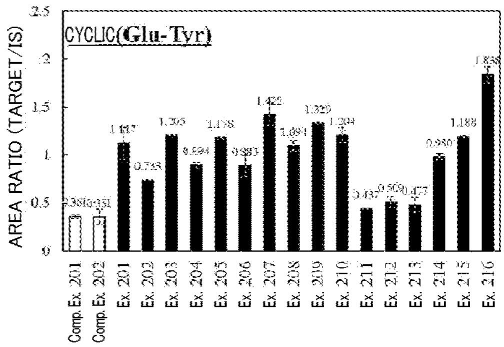


18 CYCLIC(Leu/Ile-Asp)

| | Ave. | SD |
|---|---|---|
| Comp. Ex. 201 | 0.041 | 0.003 |
| Comp. Ex. 202 | 0.039 | 0.004 |
| Ex. 201 | 0.150 | 0.002 |
| Ex. 202 | 0.152 | 0.000 |
| Ex. 203 | 0.173 | 0.002 |
| Ex. 204 | 0.182 | 0.001 |
| Ex. 205 | 0.163 | 0.015 |
| Ex. 206 | 0.197 | 0.006 |
| Ex. 207 | 0.165 | 0.005 |
| Ex. 208 | 0.202 | 0.014 |
| Ex. 209 | 0.181 | 0.007 |
| Ex. 210 | 0.203 | 0.007 |
| Ex. 211 | 0.144 | 0.007 |
| Ex. 212 | 0.137 | 0.006 |
| Ex. 213 | 0.153 | 0.036 |
| Ex. 214 | 0.183 | 0.008 |
| Ex. 215 | 0.167 | 0.033 |
| Ex. 216 | 0.176 | 0.001 |

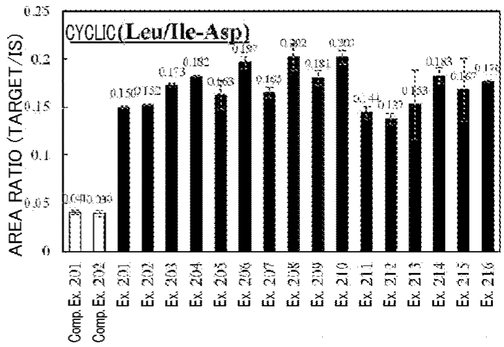


19 CYCLIC(Pro-Asp)

| | Ave. | SD |
|---|---|---|
| Comp. Ex. 201 | 0.057 | 0.005 |
| Comp. Ex. 202 | 0.065 | 0.015 |
| Ex. 201 | 0.164 | 0.007 |
| Ex. 202 | 0.168 | 0.022 |
| Ex. 203 | 0.211 | 0.028 |
| Ex. 204 | 0.232 | 0.015 |
| Ex. 205 | 0.271 | 0.049 |
| Ex. 206 | 0.253 | 0.031 |
| Ex. 207 | 0.219 | 0.025 |
| Ex. 208 | 0.254 | 0.015 |
| Ex. 209 | 0.304 | 0.003 |
| Ex. 210 | 0.315 | 0.008 |
| Ex. 211 | 0.240 | 0.006 |
| Ex. 212 | 0.232 | 0.003 |
| Ex. 213 | 0.153 | 0.004 |
| Ex. 214 | 0.141 | 0.006 |
| Ex. 215 | 0.179 | 0.000 |
| Ex. 216 | 0.184 | 0.005 |

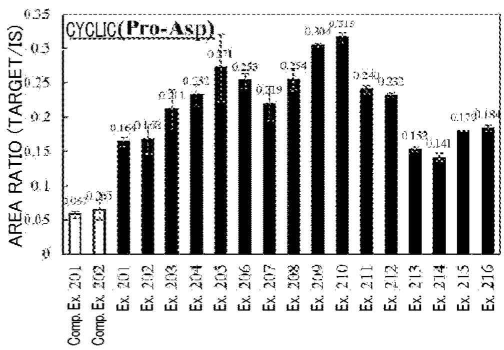


20 CYCLIC(Val-Arg)

| | Ave. | SD |
|---|---|---|
| Comp. Ex. 201 | 0.153 | 0.040 |
| Comp. Ex. 202 | 0.131 | 0.011 |
| Ex. 201 | 0.655 | 0.018 |
| Ex. 202 | 0.629 | 0.022 |
| Ex. 203 | 0.799 | 0.003 |
| Ex. 204 | 0.688 | 0.036 |
| Ex. 205 | 0.286 | 0.015 |
| Ex. 206 | 0.311 | 0.047 |
| Ex. 207 | 0.748 | 0.090 |
| Ex. 208 | 0.745 | 0.030 |
| Ex. 209 | 0.390 | 0.125 |
| Ex. 210 | 0.294 | 0.011 |
| Ex. 211 | 0.313 | 0.027 |
| Ex. 212 | 0.246 | 0.006 |
| Ex. 213 | 0.308 | 0.051 |
| Ex. 214 | 0.374 | 0.005 |
| Ex. 215 | 0.256 | 0.002 |
| Ex. 216 | 0.173 | 0.004 |

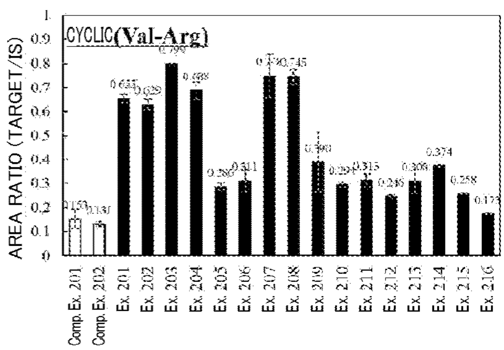


FIG. 5

21 CYCLIC(Thr-Pro)

| | Ave. | SD |
|---|---|---|
| Comp. Ex. 201 | 0.134 | 0.000 |
| Comp. Ex. 202 | 0.167 | 0.010 |
| Ex. 201 | 0.593 | 0.069 |
| Ex. 202 | 0.504 | 0.044 |
| Ex. 203 | 0.730 | 0.055 |
| Ex. 204 | 0.610 | 0.083 |
| Ex. 205 | 0.722 | 0.012 |
| Ex. 206 | 0.646 | 0.066 |
| Ex. 207 | 0.670 | 0.018 |
| Ex. 208 | 0.672 | 0.004 |
| Ex. 209 | 0.699 | 0.031 |
| Ex. 210 | 0.677 | 0.030 |
| Ex. 211 | 0.486 | 0.072 |
| Ex. 212 | 0.637 | 0.035 |
| Ex. 213 | 0.577 | 0.052 |
| Ex. 214 | 0.981 | 0.035 |
| Ex. 215 | 0.950 | 0.017 |
| Ex. 216 | 0.937 | 0.036 |

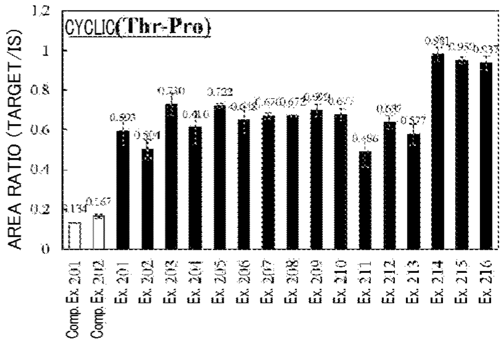


22 CYCLIC(hyPro-Pro)

| | Ave. | SD |
|---|---|---|
| Comp. Ex. 201 | nd | |
| Comp. Ex. 202 | nd | |
| Ex. 201 | 0.038 | 0.004 |
| Ex. 202 | 0.034 | 0.001 |
| Ex. 203 | 0.045 | 0.003 |
| Ex. 204 | 0.056 | 0.004 |
| Ex. 205 | 0.065 | 0.019 |
| Ex. 206 | 0.063 | 0.006 |
| Ex. 207 | 0.073 | 0.001 |
| Ex. 208 | 0.058 | 0.011 |
| Ex. 209 | 0.042 | 0.003 |
| Ex. 210 | 0.079 | 0.019 |
| Ex. 211 | 0.068 | 0.001 |
| Ex. 212 | 0.095 | 0.006 |
| Ex. 213 | 0.059 | 0.001 |
| Ex. 214 | 0.136 | 0.002 |
| Ex. 215 | 0.188 | 0.004 |
| Ex. 216 | 0.095 | 0.011 |

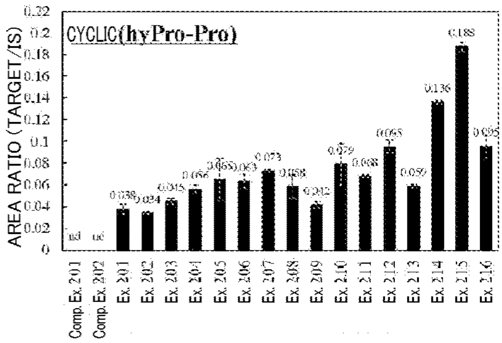


23 CYCLIC(Gly-Val)

| | Ave. | SD |
|---|---|---|
| Comp. Ex. 201 | nd | |
| Comp. Ex. 202 | nd | |
| Ex. 201 | 0.033 | 0.012 |
| Ex. 202 | 0.032 | 0.011 |
| Ex. 203 | 0.052 | 0.005 |
| Ex. 204 | 0.053 | 0.012 |
| Ex. 205 | 0.061 | 0.002 |
| Ex. 206 | 0.040 | 0.009 |
| Ex. 207 | 0.069 | 0.007 |
| Ex. 208 | 0.061 | 0.002 |
| Ex. 209 | 0.065 | 0.004 |
| Ex. 210 | 0.046 | 0.001 |
| Ex. 211 | 0.018 | 0.001 |
| Ex. 212 | 0.042 | 0.000 |
| Ex. 213 | 0.014 | 0.000 |
| Ex. 214 | 0.024 | 0.001 |
| Ex. 215 | 0.026 | 0.003 |
| Ex. 216 | 0.031 | 0.003 |

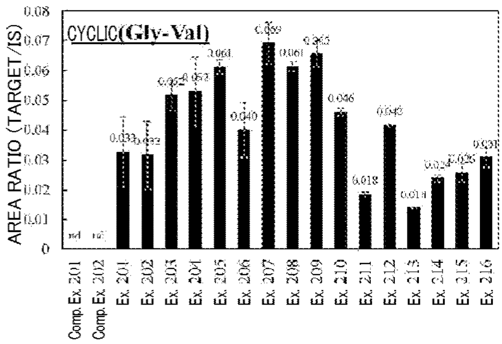


24 CYCLIC(Ala-Pro)

| | Ave. | SD |
|---|---|---|
| Comp. Ex. 201 | 0.048 | 0.003 |
| Comp. Ex. 202 | 0.061 | 0.003 |
| Ex. 201 | 0.182 | 0.009 |
| Ex. 202 | 0.236 | 0.047 |
| Ex. 203 | 0.238 | 0.007 |
| Ex. 204 | 0.328 | 0.048 |
| Ex. 205 | 0.491 | 0.100 |
| Ex. 206 | 0.444 | 0.001 |
| Ex. 207 | 0.328 | 0.029 |
| Ex. 208 | 0.396 | 0.016 |
| Ex. 209 | 0.526 | 0.056 |
| Ex. 210 | 0.613 | 0.071 |
| Ex. 211 | 0.174 | 0.008 |
| Ex. 212 | 0.146 | 0.005 |
| Ex. 213 | 0.211 | 0.009 |
| Ex. 214 | 0.331 | 0.003 |
| Ex. 215 | 0.389 | 0.024 |
| Ex. 216 | 0.417 | 0.002 |

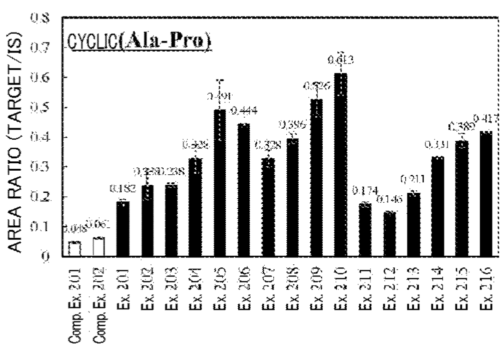


FIG. 6

25 CYCLIC(Ala-Tyr)

| | Ave. | SD |
|---|---|---|
| Comp. Ex. 201 | 0.021 | 0.000 |
| Comp. Ex. 202 | 0.017 | 0.001 |
| Ex. 201 | 0.049 | 0.001 |
| Ex. 202 | 0.041 | 0.003 |
| Ex. 203 | 0.050 | 0.008 |
| Ex. 204 | 0.053 | 0.005 |
| Ex. 205 | 0.045 | 0.014 |
| Ex. 206 | 0.054 | 0.000 |
| Ex. 207 | 0.057 | 0.008 |
| Ex. 208 | 0.057 | 0.007 |
| Ex. 209 | 0.055 | 0.009 |
| Ex. 210 | 0.070 | 0.000 |
| Ex. 211 | 0.038 | 0.001 |
| Ex. 212 | 0.041 | 0.004 |
| Ex. 213 | 0.042 | 0.003 |
| Ex. 214 | 0.043 | 0.001 |
| Ex. 215 | 0.048 | 0.001 |
| Ex. 216 | 0.044 | 0.004 |

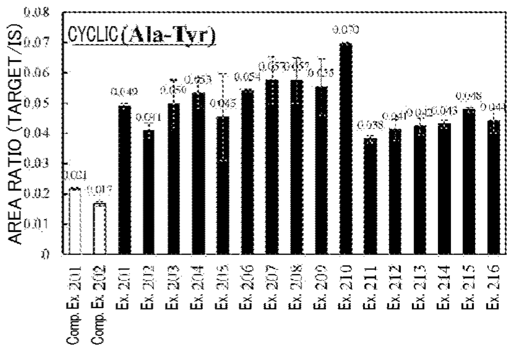


26 CYCLIC(Arg-Leu/Ile)

| | Ave. | SD |
|---|---|---|
| Comp. Ex. 201 | 0.500 | 0.052 |
| Comp. Ex. 202 | 0.444 | 0.120 |
| Ex. 201 | 1.168 | 0.169 |
| Ex. 202 | 0.944 | 0.054 |
| Ex. 203 | 1.058 | 0.219 |
| Ex. 204 | 0.986 | 0.019 |
| Ex. 205 | 0.936 | 0.280 |
| Ex. 206 | 0.750 | 0.070 |
| Ex. 207 | 1.189 | 0.074 |
| Ex. 208 | 1.051 | 0.178 |
| Ex. 209 | 0.869 | 0.051 |
| Ex. 210 | 0.669 | 0.112 |
| Ex. 211 | 0.671 | 0.011 |
| Ex. 212 | 0.676 | 0.031 |
| Ex. 213 | 0.967 | 0.055 |
| Ex. 214 | 0.960 | 0.126 |
| Ex. 215 | 0.778 | 0.007 |
| Ex. 216 | 0.998 | 0.041 |

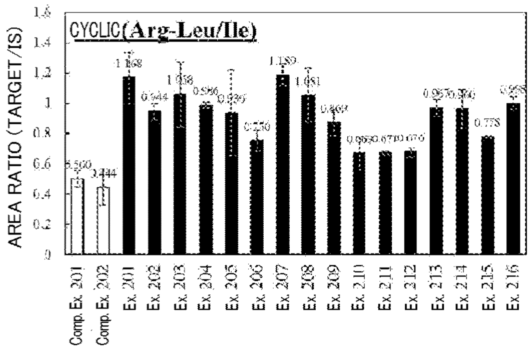


27 CYCLIC(Leu/Ile-Ser)

| | Ave. | SD |
|---|---|---|
| Comp. Ex. 201 | 0.015 | 0.001 |
| Comp. Ex. 202 | 0.017 | 0.003 |
| Ex. 201 | 0.120 | 0.000 |
| Ex. 202 | 0.121 | 0.014 |
| Ex. 203 | 0.146 | 0.016 |
| Ex. 204 | 0.135 | 0.010 |
| Ex. 205 | 0.143 | 0.021 |
| Ex. 206 | 0.167 | 0.007 |
| Ex. 207 | 0.166 | 0.014 |
| Ex. 208 | 0.183 | 0.009 |
| Ex. 209 | 0.157 | 0.001 |
| Ex. 210 | 0.184 | 0.001 |
| Ex. 211 | 0.126 | 0.005 |
| Ex. 212 | 0.120 | 0.005 |
| Ex. 213 | 0.110 | 0.009 |
| Ex. 214 | 0.103 | 0.002 |
| Ex. 215 | 0.062 | 0.000 |
| Ex. 216 | 0.065 | 0.003 |

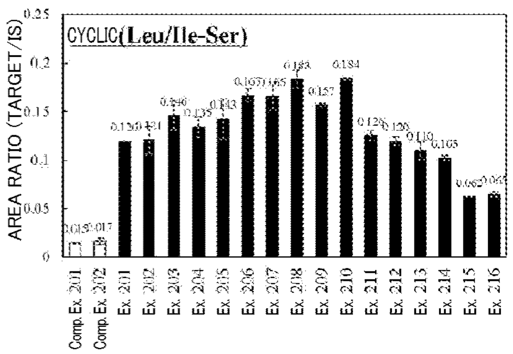


28 CYCLIC(Ala-Val)

| | Ave. | SD |
|---|---|---|
| Comp. Ex. 201 | 0.026 | 0.002 |
| Comp. Ex. 202 | 0.036 | 0.002 |
| Ex. 201 | 0.065 | 0.008 |
| Ex. 202 | 0.066 | 0.012 |
| Ex. 203 | 0.075 | 0.005 |
| Ex. 204 | 0.072 | 0.008 |
| Ex. 205 | 0.101 | 0.011 |
| Ex. 206 | 0.083 | 0.001 |
| Ex. 207 | 0.068 | 0.008 |
| Ex. 208 | 0.099 | 0.011 |
| Ex. 209 | 0.110 | 0.012 |
| Ex. 210 | 0.116 | 0.001 |
| Ex. 211 | 0.058 | 0.000 |
| Ex. 212 | 0.047 | 0.000 |
| Ex. 213 | 0.054 | 0.002 |
| Ex. 214 | 0.069 | 0.007 |
| Ex. 215 | 0.063 | 0.004 |
| Ex. 216 | 0.102 | 0.004 |

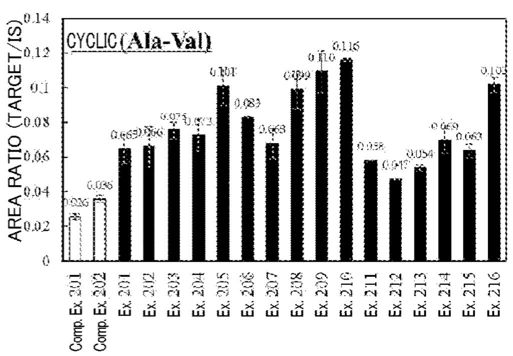


FIG. 7

29 CYCLIC(Pro-Pro)

| | Ave. | SD |
|---|---|---|
| Comp. Ex. 201 | 0.259 | 0.015 |
| Comp. Ex. 202 | 0.286 | 0.023 |
| Ex. 201 | 0.494 | 0.017 |
| Ex. 202 | 0.522 | 0.031 |
| Ex. 203 | 0.609 | 0.015 |
| Ex. 204 | 0.637 | 0.016 |
| Ex. 205 | 0.667 | 0.080 |
| Ex. 206 | 0.779 | 0.008 |
| Ex. 207 | 0.613 | 0.014 |
| Ex. 208 | 0.713 | 0.052 |
| Ex. 209 | 0.825 | 0.002 |
| Ex. 210 | 0.923 | 0.026 |
| Ex. 211 | 0.384 | 0.023 |
| Ex. 212 | 0.336 | 0.004 |
| Ex. 213 | 0.361 | 0.002 |
| Ex. 214 | 0.456 | 0.013 |
| Ex. 215 | 0.435 | 0.002 |
| Ex. 216 | 0.617 | 0.008 |

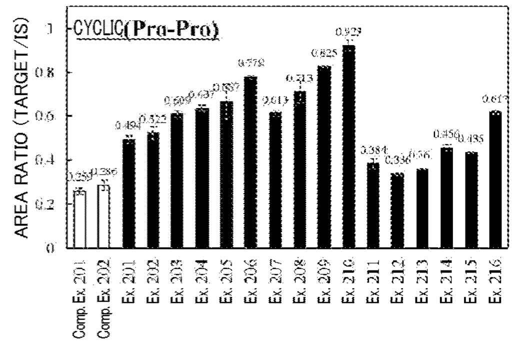


30 CYCLIC(Gly-Leu/Ile)

| | Ave. | SD |
|---|---|---|
| Comp. Ex. 201 | 0.019 | 0.002 |
| Comp. Ex. 202 | 0.017 | 0.003 |
| Ex. 201 | 0.047 | 0.001 |
| Ex. 202 | 0.049 | 0.000 |
| Ex. 203 | 0.060 | 0.005 |
| Ex. 204 | 0.060 | 0.006 |
| Ex. 205 | 0.058 | 0.014 |
| Ex. 206 | 0.073 | 0.009 |
| Ex. 207 | 0.067 | 0.006 |
| Ex. 208 | 0.074 | 0.007 |
| Ex. 209 | 0.075 | 0.005 |
| Ex. 210 | 0.082 | 0.006 |
| Ex. 211 | 0.038 | 0.001 |
| Ex. 212 | 0.039 | 0.001 |
| Ex. 213 | 0.050 | 0.001 |
| Ex. 214 | 0.072 | 0.007 |
| Ex. 215 | 0.071 | 0.007 |
| Ex. 216 | 0.073 | 0.002 |

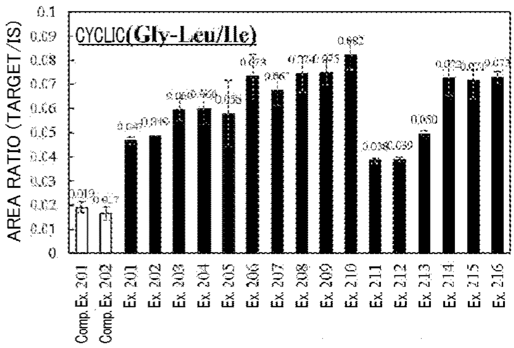


31 CYCLIC(Gly-Phe)

| | Ave. | SD |
|---|---|---|
| Comp. Ex. 201 | 0.035 | 0.004 |
| Comp. Ex. 202 | 0.039 | 0.002 |
| Ex. 201 | 0.085 | 0.003 |
| Ex. 202 | 0.088 | 0.004 |
| Ex. 203 | 0.091 | 0.007 |
| Ex. 204 | 0.106 | 0.000 |
| Ex. 205 | 0.119 | 0.009 |
| Ex. 206 | 0.142 | 0.016 |
| Ex. 207 | 0.084 | 0.004 |
| Ex. 208 | 0.109 | 0.022 |
| Ex. 209 | 0.135 | 0.004 |
| Ex. 210 | 0.167 | 0.018 |
| Ex. 211 | 0.100 | 0.004 |
| Ex. 212 | 0.089 | 0.003 |
| Ex. 213 | 0.078 | 0.003 |
| Ex. 214 | 0.106 | 0.002 |
| Ex. 215 | 0.094 | 0.007 |
| Ex. 216 | 0.086 | 0.001 |

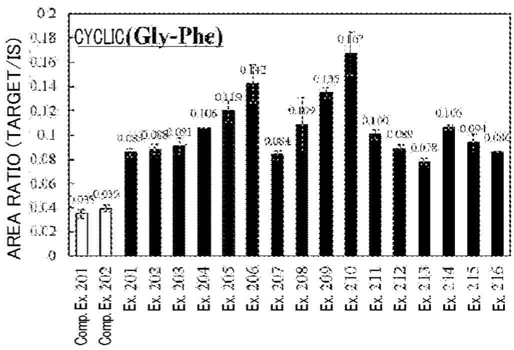


32 CYCLIC(Pro-Tyr)

| | Ave. | SD |
|---|---|---|
| Comp. Ex. 201 | 0.037 | 0.002 |
| Comp. Ex. 202 | 0.045 | 0.002 |
| Ex. 201 | 0.110 | 0.003 |
| Ex. 202 | 0.129 | 0.028 |
| Ex. 203 | 0.129 | 0.001 |
| Ex. 204 | 0.164 | 0.006 |
| Ex. 205 | 0.207 | 0.054 |
| Ex. 206 | 0.227 | 0.001 |
| Ex. 207 | 0.119 | 0.009 |
| Ex. 208 | 0.181 | 0.023 |
| Ex. 209 | 0.289 | 0.017 |
| Ex. 210 | 0.318 | 0.050 |
| Ex. 211 | 0.085 | 0.001 |
| Ex. 212 | 0.104 | 0.002 |
| Ex. 213 | 0.090 | 0.002 |
| Ex. 214 | 0.124 | 0.002 |
| Ex. 215 | 0.139 | 0.001 |
| Ex. 216 | 0.173 | 0.000 |

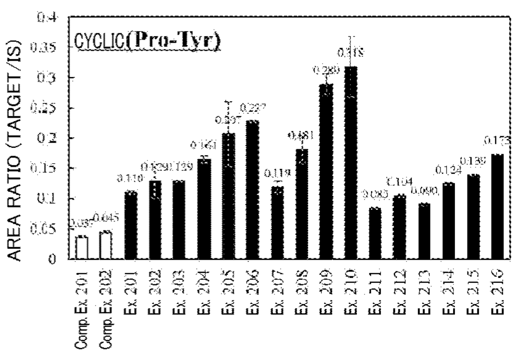


FIG. 8

33 CYCLIC(Phe-Asp)

| | Ave. | SD |
|---|---|---|
| Comp. Ex. 201 | 0.026 | 0.000 |
| Comp. Ex. 202 | 0.030 | 0.005 |
| Ex. 201 | 0.096 | 0.014 |
| Ex. 202 | 0.087 | 0.007 |
| Ex. 203 | 0.116 | 0.024 |
| Ex. 204 | 0.106 | 0.003 |
| Ex. 205 | 0.122 | 0.008 |
| Ex. 206 | 0.121 | 0.010 |
| Ex. 207 | 0.119 | 0.003 |
| Ex. 208 | 0.119 | 0.006 |
| Ex. 209 | 0.132 | 0.007 |
| Ex. 210 | 0.122 | 0.001 |
| Ex. 211 | 0.061 | 0.002 |
| Ex. 212 | 0.090 | 0.002 |
| Ex. 213 | 0.081 | 0.006 |
| Ex. 214 | 0.214 | 0.006 |
| Ex. 215 | 0.191 | 0.004 |
| Ex. 216 | 0.211 | 0.004 |

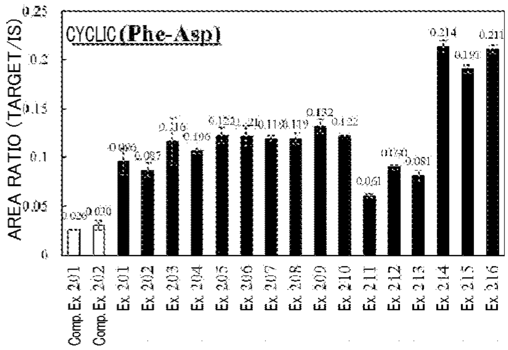


34 CYCLIC(Pro-Val)

| | Ave. | SD |
|---|---|---|
| Comp. Ex. 201 | 0.036 | 0.010 |
| Comp. Ex. 202 | 0.049 | 0.003 |
| Ex. 201 | 0.078 | 0.002 |
| Ex. 202 | 0.093 | 0.021 |
| Ex. 203 | 0.089 | 0.009 |
| Ex. 204 | 0.086 | 0.001 |
| Ex. 205 | 0.129 | 0.034 |
| Ex. 206 | 0.109 | 0.013 |
| Ex. 207 | 0.100 | 0.008 |
| Ex. 208 | 0.119 | 0.002 |
| Ex. 209 | 0.144 | 0.006 |
| Ex. 210 | 0.163 | 0.005 |
| Ex. 211 | 0.087 | 0.001 |
| Ex. 212 | 0.083 | 0.000 |
| Ex. 213 | 0.111 | 0.001 |
| Ex. 214 | 0.147 | 0.000 |
| Ex. 215 | 0.124 | 0.003 |
| Ex. 216 | 0.304 | 0.009 |

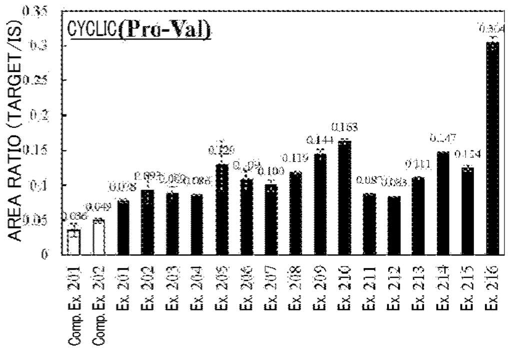


35 CYCLIC(Ala-Leu/Ile)

| | Ave. | SD |
|---|---|---|
| Comp. Ex. 201 | 0.067 | 0.007 |
| Comp. Ex. 202 | 0.069 | 0.004 |
| Ex. 201 | 0.136 | 0.010 |
| Ex. 202 | 0.124 | 0.003 |
| Ex. 203 | 0.143 | 0.015 |
| Ex. 204 | 0.144 | 0.004 |
| Ex. 205 | 0.138 | 0.019 |
| Ex. 206 | 0.147 | 0.011 |
| Ex. 207 | 0.160 | 0.005 |
| Ex. 208 | 0.160 | 0.005 |
| Ex. 209 | 0.148 | 0.017 |
| Ex. 210 | 0.157 | 0.001 |
| Ex. 211 | 0.093 | 0.006 |
| Ex. 212 | 0.115 | 0.006 |
| Ex. 213 | 0.103 | 0.000 |
| Ex. 214 | 0.143 | 0.012 |
| Ex. 215 | 0.135 | 0.018 |
| Ex. 216 | 0.173 | 0.002 |

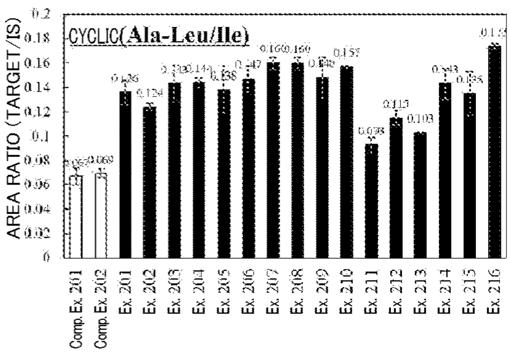


36 CYCLIC(Val-Tyr)

| | Ave. | SD |
|---|---|---|
| Comp. Ex. 201 | 0.019 | 0.004 |
| Comp. Ex. 202 | 0.019 | 0.003 |
| Ex. 201 | 0.060 | 0.006 |
| Ex. 202 | 0.064 | 0.006 |
| Ex. 203 | 0.072 | 0.003 |
| Ex. 204 | 0.072 | 0.005 |
| Ex. 205 | 0.068 | 0.010 |
| Ex. 206 | 0.081 | 0.010 |
| Ex. 207 | 0.077 | 0.005 |
| Ex. 208 | 0.078 | 0.001 |
| Ex. 209 | 0.075 | 0.004 |
| Ex. 210 | 0.084 | 0.005 |
| Ex. 211 | 0.025 | 0.001 |
| Ex. 212 | 0.051 | 0.002 |
| Ex. 213 | 0.036 | 0.001 |
| Ex. 214 | 0.047 | 0.001 |
| Ex. 215 | 0.072 | 0.002 |
| Ex. 216 | 0.081 | 0.004 |

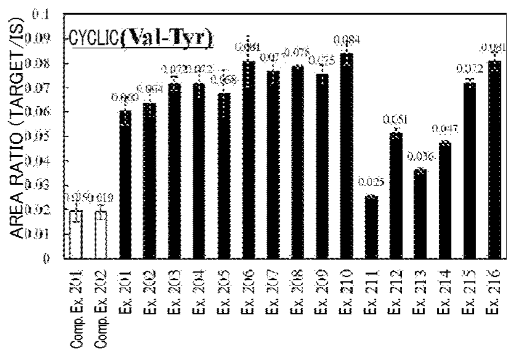


FIG. 9

37 CYCLIC(Tyr-Ser)

| | Ave. | SD |
|---|---|---|
| Comp. Ex. 201 | 0.172 | 0.004 |
| Comp. Ex. 202 | 0.202 | 0.006 |
| Ex. 201 | 0.498 | 0.015 |
| Ex. 202 | 0.376 | 0.003 |
| Ex. 203 | 0.497 | 0.040 |
| Ex. 204 | 0.416 | 0.015 |
| Ex. 205 | 0.468 | 0.057 |
| Ex. 206 | 0.413 | 0.003 |
| Ex. 207 | 0.457 | 0.018 |
| Ex. 208 | 0.394 | 0.005 |
| Ex. 209 | 0.454 | 0.025 |
| Ex. 210 | 0.377 | 0.034 |
| Ex. 211 | 0.465 | 0.100 |
| Ex. 212 | 0.268 | 0.003 |
| Ex. 213 | 0.442 | 0.021 |
| Ex. 214 | 0.393 | 0.007 |
| Ex. 215 | 0.724 | 0.036 |
| Ex. 216 | 1.488 | 0.044 |

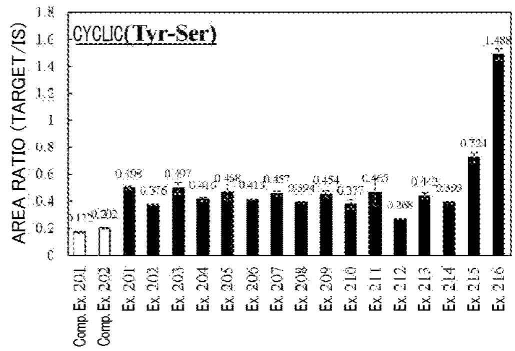


38 CYCLIC(Phe-Ala)

| | Ave. | SD |
|---|---|---|
| Comp. Ex. 201 | 0.021 | 0.001 |
| Comp. Ex. 202 | 0.021 | 0.001 |
| Ex. 201 | 0.038 | 0.002 |
| Ex. 202 | 0.033 | 0.001 |
| Ex. 203 | 0.045 | 0.006 |
| Ex. 204 | 0.041 | 0.001 |
| Ex. 205 | 0.043 | 0.001 |
| Ex. 206 | 0.041 | 0.010 |
| Ex. 207 | 0.048 | 0.004 |
| Ex. 208 | 0.045 | 0.001 |
| Ex. 209 | 0.047 | 0.005 |
| Ex. 210 | 0.045 | 0.001 |
| Ex. 211 | 0.030 | 0.001 |
| Ex. 212 | 0.034 | 0.001 |
| Ex. 213 | 0.030 | 0.001 |
| Ex. 214 | 0.036 | 0.000 |
| Ex. 215 | 0.033 | 0.000 |
| Ex. 216 | 0.045 | 0.001 |

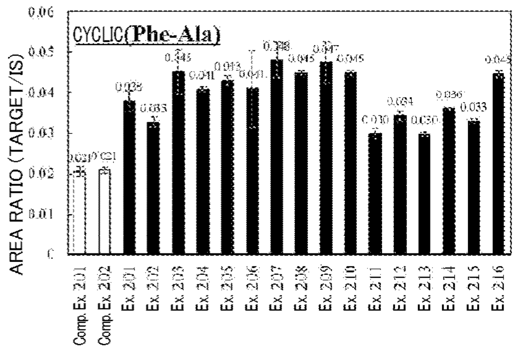


39 CYCLIC(Val-Val)

| | Ave. | SD |
|---|---|---|
| Comp. Ex. 201 | 0.041 | 0.001 |
| Comp. Ex. 202 | 0.049 | 0.004 |
| Ex. 201 | 0.098 | 0.005 |
| Ex. 202 | 0.090 | 0.000 |
| Ex. 203 | 0.106 | 0.005 |
| Ex. 204 | 0.104 | 0.003 |
| Ex. 205 | 0.137 | 0.011 |
| Ex. 206 | 0.117 | 0.001 |
| Ex. 207 | 0.117 | 0.007 |
| Ex. 208 | 0.130 | 0.010 |
| Ex. 209 | 0.133 | 0.005 |
| Ex. 210 | 0.127 | 0.013 |
| Ex. 211 | 0.070 | 0.002 |
| Ex. 212 | 0.063 | 0.001 |
| Ex. 213 | 0.089 | 0.000 |
| Ex. 214 | 0.095 | 0.001 |
| Ex. 215 | 0.094 | 0.003 |
| Ex. 216 | 0.122 | 0.003 |

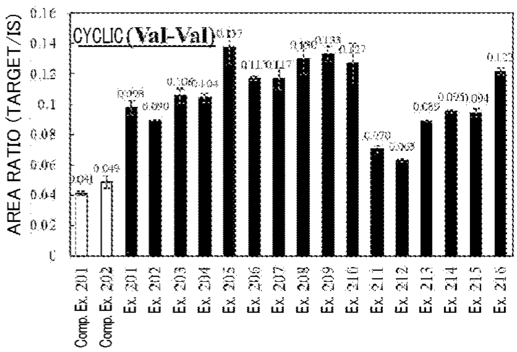


40 CYCLIC(Pro-Leu/Ile)

| | Ave. | SD |
|---|---|---|
| Comp. Ex. 201 | 0.339 | 0.013 |
| Comp. Ex. 202 | 0.425 | 0.007 |
| Ex. 201 | 0.613 | 0.028 |
| Ex. 202 | 0.643 | 0.070 |
| Ex. 203 | 0.710 | 0.022 |
| Ex. 204 | 0.732 | 0.016 |
| Ex. 205 | 0.810 | 0.053 |
| Ex. 206 | 0.861 | 0.022 |
| Ex. 207 | 0.671 | 0.075 |
| Ex. 208 | 0.802 | 0.100 |
| Ex. 209 | 0.921 | 0.007 |
| Ex. 210 | 0.972 | 0.037 |
| Ex. 211 | 0.531 | 0.014 |
| Ex. 212 | 0.569 | 0.036 |
| Ex. 213 | 0.605 | 0.027 |
| Ex. 214 | 0.913 | 0.018 |
| Ex. 215 | 1.008 | 0.005 |
| Ex. 216 | 1.334 | 0.062 |

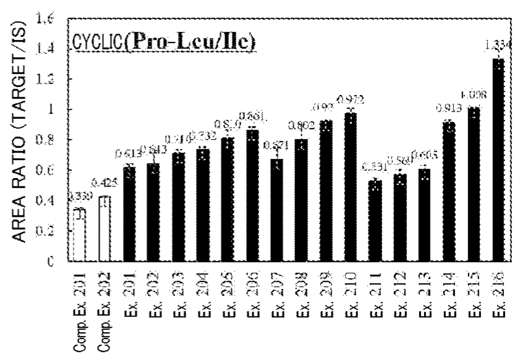


FIG. 10

41 CYCLIC(Phe-Tyr)

| | Ave. | SD |
|---|---|---|
| Comp. Ex. 201 | 0.112 | 0.003 |
| Comp. Ex. 202 | 0.124 | 0.006 |
| Ex. 201 | 0.202 | 0.011 |
| Ex. 202 | 0.206 | 0.011 |
| Ex. 203 | 0.189 | 0.019 |
| Ex. 204 | 0.218 | 0.008 |
| Ex. 205 | 0.200 | 0.000 |
| Ex. 206 | 0.237 | 0.002 |
| Ex. 207 | 0.171 | 0.008 |
| Ex. 208 | 0.214 | 0.037 |
| Ex. 209 | 0.209 | 0.000 |
| Ex. 210 | 0.225 | 0.002 |
| Ex. 211 | 0.982 | 0.003 |
| Ex. 212 | 0.692 | 0.020 |
| Ex. 213 | 0.717 | 0.138 |
| Ex. 214 | 0.458 | 0.091 |
| Ex. 215 | 0.278 | 0.010 |
| Ex. 216 | 0.175 | 0.009 |

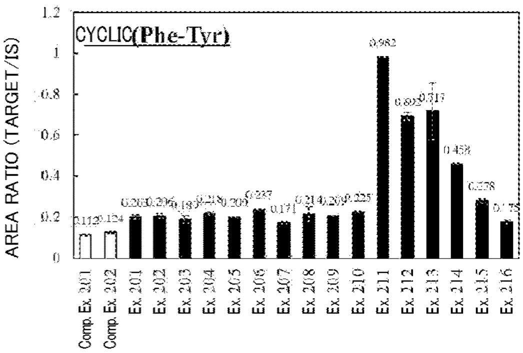


42 CYCLIC(Phe-Thr)

| | Ave. | SD |
|---|---|---|
| Comp. Ex. 201 | 0.007 | 0.001 |
| Comp. Ex. 202 | 0.008 | 0.002 |
| Ex. 201 | 0.084 | 0.017 |
| Ex. 202 | 0.032 | 0.009 |
| Ex. 203 | 0.149 | 0.002 |
| Ex. 204 | 0.046 | 0.005 |
| Ex. 205 | 0.078 | 0.008 |
| Ex. 206 | 0.045 | 0.005 |
| Ex. 207 | 0.112 | 0.003 |
| Ex. 208 | 0.057 | 0.003 |
| Ex. 209 | 0.063 | 0.004 |
| Ex. 210 | 0.034 | 0.000 |
| Ex. 211 | 0.094 | 0.000 |
| Ex. 212 | 0.091 | 0.002 |
| Ex. 213 | 0.084 | 0.008 |
| Ex. 214 | 0.169 | 0.001 |
| Ex. 215 | 0.203 | 0.007 |
| Ex. 216 | 0.194 | 0.012 |

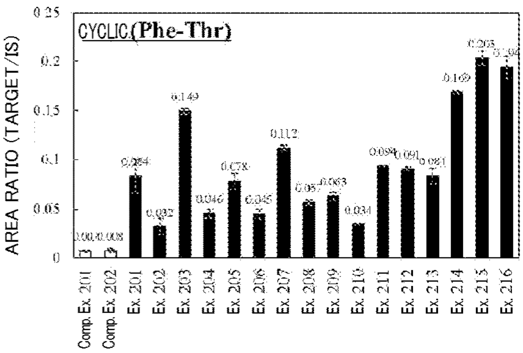


43 CYCLIC(Phe-Pro)

| | Ave. | SD |
|---|---|---|
| Comp. Ex. 201 | 0.414 | 0.008 |
| Comp. Ex. 202 | 0.427 | 0.012 |
| Ex. 201 | 0.588 | 0.014 |
| Ex. 202 | 0.589 | 0.012 |
| Ex. 203 | 0.624 | 0.040 |
| Ex. 204 | 0.646 | 0.014 |
| Ex. 205 | 0.585 | 0.135 |
| Ex. 206 | 0.703 | 0.016 |
| Ex. 207 | 0.623 | 0.003 |
| Ex. 208 | 0.659 | 0.001 |
| Ex. 209 | 0.628 | 0.092 |
| Ex. 210 | 0.711 | 0.125 |
| Ex. 211 | 0.488 | 0.010 |
| Ex. 212 | 0.574 | 0.012 |
| Ex. 213 | 0.523 | 0.000 |
| Ex. 214 | 0.903 | 0.035 |
| Ex. 215 | 1.062 | 0.017 |
| Ex. 216 | 1.140 | 0.004 |

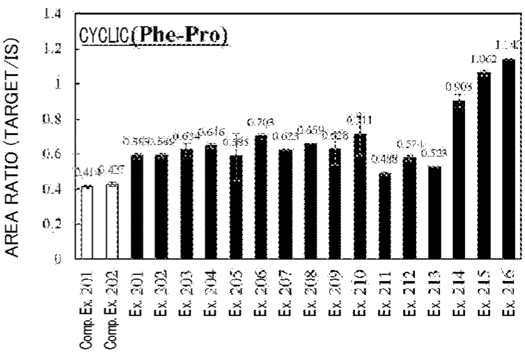


44 CYCLIC(Leu/Ile-Val)

| | Ave. | SD |
|---|---|---|
| Comp. Ex. 201 | 0.087 | 0.018 |
| Comp. Ex. 202 | 0.083 | 0.009 |
| Ex. 201 | 0.144 | 0.006 |
| Ex. 202 | 0.146 | 0.014 |
| Ex. 203 | 0.179 | 0.006 |
| Ex. 204 | 0.159 | 0.004 |
| Ex. 205 | 0.196 | 0.002 |
| Ex. 206 | 0.175 | 0.014 |
| Ex. 207 | 0.207 | 0.015 |
| Ex. 208 | 0.184 | 0.019 |
| Ex. 209 | 0.122 | 0.023 |
| Ex. 210 | 0.152 | 0.048 |
| Ex. 211 | 0.111 | 0.012 |
| Ex. 212 | 0.128 | 0.006 |
| Ex. 213 | 0.106 | 0.003 |
| Ex. 214 | 0.186 | 0.005 |
| Ex. 215 | 0.252 | 0.002 |
| Ex. 216 | 0.261 | 0.010 |

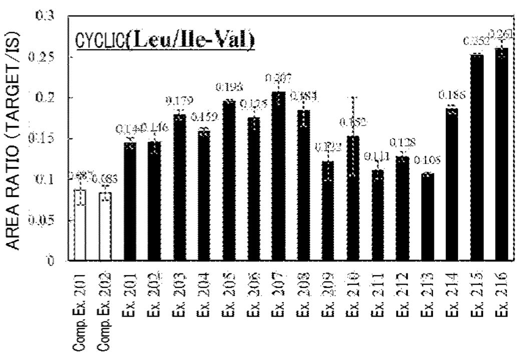


FIG. 11

45 CYCLIC(Val-Phe)

| | Ave. | SD |
|---|---|---|
| Comp. Ex. 201 | 0.009 | 0.003 |
| Comp. Ex. 202 | 0.010 | 0.001 |
| Ex. 201 | 0.014 | 0.001 |
| Ex. 202 | 0.016 | 0.001 |
| Ex. 203 | 0.023 | 0.003 |
| Ex. 204 | 0.025 | 0.000 |
| Ex. 205 | 0.024 | 0.004 |
| Ex. 206 | 0.022 | 0.007 |
| Ex. 207 | 0.029 | 0.001 |
| Ex. 208 | 0.028 | 0.004 |
| Ex. 209 | 0.031 | 0.009 |
| Ex. 210 | 0.025 | 0.003 |
| Ex. 211 | 0.057 | 0.004 |
| Ex. 212 | 0.044 | 0.000 |
| Ex. 213 | 0.044 | 0.003 |
| Ex. 214 | 0.029 | 0.002 |
| Ex. 215 | 0.013 | 0.001 |
| Ex. 216 | 0.014 | 0.000 |

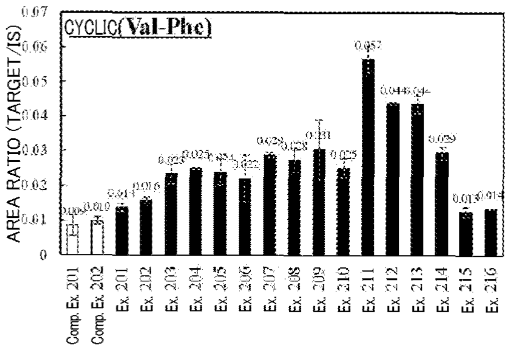


46 CYCLIC(Tyr-Asp)

| | Ave. | SD |
|---|---|---|
| Comp. Ex. 201 | nd | |
| Comp. Ex. 202 | nd | |
| Ex. 201 | 0.052 | 0.001 |
| Ex. 202 | 0.025 | 0.001 |
| Ex. 203 | 0.087 | 0.002 |
| Ex. 204 | 0.073 | 0.000 |
| Ex. 205 | 0.134 | 0.005 |
| Ex. 206 | 0.095 | 0.004 |
| Ex. 207 | 0.141 | 0.004 |
| Ex. 208 | 0.087 | 0.010 |
| Ex. 209 | 0.165 | 0.028 |
| Ex. 210 | 0.127 | 0.014 |
| Ex. 211 | 0.053 | 0.004 |
| Ex. 212 | 0.056 | 0.010 |
| Ex. 213 | 0.053 | 0.010 |
| Ex. 214 | 0.105 | 0.001 |
| Ex. 215 | 0.115 | 0.002 |
| Ex. 216 | 0.175 | 0.001 |

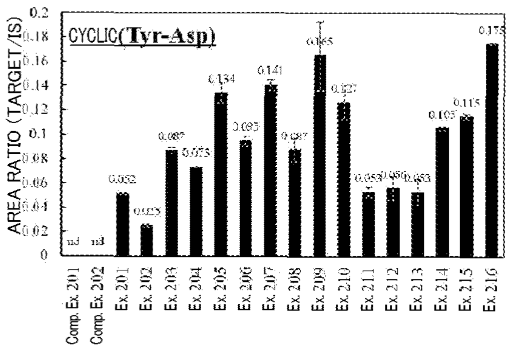


47 CYCLIC(Ala-Asp)

| | Ave. | SD |
|---|---|---|
| Comp. Ex. 201 | 0.000 | |
| Comp. Ex. 202 | 0.000 | |
| Ex. 201 | 0.011 | 0.000 |
| Ex. 202 | 0.012 | 0.001 |
| Ex. 203 | 0.017 | 0.000 |
| Ex. 204 | 0.014 | 0.001 |
| Ex. 205 | 0.024 | 0.002 |
| Ex. 206 | 0.022 | 0.003 |
| Ex. 207 | 0.009 | 0.001 |
| Ex. 208 | 0.013 | 0.001 |
| Ex. 209 | 0.029 | 0.001 |
| Ex. 210 | 0.031 | 0.000 |
| Ex. 211 | 0.022 | 0.001 |
| Ex. 212 | 0.022 | 0.000 |
| Ex. 213 | 0.020 | 0.001 |
| Ex. 214 | 0.027 | 0.001 |
| Ex. 215 | 0.022 | 0.001 |
| Ex. 216 | 0.019 | 0.005 |

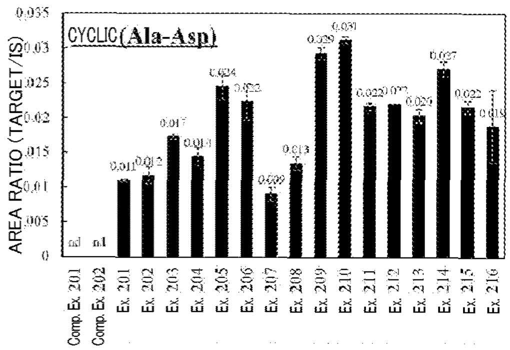


48 CYCLIC(Val-Ser)

| | Ave. | SD |
|---|---|---|
| Comp. Ex. 201 | 0.059 | 0.016 |
| Comp. Ex. 202 | 0.048 | 0.006 |
| Ex. 201 | 0.146 | 0.013 |
| Ex. 202 | 0.157 | 0.005 |
| Ex. 203 | 0.166 | 0.008 |
| Ex. 204 | 0.173 | 0.014 |
| Ex. 205 | 0.167 | 0.007 |
| Ex. 206 | 0.167 | 0.017 |
| Ex. 207 | 0.170 | 0.005 |
| Ex. 208 | 0.179 | 0.013 |
| Ex. 209 | 0.181 | 0.015 |
| Ex. 210 | 0.200 | 0.006 |
| Ex. 211 | 0.101 | 0.004 |
| Ex. 212 | 0.113 | 0.002 |
| Ex. 213 | 0.117 | 0.002 |
| Ex. 214 | 0.095 | 0.001 |
| Ex. 215 | 0.087 | 0.003 |
| Ex. 216 | 0.126 | 0.015 |

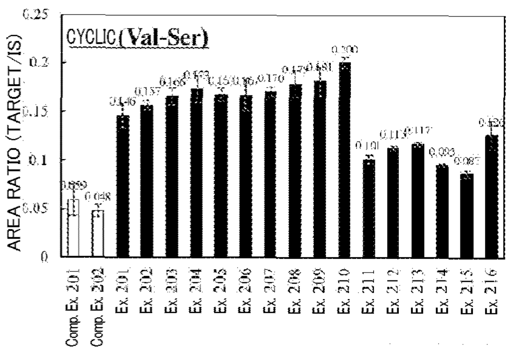


FIG. 12

SULFUROL

| | Ave. | SD |
|---|---|---|
| Comp. Ex. 201 | 0.075 | 0.000 |
| Comp. Ex. 202 | 0.068 | 0.004 |
| Ex. 201 | 0.116 | 0.007 |
| Ex. 202 | 0.096 | 0.001 |
| Ex. 203 | 0.140 | 0.006 |
| Ex. 204 | 0.132 | 0.001 |
| Ex. 205 | 0.128 | 0.013 |
| Ex. 206 | 0.115 | 0.002 |
| Ex. 207 | 0.134 | 0.012 |
| Ex. 208 | 0.140 | 0.001 |
| Ex. 209 | 0.116 | 0.008 |
| Ex. 210 | 0.113 | 0.006 |
| Ex. 211 | 0.149 | 0.002 |
| Ex. 212 | 0.121 | 0.004 |
| Ex. 213 | 0.142 | 0.018 |
| Ex. 214 | 0.189 | 0.011 |
| Ex. 215 | 0.183 | 0.012 |
| Ex. 216 | 0.239 | 0.004 |

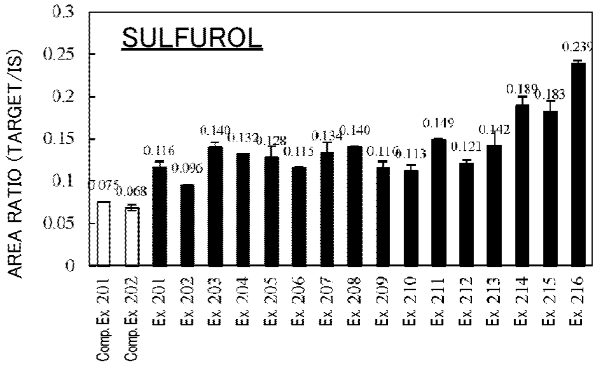


SULFUROL ACETATE

| | Ave. | SD |
|---|---|---|
| Comp. Ex. 201 | 0.011 | 0.001 |
| Comp. Ex. 202 | 0.010 | 0.001 |
| Ex. 201 | 0.023 | 0.002 |
| Ex. 202 | 0.023 | 0.003 |
| Ex. 203 | 0.040 | 0.006 |
| Ex. 204 | 0.034 | 0.002 |
| Ex. 205 | 0.031 | 0.003 |
| Ex. 206 | 0.028 | 0.003 |
| Ex. 207 | 0.046 | 0.005 |
| Ex. 208 | 0.037 | 0.001 |
| Ex. 209 | 0.029 | 0.001 |
| Ex. 210 | 0.023 | 0.000 |
| Ex. 211 | 0.015 | 0.000 |
| Ex. 212 | 0.024 | 0.003 |
| Ex. 213 | 0.025 | 0.002 |
| Ex. 214 | 0.038 | 0.002 |
| Ex. 215 | 0.073 | 0.010 |
| Ex. 216 | 0.113 | 0.002 |

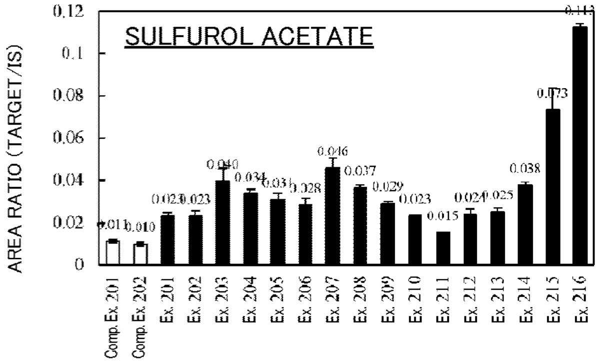


TARTARIC ACID

| | Ave. | SD |
|---|---|---|
| Comp. Ex. 201 | 0.463 | 0.120 |
| Comp. Ex. 202 | 0.517 | 0.034 |
| Ex. 201 | 0.790 | 0.165 |
| Ex. 202 | 0.992 | 0.187 |
| Ex. 203 | 0.888 | 0.092 |
| Ex. 204 | 1.066 | 0.013 |
| Ex. 205 | 0.989 | 0.122 |
| Ex. 206 | 1.070 | 0.075 |
| Ex. 207 | 1.157 | 0.092 |
| Ex. 208 | 1.086 | 0.044 |
| Ex. 209 | 1.008 | 0.011 |
| Ex. 210 | 1.070 | 0.047 |
| Ex. 211 | 1.116 | 0.041 |
| Ex. 212 | 1.407 | 0.004 |
| Ex. 213 | 1.296 | 0.180 |
| Ex. 214 | 2.741 | 0.002 |
| Ex. 215 | 3.362 | 0.212 |
| Ex. 216 | 4.163 | 0.126 |

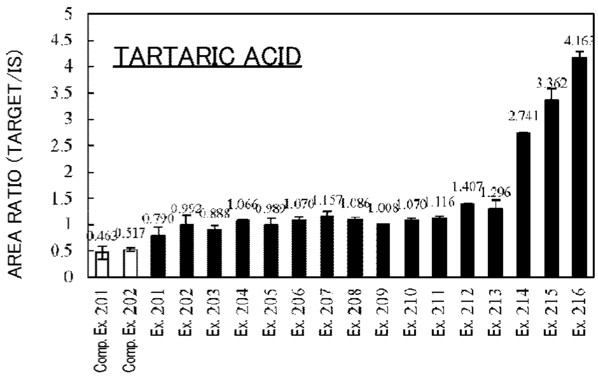


trans-ACONITIC ACID

| | Ave. | SD |
|---|---|---|
| Comp. Ex. 201 | 0.318 | 0.002 |
| Comp. Ex. 202 | 0.328 | 0.003 |
| Ex. 201 | 0.478 | 0.011 |
| Ex. 202 | 0.415 | 0.034 |
| Ex. 203 | 0.514 | 0.015 |
| Ex. 204 | 0.479 | 0.066 |
| Ex. 205 | 0.596 | 0.032 |
| Ex. 206 | 0.504 | 0.039 |
| Ex. 207 | 0.678 | 0.023 |
| Ex. 208 | 0.578 | 0.018 |
| Ex. 209 | 0.677 | 0.031 |
| Ex. 210 | 0.567 | 0.028 |
| Ex. 211 | 0.748 | 0.071 |
| Ex. 212 | 1.094 | 0.112 |
| Ex. 213 | 0.724 | 0.015 |
| Ex. 214 | 1.727 | 0.361 |
| Ex. 215 | 2.493 | 0.024 |
| Ex. 216 | 2.956 | 0.201 |

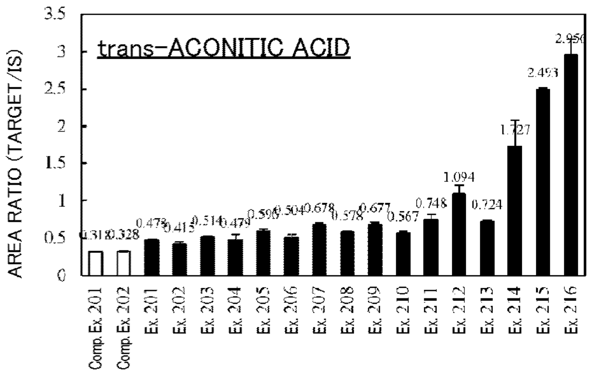


FIG. 13

1 CYCLIC(Phe-Phe)

| | Ave. | SD |
|---|---|---|
| Comp. Ex.301 | 0.023 | 0.001 |
| Ex. 301 | 0.034 | 0.007 |
| Ex. 302 | 0.029 | 0.003 |
| Ex. 303 | 0.044 | 0.011 |
| Ex. 304 | 0.036 | 0.001 |
| Ex. 305 | 0.034 | 0.001 |
| Ex. 306 | 0.055 | 0.010 |
| Ex. 308 | 0.055 | 0.005 |
| Ex. 307 | 0.038 | 0.011 |
| Ex. 309 | 0.049 | 0.000 |
| Ex. 310 | 0.058 | 0.015 |
| Ex. 311 | 0.032 | 0.004 |
| Ex. 312 | 0.048 | 0.001 |
| Ex. 314 | 0.041 | 0.004 |
| Ex. 313 | 0.061 | 0.003 |
| Ex. 315 | 0.027 | 0.003 |
| Ex. 316 | 0.028 | 0.001 |
| Ex. 317 | 0.030 | 0.002 |
| Ex. 318 | 0.037 | 0.001 |
| Ex. 319 | 0.026 | 0.000 |

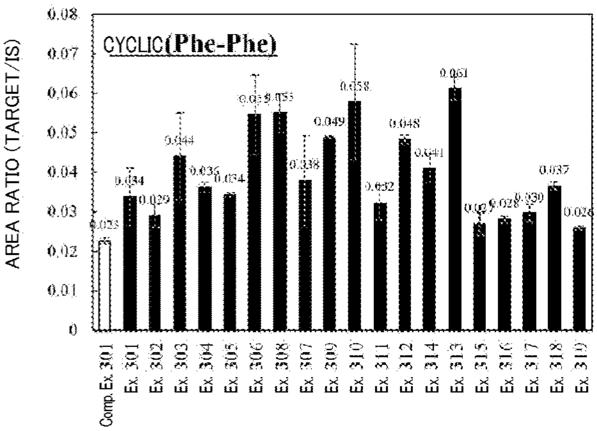


2 CYCLIC(Phe-Leu/Ile)

| | Ave. | SD |
|---|---|---|
| Comp. Ex.301 | 0.038 | 0.007 |
| Ex. 301 | 0.062 | 0.006 |
| Ex. 302 | 0.072 | 0.002 |
| Ex. 303 | 0.070 | 0.005 |
| Ex. 304 | 0.096 | 0.001 |
| Ex. 305 | 0.124 | 0.009 |
| Ex. 306 | 0.164 | 0.010 |
| Ex. 308 | 0.156 | 0.000 |
| Ex. 307 | 0.139 | 0.026 |
| Ex. 309 | 0.137 | 0.019 |
| Ex. 310 | 0.210 | 0.015 |
| Ex. 311 | 0.142 | 0.011 |
| Ex. 312 | 0.213 | 0.000 |
| Ex. 314 | 0.186 | 0.012 |
| Ex. 313 | 0.310 | 0.028 |
| Ex. 315 | 0.060 | 0.001 |
| Ex. 316 | 0.099 | 0.007 |
| Ex. 317 | 0.109 | 0.014 |
| Ex. 318 | 0.125 | 0.020 |
| Ex. 319 | 0.150 | 0.013 |

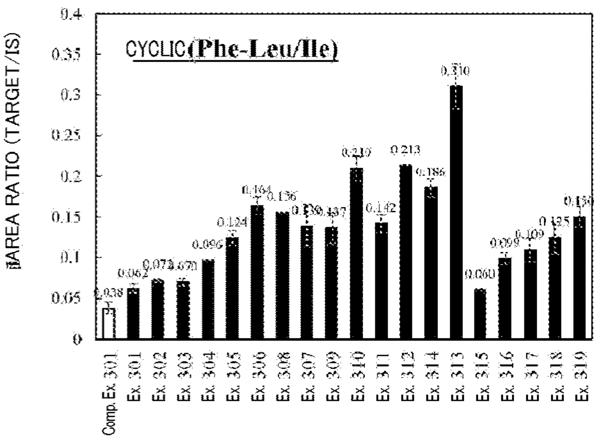


3 CYCLIC(Leu/Ile-Val)

| | Ave. | SD |
|---|---|---|
| Comp. Ex.301 | 0.010 | 0.002 |
| Ex. 301 | 0.024 | 0.002 |
| Ex. 302 | 0.033 | 0.005 |
| Ex. 303 | 0.031 | 0.008 |
| Ex. 304 | 0.046 | 0.013 |
| Ex. 305 | 0.065 | 0.006 |
| Ex. 306 | 0.090 | 0.005 |
| Ex. 308 | 0.116 | 0.006 |
| Ex. 307 | 0.055 | 0.010 |
| Ex. 309 | 0.100 | 0.009 |
| Ex. 310 | 0.129 | 0.027 |
| Ex. 311 | 0.044 | 0.001 |
| Ex. 312 | 0.070 | 0.007 |
| Ex. 314 | 0.081 | 0.009 |
| Ex. 313 | 0.129 | 0.011 |
| Ex. 315 | 0.021 | 0.009 |
| Ex. 316 | 0.051 | 0.004 |
| Ex. 317 | 0.106 | 0.006 |
| Ex. 318 | 0.146 | 0.008 |
| Ex. 319 | 0.236 | 0.015 |

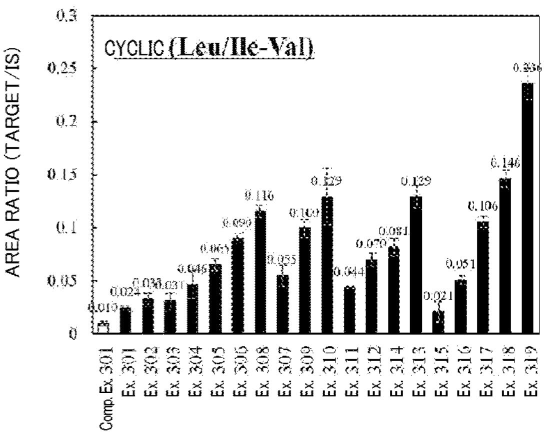


FIG. 14

4 CYCLIC(Phe-Pro)

| | Ave. | SD |
|---|---|---|
| Comp. Ex.301 | 0.013 | 0.003 |
| Ex. 301 | 0.039 | 0.002 |
| Ex. 302 | 0.056 | 0.002 |
| Ex. 303 | 0.047 | 0.002 |
| Ex. 304 | 0.071 | 0.003 |
| Ex. 305 | 0.119 | 0.006 |
| Ex. 306 | 0.166 | 0.018 |
| Ex. 308 | 0.166 | 0.003 |
| Ex. 307 | 0.137 | 0.021 |
| Ex. 309 | 0.155 | 0.025 |
| Ex. 310 | 0.232 | 0.009 |
| Ex. 311 | 0.119 | 0.015 |
| Ex. 312 | 0.200 | 0.014 |
| Ex. 314 | 0.212 | 0.003 |
| Ex. 313 | 0.302 | 0.011 |
| Ex. 315 | 0.034 | 0.001 |
| Ex. 316 | 0.068 | 0.006 |
| Ex. 317 | 0.125 | 0.007 |
| Ex. 318 | 0.103 | 0.009 |
| Ex. 319 | 0.201 | 0.026 |

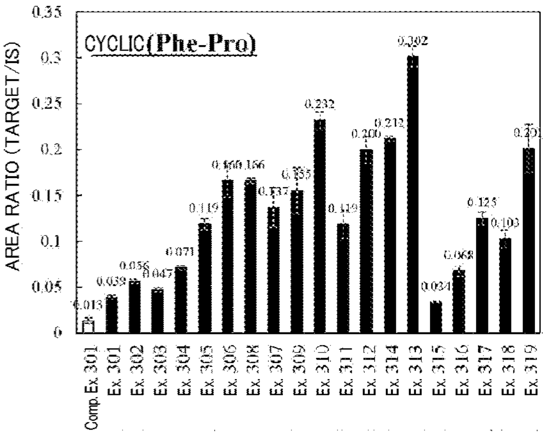


5 CYCLIC(Phe-Tyr)

| | Ave. | SD |
|---|---|---|
| Comp. Ex.301 | nd | |
| Ex. 301 | 0.003 | 0.000 |
| Ex. 302 | 0.003 | 0.001 |
| Ex. 303 | 0.003 | 0.000 |
| Ex. 304 | 0.005 | 0.001 |
| Ex. 305 | 0.006 | 0.000 |
| Ex. 306 | 0.007 | 0.001 |
| Ex. 308 | 0.006 | 0.001 |
| Ex. 307 | 0.005 | 0.000 |
| Ex. 309 | 0.006 | 0.000 |
| Ex. 310 | 0.009 | 0.002 |
| Ex. 311 | 0.005 | 0.003 |
| Ex. 312 | 0.010 | 0.001 |
| Ex. 314 | 0.008 | 0.003 |
| Ex. 313 | 0.012 | 0.001 |
| Ex. 315 | 0.022 | 0.001 |
| Ex. 316 | 0.011 | 0.000 |
| Ex. 317 | 0.013 | 0.002 |
| Ex. 318 | 0.011 | 0.000 |
| Ex. 319 | 0.018 | 0.002 |

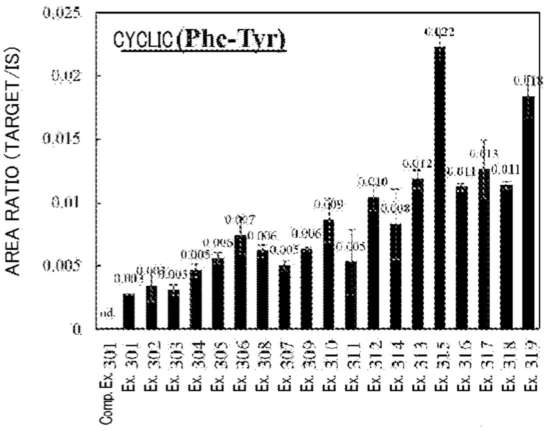


6 CYCLIC(Pro-Leu/Ile)

| | Ave. | SD |
|---|---|---|
| Comp. Ex.301 | 0.031 | 0.005 |
| Ex. 301 | 0.053 | 0.003 |
| Ex. 302 | 0.093 | 0.006 |
| Ex. 303 | 0.066 | 0.003 |
| Ex. 304 | 0.123 | 0.004 |
| Ex. 305 | 0.198 | 0.010 |
| Ex. 306 | 0.345 | 0.080 |
| Ex. 308 | 0.336 | 0.010 |
| Ex. 307 | 0.224 | 0.040 |
| Ex. 309 | 0.348 | 0.090 |
| Ex. 310 | 0.743 | 0.062 |
| Ex. 311 | 0.204 | 0.016 |
| Ex. 312 | 0.510 | 0.047 |
| Ex. 314 | 0.604 | 0.004 |
| Ex. 313 | 1.073 | 0.119 |
| Ex. 315 | 0.100 | 0.002 |
| Ex. 316 | 0.243 | 0.008 |
| Ex. 317 | 0.310 | 0.008 |
| Ex. 318 | 0.252 | 0.010 |
| Ex. 319 | 0.702 | 0.071 |

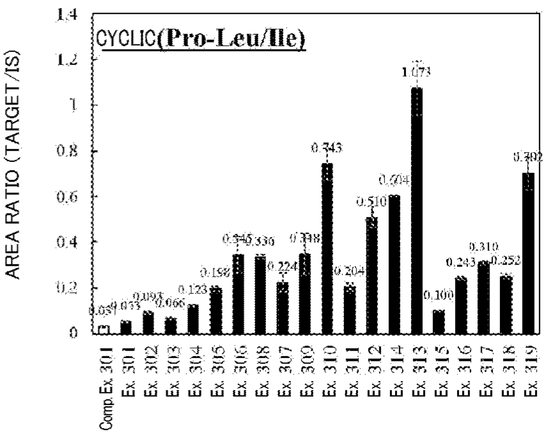


FIG. 15

7 CYCLIC(Val-Val)

| | Ave. | SD |
|---|---|---|
| Comp. Ex.301 | 0.002 | 0.000 |
| Ex. 301 | 0.009 | 0.003 |
| Ex. 302 | 0.006 | 0.002 |
| Ex. 303 | 0.014 | 0.003 |
| Ex. 304 | 0.011 | 0.002 |
| Ex. 305 | 0.016 | 0.004 |
| Ex. 306 | 0.014 | 0.001 |
| Ex. 308 | 0.014 | 0.001 |
| Ex. 307 | 0.019 | 0.001 |
| Ex. 309 | 0.019 | 0.001 |
| Ex. 310 | 0.020 | 0.001 |
| Ex. 311 | 0.017 | 0.002 |
| Ex. 312 | 0.017 | 0.004 |
| Ex. 314 | 0.022 | 0.002 |
| Ex. 313 | 0.022 | 0.002 |
| Ex. 315 | 0.015 | 0.003 |
| Ex. 316 | 0.006 | 0.001 |
| Ex. 317 | 0.009 | 0.000 |
| Ex. 318 | 0.013 | 0.000 |
| Ex. 319 | 0.009 | 0.001 |

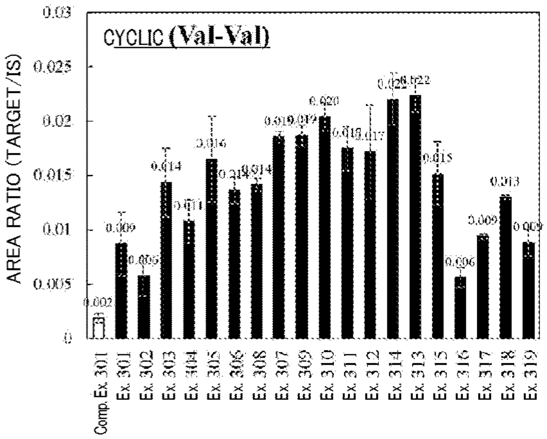


8 CYCLIC(Phe-Ala)

| | Ave. | SD |
|---|---|---|
| Comp. Ex.301 | nd | |
| Ex. 301 | 0.005 | 0.000 |
| Ex. 302 | 0.007 | 0.002 |
| Ex. 303 | 0.007 | 0.002 |
| Ex. 304 | 0.009 | 0.002 |
| Ex. 305 | 0.012 | 0.001 |
| Ex. 306 | 0.020 | 0.007 |
| Ex. 308 | 0.016 | 0.001 |
| Ex. 307 | 0.023 | 0.002 |
| Ex. 309 | 0.019 | 0.001 |
| Ex. 310 | 0.024 | 0.007 |
| Ex. 311 | 0.007 | 0.001 |
| Ex. 312 | 0.013 | 0.001 |
| Ex. 314 | 0.013 | 0.002 |
| Ex. 313 | 0.021 | 0.002 |
| Ex. 315 | 0.003 | 0.001 |
| Ex. 316 | 0.008 | 0.001 |
| Ex. 317 | 0.016 | 0.003 |
| Ex. 318 | 0.020 | 0.001 |
| Ex. 319 | 0.033 | 0.002 |

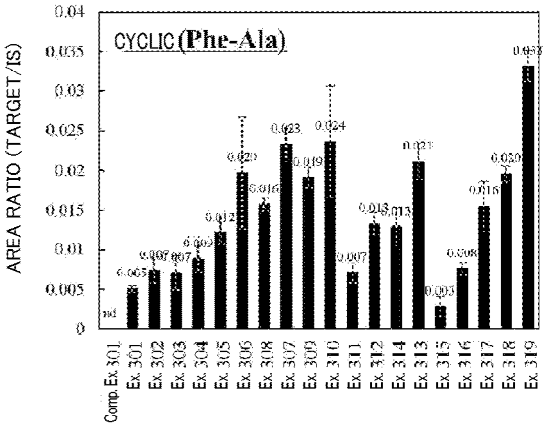


9 CYCLIC(Tyr-Ser)

| | Ave. | SD |
|---|---|---|
| Comp. Ex.301 | nd | |
| Ex. 301 | 0.013 | 0.002 |
| Ex. 302 | 0.005 | 0.003 |
| Ex. 303 | 0.018 | 0.002 |
| Ex. 304 | 0.009 | 0.002 |
| Ex. 305 | 0.037 | 0.005 |
| Ex. 306 | 0.018 | 0.001 |
| Ex. 308 | 0.022 | 0.002 |
| Ex. 307 | 0.037 | 0.002 |
| Ex. 309 | 0.054 | 0.009 |
| Ex. 310 | 0.053 | 0.003 |
| Ex. 311 | 0.030 | 0.000 |
| Ex. 312 | 0.017 | 0.003 |
| Ex. 314 | 0.055 | 0.001 |
| Ex. 313 | 0.036 | 0.003 |
| Ex. 315 | 0.045 | 0.004 |
| Ex. 316 | 0.039 | 0.004 |
| Ex. 317 | 0.032 | 0.004 |
| Ex. 318 | 0.022 | 0.002 |
| Ex. 319 | 0.029 | 0.007 |

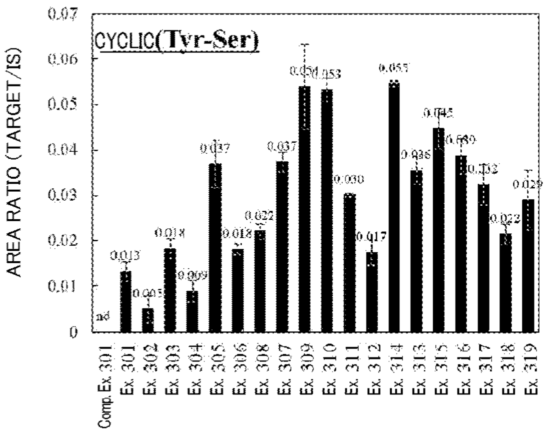


FIG. 16

10 CYCLIC(Pro-Val)

| | Ave. | SD |
|---|---|---|
| Comp. Ex.301 | 0.012 | 0.001 |
| Ex. 301 | 0.018 | 0.003 |
| Ex. 302 | 0.032 | 0.005 |
| Ex. 303 | 0.017 | 0.000 |
| Ex. 304 | 0.023 | 0.003 |
| Ex. 305 | 0.032 | 0.003 |
| Ex. 306 | 0.041 | 0.003 |
| Ex. 308 | 0.030 | 0.004 |
| Ex. 307 | 0.035 | 0.002 |
| Ex. 309 | 0.028 | 0.001 |
| Ex. 310 | 0.048 | 0.004 |
| Ex. 311 | 0.040 | 0.006 |
| Ex. 312 | 0.053 | 0.012 |
| Ex. 314 | 0.044 | 0.002 |
| Ex. 313 | 0.058 | 0.009 |
| Ex. 315 | 0.016 | 0.002 |
| Ex. 316 | 0.032 | 0.001 |
| Ex. 317 | 0.056 | 0.007 |
| Ex. 318 | 0.049 | 0.010 |
| Ex. 319 | 0.045 | 0.003 |

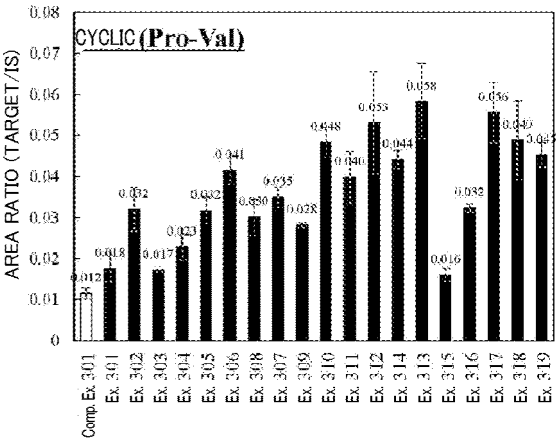


11 CYCLIC(Pro-Tyr)

| | Ave. | SD |
|---|---|---|
| Comp. Ex.301 | 0.005 | 0.000 |
| Ex. 301 | 0.013 | 0.001 |
| Ex. 302 | 0.021 | 0.003 |
| Ex. 303 | 0.014 | 0.002 |
| Ex. 304 | 0.024 | 0.000 |
| Ex. 305 | 0.030 | 0.002 |
| Ex. 306 | 0.042 | 0.014 |
| Ex. 308 | 0.036 | 0.013 |
| Ex. 307 | 0.036 | 0.005 |
| Ex. 309 | 0.036 | 0.006 |
| Ex. 310 | 0.046 | 0.007 |
| Ex. 311 | 0.034 | 0.004 |
| Ex. 312 | 0.064 | 0.008 |
| Ex. 314 | 0.066 | 0.001 |
| Ex. 313 | 0.078 | 0.016 |
| Ex. 315 | 0.012 | 0.003 |
| Ex. 316 | 0.043 | 0.001 |
| Ex. 317 | 0.080 | 0.006 |
| Ex. 318 | 0.093 | 0.001 |
| Ex. 319 | 0.079 | 0.013 |

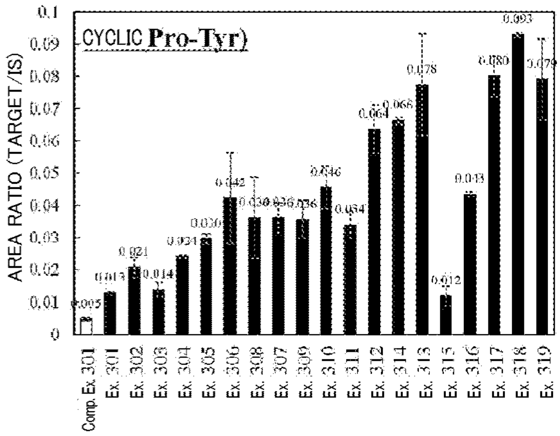


12 CYCLIC(Pro-Pro)

| | Ave. | SD |
|---|---|---|
| Comp. Ex.301 | 0.031 | 0.003 |
| Ex. 301 | 0.066 | 0.007 |
| Ex. 302 | 0.115 | 0.002 |
| Ex. 303 | 0.096 | 0.007 |
| Ex. 304 | 0.136 | 0.001 |
| Ex. 305 | 0.301 | 0.011 |
| Ex. 306 | 0.376 | 0.083 |
| Ex. 308 | 0.293 | 0.086 |
| Ex. 307 | 0.381 | 0.002 |
| Ex. 309 | 0.407 | 0.065 |
| Ex. 310 | 0.536 | 0.044 |
| Ex. 311 | 0.292 | 0.041 |
| Ex. 312 | 0.465 | 0.019 |
| Ex. 314 | 0.592 | 0.044 |
| Ex. 313 | 0.726 | 0.012 |
| Ex. 315 | 0.063 | 0.005 |
| Ex. 316 | 0.194 | 0.021 |
| Ex. 317 | 0.556 | 0.052 |
| Ex. 318 | 0.589 | 0.008 |
| Ex. 319 | 0.148 | 0.022 |

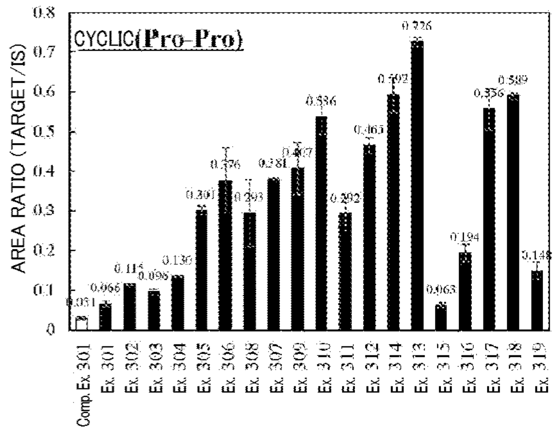


FIG. 17

13 CYCLIC(Leu/Ile-Ser)

| | Ave. | SD |
|---|---|---|
| Comp. Ex. 301 | nd | |
| Ex. 301 | 0.020 | 0.004 |
| Ex. 302 | 0.017 | 0.003 |
| Ex. 303 | 0.014 | 0.002 |
| Ex. 304 | 0.020 | 0.001 |
| Ex. 305 | 0.037 | 0.010 |
| Ex. 306 | 0.037 | 0.009 |
| Ex. 308 | 0.030 | 0.012 |
| Ex. 307 | 0.046 | 0.004 |
| Ex. 309 | 0.044 | 0.008 |
| Ex. 310 | 0.057 | 0.009 |
| Ex. 311 | 0.025 | 0.006 |
| Ex. 312 | 0.029 | 0.000 |
| Ex. 314 | 0.037 | 0.001 |
| Ex. 313 | 0.066 | 0.007 |
| Ex. 315 | 0.087 | 0.007 |
| Ex. 316 | 0.046 | 0.003 |
| Ex. 317 | 0.034 | 0.003 |
| Ex. 318 | 0.030 | 0.005 |
| Ex. 319 | 0.032 | 0.004 |

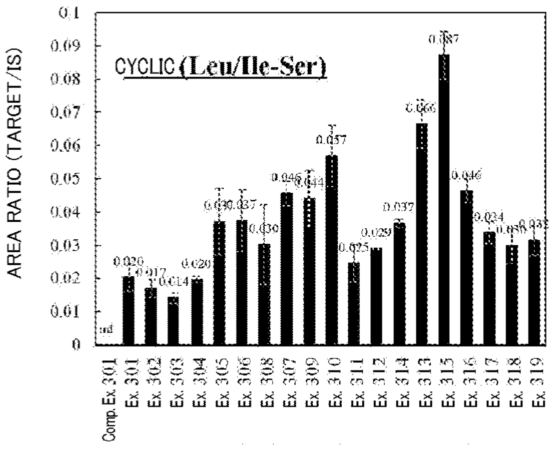


14 CYCLIC(Arg-Leu/Ile)

| | Ave. | SD |
|---|---|---|
| Comp. Ex. 301 | 0.063 | 0.011 |
| Ex. 301 | 0.168 | 0.041 |
| Ex. 302 | 0.186 | 0.001 |
| Ex. 303 | 0.179 | 0.061 |
| Ex. 304 | 0.234 | 0.103 |
| Ex. 305 | 0.307 | 0.071 |
| Ex. 306 | 0.424 | 0.109 |
| Ex. 308 | 0.236 | 0.003 |
| Ex. 307 | 0.445 | 0.026 |
| Ex. 309 | 0.336 | 0.010 |
| Ex. 310 | 0.422 | 0.044 |
| Ex. 311 | 0.267 | 0.047 |
| Ex. 312 | 0.383 | 0.030 |
| Ex. 314 | 0.311 | 0.060 |
| Ex. 313 | 0.487 | 0.019 |
| Ex. 315 | 0.198 | 0.018 |
| Ex. 316 | 0.610 | 0.019 |
| Ex. 317 | 0.778 | 0.024 |
| Ex. 318 | 0.661 | 0.034 |
| Ex. 319 | 0.189 | 0.009 |

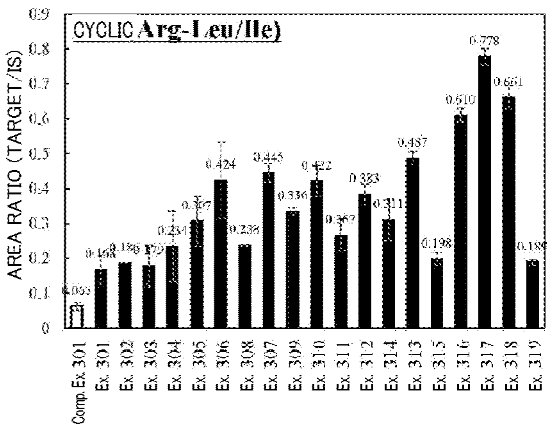


15 CYCLIC(Ala-Tyr)

| | Ave. | SD |
|---|---|---|
| Comp. Ex. 301 | nd | |
| Ex. 301 | 0.014 | 0.007 |
| Ex. 302 | 0.018 | 0.011 |
| Ex. 303 | 0.016 | 0.006 |
| Ex. 304 | 0.017 | 0.004 |
| Ex. 305 | 0.058 | 0.011 |
| Ex. 306 | 0.049 | 0.006 |
| Ex. 308 | 0.025 | 0.013 |
| Ex. 307 | 0.044 | 0.016 |
| Ex. 309 | 0.076 | 0.004 |
| Ex. 310 | 0.068 | 0.018 |
| Ex. 311 | 0.028 | 0.017 |
| Ex. 312 | 0.048 | 0.001 |
| Ex. 314 | 0.091 | 0.016 |
| Ex. 313 | 0.076 | 0.006 |
| Ex. 315 | 0.031 | 0.002 |
| Ex. 316 | 0.025 | 0.007 |
| Ex. 317 | 0.033 | 0.001 |
| Ex. 318 | 0.026 | 0.002 |
| Ex. 319 | 0.042 | 0.003 |

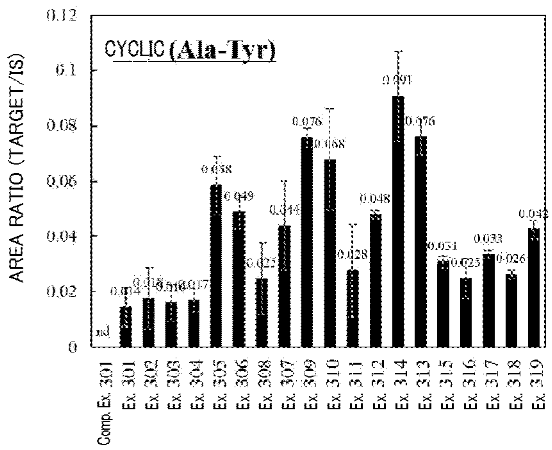


FIG. 18

16 CYCLIC(Gly-Tyr)

| | Ave. | SD |
|---|---|---|
| Comp. Ex.301 | nd | |
| Ex. 301 | 0.003 | 0.001 |
| Ex. 302 | 0.004 | 0.001 |
| Ex. 303 | 0.007 | 0.000 |
| Ex. 304 | 0.008 | 0.001 |
| Ex. 305 | 0.018 | 0.002 |
| Ex. 306 | 0.026 | 0.000 |
| Ex. 308 | 0.016 | 0.001 |
| Ex. 307 | 0.021 | 0.001 |
| Ex. 309 | 0.018 | 0.000 |
| Ex. 310 | 0.021 | 0.007 |
| Ex. 311 | 0.015 | 0.001 |
| Ex. 312 | 0.010 | 0.000 |
| Ex. 314 | 0.018 | 0.003 |
| Ex. 313 | 0.034 | 0.005 |
| Ex. 315 | 0.016 | 0.001 |
| Ex. 316 | 0.023 | 0.001 |
| Ex. 317 | 0.036 | 0.007 |
| Ex. 318 | 0.037 | 0.014 |
| Ex. 319 | 0.045 | 0.011 |

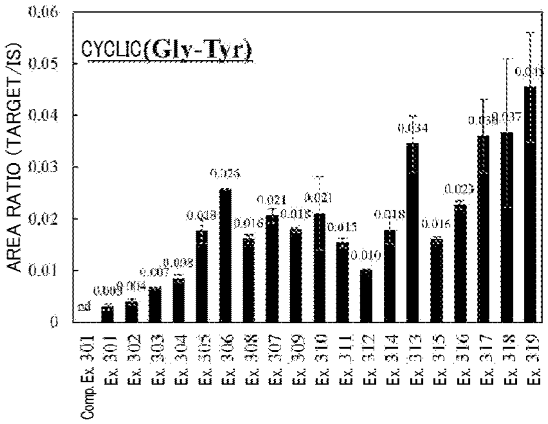


17 CYCLIC(Ala-Pro)

| | Ave. | SD |
|---|---|---|
| Comp. Ex.301 | nd | 0.000 |
| Ex. 301 | 0.037 | 0.016 |
| Ex. 302 | 0.046 | 0.016 |
| Ex. 303 | 0.049 | 0.007 |
| Ex. 304 | 0.054 | 0.002 |
| Ex. 305 | 0.078 | 0.004 |
| Ex. 306 | 0.098 | 0.015 |
| Ex. 308 | 0.066 | 0.005 |
| Ex. 307 | 0.093 | 0.019 |
| Ex. 309 | 0.096 | 0.003 |
| Ex. 310 | 0.118 | 0.001 |
| Ex. 311 | 0.068 | 0.013 |
| Ex. 312 | 0.093 | 0.014 |
| Ex. 314 | 0.115 | 0.017 |
| Ex. 313 | 0.149 | 0.021 |
| Ex. 315 | 0.029 | 0.002 |
| Ex. 316 | 0.058 | 0.004 |
| Ex. 317 | 0.095 | 0.008 |
| Ex. 318 | 0.096 | 0.002 |
| Ex. 319 | 0.141 | 0.003 |

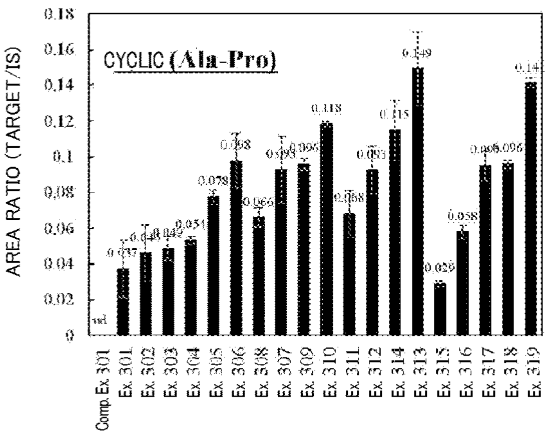


18 CYCLIC(Gly-Val)

| | Ave. | SD |
|---|---|---|
| Comp. Ex.301 | nd | |
| Ex. 301 | 0.002 | 0.001 |
| Ex. 302 | 0.005 | 0.001 |
| Ex. 303 | 0.008 | 0.002 |
| Ex. 304 | 0.006 | 0.000 |
| Ex. 305 | 0.011 | 0.002 |
| Ex. 306 | 0.014 | 0.005 |
| Ex. 308 | 0.014 | 0.005 |
| Ex. 307 | 0.016 | 0.007 |
| Ex. 309 | 0.016 | 0.007 |
| Ex. 310 | 0.028 | 0.002 |
| Ex. 311 | 0.014 | 0.002 |
| Ex. 312 | 0.019 | 0.001 |
| Ex. 314 | 0.026 | 0.005 |
| Ex. 313 | 0.039 | 0.012 |
| Ex. 315 | 0.040 | 0.002 |
| Ex. 316 | 0.016 | 0.004 |
| Ex. 317 | 0.022 | 0.000 |
| Ex. 318 | 0.013 | 0.001 |
| Ex. 319 | 0.007 | 0.001 |

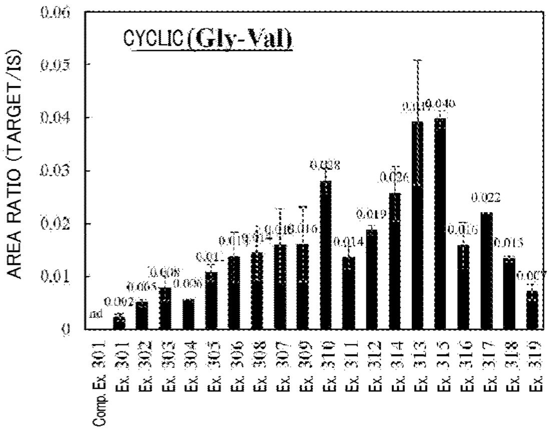


FIG. 19

19 CYCLIC(hyPro-Pro)

| | Ave. | SD |
|---|---|---|
| Comp. Ex.301 | nd | 0.000 |
| Ex. 301 | 0.028 | 0.003 |
| Ex. 302 | 0.026 | 0.007 |
| Ex. 303 | 0.026 | 0.003 |
| Ex. 304 | 0.038 | 0.008 |
| Ex. 305 | 0.063 | 0.012 |
| Ex. 306 | 0.072 | 0.004 |
| Ex. 308 | 0.087 | 0.028 |
| Ex. 307 | 0.089 | 0.008 |
| Ex. 309 | 0.074 | 0.008 |
| Ex. 310 | 0.098 | 0.012 |
| Ex. 311 | 0.081 | 0.023 |
| Ex. 312 | 0.077 | 0.004 |
| Ex. 314 | 0.084 | 0.012 |
| Ex. 313 | 0.116 | 0.002 |
| Ex. 315 | 0.024 | 0.001 |
| Ex. 316 | 0.071 | 0.006 |
| Ex. 317 | 0.136 | 0.006 |
| Ex. 318 | 0.132 | 0.013 |
| Ex. 319 | 0.081 | 0.001 |

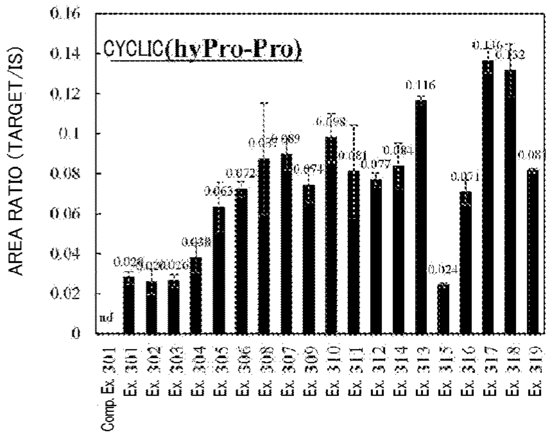


20 CYCLIC(Thr-Pro)

| | Ave. | SD |
|---|---|---|
| Comp. Ex.301 | 0.026 | 0.001 |
| Ex. 301 | 0.047 | 0.006 |
| Ex. 302 | 0.047 | 0.004 |
| Ex. 303 | 0.055 | 0.002 |
| Ex. 304 | 0.064 | 0.010 |
| Ex. 305 | 0.116 | 0.013 |
| Ex. 306 | 0.117 | 0.005 |
| Ex. 308 | 0.123 | 0.010 |
| Ex. 307 | 0.135 | 0.002 |
| Ex. 309 | 0.149 | 0.000 |
| Ex. 310 | 0.187 | 0.020 |
| Ex. 311 | 0.113 | 0.025 |
| Ex. 312 | 0.128 | 0.001 |
| Ex. 314 | 0.175 | 0.016 |
| Ex. 313 | 0.203 | 0.021 |
| Ex. 315 | 0.050 | 0.000 |
| Ex. 316 | 0.071 | 0.003 |
| Ex. 317 | 0.227 | 0.021 |
| Ex. 318 | 0.181 | 0.001 |
| Ex. 319 | 0.081 | 0.003 |

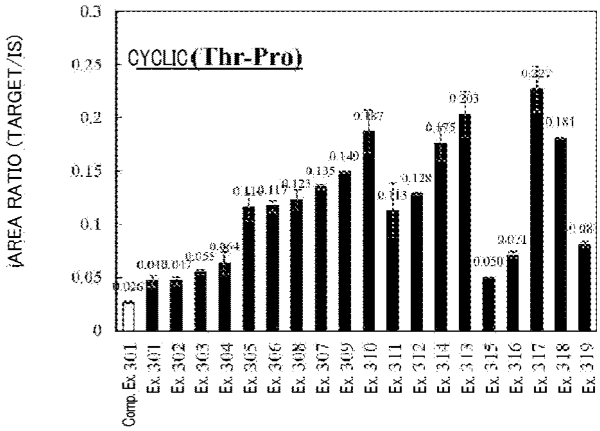


21 CYCLIC(Val-Arg)

| | Ave. | SD |
|---|---|---|
| Comp. Ex.301 | 0.031 | 0.003 |
| Ex. 301 | 0.082 | 0.009 |
| Ex. 302 | 0.088 | 0.004 |
| Ex. 303 | 0.086 | 0.015 |
| Ex. 304 | 0.087 | 0.020 |
| Ex. 305 | 0.137 | 0.020 |
| Ex. 306 | 0.174 | 0.005 |
| Ex. 308 | 0.135 | 0.023 |
| Ex. 307 | 0.194 | 0.002 |
| Ex. 309 | 0.131 | 0.002 |
| Ex. 310 | 0.172 | 0.001 |
| Ex. 311 | 0.080 | 0.001 |
| Ex. 312 | 0.158 | 0.009 |
| Ex. 314 | 0.150 | 0.012 |
| Ex. 313 | 0.195 | 0.013 |
| Ex. 315 | 0.159 | 0.006 |
| Ex. 316 | 0.150 | 0.015 |
| Ex. 317 | 0.163 | 0.014 |
| Ex. 318 | 0.159 | 0.013 |
| Ex. 319 | 0.096 | 0.002 |

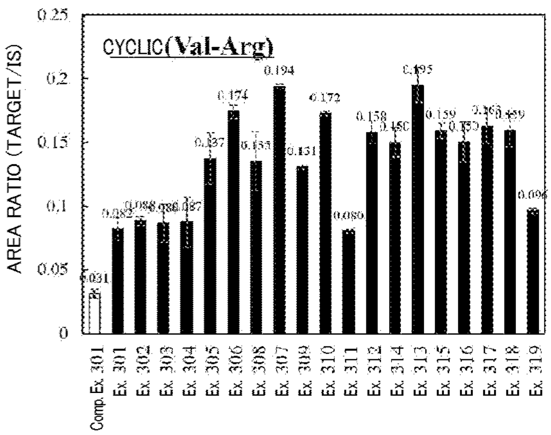


FIG. 20

22 CYCLIC(Pro-Asp)

| | Ave. | SD |
|---|---|---|
| Comp. Ex.301 | 0.029 | 0.000 |
| Ex. 301 | 0.052 | 0.002 |
| Ex. 302 | 0.057 | 0.005 |
| Ex. 303 | 0.054 | 0.012 |
| Ex. 304 | 0.081 | 0.003 |
| Ex. 305 | 0.104 | 0.009 |
| Ex. 306 | 0.131 | 0.001 |
| Ex. 308 | 0.102 | 0.011 |
| Ex. 307 | 0.136 | 0.008 |
| Ex. 309 | 0.126 | 0.002 |
| Ex. 310 | 0.149 | 0.000 |
| Ex. 311 | 0.105 | 0.032 |
| Ex. 312 | 0.130 | 0.002 |
| Ex. 314 | 0.151 | 0.009 |
| Ex. 313 | 0.168 | 0.011 |
| Ex. 315 | 0.052 | 0.003 |
| Ex. 316 | 0.106 | 0.012 |
| Ex. 317 | 0.158 | 0.001 |
| Ex. 318 | 0.196 | 0.024 |
| Ex. 319 | 0.166 | 0.052 |

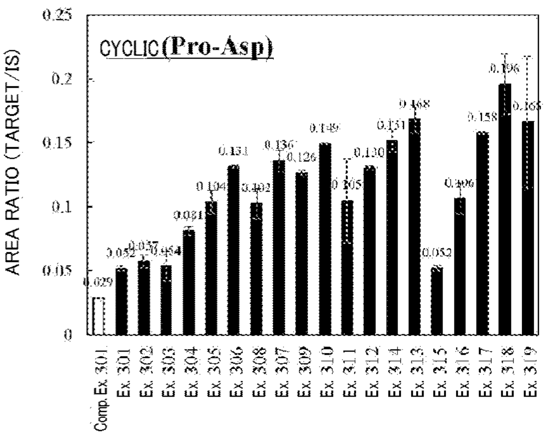


23 CYCLIC(Arg-Pro)

| | Ave. | SD |
|---|---|---|
| Comp. Ex.301 | 0.023 | 0.011 |
| Ex. 301 | 0.035 | 0.013 |
| Ex. 302 | 0.075 | 0.009 |
| Ex. 303 | 0.038 | 0.009 |
| Ex. 304 | 0.074 | 0.035 |
| Ex. 305 | 0.114 | 0.011 |
| Ex. 306 | 0.255 | 0.008 |
| Ex. 308 | 0.069 | 0.025 |
| Ex. 307 | 0.188 | 0.046 |
| Ex. 309 | 0.163 | 0.039 |
| Ex. 310 | 0.275 | 0.070 |
| Ex. 311 | 0.107 | 0.046 |
| Ex. 312 | 0.269 | 0.022 |
| Ex. 314 | 0.227 | 0.009 |
| Ex. 313 | 0.289 | 0.033 |
| Ex. 315 | 0.094 | 0.007 |
| Ex. 316 | 0.128 | 0.011 |
| Ex. 317 | 0.289 | 0.046 |
| Ex. 318 | 0.209 | 0.015 |
| Ex. 319 | 0.115 | 0.002 |

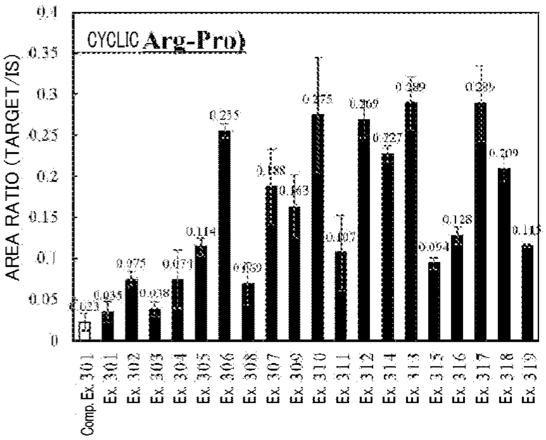


24 CYCLIC(Glu-Tyr)

| | Ave. | SD |
|---|---|---|
| Comp. Ex.301 | 0.027 | 0.016 |
| Ex. 301 | 0.110 | 0.014 |
| Ex. 302 | 0.065 | 0.013 |
| Ex. 303 | 0.135 | 0.001 |
| Ex. 304 | 0.087 | 0.020 |
| Ex. 305 | 0.307 | 0.047 |
| Ex. 306 | 0.212 | 0.027 |
| Ex. 308 | 0.290 | 0.013 |
| Ex. 307 | 0.208 | 0.003 |
| Ex. 309 | 0.363 | 0.041 |
| Ex. 310 | 0.352 | 0.044 |
| Ex. 311 | 0.297 | 0.003 |
| Ex. 312 | 0.168 | 0.005 |
| Ex. 314 | 0.380 | 0.042 |
| Ex. 313 | 0.341 | 0.056 |
| Ex. 315 | 0.125 | 0.005 |
| Ex. 316 | 0.254 | 0.007 |
| Ex. 317 | 0.411 | 0.003 |
| Ex. 318 | 0.474 | 0.013 |
| Ex. 319 | 0.268 | 0.004 |

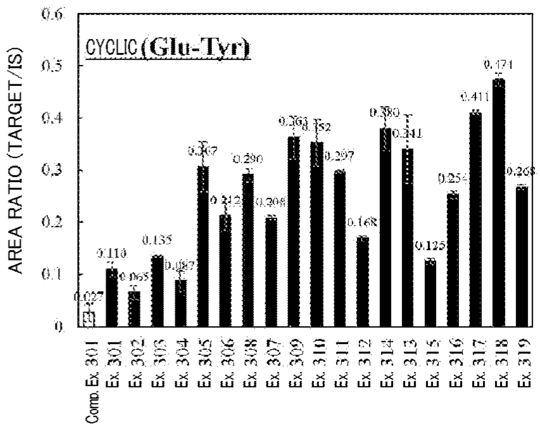


FIG. 21

**25** CYCLIC(Pro-His)

| | Ave. | SD |
|---|---|---|
| Comp. Ex. 301 | 0.015 | 0.003 |
| Ex. 301 | 0.022 | 0.000 |
| Ex. 302 | 0.050 | 0.007 |
| Ex. 303 | 0.033 | 0.009 |
| Ex. 304 | 0.068 | 0.008 |
| Ex. 305 | 0.101 | 0.007 |
| Ex. 306 | 0.277 | 0.025 |
| Ex. 308 | 0.159 | 0.003 |
| Ex. 307 | 0.182 | 0.005 |
| Ex. 309 | 0.181 | 0.021 |
| Ex. 310 | 0.275 | 0.045 |
| Ex. 311 | 0.113 | 0.047 |
| Ex. 312 | 0.150 | 0.001 |
| Ex. 314 | 0.185 | 0.017 |
| Ex. 313 | 0.347 | 0.022 |
| Ex. 315 | 0.295 | 0.015 |
| Ex. 316 | 0.095 | 0.014 |
| Ex. 317 | 0.109 | 0.009 |
| Ex. 318 | 0.084 | 0.007 |
| Ex. 319 | 0.084 | 0.011 |

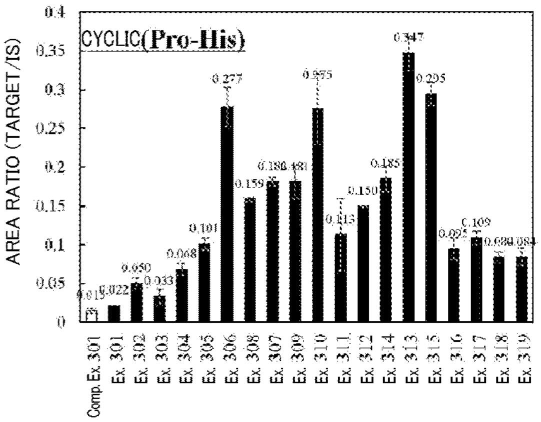


**26** CYCLIC(Glu-Asp)

| | Ave. | SD |
|---|---|---|
| Comp. Ex. 301 | 0.067 | 0.001 |
| Ex. 301 | 0.091 | 0.002 |
| Ex. 302 | 0.091 | 0.008 |
| Ex. 303 | 0.096 | 0.006 |
| Ex. 304 | 0.095 | 0.000 |
| Ex. 305 | 0.130 | 0.015 |
| Ex. 306 | 0.125 | 0.007 |
| Ex. 308 | 0.126 | 0.001 |
| Ex. 307 | 0.134 | 0.001 |
| Ex. 309 | 0.147 | 0.002 |
| Ex. 310 | 0.178 | 0.017 |
| Ex. 311 | 0.125 | 0.005 |
| Ex. 312 | 0.129 | 0.003 |
| Ex. 314 | 0.154 | 0.014 |
| Ex. 313 | 0.193 | 0.003 |
| Ex. 315 | 0.097 | 0.004 |
| Ex. 316 | 0.147 | 0.020 |
| Ex. 317 | 0.171 | 0.000 |
| Ex. 318 | 0.127 | 0.013 |
| Ex. 319 | 0.121 | 0.012 |

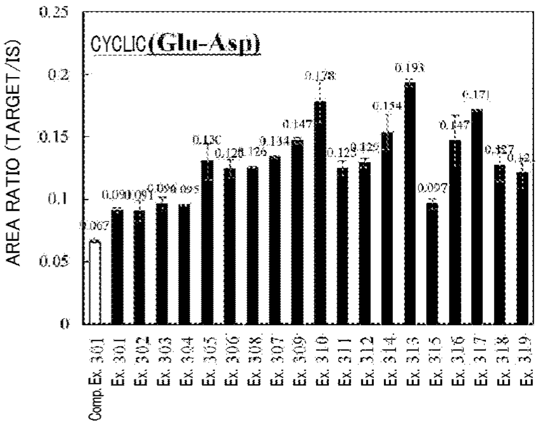


**27** CYCLIC(Gly-Arg)

| | Ave. | SD |
|---|---|---|
| Comp. Ex. 301 | 0.406 | 0.043 |
| Ex. 301 | 1.222 | 0.128 |
| Ex. 302 | 1.753 | 0.235 |
| Ex. 303 | 1.499 | 0.188 |
| Ex. 304 | 1.996 | 0.159 |
| Ex. 305 | 2.244 | 0.185 |
| Ex. 306 | 2.543 | 0.109 |
| Ex. 308 | 2.251 | 0.082 |
| Ex. 307 | 2.481 | 0.055 |
| Ex. 309 | 2.389 | 0.262 |
| Ex. 310 | 2.467 | 0.310 |
| Ex. 311 | 2.396 | 0.127 |
| Ex. 312 | 2.721 | 0.040 |
| Ex. 314 | 2.702 | 0.297 |
| Ex. 313 | 2.745 | 0.087 |
| Ex. 315 | 0.713 | 0.003 |
| Ex. 316 | 1.066 | 0.005 |
| Ex. 317 | 1.229 | 0.138 |
| Ex. 318 | 2.032 | 0.174 |
| Ex. 319 | 2.420 | 0.185 |

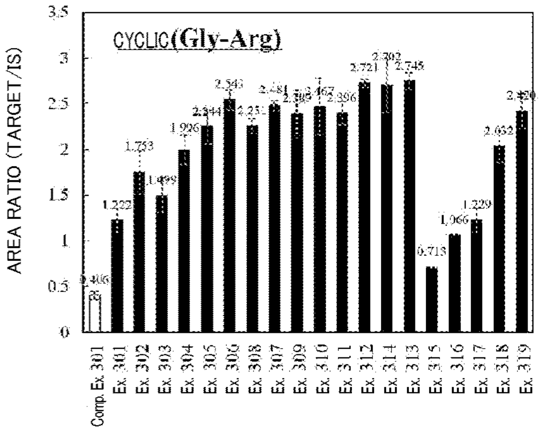


FIG. 22

**28** CYCLIC(Gly-His)

| | Ave. | SD |
|---|---|---|
| Comp. Ex.301 | 0.003 | 0.001 |
| Ex. 301 | 0.011 | 0.003 |
| Ex. 302 | 0.015 | 0.003 |
| Ex. 303 | 0.013 | 0.002 |
| Ex. 304 | 0.019 | 0.001 |
| Ex. 305 | 0.027 | 0.003 |
| Ex. 306 | 0.028 | 0.010 |
| Ex. 308 | 0.018 | 0.001 |
| Ex. 307 | 0.032 | 0.004 |
| Ex. 309 | 0.031 | 0.009 |
| Ex. 310 | 0.043 | 0.009 |
| Ex. 311 | 0.022 | 0.003 |
| Ex. 312 | 0.034 | 0.000 |
| Ex. 314 | 0.037 | 0.003 |
| Ex. 313 | 0.053 | 0.011 |
| Ex. 315 | 0.009 | 0.002 |
| Ex. 316 | 0.026 | 0.001 |
| Ex. 317 | 0.037 | 0.002 |
| Ex. 318 | 0.031 | 0.003 |
| Ex. 319 | 0.025 | 0.001 |

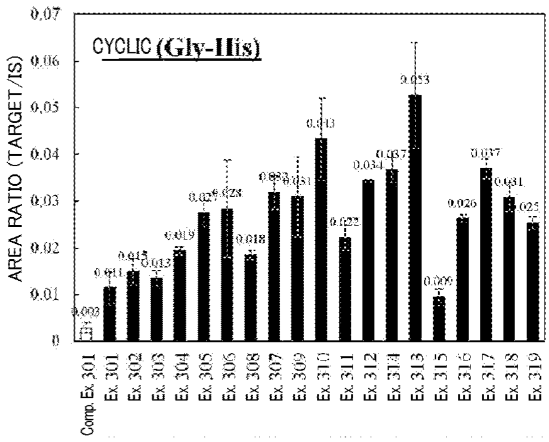


**29** CYCLIC(Leu/Ile-Leu/Ile)

| | Ave. | SD |
|---|---|---|
| Comp. Ex.301 | 0.050 | 0.005 |
| Ex. 301 | 0.086 | 0.013 |
| Ex. 302 | 0.090 | 0.009 |
| Ex. 303 | 0.102 | 0.014 |
| Ex. 304 | 0.108 | 0.012 |
| Ex. 305 | 0.147 | 0.010 |
| Ex. 306 | 0.160 | 0.007 |
| Ex. 308 | 0.146 | 0.002 |
| Ex. 307 | 0.169 | 0.005 |
| Ex. 309 | 0.165 | 0.019 |
| Ex. 310 | 0.196 | 0.031 |
| Ex. 311 | 0.140 | 0.005 |
| Ex. 312 | 0.167 | 0.008 |
| Ex. 314 | 0.180 | 0.009 |
| Ex. 313 | 0.233 | 0.008 |
| Ex. 315 | 0.119 | 0.018 |
| Ex. 316 | 0.192 | 0.023 |
| Ex. 317 | 0.163 | 0.006 |
| Ex. 318 | 0.168 | 0.011 |
| Ex. 319 | 0.255 | 0.006 |

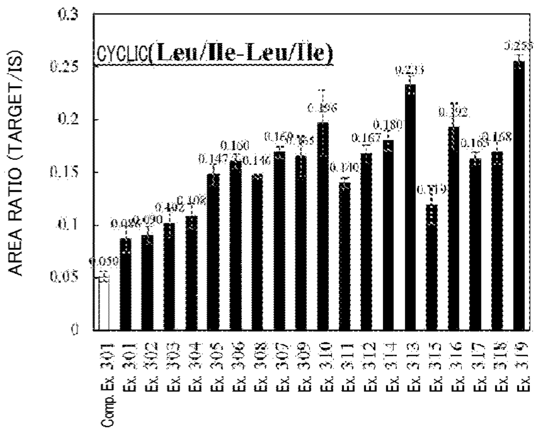


**30** CYCLIC(Glu-Glu)

| | Ave. | SD |
|---|---|---|
| Comp. Ex.301 | n.d. | |
| Ex. 301 | 0.025 | 0.005 |
| Ex. 302 | 0.024 | 0.001 |
| Ex. 303 | 0.025 | 0.005 |
| Ex. 304 | 0.022 | 0.001 |
| Ex. 305 | 0.038 | 0.009 |
| Ex. 306 | 0.034 | 0.000 |
| Ex. 308 | 0.039 | 0.000 |
| Ex. 307 | 0.036 | 0.006 |
| Ex. 309 | 0.037 | 0.004 |
| Ex. 310 | 0.035 | 0.000 |
| Ex. 311 | 0.052 | 0.002 |
| Ex. 312 | 0.034 | 0.001 |
| Ex. 314 | 0.049 | 0.001 |
| Ex. 313 | 0.042 | 0.007 |
| Ex. 315 | 0.029 | 0.006 |
| Ex. 316 | 0.034 | 0.011 |
| Ex. 317 | 0.050 | 0.010 |
| Ex. 318 | 0.042 | 0.002 |
| Ex. 319 | 0.016 | 0.002 |

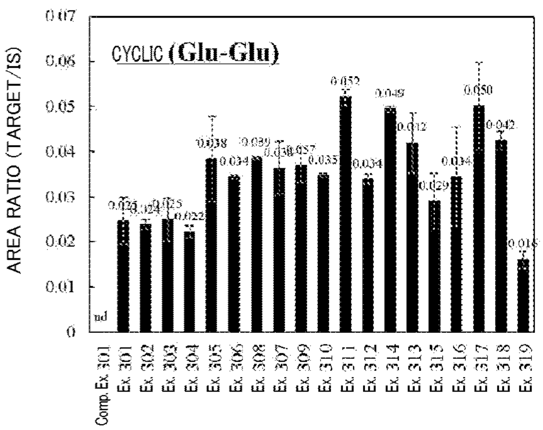


FIG. 23

31 CYCLIC(Leu/Ile-Asp)

| | Ave. | SD |
|---|---|---|
| Comp. Ex.301 | 0.007 | 0.000 |
| Ex. 301 | 0.016 | 0.001 |
| Ex. 302 | 0.022 | 0.002 |
| Ex. 303 | 0.015 | 0.001 |
| Ex. 304 | 0.028 | 0.001 |
| Ex. 305 | 0.039 | 0.000 |
| Ex. 306 | 0.054 | 0.000 |
| Ex. 308 | 0.055 | 0.001 |
| Ex. 307 | 0.038 | 0.002 |
| Ex. 309 | 0.050 | 0.001 |
| Ex. 310 | 0.063 | 0.003 |
| Ex. 311 | 0.037 | 0.003 |
| Ex. 312 | 0.052 | 0.000 |
| Ex. 314 | 0.057 | 0.006 |
| Ex. 313 | 0.081 | 0.003 |
| Ex. 315 | 0.018 | 0.001 |
| Ex. 316 | 0.021 | 0.001 |
| Ex. 317 | 0.037 | 0.015 |
| Ex. 318 | 0.023 | 0.005 |
| Ex. 319 | 0.034 | 0.003 |

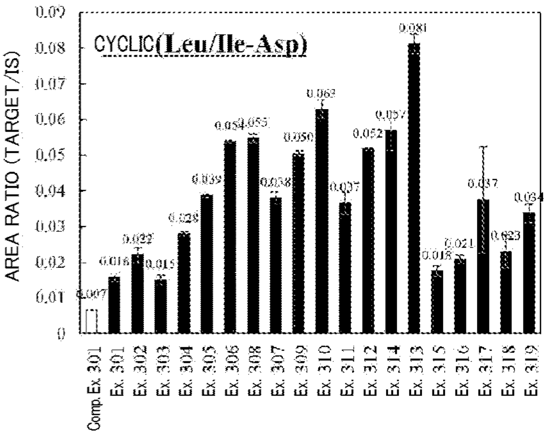


32 CYCLIC(Phe-Asp)

| | Ave. | SD |
|---|---|---|
| Comp. Ex.301 | 0.010 | 0.000 |
| Ex. 301 | 0.016 | 0.000 |
| Ex. 302 | 0.020 | 0.002 |
| Ex. 303 | 0.016 | 0.001 |
| Ex. 304 | 0.021 | 0.002 |
| Ex. 305 | 0.026 | 0.003 |
| Ex. 306 | 0.028 | 0.005 |
| Ex. 308 | 0.028 | 0.001 |
| Ex. 307 | 0.031 | 0.002 |
| Ex. 309 | 0.030 | 0.001 |
| Ex. 310 | 0.033 | 0.002 |
| Ex. 311 | 0.029 | 0.004 |
| Ex. 312 | 0.030 | 0.004 |
| Ex. 314 | 0.035 | 0.000 |
| Ex. 313 | 0.041 | 0.005 |
| Ex. 315 | 0.020 | 0.005 |
| Ex. 316 | 0.027 | 0.002 |
| Ex. 317 | 0.059 | 0.007 |
| Ex. 318 | 0.050 | 0.001 |
| Ex. 319 | 0.023 | 0.001 |

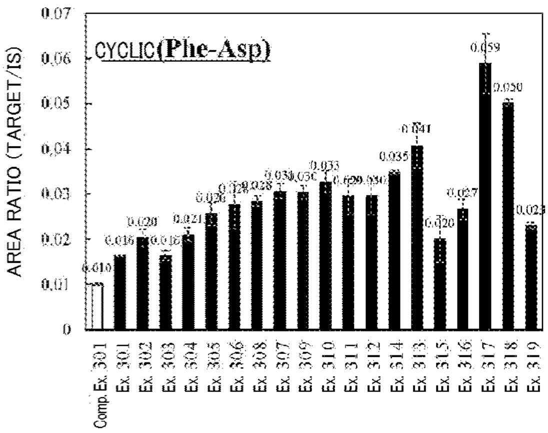


FIG. 24

SULFUROL

| | Ave. | SD |
|---|---|---|
| Comp. Ex.301 | 0.156 | 0.015 |
| Ex. 301 | 0.365 | 0.046 |
| Ex. 302 | 0.398 | 0.001 |
| Ex. 303 | 0.388 | 0.062 |
| Ex. 304 | 0.365 | 0.041 |
| Ex. 305 | 0.527 | 0.056 |
| Ex. 306 | 0.461 | 0.039 |
| Ex. 308 | 0.470 | 0.002 |
| Ex. 307 | 0.554 | 0.006 |
| Ex. 309 | 0.436 | 0.039 |
| Ex. 310 | 0.439 | 0.077 |
| Ex. 311 | 0.538 | 0.006 |
| Ex. 312 | 0.493 | 0.008 |
| Ex. 314 | 0.581 | 0.023 |
| Ex. 313 | 0.520 | 0.010 |
| Ex. 315 | 0.346 | 0.014 |
| Ex. 316 | 0.565 | 0.009 |
| Ex. 317 | 0.919 | 0.051 |
| Ex. 318 | 0.837 | 0.086 |
| Ex. 319 | 0.444 | 0.016 |

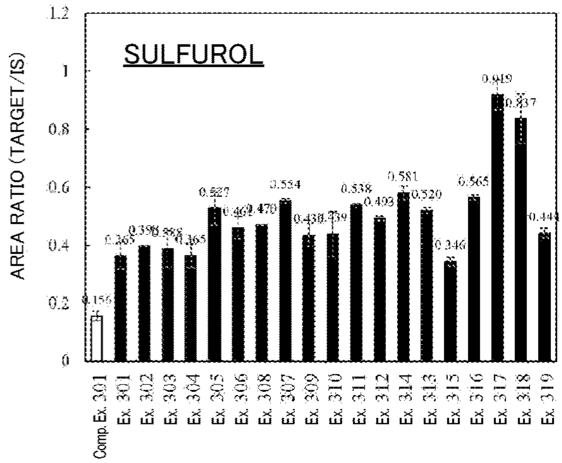


SULFUROL ACETATE

| | Ave. | SD |
|---|---|---|
| Comp. Ex.301 | 0.015 | 0.003 |
| Ex. 301 | 0.042 | 0.006 |
| Ex. 302 | 0.034 | 0.002 |
| Ex. 303 | 0.058 | 0.008 |
| Ex. 304 | 0.045 | 0.005 |
| Ex. 305 | 0.127 | 0.009 |
| Ex. 306 | 0.100 | 0.001 |
| Ex. 308 | 0.151 | 0.001 |
| Ex. 307 | 0.117 | 0.005 |
| Ex. 309 | 0.162 | 0.009 |
| Ex. 310 | 0.118 | 0.003 |
| Ex. 311 | 0.109 | 0.009 |
| Ex. 312 | 0.088 | 0.004 |
| Ex. 314 | 0.151 | 0.001 |
| Ex. 313 | 0.136 | 0.007 |
| Ex. 315 | 0.031 | 0.001 |
| Ex. 316 | 0.085 | 0.002 |
| Ex. 317 | 0.163 | 0.042 |
| Ex. 318 | 0.170 | 0.004 |
| Ex. 319 | 0.132 | 0.010 |

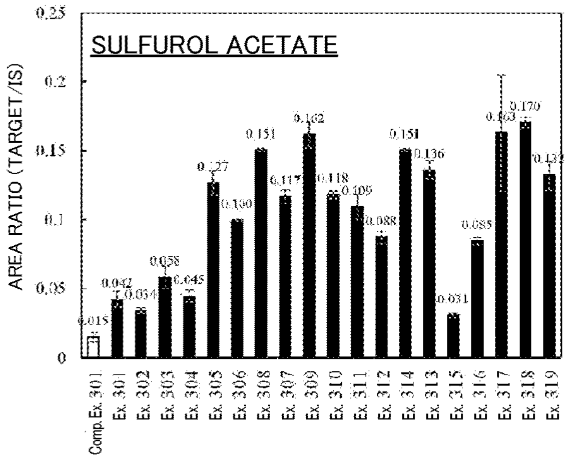


cis-ACONITIC ACID

| | Ave. | SD |
|---|---|---|
| Comp. Ex.301 | 0.157 | 0.005 |
| Ex. 301 | 0.208 | 0.015 |
| Ex. 302 | 0.199 | 0.003 |
| Ex. 303 | 0.223 | 0.006 |
| Ex. 304 | 0.229 | 0.002 |
| Ex. 305 | 0.378 | 0.031 |
| Ex. 306 | 0.346 | 0.033 |
| Ex. 308 | 0.369 | 0.015 |
| Ex. 307 | 0.389 | 0.002 |
| Ex. 309 | 0.389 | 0.032 |
| Ex. 310 | 0.347 | 0.025 |
| Ex. 311 | 0.329 | 0.004 |
| Ex. 312 | 0.282 | 0.002 |
| Ex. 314 | 0.380 | 0.007 |
| Ex. 313 | 0.344 | 0.032 |
| Ex. 315 | 0.192 | 0.020 |
| Ex. 316 | 0.313 | 0.015 |
| Ex. 317 | 0.343 | 0.013 |
| Ex. 318 | 0.399 | 0.012 |
| Ex. 319 | 0.242 | 0.002 |

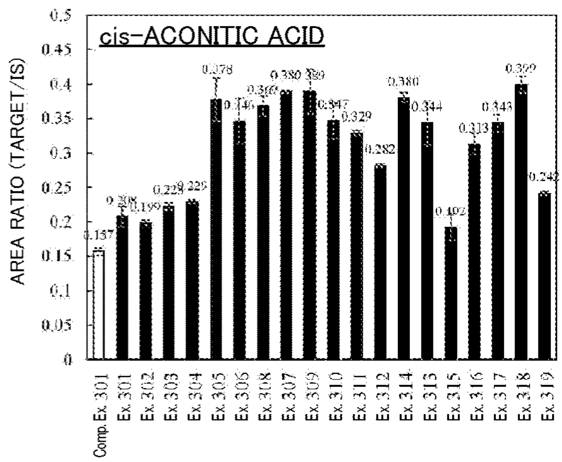


FIG. 25

trans-ACONITIC ACID

| | Ave. | SD |
|---|---|---|
| Comp. Ex.301 | 0.238 | 0.004 |
| Ex. 301 | 0.326 | 0.030 |
| Ex. 302 | 0.308 | 0.046 |
| Ex. 303 | 0.343 | 0.071 |
| Ex. 304 | 0.383 | 0.035 |
| Ex. 305 | 0.626 | 0.011 |
| Ex. 306 | 0.594 | 0.028 |
| Ex. 308 | 0.640 | 0.056 |
| Ex. 307 | 0.562 | 0.064 |
| Ex. 309 | 0.591 | 0.032 |
| Ex. 310 | 0.569 | 0.031 |
| Ex. 311 | 0.601 | 0.008 |
| Ex. 312 | 0.551 | 0.002 |
| Ex. 314 | 0.585 | 0.015 |
| Ex. 313 | 0.584 | 0.038 |
| Ex. 315 | 0.705 | 0.089 |
| Ex. 316 | 0.723 | 0.059 |
| Ex. 317 | 1.177 | 0.078 |
| Ex. 318 | 1.190 | 0.029 |
| Ex. 319 | 0.866 | 0.179 |

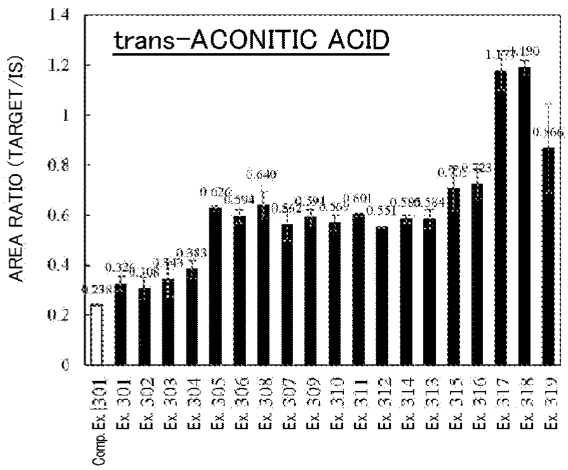


TARTARIC ACID

| | Ave. | SD |
|---|---|---|
| Comp. Ex.301 | 0.543 | 0.004 |
| Ex. 301 | 0.662 | 0.063 |
| Ex. 302 | 0.734 | 0.068 |
| Ex. 303 | 0.663 | 0.051 |
| Ex. 304 | 0.781 | 0.053 |
| Ex. 305 | 0.785 | 0.017 |
| Ex. 306 | 0.871 | 0.064 |
| Ex. 308 | 0.971 | 0.024 |
| Ex. 307 | 0.822 | 0.003 |
| Ex. 309 | 0.714 | 0.054 |
| Ex. 310 | 0.787 | 0.027 |
| Ex. 311 | 0.746 | 0.006 |
| Ex. 312 | 0.930 | 0.021 |
| Ex. 314 | 0.746 | 0.051 |
| Ex. 313 | 0.828 | 0.044 |
| Ex. 315 | 1.242 | 0.019 |
| Ex. 316 | 1.172 | 0.018 |
| Ex. 317 | 0.994 | 0.018 |
| Ex. 318 | 0.847 | 0.007 |
| Ex. 319 | 0.601 | 0.004 |

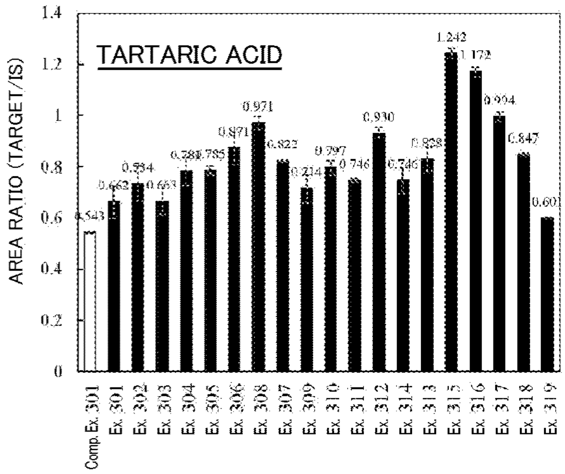


MALIC ACID

| | Ave. | SD |
|---|---|---|
| Comp. Ex.301 | 20.45 | 0.295 |
| Ex. 301 | 23.19 | 1.043 |
| Ex. 302 | 22.37 | 0.136 |
| Ex. 303 | 24.57 | 1.314 |
| Ex. 304 | 22.85 | 0.473 |
| Ex. 305 | 24.86 | 1.553 |
| Ex. 306 | 25.15 | 1.592 |
| Ex. 308 | 25.06 | 1.830 |
| Ex. 307 | 26.02 | 1.543 |
| Ex. 309 | 23.90 | 0.279 |
| Ex. 310 | 22.78 | 0.264 |
| Ex. 311 | 22.32 | 0.015 |
| Ex. 312 | 22.29 | 1.651 |
| Ex. 314 | 22.66 | 1.584 |
| Ex. 313 | 23.41 | 0.934 |
| Ex. 315 | 28.02 | 0.651 |
| Ex. 316 | 30.23 | 0.327 |
| Ex. 317 | 34.64 | 2.051 |
| Ex. 318 | 36.15 | 0.828 |
| Ex. 319 | 34.83 | 0.264 |

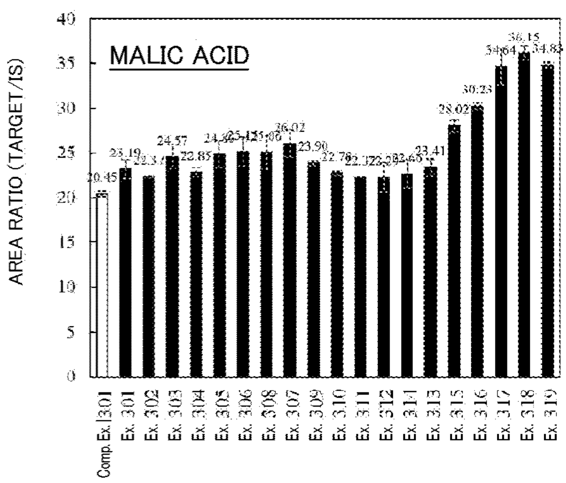


FIG. 26

CITRIC ACID

| | Ave. | SD |
|---|---|---|
| Comp. Ex. 301 | 33.75 | 0.587 |
| Ex. 301 | 41.82 | 4.388 |
| Ex. 302 | 40.91 | 2.871 |
| Ex. 303 | 43.54 | 2.642 |
| Ex. 304 | 42.72 | 2.059 |
| Ex. 305 | 45.95 | 0.069 |
| Ex. 306 | 46.87 | 2.583 |
| Ex. 308 | 46.63 | 3.082 |
| Ex. 307 | 47.50 | 2.814 |
| Ex. 309 | 41.65 | 3.430 |
| Ex. 310 | 39.86 | 1.183 |
| Ex. 311 | 46.27 | 0.394 |
| Ex. 312 | 41.89 | 0.135 |
| Ex. 314 | 44.96 | 1.893 |
| Ex. 313 | 45.13 | 2.699 |
| Ex. 315 | 59.36 | 5.421 |
| Ex. 316 | 57.09 | 1.440 |
| Ex. 317 | 52.32 | 1.535 |
| Ex. 318 | 56.01 | 2.391 |
| Ex. 319 | 39.96 | 0.146 |

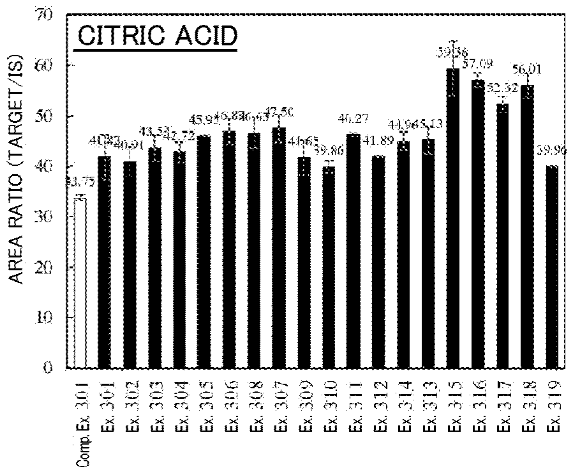


FIG. 27

CARVEOL

| | Ave. | SD |
|---|---|---|
| Comp. Ex. 301 | 0.04 | 0.001 |
| Ex. 301 | 0.08 | 0.007 |
| Ex. 302 | 0.07 | 0.002 |
| Ex. 303 | 0.08 | 0.004 |
| Ex. 304 | 0.09 | 0.009 |
| Ex. 305 | 0.13 | 0.003 |
| Ex. 306 | 0.13 | 0.000 |
| Ex. 308 | 0.14 | 0.006 |
| Ex. 307 | 0.12 | 0.004 |
| Ex. 309 | 0.13 | 0.014 |
| Ex. 310 | 0.13 | 0.004 |
| Ex. 311 | 0.11 | 0.003 |
| Ex. 312 | 0.12 | 0.008 |
| Ex. 314 | 0.11 | 0.010 |
| Ex. 313 | 0.12 | 0.001 |
| Ex. 315 | 0.15 | 0.005 |
| Ex. 316 | 0.16 | 0.001 |
| Ex. 317 | 0.17 | 0.000 |
| Ex. 318 | 0.18 | 0.003 |
| Ex. 319 | 0.07 | 0.001 |

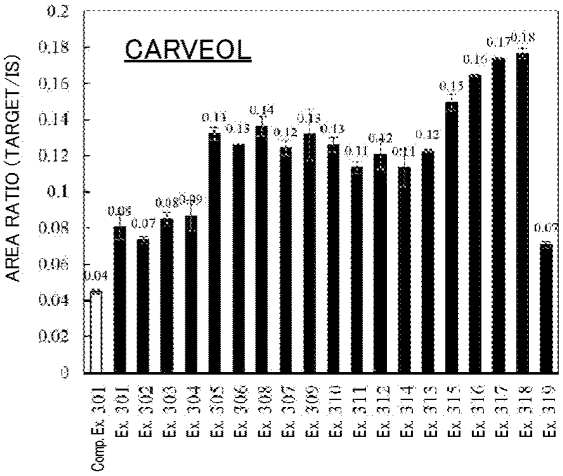


NERORIDOL

| | Ave. | SD |
|---|---|---|
| Comp. Ex. 301 | 0.17 | 0.005 |
| Ex. 301 | 0.22 | 0.011 |
| Ex. 302 | 0.24 | 0.006 |
| Ex. 303 | 0.23 | 0.016 |
| Ex. 304 | 0.26 | 0.024 |
| Ex. 305 | 0.28 | 0.011 |
| Ex. 306 | 0.27 | 0.003 |
| Ex. 308 | 0.29 | 0.008 |
| Ex. 307 | 0.30 | 0.015 |
| Ex. 309 | 0.30 | 0.004 |
| Ex. 310 | 0.34 | 0.018 |
| Ex. 311 | 0.26 | 0.005 |
| Ex. 312 | 0.29 | 0.013 |
| Ex. 314 | 0.25 | 0.028 |
| Ex. 313 | 0.29 | 0.013 |
| Ex. 315 | 0.28 | 0.008 |
| Ex. 316 | 0.27 | 0.003 |
| Ex. 317 | 0.29 | 0.017 |
| Ex. 318 | 0.30 | 0.001 |
| Ex. 319 | 0.19 | 0.011 |

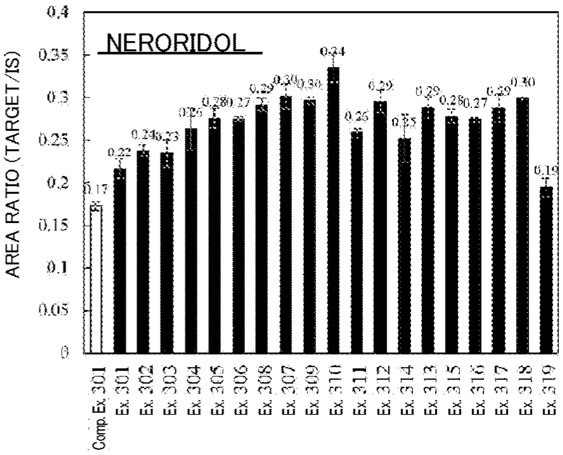


α-TERPINENE-7-AL

| | Ave. | SD |
|---|---|---|
| Comp. Ex. 301 | 46.67 | 0.320 |
| Ex. 301 | 122.21 | 4.523 |
| Ex. 302 | 139.44 | 5.484 |
| Ex. 303 | 135.75 | 6.783 |
| Ex. 304 | 180.40 | 11.146 |
| Ex. 305 | 233.71 | 5.893 |
| Ex. 306 | 236.31 | 3.184 |
| Ex. 308 | 252.92 | 10.277 |
| Ex. 307 | 245.65 | 2.058 |
| Ex. 309 | 248.05 | 0.130 |
| Ex. 310 | 259.04 | 9.127 |
| Ex. 311 | 215.63 | 8.490 |
| Ex. 312 | 238.58 | 1.158 |
| Ex. 314 | 250.27 | 11.962 |
| Ex. 313 | 251.82 | 5.663 |
| Ex. 315 | 137.21 | 0.648 |
| Ex. 316 | 278.68 | 1.237 |
| Ex. 317 | 262.27 | 2.604 |
| Ex. 318 | 235.41 | 6.403 |
| Ex. 319 | 153.43 | 1.843 |

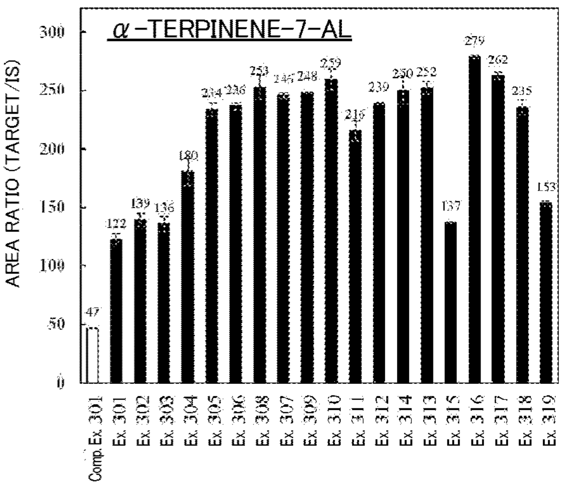


FIG. 28

1 CYCLIC(Gly-Thr)

| | Ave. | SD |
|---|---|---|
| Comp. Ex. 401 | 0.026 | 0.001 |
| Ex. 401 | 0.032 | 0.003 |
| Ex. 402 | 0.035 | 0.001 |
| Ex. 403 | 0.071 | 0.009 |
| Ex. 404 | 0.066 | 0.004 |

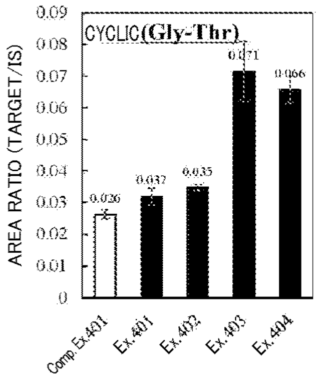


2 CYCLIC(Gly-His)

| | Ave. | SD |
|---|---|---|
| Comp. Ex. 401 | 0.002 | 0.000 |
| Ex. 401 | 0.005 | 0.000 |
| Ex. 402 | 0.006 | 0.000 |
| Ex. 403 | 0.006 | 0.001 |
| Ex. 404 | 0.004 | 0.001 |

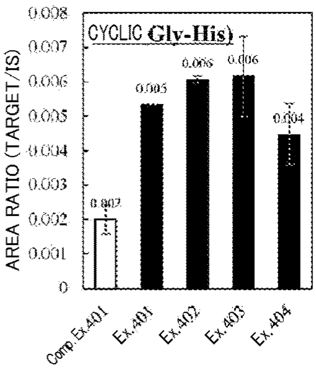


3 CYCLIC(Ala-Asp)

| | Ave. | SD |
|---|---|---|
| Comp. Ex. 401 | 0.021 | 0.002 |
| Ex. 401 | 0.023 | 0.002 |
| Ex. 402 | 0.031 | 0.002 |
| Ex. 403 | 0.048 | 0.001 |
| Ex. 404 | 0.033 | 0.002 |

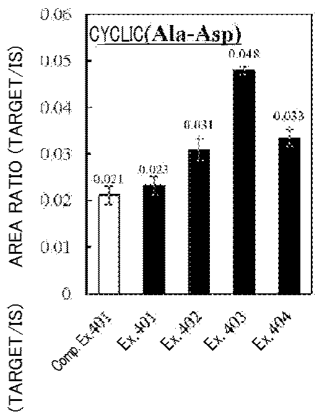


4 CYCLIC(Val-Ser)

| | Ave. | SD |
|---|---|---|
| Comp. Ex. 401 | nd | |
| Ex. 401 | 0.077 | 0.005 |
| Ex. 402 | 0.177 | 0.020 |
| Ex. 403 | 0.178 | 0.025 |
| Ex. 404 | 0.193 | 0.036 |

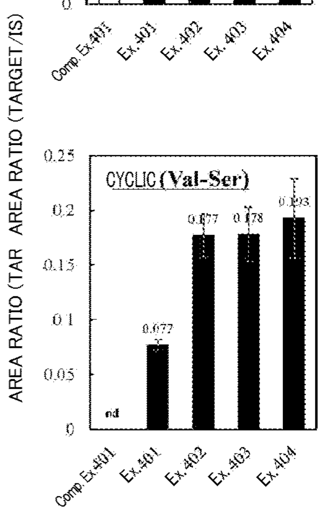


FIG. 29

5 CYCLIC (Gly-Arg)

| | Ave. | SD |
|---|---|---|
| Comp. Ex. 401 | 0.012 | 0.001 |
| Ex. 401 | 0.026 | 0.002 |
| Ex. 402 | 0.079 | 0.000 |
| Ex. 403 | 0.114 | 0.001 |
| Ex. 404 | 0.121 | 0.000 |

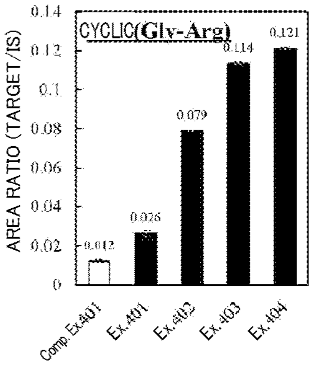


6 CYCLIC (Ala-His)

| | Ave. | SD |
|---|---|---|
| Comp. Ex. 401 | 0.013 | 0.000 |
| Ex. 401 | 0.007 | 0.002 |
| Ex. 402 | 0.022 | 0.003 |
| Ex. 403 | 0.023 | 0.001 |
| Ex. 404 | 0.022 | 0.005 |

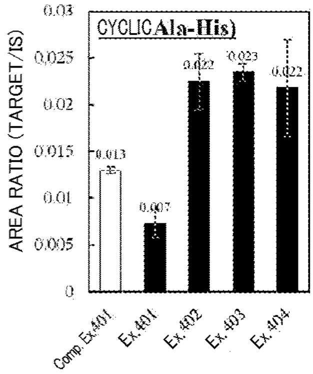


7 CYCLIC (Glu-Asp)

| | Ave. | SD |
|---|---|---|
| Comp. Ex. 401 | 0.006 | 0.001 |
| Ex. 401 | 0.012 | 0.003 |
| Ex. 402 | 0.023 | 0.000 |
| Ex. 403 | 0.019 | 0.001 |
| Ex. 404 | 0.014 | 0.001 |

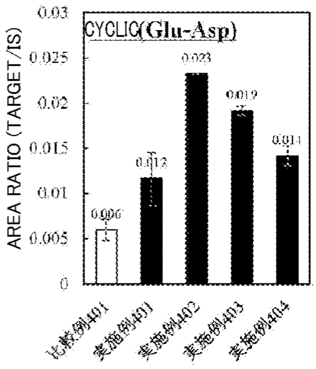


8 CYCLIC (Phe-Phe)

| | Ave. | SD |
|---|---|---|
| Comp. Ex. 401 | 0.022 | 0.006 |
| Ex. 401 | 0.073 | 0.008 |
| Ex. 402 | 0.081 | 0.010 |
| Ex. 403 | 0.157 | 0.034 |
| Ex. 404 | 0.124 | 0.015 |

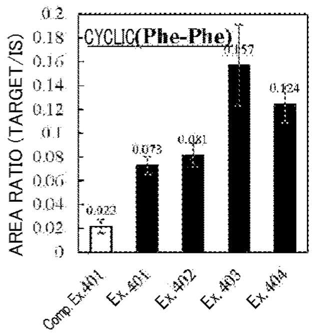


FIG. 30

**9** CYCLIC(Leu/Ile-Leu/Ile)

| | Ave. | SD |
|---|---|---|
| Comp. Ex. 401 | 0.008 | 0.004 |
| Ex. 401 | 0.044 | 0.001 |
| Ex. 402 | 0.041 | 0.005 |
| Ex. 403 | 0.054 | 0.008 |
| Ex. 404 | 0.049 | 0.016 |

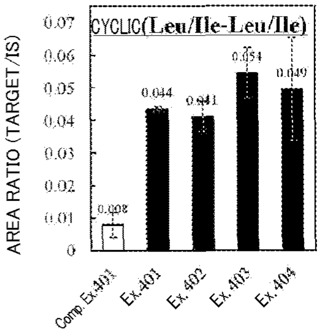


**10** CYCLIC(Ala-Arg)

| | Ave. | SD |
|---|---|---|
| Comp. Ex. 401 | 0.008 | 0.002 |
| Ex. 401 | 0.038 | 0.003 |
| Ex. 402 | 0.067 | 0.000 |
| Ex. 403 | 0.083 | 0.002 |
| Ex. 404 | 0.075 | 0.002 |

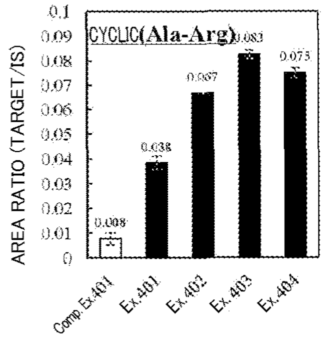


**11** CYCLIC(Leu/Ile-Val)

| | Ave. | SD |
|---|---|---|
| Comp. Ex. 401 | 0.014 | 0.004 |
| Ex. 401 | 0.030 | 0.003 |
| Ex. 402 | 0.026 | 0.000 |
| Ex. 403 | 0.028 | 0.006 |
| Ex. 404 | 0.027 | 0.001 |

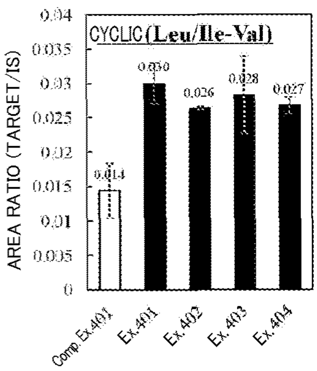


**12** CYCLIC(Phe-Pro)

| | Ave. | SD |
|---|---|---|
| Comp. Ex. 401 | 0.009 | 0.002 |
| Ex. 401 | 0.031 | 0.004 |
| Ex. 402 | 0.026 | 0.008 |
| Ex. 403 | 0.041 | 0.004 |
| Ex. 404 | 0.043 | 0.000 |

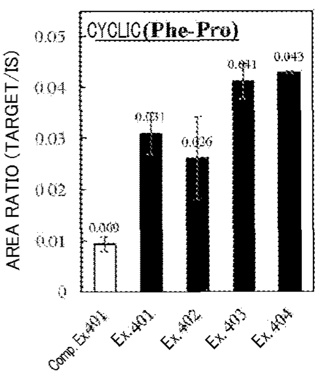


FIG. 31

13 CYCLIC (Phe-Leu/Ile)

| | Ave. | SD |
|---|---|---|
| Comp. Ex. 401 | 0.024 | 0.001 |
| Ex. 401 | 0.057 | 0.005 |
| Ex. 402 | 0.054 | 0.001 |
| Ex. 403 | 0.067 | 0.001 |
| Ex. 404 | 0.064 | 0.001 |

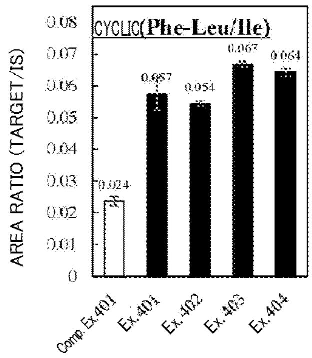


14 CYCLIC (Pro-Pro)

| | Ave. | SD |
|---|---|---|
| Comp. Ex. 401 | nd | |
| Ex. 401 | 0.044 | 0.000 |
| Ex. 402 | 0.023 | 0.006 |
| Ex. 403 | 0.051 | 0.001 |
| Ex. 404 | 0.081 | 0.000 |

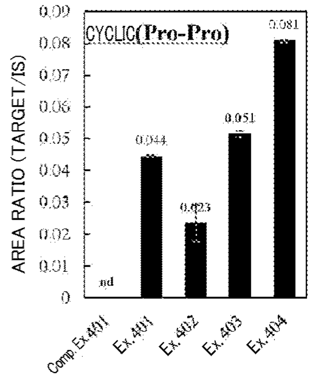


15 CYCLIC (Glu-Phe)

| | Ave. | SD |
|---|---|---|
| Comp. Ex. 401 | 0.022 | 0.014 |
| Ex. 401 | 0.135 | 0.011 |
| Ex. 402 | 0.137 | 0.021 |
| Ex. 403 | 0.277 | 0.023 |
| Ex. 404 | 0.261 | 0.030 |

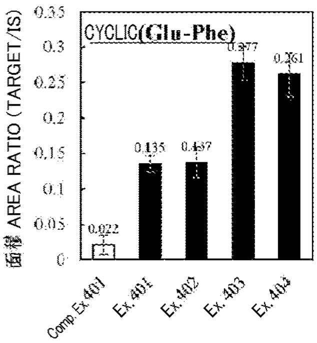


16 CYCLIC (Arg-Leu/Ile)

| | Ave. | SD |
|---|---|---|
| Comp. Ex. 401 | 0.023 | 0.016 |
| Ex. 401 | 0.069 | 0.007 |
| Ex. 402 | 0.055 | 0.004 |
| Ex. 403 | 0.073 | 0.002 |
| Ex. 404 | 0.090 | 0.003 |

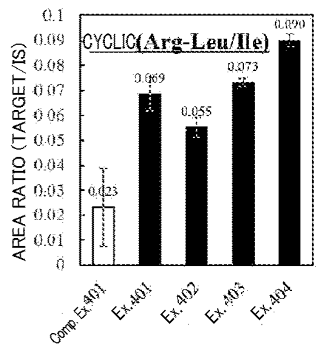


FIG. 32

**17** CYCLIC (Pro-Leu/Ile)

| | Ave. | SD |
|---|---|---|
| Comp. Ex. 401 | 0. 097 | 0. 002 |
| Ex. 401 | 0. 116 | 0. 001 |
| Ex. 402 | 0. 094 | 0. 011 |
| Ex. 403 | 0. 135 | 0. 004 |
| Ex. 404 | 0. 123 | 0. 004 |

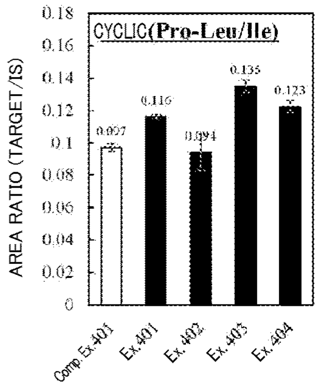


**18** CYCLIC (Pro-Glu)

| | Ave. | SD |
|---|---|---|
| Comp. Ex. 401 | nd | |
| Ex. 401 | 0. 009 | 0. 003 |
| Ex. 402 | 0. 008 | 0. 001 |
| Ex. 403 | 0. 032 | 0. 003 |
| Ex. 404 | 0. 034 | 0. 001 |

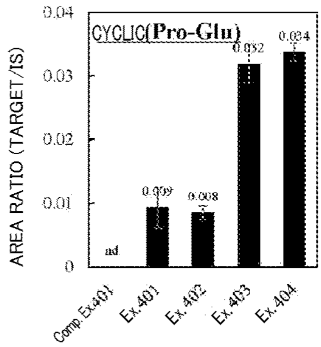


**19** CYCLIC (Glu-Leu/Ile)

| | Ave. | SD |
|---|---|---|
| Comp. Ex. 401 | 0. 010 | 0. 001 |
| Ex. 401 | 0. 018 | 0. 001 |
| Ex. 402 | 0. 029 | 0. 004 |
| Ex. 403 | 0. 029 | 0. 005 |
| Ex. 404 | 0. 030 | 0. 003 |

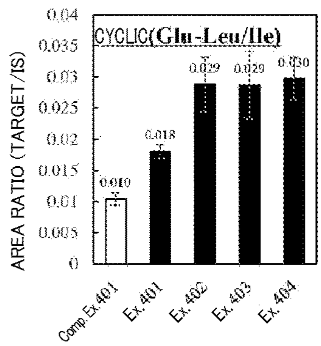


**20** CYCLIC (Ala-Pro)

| | Ave. | SD |
|---|---|---|
| Comp. Ex. 401 | 0. 028 | 0. 001 |
| Ex. 401 | 0. 029 | 0. 001 |
| Ex. 402 | 0. 052 | 0. 001 |
| Ex. 403 | 0. 057 | 0. 001 |
| Ex. 404 | 0. 041 | 0. 001 |

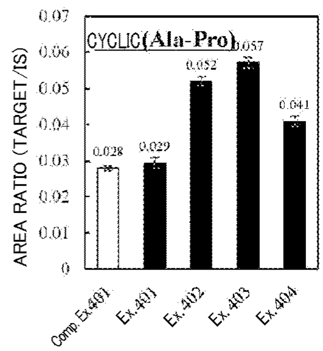


FIG. 33

**21** CYCLIC(Pro-Val)

| | Ave. | SD |
|---|---|---|
| Comp. Ex. 401 | 0. 009 | 0. 005 |
| Ex. 401 | 0. 037 | 0. 003 |
| Ex. 402 | 0. 066 | 0. 005 |
| Ex. 403 | 0. 072 | 0. 001 |
| Ex. 404 | 0. 047 | 0. 007 |

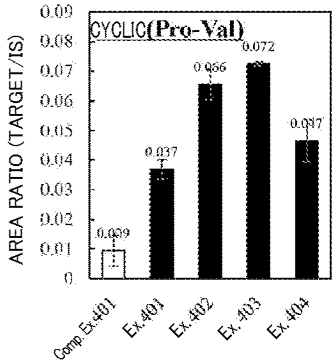


**22** CYCLIC(Pro-His)

| | Ave. | SD |
|---|---|---|
| Comp. Ex. 401 | 0. 016 | 0. 007 |
| Ex. 401 | 0. 100 | 0. 009 |
| Ex. 402 | 0. 069 | 0. 027 |
| Ex. 403 | 0. 152 | 0. 027 |
| Ex. 404 | 0. 148 | 0. 005 |

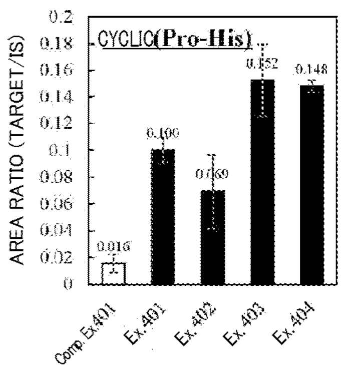


FIG. 34

SULFUROL

| | Ave. | SD |
|---|---|---|
| Comp. Ex. 401 | 0.216 | 0.008 |
| Ex. 401 | 0.310 | 0.007 |
| Ex. 402 | 0.352 | 0.023 |
| Ex. 403 | 0.530 | 0.022 |
| Ex. 404 | 0.460 | 0.069 |

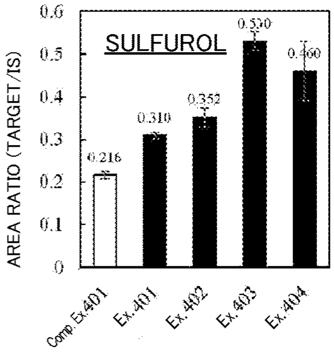


TARTARIC ACID

| | Ave. | SD |
|---|---|---|
| Comp. Ex. 401 | 0.100 | 0.002 |
| Ex. 401 | 0.142 | 0.002 |
| Ex. 402 | 0.146 | 0.009 |
| Ex. 403 | 0.231 | 0.011 |
| Ex. 404 | 0.239 | 0.022 |

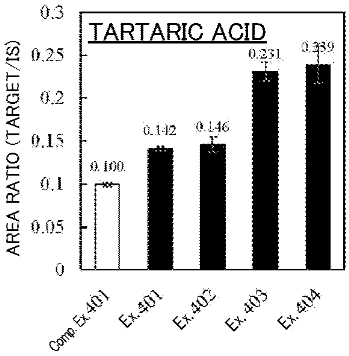


MALIC ACID

| | Ave. | SD |
|---|---|---|
| Comp. Ex. 401 | 1.065 | 0.017 |
| Ex. 401 | 1.226 | 0.022 |
| Ex. 402 | 1.304 | 0.025 |
| Ex. 403 | 1.556 | 0.013 |
| Ex. 404 | 1.516 | 0.019 |

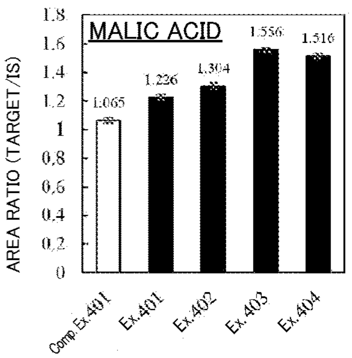


CITRIC ACID

| | Ave. | SD |
|---|---|---|
| Comp. Ex. 401 | 1.756 | 0.118 |
| Ex. 401 | 1.495 | 0.012 |
| Ex. 402 | 2.872 | 0.011 |
| Ex. 403 | 2.260 | 0.040 |
| Ex. 404 | 2.446 | 0.164 |

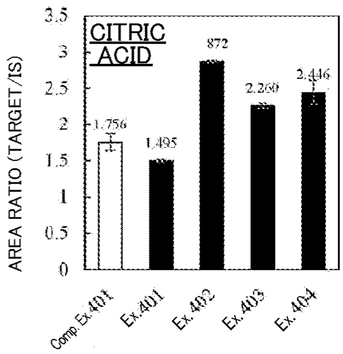


FIG. 35

**1** CYCLIC(Glu-His)

| | Ave. | SD |
|---|---|---|
| Comp. Ex. 501 | 0.020 | 0.006 |
| Comp. Ex. 502 | 0.066 | 0.010 |
| Comp. Ex. 503 | 0.117 | 0.006 |
| Comp. Ex. 504 | 0.126 | 0.023 |
| Comp. Ex. 505 | 0.164 | 0.006 |
| Comp. Ex. 506 | 0.078 | 0.017 |
| Ex. 501 | 1.042 | 0.069 |
| Ex. 502 | 0.794 | 0.024 |
| Ex. 503 | 0.340 | 0.026 |
| Ex. 504 | 0.370 | 0.026 |
| Ex. 505 | 0.299 | 0.018 |

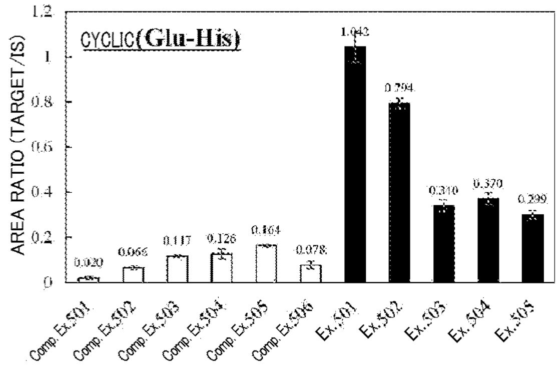


**2** CYCLIC(Gly-Arg)

| | Ave. | SD |
|---|---|---|
| Comp. Ex. 501 | 0.021 | 0.006 |
| Comp. Ex. 502 | 0.043 | 0.005 |
| Comp. Ex. 503 | 0.173 | 0.027 |
| Comp. Ex. 504 | 0.244 | 0.023 |
| Comp. Ex. 505 | 0.242 | 0.023 |
| Comp. Ex. 506 | 0.065 | 0.006 |
| Ex. 501 | 0.355 | 0.016 |
| Ex. 502 | 0.578 | 0.019 |
| Ex. 503 | 0.328 | 0.008 |
| Ex. 504 | 0.505 | 0.031 |
| Ex. 505 | 0.312 | 0.007 |

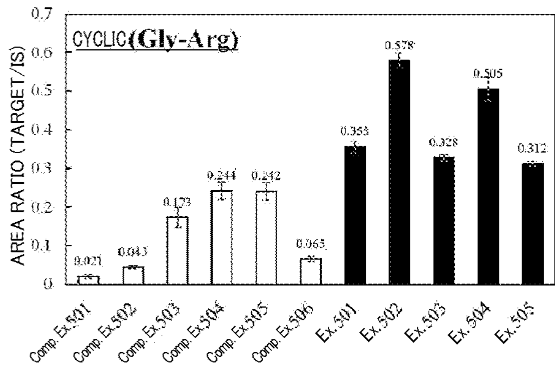


**3** CYCLIC(Glu-Gly)

| | Ave. | SD |
|---|---|---|
| Comp. Ex. 501 | 0.073 | 0.008 |
| Comp. Ex. 502 | 0.089 | 0.002 |
| Comp. Ex. 503 | 0.086 | 0.010 |
| Comp. Ex. 504 | 0.113 | 0.021 |
| Comp. Ex. 505 | 0.115 | 0.013 |
| Comp. Ex. 506 | 0.115 | 0.009 |
| Ex. 501 | 0.568 | 0.016 |
| Ex. 502 | 0.620 | 0.023 |
| Ex. 503 | 0.428 | 0.006 |
| Ex. 504 | 0.250 | 0.013 |
| Ex. 505 | 0.212 | 0.011 |

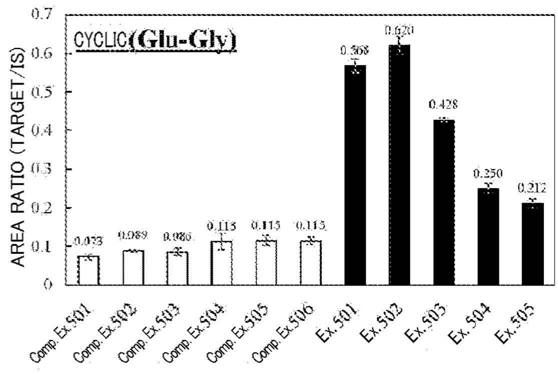


**4** CYCLIC(Ala-His)

| | Ave. | SD |
|---|---|---|
| Comp. Ex. 501 | 0.010 | 0.001 |
| Comp. Ex. 502 | 0.010 | 0.002 |
| Comp. Ex. 503 | 0.066 | 0.014 |
| Comp. Ex. 504 | 0.093 | 0.006 |
| Comp. Ex. 505 | 0.107 | 0.006 |
| Comp. Ex. 506 | 0.050 | 0.007 |
| Ex. 501 | 0.292 | 0.016 |
| Ex. 502 | 0.263 | 0.020 |
| Ex. 503 | 0.249 | 0.021 |
| Ex. 504 | 0.170 | 0.023 |
| Ex. 505 | 0.123 | 0.003 |

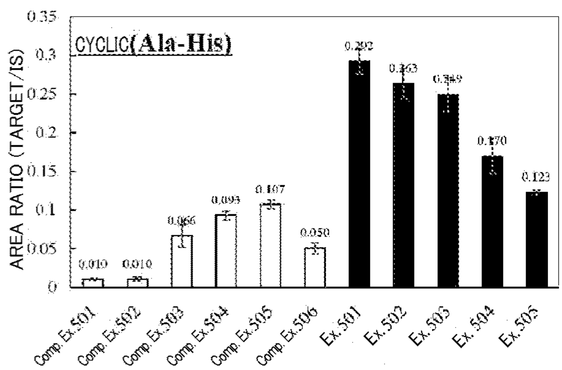


FIG. 36

5 CYCLIC(Ala-Arg)

| | Ave. | SD |
|---|---|---|
| Comp. Ex. 501 | 0.047 | 0.003 |
| Comp. Ex. 502 | 0.080 | 0.011 |
| Comp. Ex. 503 | 0.097 | 0.020 |
| Comp. Ex. 504 | 0.182 | 0.010 |
| Comp. Ex. 505 | 0.177 | 0.014 |
| Comp. Ex. 506 | 0.105 | 0.013 |
| Ex. 501 | 0.390 | 0.001 |
| Ex. 502 | 0.506 | 0.022 |
| Ex. 503 | 0.358 | 0.020 |
| Ex. 504 | 0.342 | 0.010 |
| Ex. 505 | 0.237 | 0.006 |

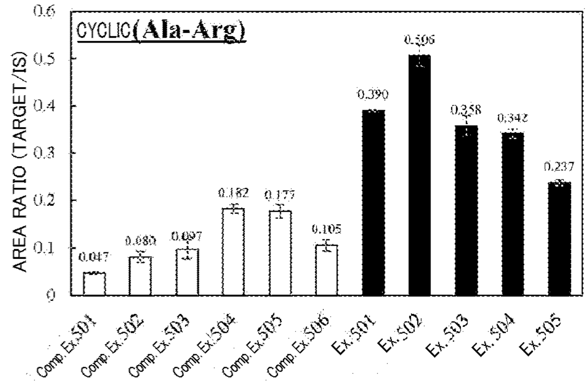


6 CYCLIC(Glu-Asp)

| | Ave. | SD |
|---|---|---|
| Comp. Ex. 501 | nd | |
| Comp. Ex. 502 | nd | |
| Comp. Ex. 503 | 0.035 | 0.015 |
| Comp. Ex. 504 | 0.045 | 0.002 |
| Comp. Ex. 505 | 0.089 | 0.003 |
| Comp. Ex. 506 | 0.115 | 0.006 |
| Ex. 501 | 0.435 | 0.026 |
| Ex. 502 | 0.437 | 0.013 |
| Ex. 503 | 0.306 | 0.005 |
| Ex. 504 | 0.388 | 0.013 |
| Ex. 505 | 0.136 | 0.008 |

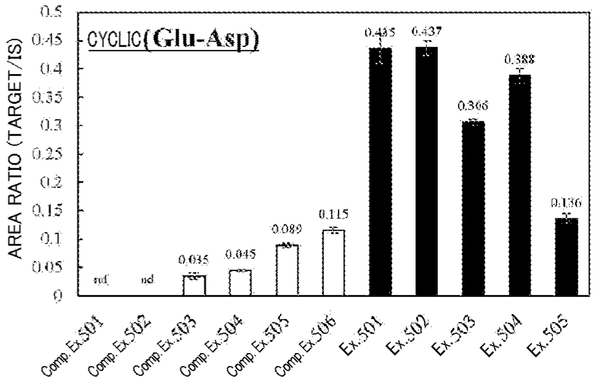


7 CYCLIC(Leu/Ile-Asp)

| | Ave. | SD |
|---|---|---|
| Comp. Ex. 501 | nd | |
| Comp. Ex. 502 | nd | |
| Comp. Ex. 503 | 0.009 | 0.001 |
| Comp. Ex. 504 | 0.012 | 0.003 |
| Comp. Ex. 505 | 0.025 | 0.002 |
| Comp. Ex. 506 | 0.029 | 0.002 |
| Ex. 501 | 0.082 | 0.004 |
| Ex. 502 | 0.088 | 0.005 |
| Ex. 503 | 0.088 | 0.006 |
| Ex. 504 | 0.042 | 0.003 |
| Ex. 505 | 0.040 | 0.006 |

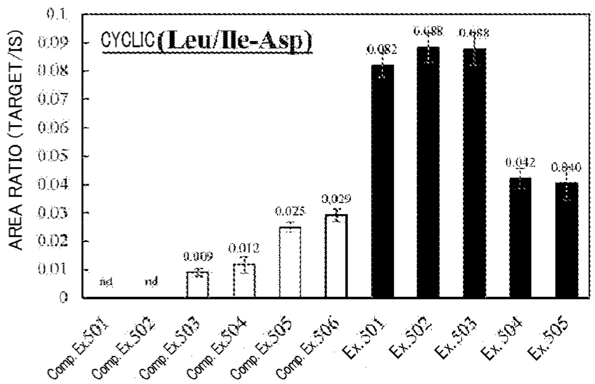


8 CYCLIC(Thr-Pro)

| | Ave. | SD |
|---|---|---|
| Comp. Ex. 501 | nd | |
| Comp. Ex. 502 | 0.090 | 0.007 |
| Comp. Ex. 503 | 0.330 | 0.016 |
| Comp. Ex. 504 | 0.171 | 0.009 |
| Comp. Ex. 505 | 0.359 | 0.013 |
| Comp. Ex. 506 | 0.388 | 0.035 |
| Ex. 501 | 0.853 | 0.015 |
| Ex. 502 | 1.175 | 0.015 |
| Ex. 503 | 0.691 | 0.021 |
| Ex. 504 | 0.837 | 0.031 |
| Ex. 505 | 0.614 | 0.010 |

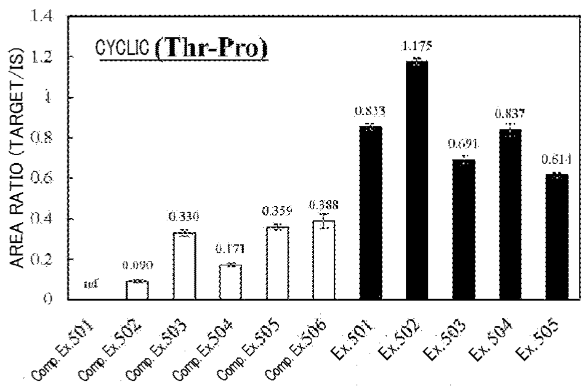


FIG. 37

**9** CYCLIC(**Pro-His**)

| | Ave. | SD |
|---|---|---|
| Comp. Ex. 501 | nd | |
| Comp. Ex. 502 | 0.017 | 0.000 |
| Comp. Ex. 503 | 0.018 | 0.001 |
| Comp. Ex. 504 | 0.021 | 0.003 |
| Comp. Ex. 505 | 0.034 | 0.002 |
| Comp. Ex. 506 | 0.077 | 0.008 |
| Ex. 501 | 0.137 | 0.011 |
| Ex. 502 | 0.178 | 0.003 |
| Ex. 503 | 0.115 | 0.010 |
| Ex. 504 | 0.101 | 0.010 |
| Ex. 505 | 0.093 | 0.009 |

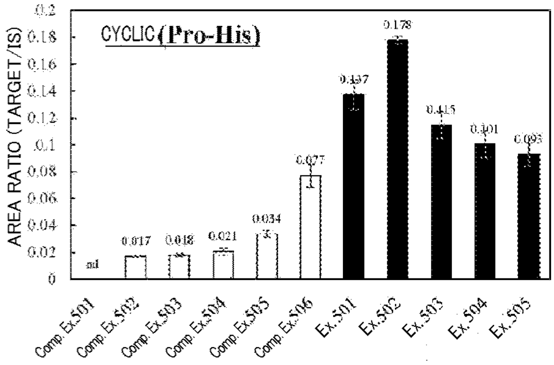


**10** CYCLIC(**Pro-Pro**)

| | Ave. | SD |
|---|---|---|
| Comp. Ex.501 | nd | |
| Comp. Ex.502 | 0.164 | 0.003 |
| Comp. Ex.503 | 0.179 | 0.010 |
| Comp. Ex.504 | 0.269 | 0.010 |
| Comp. Ex.505 | 0.380 | 0.014 |
| Comp. Ex.506 | 0.301 | 0.011 |
| Ex. 501 | 1.060 | 0.043 |
| Ex. 502 | 1.308 | 0.052 |
| Ex. 503 | 1.231 | 0.024 |
| Ex. 504 | 0.700 | 0.033 |
| Ex. 505 | 0.572 | 0.032 |

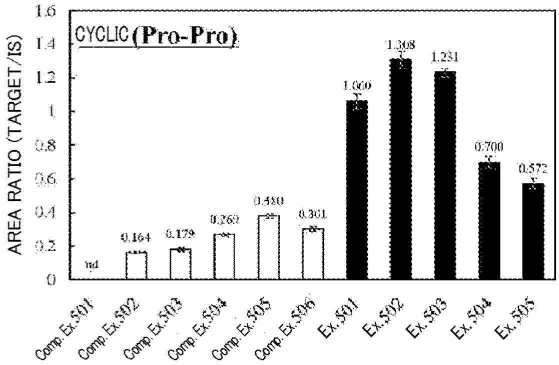


**11** CYCLIC(**Glu-Tyr**)

| | Ave. | SD |
|---|---|---|
| Comp. Ex. 501 | nd | |
| Comp. Ex. 502 | nd | |
| Comp. Ex. 503 | 0.012 | 0.002 |
| Comp. Ex. 504 | 0.010 | 0.001 |
| Comp. Ex. 505 | 0.017 | 0.002 |
| Comp. Ex. 506 | 0.058 | 0.006 |
| Ex. 501 | 0.151 | 0.007 |
| Ex. 502 | 0.122 | 0.006 |
| Ex. 503 | 0.108 | 0.007 |
| Ex. 504 | 0.092 | 0.009 |
| Ex. 505 | 0.087 | 0.004 |

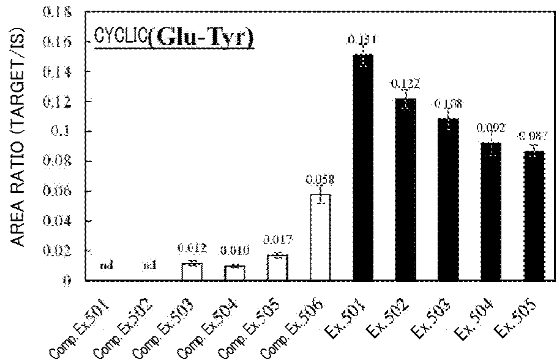


**12** CYCLIC(**Arg-Leu/Ile**)

| | Ave. | SD |
|---|---|---|
| Comp. Ex. 501 | nd | |
| Comp. Ex. 502 | nd | |
| Comp. Ex. 503 | 0.008 | 0.003 |
| Comp. Ex. 504 | 0.006 | 0.002 |
| Comp. Ex. 505 | 0.014 | 0.003 |
| Comp. Ex. 506 | 0.009 | 0.002 |
| Ex. 501 | 0.060 | 0.004 |
| Ex. 502 | 0.051 | 0.005 |
| Ex. 503 | 0.023 | 0.002 |
| Ex. 504 | 0.044 | 0.005 |
| Ex. 505 | 0.041 | 0.001 |

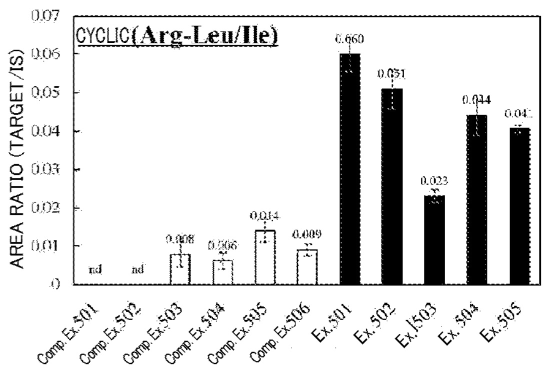


FIG. 38

13 CYCLIC(Ala-Pro)

| | Ave. | SD |
|---|---|---|
| Comp. Ex. 501 | nd | |
| Comp. Ex. 502 | nd | |
| Comp. Ex. 503 | 0.278 | 0.067 |
| Comp. Ex. 504 | 0.455 | 0.019 |
| Comp. Ex. 505 | 0.489 | 0.039 |
| Comp. Ex. 506 | 0.386 | 0.012 |
| Ex. 501 | 0.991 | 0.023 |
| Ex. 502 | 1.338 | 0.043 |
| Ex. 503 | 0.970 | 0.036 |
| Ex. 504 | 0.892 | 0.055 |
| Ex. 505 | 0.802 | 0.022 |

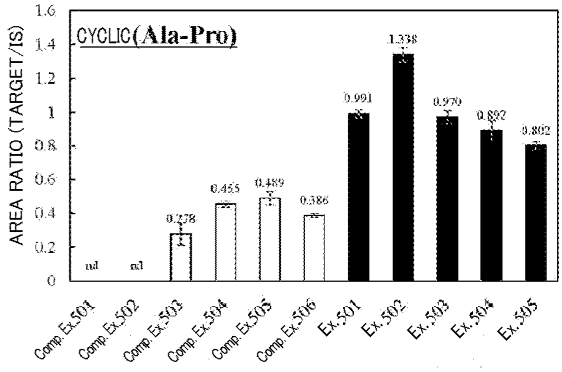


14 CYCLIC(Pro-Val)

| | Ave. | SD |
|---|---|---|
| Comp. Ex.501 | nd | |
| Comp. Ex.502 | 0.117 | 0.006 |
| Comp. Ex.503 | 0.217 | 0.021 |
| Comp. Ex.504 | 0.436 | 0.061 |
| Comp. Ex.505 | 0.429 | 0.025 |
| Comp. Ex.506 | 0.613 | 0.015 |
| Ex. 501 | 2.598 | 0.270 |
| Ex. 502 | 2.621 | 0.334 |
| Ex. 503 | 1.984 | 0.037 |
| Ex. 504 | 1.136 | 0.013 |
| Ex. 505 | 0.694 | 0.001 |

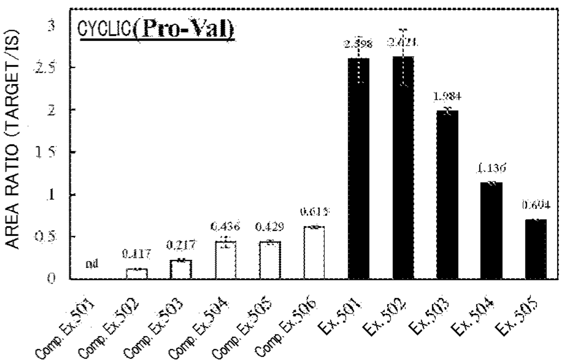


15 CYCLIC(Phe-Ala)

| | Ave. | SD |
|---|---|---|
| Comp. Ex.501 | nd | |
| Comp. Ex.502 | 0.016 | 0.002 |
| Comp. Ex.503 | 0.017 | 0.004 |
| Comp. Ex.504 | 0.042 | 0.005 |
| Comp. Ex.505 | 0.048 | 0.004 |
| Comp. Ex.506 | 0.027 | 0.001 |
| Ex. 501 | 0.163 | 0.008 |
| Ex. 502 | 0.156 | 0.007 |
| Ex. 503 | 0.167 | 0.002 |
| Ex. 504 | 0.095 | 0.007 |
| Ex. 505 | 0.078 | 0.008 |

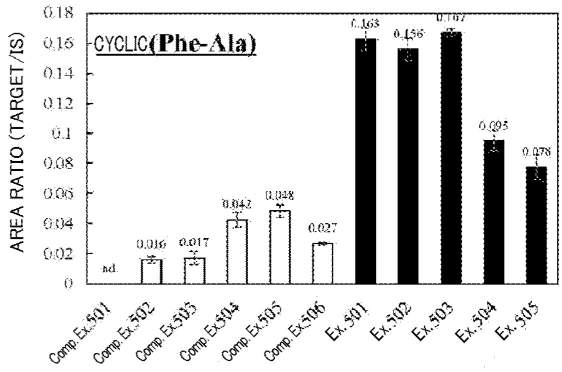


16 CYCLIC(Phe-Pro)

| | Ave. | SD |
|---|---|---|
| Comp. Ex. 501 | nd | |
| Comp. Ex. 502 | 0.013 | 0.001 |
| Comp. Ex. 503 | 0.046 | 0.003 |
| Comp. Ex. 504 | 0.062 | 0.004 |
| Comp. Ex. 505 | 0.052 | 0.007 |
| Comp. Ex. 506 | 0.061 | 0.005 |
| Ex. 501 | 0.162 | 0.005 |
| Ex. 502 | 0.198 | 0.002 |
| Ex. 503 | 0.119 | 0.007 |
| Ex. 504 | 0.097 | 0.009 |
| Ex. 505 | 0.087 | 0.006 |

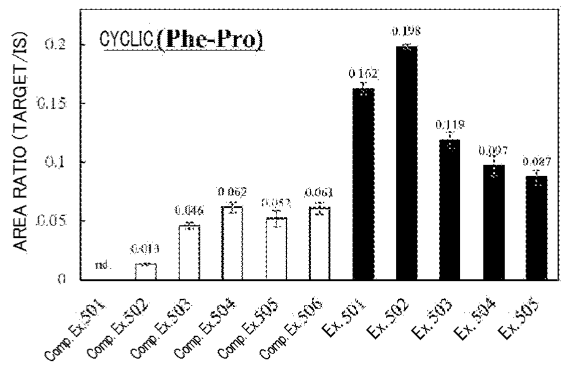


FIG. 39

17 CYCLIC(Phe-Tyr)

| | Ave. | SD |
|---|---|---|
| Comp. Ex. 501 | nd | |
| Comp. Ex. 502 | 0.028 | 0.001 |
| Comp. Ex. 503 | 0.066 | 0.006 |
| Comp. Ex. 504 | 0.149 | 0.020 |
| Comp. Ex. 505 | 0.114 | 0.011 |
| Comp. Ex. 506 | 0.131 | 0.009 |
| Ex. 501 | 0.282 | 0.022 |
| Ex. 502 | 0.257 | 0.018 |
| Ex. 503 | 0.207 | 0.013 |
| Ex. 504 | 0.214 | 0.017 |
| Ex. 505 | 0.192 | 0.009 |

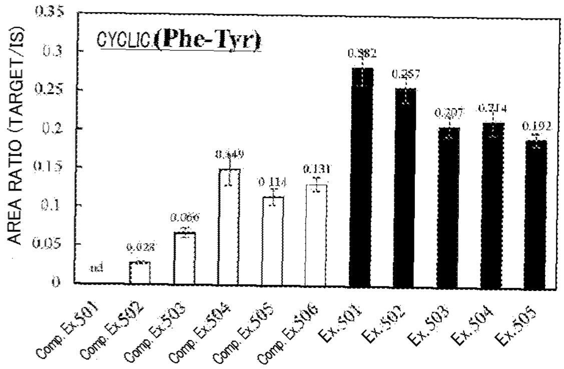


18 CYCLIC(Tyr-His)

| | Ave. | SD |
|---|---|---|
| Comp. Ex. 501 | nd | |
| Comp. Ex. 502 | nd | |
| Comp. Ex. 503 | nd | |
| Comp. Ex. 504 | 0.003 | 0.001 |
| Comp. Ex. 505 | 0.010 | 0.002 |
| Comp. Ex. 506 | 0.008 | 0.002 |
| Ex. 501 | 0.057 | 0.003 |
| Ex. 502 | 0.050 | 0.004 |
| Ex. 503 | 0.053 | 0.004 |
| Ex. 504 | 0.032 | 0.003 |
| Ex. 505 | 0.026 | 0.002 |

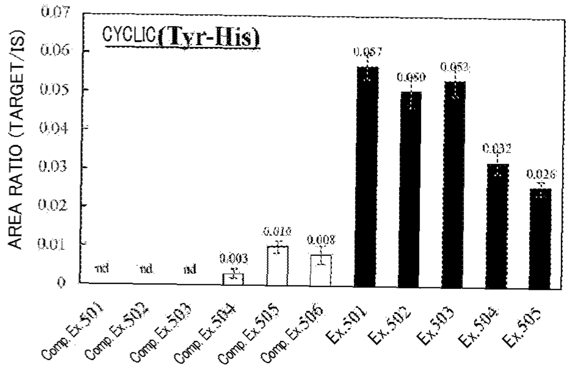


19 CYCLIC(Leu/Ile-His)

| | Ave. | SD |
|---|---|---|
| Comp. Ex. 501 | nd | |
| Comp. Ex. 502 | 0.008 | 0.001 |
| Comp. Ex. 503 | 0.012 | 0.003 |
| Comp. Ex. 504 | 0.019 | 0.004 |
| Comp. Ex. 505 | 0.027 | 0.009 |
| Comp. Ex. 506 | 0.022 | 0.004 |
| Ex. 501 | 0.221 | 0.015 |
| Ex. 502 | 0.205 | 0.020 |
| Ex. 503 | 0.212 | 0.023 |
| Ex. 504 | 0.184 | 0.002 |
| Ex. 505 | 0.103 | 0.006 |

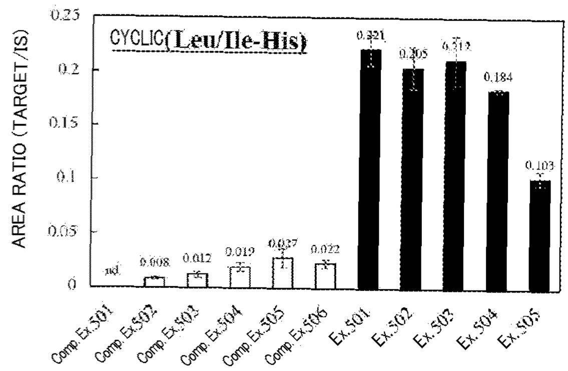


20 CYCLIC(Pro-Met)

| | Ave. | SD |
|---|---|---|
| Comp. Ex. 501 | 0.005 | 0.001 |
| Comp. Ex. 502 | 0.023 | 0.003 |
| Comp. Ex. 503 | 0.061 | 0.002 |
| Comp. Ex. 504 | 0.075 | 0.000 |
| Comp. Ex. 505 | 0.134 | 0.018 |
| Comp. Ex. 506 | 0.289 | 0.019 |
| Ex. 501 | 0.732 | 0.023 |
| Ex. 502 | 0.650 | 0.028 |
| Ex. 503 | 0.559 | 0.033 |
| Ex. 504 | 0.367 | 0.012 |
| Ex. 505 | 0.338 | 0.026 |

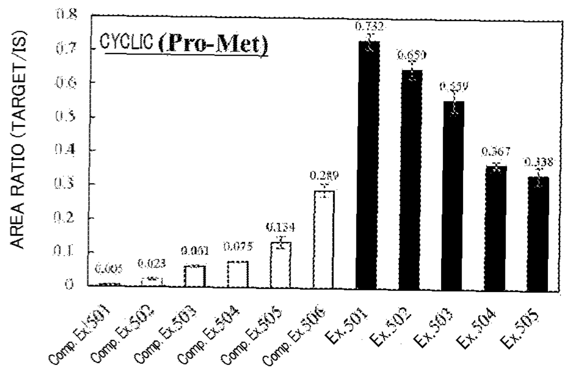


FIG. 40

**21** CYCLIC (Val-Glu)

| | Ave. | SD |
|---|---|---|
| Comp. Ex. 501 | nd | |
| Comp. Ex. 502 | nd | |
| Comp. Ex. 503 | 0.016 | 0.002 |
| Comp. Ex. 504 | 0.033 | 0.004 |
| Comp. Ex. 505 | 0.024 | 0.006 |
| Comp. Ex. 506 | 0.017 | 0.004 |
| Ex. 501 | 0.889 | 0.044 |
| Ex. 502 | 0.444 | 0.041 |
| Ex. 503 | 0.455 | 0.026 |
| Ex. 504 | 0.215 | 0.010 |
| Ex. 505 | 0.181 | 0.011 |

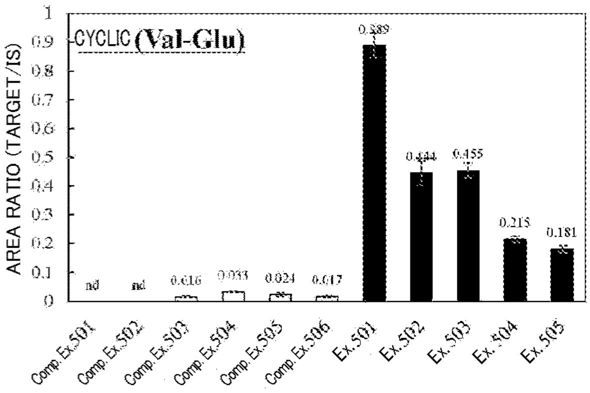


FIG. 41

**22** ALLYL METHYL DISULFIDE

| | Ave. | SD |
|---|---|---|
| Comp. Ex.501 | nd | |
| Comp. Ex.502 | 0.002 | 0.000 |
| Comp. Ex.503 | 0.002 | 0.000 |
| Comp. Ex.504 | 0.001 | 0.000 |
| Comp. Ex.505 | 0.002 | 0.000 |
| Comp. Ex.506 | 0.004 | 0.000 |
| Ex. 501 | 0.019 | 0.001 |
| Ex. 502 | 0.011 | 0.000 |
| Ex. 503 | 0.021 | 0.001 |
| Ex. 504 | 0.016 | 0.001 |
| Ex. 505 | 0.009 | 0.001 |

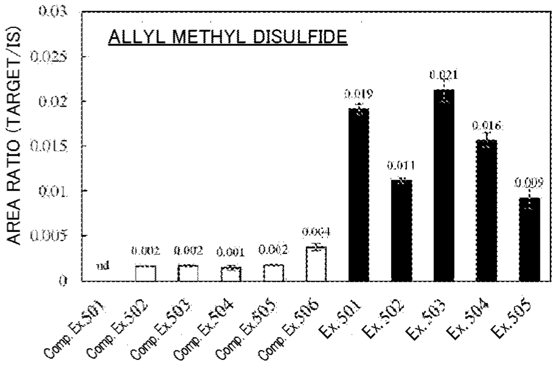


**23** 2,5-DIMETHYLPYRAZINE

| | Ave. | SD |
|---|---|---|
| Comp. Ex.501 | 0.014 | 0.005 |
| Comp. Ex.502 | 0.219 | 0.010 |
| Comp. Ex.503 | 0.100 | 0.001 |
| Comp. Ex.504 | 0.059 | 0.005 |
| Comp. Ex.505 | 0.144 | 0.001 |
| Comp. Ex.506 | 0.387 | 0.017 |
| Ex. 501 | 0.951 | 0.025 |
| Ex. 502 | 1.057 | 0.029 |
| Ex. 503 | 0.849 | 0.047 |
| Ex. 504 | 0.977 | 0.034 |
| Ex. 505 | 1.056 | 0.082 |

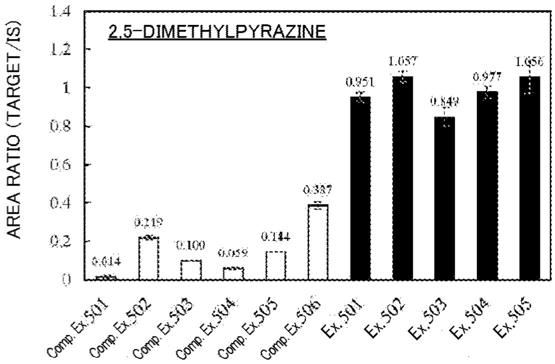


**24** 2-ETHYL-6-METHYLPYRAZINE

| | Ave. | SD |
|---|---|---|
| Comp. Ex.501 | 0.005 | 0.002 |
| Comp. Ex.502 | 0.006 | 0.001 |
| Comp. Ex.503 | 0.019 | 0.000 |
| Comp. Ex.504 | 0.008 | 0.001 |
| Comp. Ex.505 | 0.026 | 0.000 |
| Comp. Ex.506 | 0.079 | 0.008 |
| Ex. 501 | 0.234 | 0.011 |
| Ex. 502 | 0.205 | 0.010 |
| Ex. 503 | 0.214 | 0.015 |
| Ex. 504 | 0.245 | 0.008 |
| Ex. 505 | 0.335 | 0.028 |

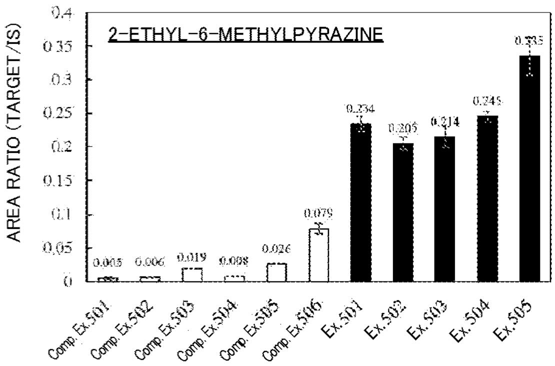


**25** 2,3,5-TRIMETHYLPYRAZINE

| | Ave. | SD |
|---|---|---|
| Comp. Ex.501 | 0.020 | 0.008 |
| Comp. Ex.502 | 0.036 | 0.002 |
| Comp. Ex.503 | 0.019 | 0.001 |
| Comp. Ex.504 | 0.011 | 0.001 |
| Comp. Ex.505 | 0.031 | 0.001 |
| Comp. Ex.506 | 0.095 | 0.009 |
| Ex. 501 | 0.252 | 0.010 |
| Ex. 502 | 0.269 | 0.014 |
| Ex. 503 | 0.231 | 0.016 |
| Ex. 504 | 0.238 | 0.008 |
| Ex. 505 | 0.202 | 0.009 |

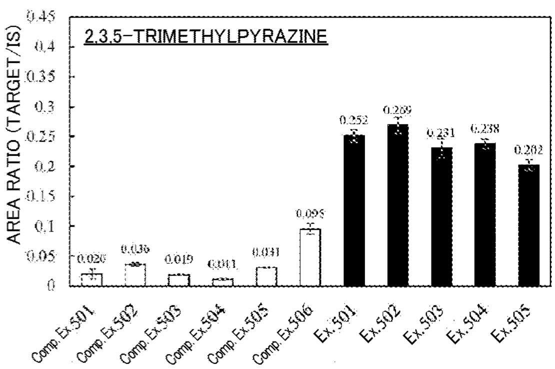


FIG. 42

**26** 2,6-DIETHYLPYRAZINE

| | Ave. | SD |
|---|---|---|
| Comp. Ex.501 | nd | |
| Comp. Ex.502 | 0.002 | 0.000 |
| Comp. Ex.503 | 0.001 | 0.000 |
| Comp. Ex.504 | nd | |
| Comp. Ex.505 | 0.002 | 0.000 |
| Comp. Ex.506 | 0.006 | 0.001 |
| Ex. 501 | 0.026 | 0.001 |
| Ex. 502 | 0.016 | 0.001 |
| Ex. 503 | 0.027 | 0.002 |
| Ex. 504 | 0.021 | 0.001 |
| Ex. 505 | 0.023 | 0.001 |

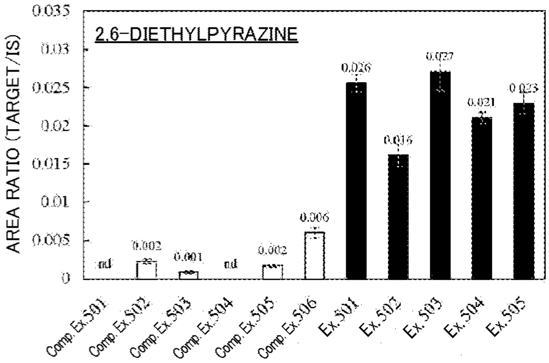


**27** 2,5-DIMETHYLPYRAZINE

| | Ave. | SD |
|---|---|---|
| Comp. Ex.501 | 0.005 | 0.003 |
| Comp. Ex.502 | 0.018 | 0.002 |
| Comp. Ex.503 | 0.026 | 0.001 |
| Comp. Ex.504 | 0.008 | 0.001 |
| Comp. Ex.505 | 0.034 | 0.001 |
| Comp. Ex.506 | 0.108 | 0.008 |
| Ex. 501 | 0.482 | 0.030 |
| Ex. 502 | 0.292 | 0.020 |
| Ex. 503 | 0.407 | 0.035 |
| Ex. 504 | 0.515 | 0.021 |
| Ex. 505 | 0.874 | 0.064 |

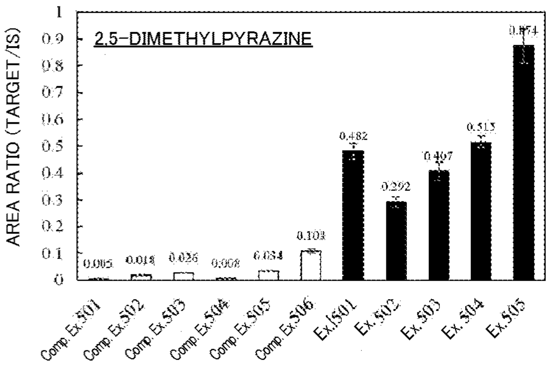


**28** 2,3-DIETHYLPYRAZINE

| | Ave. | SD |
|---|---|---|
| Comp. Ex.501 | 0.001 | 0.000 |
| Comp. Ex.502 | 0.002 | 0.001 |
| Comp. Ex.503 | 0.003 | 0.000 |
| Comp. Ex.504 | 0.002 | 0.000 |
| Comp. Ex.505 | 0.007 | 0.000 |
| Comp. Ex.506 | 0.018 | 0.002 |
| Ex. 501 | 0.064 | 0.003 |
| Ex. 502 | 0.048 | 0.002 |
| Ex. 503 | 0.060 | 0.004 |
| Ex. 504 | 0.059 | 0.003 |
| Ex. 505 | 0.062 | 0.001 |

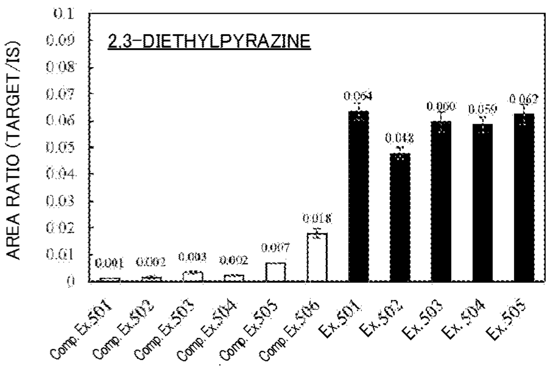


**29** ALLYLMETHYL TRISULFIDE

| | Ave. | SD |
|---|---|---|
| Comp. Ex.501 | 0.000 | 0.000 |
| Comp. Ex.502 | 0.005 | 0.001 |
| Comp. Ex.503 | 0.011 | 0.002 |
| Comp. Ex.504 | 0.013 | 0.001 |
| Comp. Ex.505 | 0.011 | 0.002 |
| Comp. Ex.506 | 0.015 | 0.001 |
| Ex. 501 | 0.061 | 0.003 |
| Ex. 502 | 0.057 | 0.007 |
| Ex. 503 | 0.045 | 0.004 |
| Ex. 504 | 0.079 | 0.005 |
| Ex. 505 | 0.034 | 0.002 |

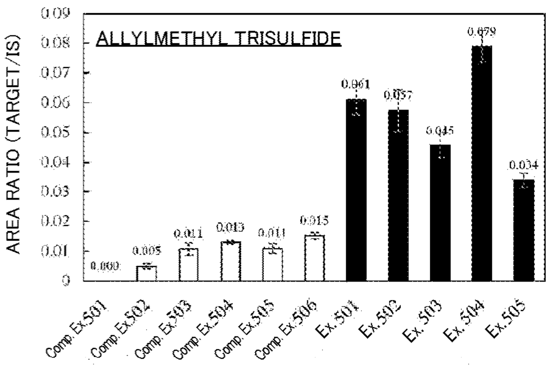


FIG. 43

**30** 3-METHYL-1,2,4-TRITHIANE

| | Ave. | SD |
|---|---|---|
| Comp. Ex.501 | nd | |
| Comp. Ex.502 | 0.105 | 0.006 |
| Comp. Ex.503 | 0.463 | 0.026 |
| Comp. Ex.504 | 0.673 | 0.019 |
| Comp. Ex.505 | 0.473 | 0.011 |
| Comp. Ex.506 | 0.290 | 0.010 |
| Ex. 501 | 1.250 | 0.026 |
| Ex. 502 | 1.218 | 0.023 |
| Ex. 503 | 1.442 | 0.086 |
| Ex. 504 | 1.013 | 0.047 |
| Ex. 505 | 0.739 | 0.044 |

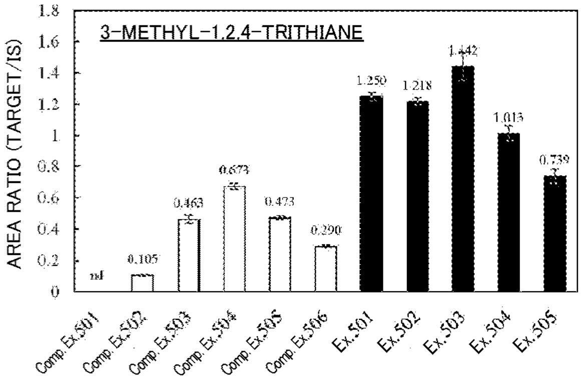


**31** 2-DODECENAL

| | Ave. | SD |
|---|---|---|
| Comp. Ex. 501 | 0.060 | 0.004 |
| Comp. Ex. 502 | 0.087 | 0.008 |
| Comp. Ex. 503 | 0.097 | 0.009 |
| Comp. Ex. 504 | 0.111 | 0.013 |
| Comp. Ex. 505 | 0.108 | 0.011 |
| Comp. Ex. 506 | 0.031 | 0.007 |
| Ex. 501 | 0.133 | 0.012 |
| Ex. 502 | 0.123 | 0.014 |
| Ex. 503 | 0.136 | 0.007 |
| Ex. 504 | 0.160 | 0.003 |
| Ex. 505 | 0.182 | 0.005 |

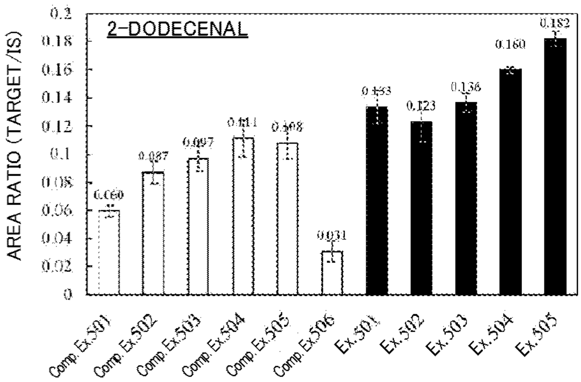


**32** SULFUROL

| | Ave. | SD |
|---|---|---|
| Comp. Ex.501 | 0.089 | 0.002 |
| Comp. Ex.502 | 0.346 | 0.045 |
| Comp. Ex.503 | 0.455 | 0.003 |
| Comp. Ex.504 | 0.524 | 0.049 |
| Comp. Ex.505 | 0.592 | 0.003 |
| Comp. Ex.506 | 0.251 | 0.026 |
| Ex. 501 | 0.653 | 0.030 |
| Ex. 502 | 0.804 | 0.081 |
| Ex. 503 | 0.659 | 0.052 |
| Ex. 504 | 0.637 | 0.008 |
| Ex. 505 | 0.879 | 0.036 |

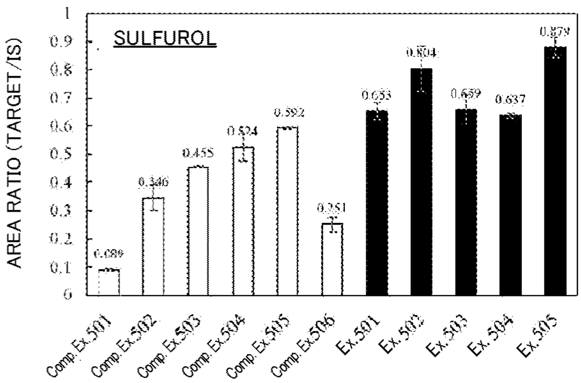


FIG. 44

## INTERNATIONAL SEARCH REPORT

International application No.
**PCT/JP2024/003190**

**A. CLASSIFICATION OF SUBJECT MATTER**

***A23L 27/10***(2016.01)i; ***A23L 23/10***(2016.01)i; ***A23L 27/00***(2016.01)i; ***A23L 27/18***(2016.01)i; ***C07K 5/06***(2006.01)i
FI: A23L27/10 C; A23L23/10; A23L27/00 Z; A23L27/18; C07K5/06

According to International Patent Classification (IPC) or to both national classification and IPC

**B. FIELDS SEARCHED**

Minimum documentation searched (classification system followed by classification symbols)

A23L27/10; A23L23/10; A23L27/00; A23L27/18; C07K5/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS/FSTA/AGRICOLA (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2020-103257 A (HOUSE FOODS CORPORATION) 09 July 2020 (2020-07-09) claims 1-14, tables 2-2, 3, 4-1 | 1-4, 7-12, 16-20, 22-24, 28-29, 32, 36-37, 39 |
| A | claims 1-14, tables 2-2, 3, 4-1 | 5-6, 13-15, 21, 25-27, 30-31, 33-35, 38 |
| X | JP 2002-325553 A (HOUSE FOODS CORPORATION) 12 November 2002 (2002-11-12) claims 1-4, paragraph [0002], example 1 | 1-2, 5-10, 25-27, 30-31, 38-39 |
| A | claims 1-4, paragraph [0002], example 1 | 3-4, 11-24, 28-29, 32-37 |
| X | JP 2000-236844 A (LION CORPORATION) 05 September 2000 (2000-09-05) claims 1-2, table 4 | 1-3, 7-10, 25-28, 39 |
| A | claims 1-2, table 4 | 4-6, 11-24, 29-38 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2024** | **16 April 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | Telephone No. |

Form PCT/ISA/210 (second sheet) (July 2022)

INTERNATIONAL SEARCH REPORT

International application No.
PCT/JP2024/003190

C. DOCUMENTS CONSIDERED TO BE RELEVANT

| Category* | Citation of document, with indication, where appropriate, of the relevant passages | Relevant to claim No. |
|---|---|---|
| X | パプリカオイルが味の決め手！「レンズ豆のスープ」で真夏のアンチエイジング, [online]. 08 August 2022, [retrieved on 15 March 2024], Retrieved from the Internet:<URL: https://www.kateigaho.com/article/detail/144631, non-official translation (Paprika oil is the key to the taste! Midsummer anti-aging with “lentil soup”) in particular, section "preparation method" | 1, 11, 14-18, 34-35, 39 |
| A | in particular, section "preparation method" | 2-10, 12-13, 19-33, 36-38 |
| X | スパイスのおはなし「アサフォエティダ」と「アジョワン」, [online]. 15 July 2012, [retrieved on 15 March 2024], Retrieved from the Internet:<URL: https://ameblo.jp/rawatpsddunhotmailcom/entry-11303191699.html>non-official translation (Story of spices “Asafoetida” and “Ajowan”) in particular, section "asafetida" | 1, 19, 21-24, 37, 39 |
| A | in particular, section "asafetida" | 2-18, 20, 25-36, 38 |

Form PCT/ISA/210 (second sheet) (July 2022)

**INTERNATIONAL SEARCH REPORT**

International application No. **PCT/JP2024/003190**

**Box No. III Observations where unity of invention is lacking (Continuation of item 3 of first sheet)**

This International Searching Authority found multiple inventions in this international application, as follows:

(Invention 1) Claim 1, claims 2-10, and 28-31, which select cumin, claim 39 citing any of claims 1 and 2-10 which select cumin
Document 1 discloses "composition for flavor enhancement obtained by heating cumin such that the heating value thereof becomes 63.5 or 65.0", document 2 discloses a "composition for flavor enhancement obtained by heating cumin with oil at a heating value of 20 to 4000", and claim 1, claims 2-10, and 28-31, which select cumin, claim 39 citing any of claims 1 and 2-10 which select cumin lack novelty in light of document 1 or 2, and thus does not have a special technical feature. Accordingly, claim 1, claims 2-10, and claims 28-31, which select cumin, claim 39 citing any of claims 1 and 2-10 which select cumin are classified as invention 1.

(Invention 2) Claim 1, claims 11-18, and 32-35, which select paprika, claim 39 citing any of claims 1 and 11-18 which select paprika
It cannot be said that claim 1, claims 11-18, and 32-35, which select paprika, claim 39 citing any of claims 1 and 11-18 which select paprika have a technical feature identical or corresponding to that of claim 1, claims 2-10, and 28-31, which select cumin, claim 39 citing any of claims 1 and 2-10 which select cumin, classified as invention 1.
In addition, claim 1, claims 11-18, and 32-35, which select paprika, claim 39 citing any of claims 1 and 11-18 which select paprika are not substantially identical or equivalent to any of the claims classified as invention 1.
Therefore, claim 1, claims 11-18, and 32-35, which select paprika, claim 39 citing any of claims 1 and 11-18 which select paprika cannot be classified as invention 1.
In addition, claim 1, claims 11-18, and 32-35, which select paprika, claim 39 citing any of claims 1 and 11-18 which select paprika have the special technical feature of a "composition for flavor enhancement containing heat-treated paprika", and thus are classified as invention 2.

(Invention 3) Claim 1, claims 19-24, and 36-37, which select asafetida, claim 39 citing any of claims 1 and 19-24 which select asafetida
It cannot be said that claim 1, claims 19-24, and 36-37, which select asafetida, claim 39 citing any of claims 1 and 19-24 which select asafetida have a technical feature identical or corresponding to that of claim 1, claims 2-10, and 28-31, which select cumin, claim 39 citing any of claims 1 and 2-10 which select cumin, classified as invention 1, or claim 1, claims 11-18, and 32-35, which select paprika, claim 39 citing any of claims 1 and 11-18 which select paprika, classified as invention 2.
In addition, claim 1, claims 19-24, and 36-37, which select asafetida, claim 39 citing any of claims 1 and 19-24 which select asafetida are not substantially identical or equivalent to any of the claims classified as invention 1 or invention 2.
Accordingly, claim 1, claims 19-24, and 36-37, which select asafetida, claim 39 citing any of claims 1 and 19-24 which select asafetida cannot be classified as either invention 1 or invention 2.
In addition, claim 1, claims 19-24, and 36-37, which select asafetida, claim 39 citing any of claims 1 and 19-24 which select asafetida have the special technical feature of a "composition for flavor enhancement containing heat-treated asafetida," and thus classified as invention 3.

(Invention 4) Claim 1, claims 25-27, and 38, which select mustard seed, claim 39 citing any of claims 1 and 25-27 which select mustard seed
It cannot be said that claim 1, claims 25-27, and 38, which select mustard seed, claim 39 citing any of claims 1 and 25-27 which select mustard seed have a technical feature identical or corresponding to that of claim 1, claims 2-10, and 28-31, which select cumin, claim 39 citing any of claims 1 and 2-10 which select cumin classified as invention 1, claim 1, claims 11-18, and 32-35, which select paprika, claim 39 citing any of claims 1 and 11-18 which select paprika classified as invention 2, or claim 1, claims 19-24, and claims 36-37, which select asafetida, claim 39 citing claims 1 and 19-24 which select asafetida classified as invention 3.
In addition, claim 1, claims 25-27, and 38, which select mustard seed, claim 39 citing any of claims 1 and 25-27 which select mustard seed are not substantially identical or equivalent to any of the claims classified as invention 1, invention 2, or invention 3.
Accordingly, claim 1, claims 25-27, and 38, which select mustard seed, claim 39 citing any of claims 1 and 25-27 which select mustard seed cannot be classified as either invention 1 or invention 2.
In addition, claim 1, claims 25-27, and 38, which select mustard seed, claim 39 citing any of claims 1 and 25-27 which select mustard seed have the special technical feature of a "composition for flavor enhancement containing heat-treated mustard seeds," and thus classified as invention 4.

Form PCT/ISA/210 (continuation of first sheet) (July 2022)

**INTERNATIONAL SEARCH REPORT**

International application No.
**PCT/JP2024/003190**

**Box No. III Observations where unity of invention is lacking (Continuation of item 3 of first sheet)**

Document 1 : JP 2020-103257 A (House Foods Group Inc) 09 July 2020 (2020-07-09)
Document 2 : JP 2002-325553 A (House Foods Group Inc) 12 November 2002 (2002-11-12)

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/003190**

| Patent document cited in search report | Publication date (day/month/year) | Patent family member(s) | Publication date (day/month/year) |
|---|---|---|---|
| JP 2020-103257 A | 09 July 2020 | JP 2020-108388 A | |
| JP 2002-325553 A | 12 November 2002 | (Family: none) | |
| JP 2000-236844 A | 05 September 2000 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018102142 A
- JP 2012239398 A
- JP 2020103257 A
- JP 2020202765 A
- JP 2023013575 A
- JP 2023013576 A
- JP 2023013623 A
- JP 2023074823 A